# EUROPEAN PATENT SPECIFICATION

(11) **EP 1 140 909 B1**
(45) Date of publication and mention of the grant of the patent: **06.07.2005**
(21) Application number: 99965906.3
(22) Date of filing: 22.12.1999
(51) Int. Cl.: C07D 403/12, C07D 401/14, C07D 241/04, A61K 31/496, A61P 35/00

(54) **NOVEL INHIBITORS OF FARNESYL-PROTEIN TRANSFERASE**
FARNESYL-PROTEIN TRANSFERASE INHIBITOREN
INHIBITEURS DE FARNESYL PROTEINE TRANSFERASE

(30) Priority: 23.12.1998 US 220314
(43) Date of publication of application: 10.10.2001
(73) Proprietor: Schering Corporation, Kenilworth, NJ 07033-0530 (US); Pharmacopeia Drug Discovery, Inc., Cranbury, New Jersey 08512 (US)
(72) Inventor: COOPER, Alan, B., West Caldwell, NJ 07006 (US); DOLL, Ronald, J., Maplewood, NJ 07040 (US); FERREIRA, Johan, A., Bensalem, PA 19020 (US); GANGULY, Ashit, Upper Montclair, NJ 07043 (US); GIRIJAVALLABHAN, Viyyoor, M., Parsippany, NJ 07054 (US); TAVERAS, Arthur, G., Denville, NJ 07834 (US); CHAO, Jianping, Summit, NJ 07901 (US); BALDWIN, John, J., Gwynedd Valley, PA 19437 (US); HUANG, Chia-Yu, Plainsboro, NJ 08536 (US); LI, Ge, West Windsor, NJ 08550 (US)
(74) Representative: Gross, Ulrich-Maria
(86) International application number: PCT/US1999/027958
(87) International publication number: WO 2000/039119

(56) References cited:
- WO-A-94/13646
- WO-A-95/00497
- US-A- 5 880 128
- J.G. BREITENBUCHER: "GENERATION OF A PIPERAZINE-2-CARBOXAMIDE LIBRARY:" TETRAHEDRON LETTERS., vol. 39, no. 11, 1998, pages 1295-8, XP004107918 ELSEVIER SCIENCE PUBLISHERS, AMSTERDAM., NL ISSN: 0040-4039

## Description

### BACKGROUND

Patent application WO 95/00497 published 5 January 1995 under the Patent Cooperation Treaty (PCT) describes compounds which inhibit the enzyme, famesyl-protein transferase (FTase) and the famesyiation of the oncogene protein Ras. Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer.

To acquire transforming potential, the precursor of the Ras oncoproiein must undergo famesylation of the cysteine residue located in a carboxyl-terminal tetrapeptide. Inhibitors of the enzyme that cata zes this modification, famesyl protein transferase, have therefore been suggested as anticancer agents for tumors in which Ras contributes to transformation. Mutated, oncogenic forms of Ras are frequently found in many human cancers, most notably in more than 50% of colon and pancreatic carcinomas (Kohl et al., Science, Vol. 260, 1834 to 1837, 1993).

Patent application WO 94/13646 relates to N,N-diacylpiperazine tachykinin antagonists which are useful in the treatment of inflammatory diseases, pain or migraine, and asthma. Furthermore, such diacylpiperazines are calcium channel blockers useful in the treatment of cardiovascular conditions.

US Patent No. 5,880,128 discloses carbonyl piperazinyl and piperidinyl compounds inhibiting Ras function and therefore inhibiting the abnormal growth of cells.

A method for preparing a library of piperazine-2-carboxamides using solid support synthesis techniques is described in Breitenbucher, J.G., Johnsen, C.R., Haight, M., Phelan, J.C., Tetrahedron Letters, 39 (1998), 1295-1298.

In view of the current interest in inhibitors of farnesyl protein transferase, a welcome contribution to the art would be additional compounds useful for the inhibition of famesyl protein transferase. Such a contribution is provided by this invention.

### SUMMARY OF THE INVENTION

Inhibition of famesyl protein transferase by compounds of this invention has not been reported previously. Thus, this invention provides compounds suitable for inhibiting farnesyl protein transferase which: (i) potently inhibit farnesyl protein transferase, but not geranylgeranyl protein transferase I, in vitro; (ii) block the phenotypic change induced by a form of transforming Ras which is a famesyl acceptor but not by a form of transforming Ras engineered to be a geranylgeranyl acceptor; (iii) block intracellular processing of Ras which is a famesyl acceptor but not of Ras engineered to be a geranylgeranyl acceptor; and (iv) block abnormal cell growth in culture induced by transforming Ras.

This invention provides the use of compounds for the manufacture of a medicament for inhibiting the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene: (2) tumor cells in which the Ras protein is activated as a result of oncogenic mutation in another gene; and (3) benign and malignant cells of other proliferative diseases in which aberrant Ras activation occurs.

Compounds of the present invention are selected from the group consisting of:
the compound of 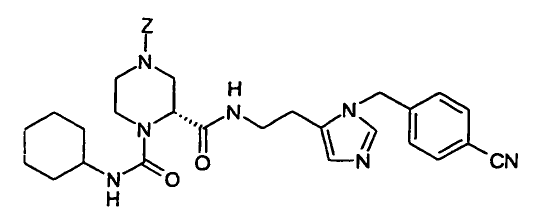 wherein Z is selected from: 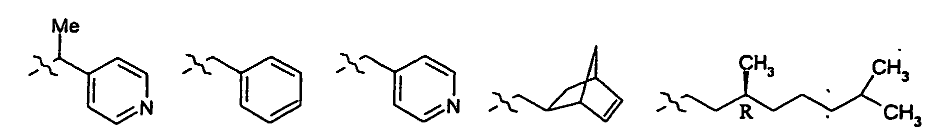 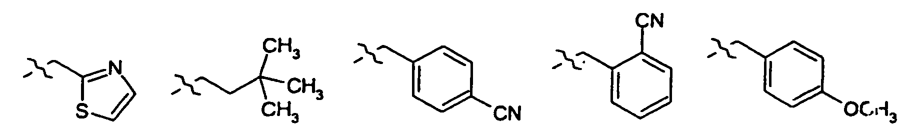 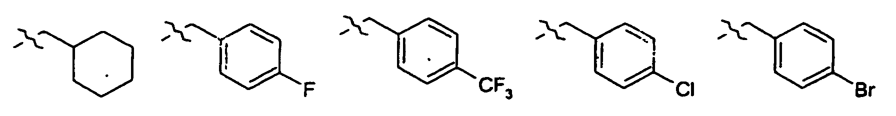 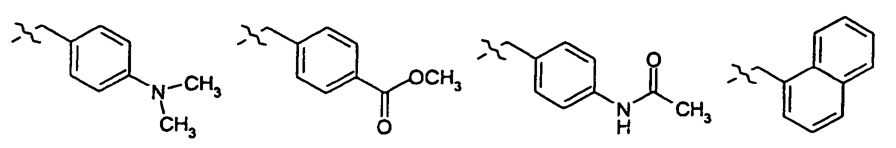 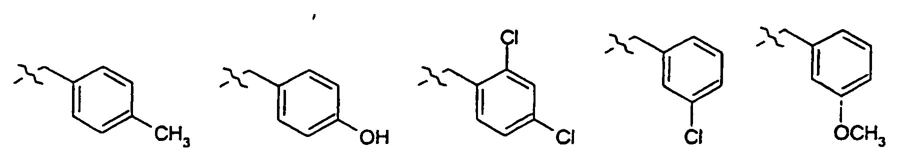 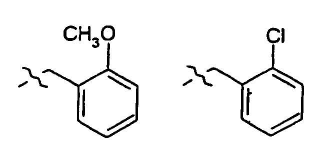 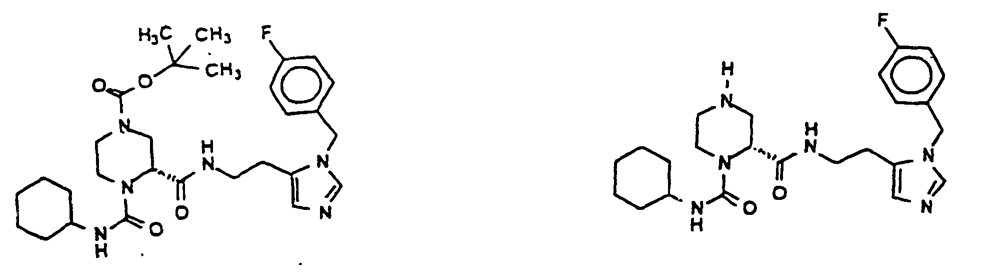 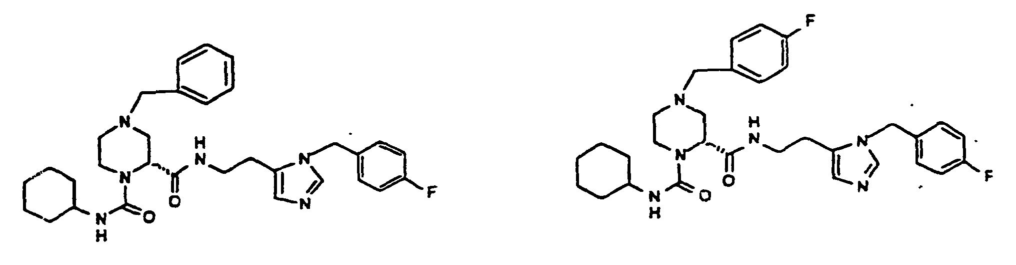 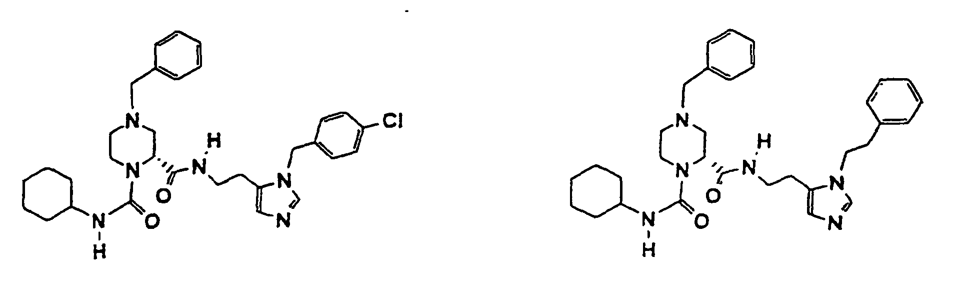
the compound of 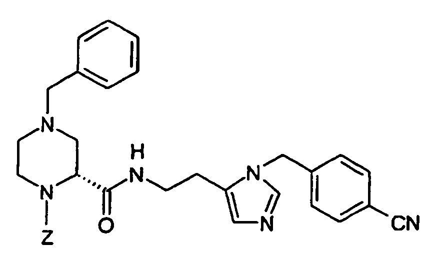 wherein Z is selected from: 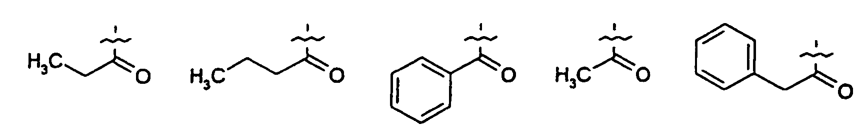 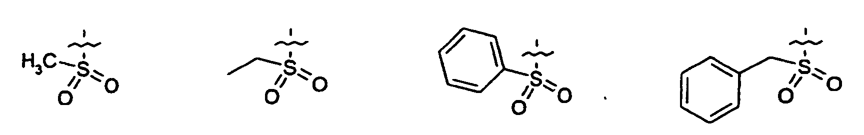 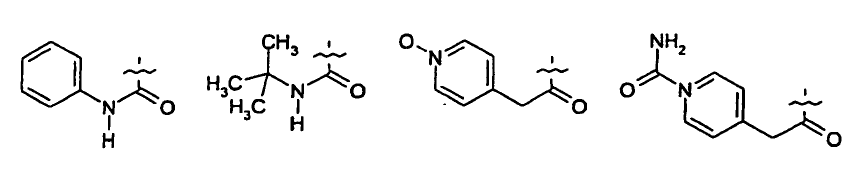 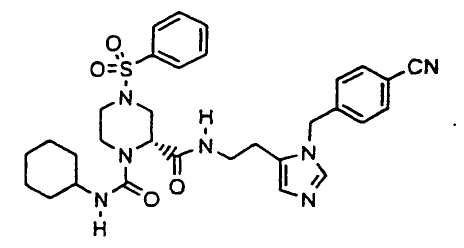 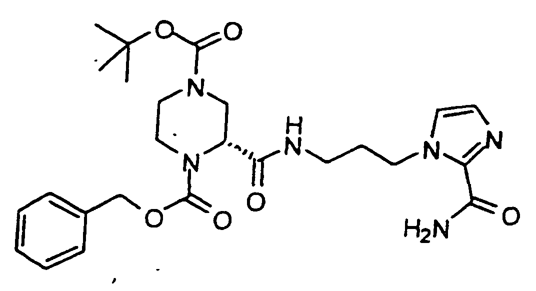 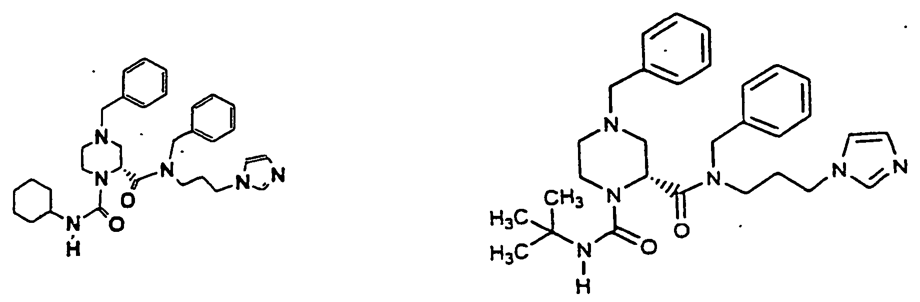 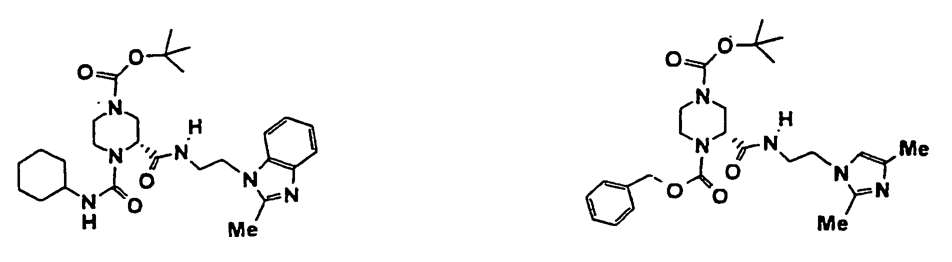 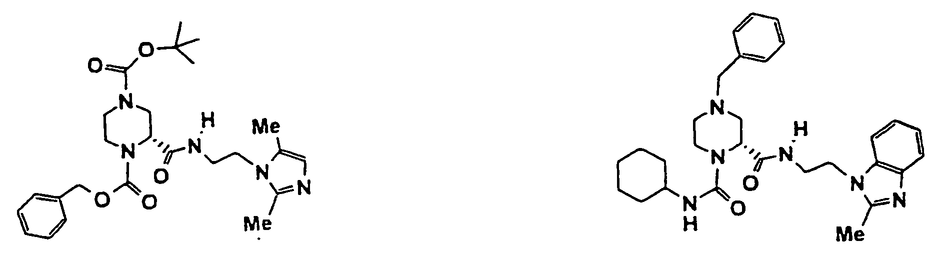 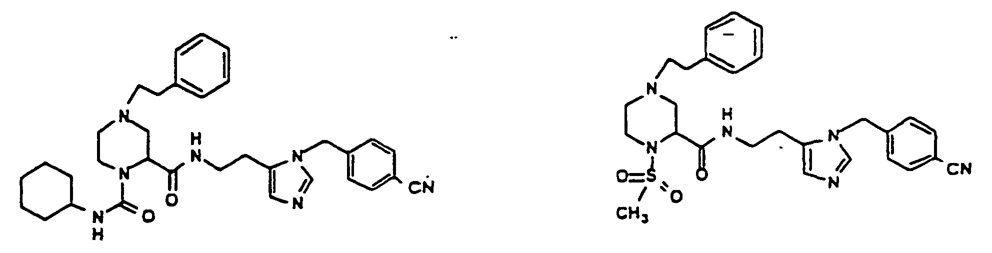 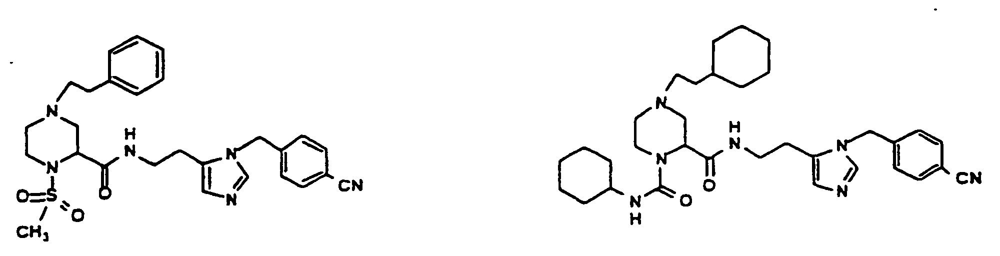 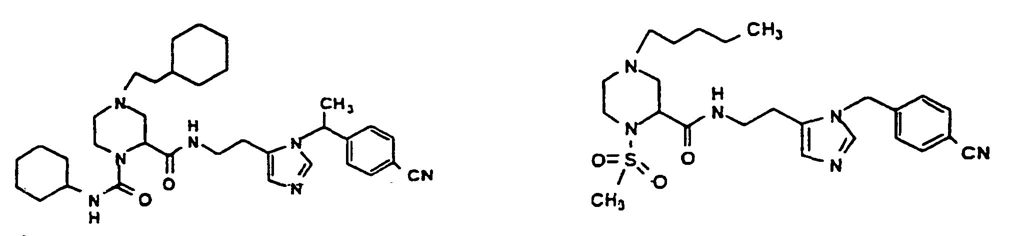 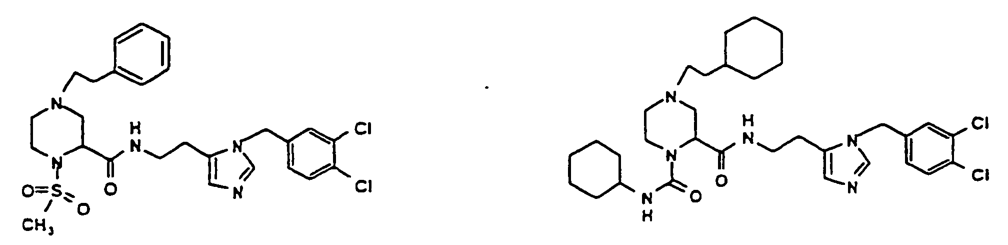 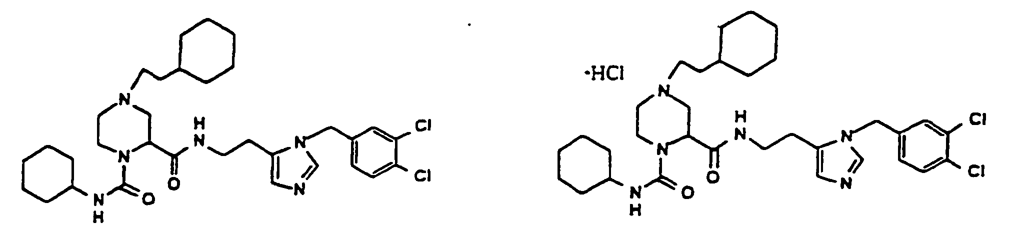 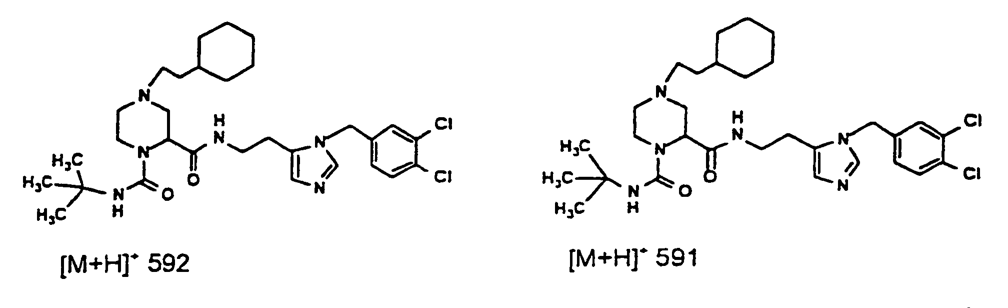 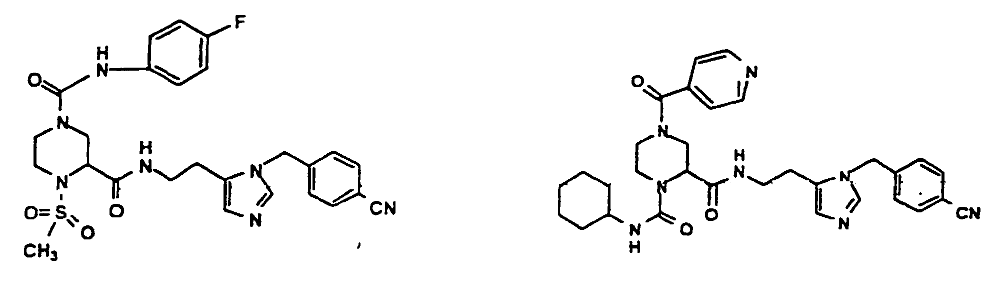 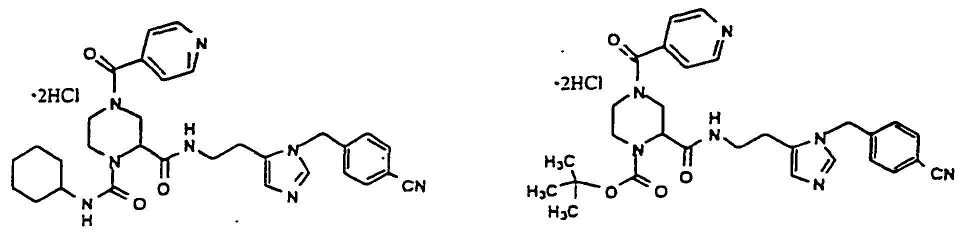 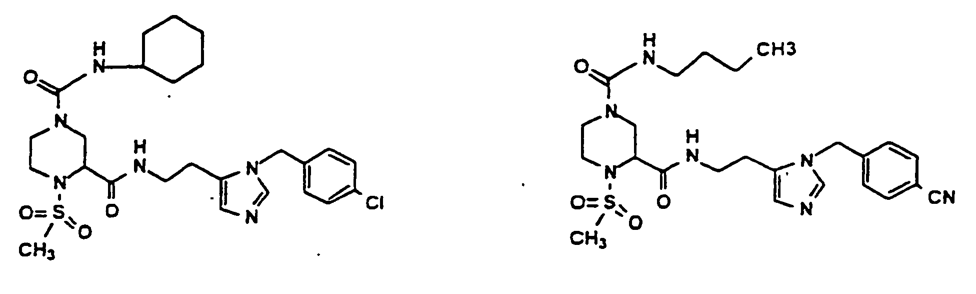 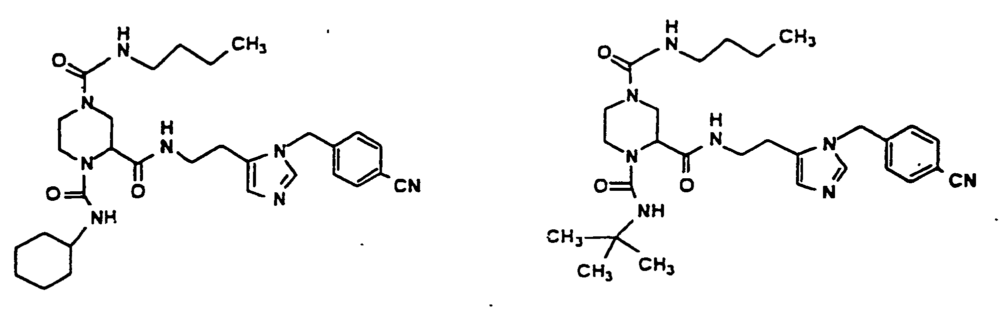 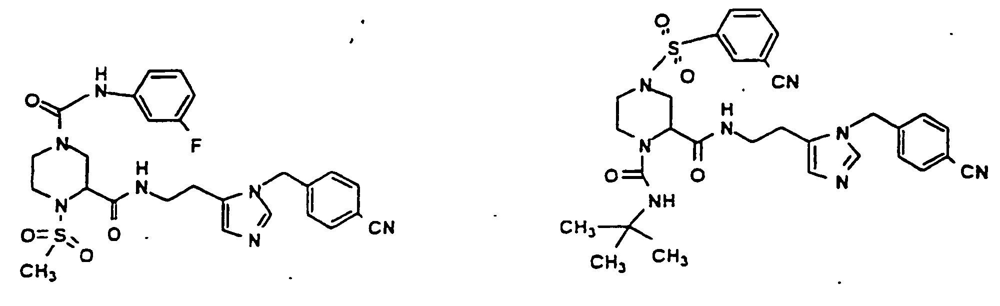 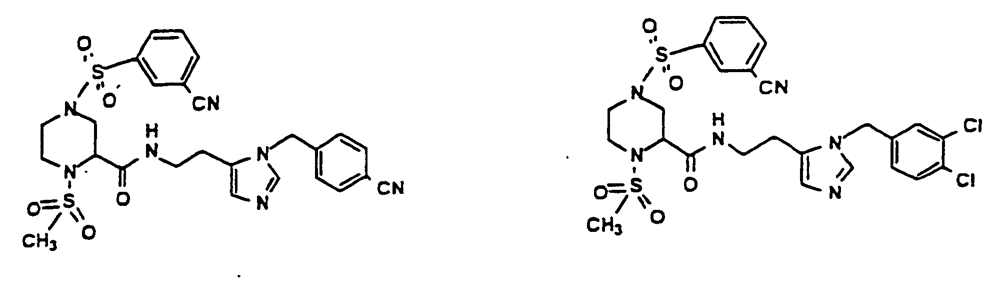 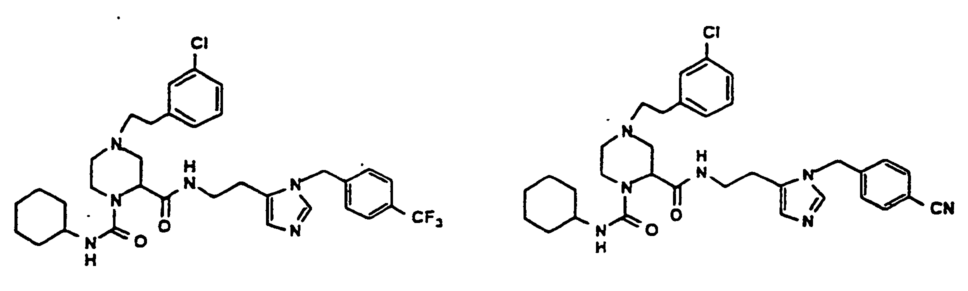 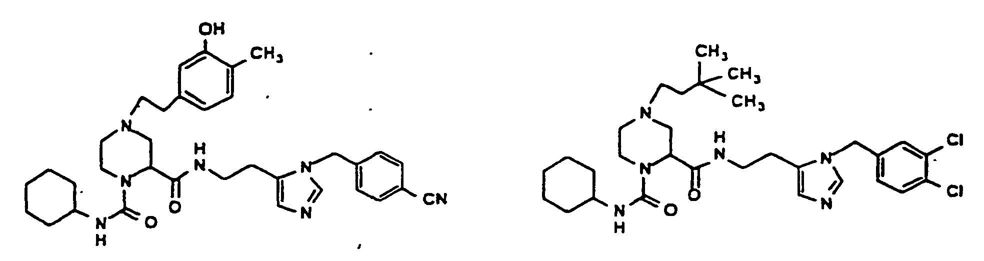 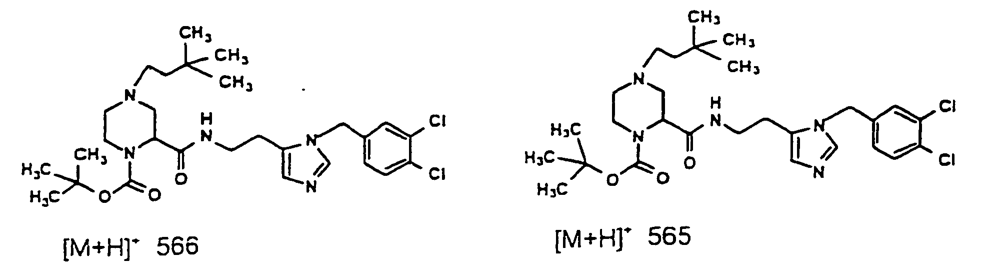 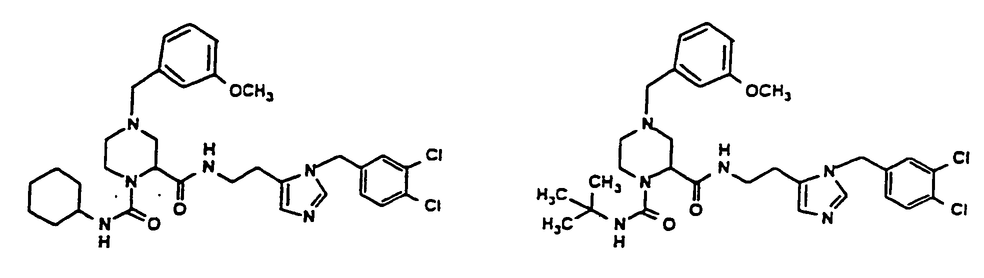 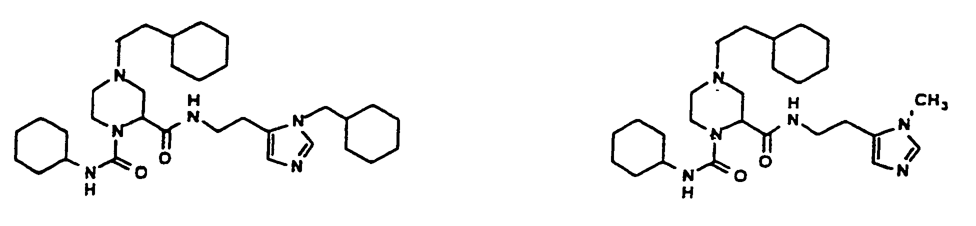 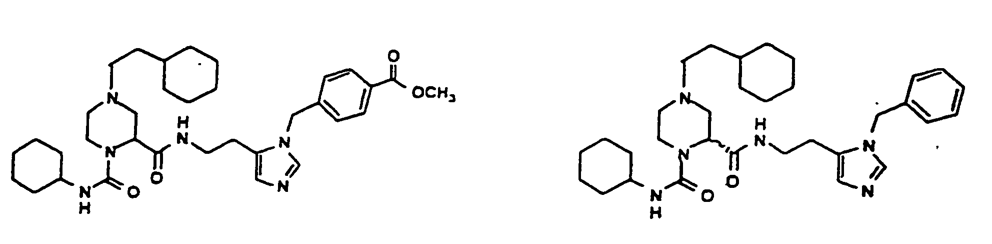 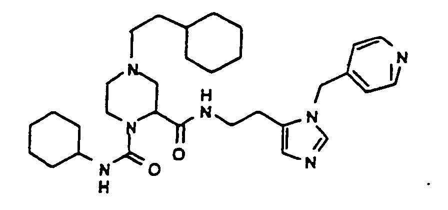
the compound of 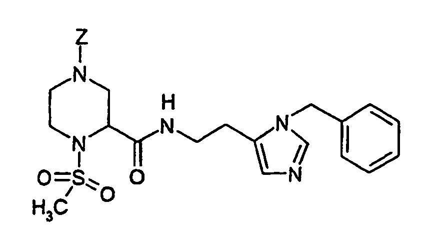 wherein Z is selected from 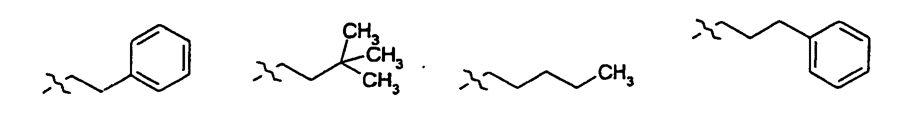 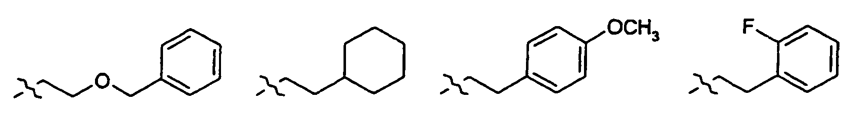 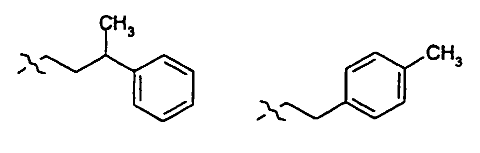
the compound of 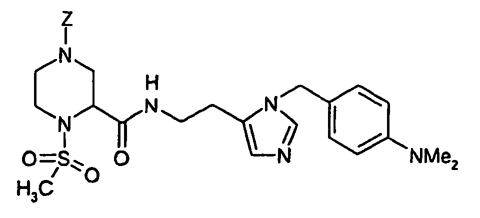 wherein Z is selected from 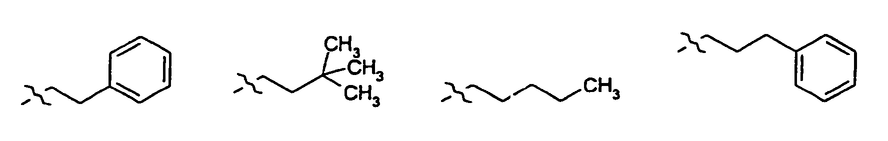 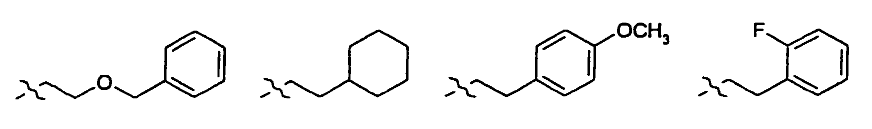 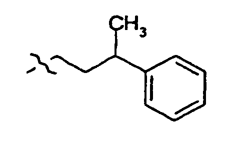
the compound of 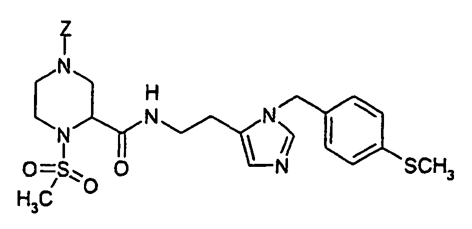 wherein Z is selected from 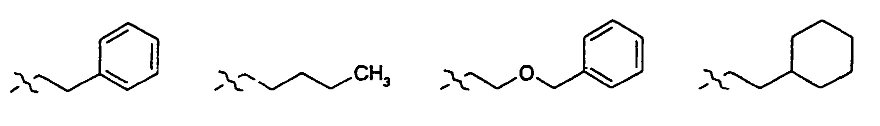 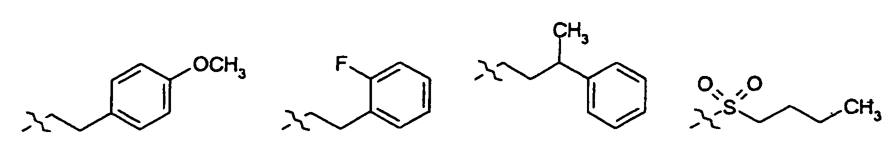 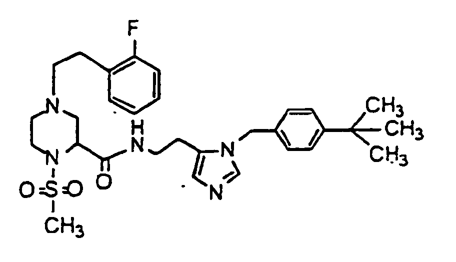
the compound of 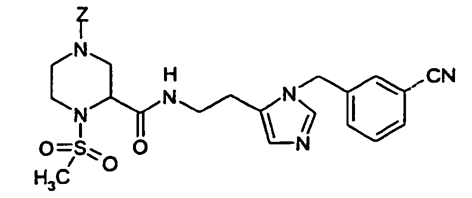 wherein Z is selected from 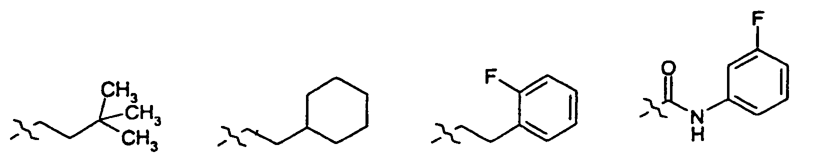 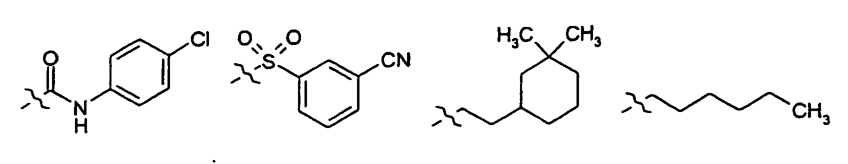
the compound of 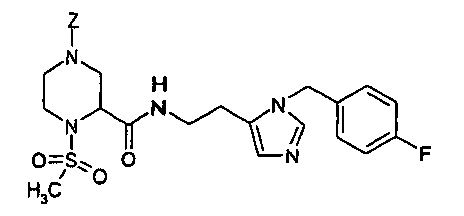 wherein Z is selected from 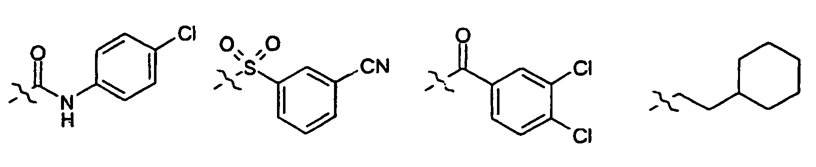 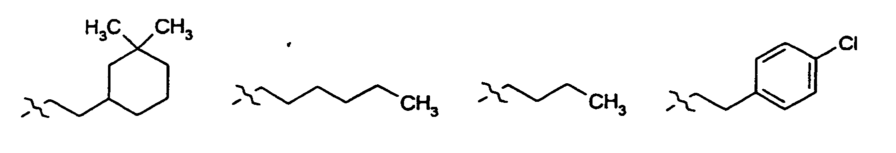 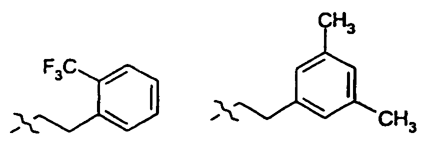 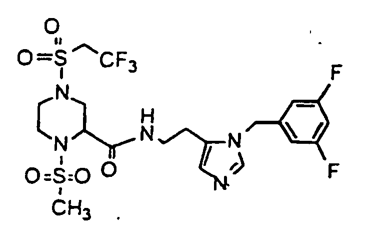 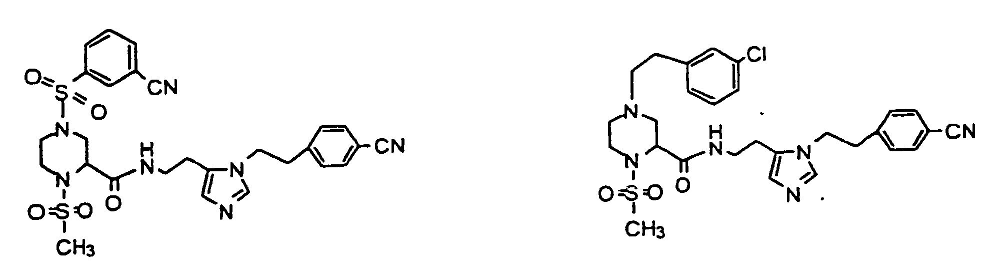
the compound of 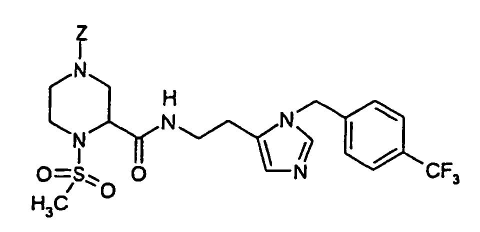 wherein Z is selected from 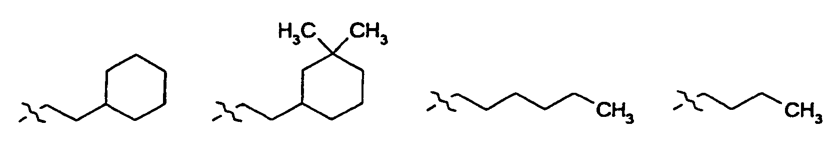 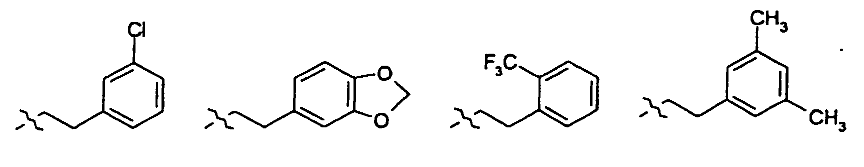
the compound of 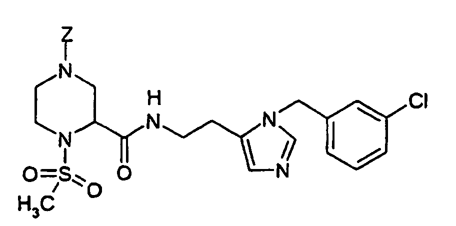 wherein Z is selected from 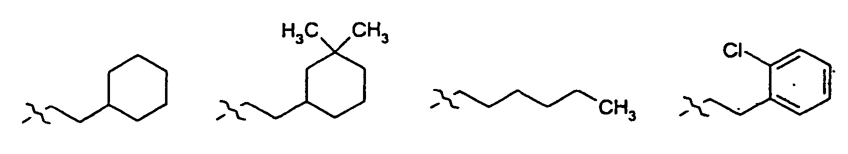 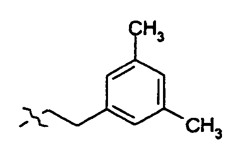
the compound of 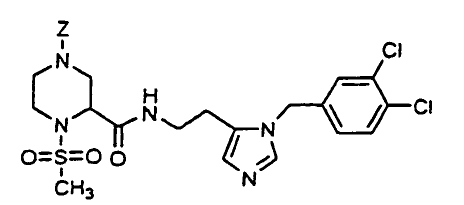 wherein Z is selected from: 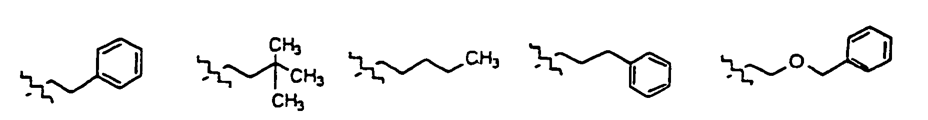 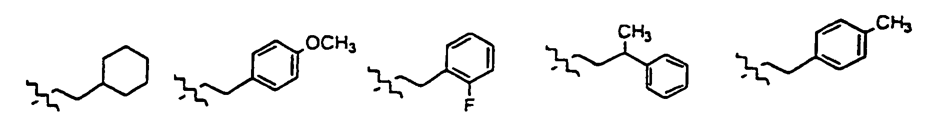 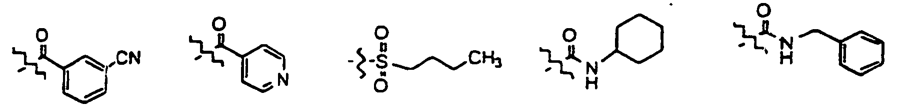 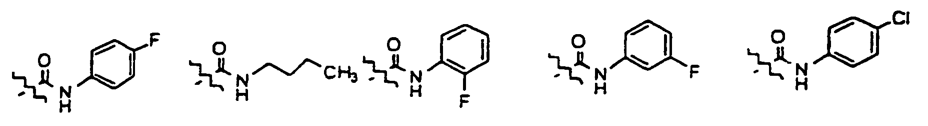 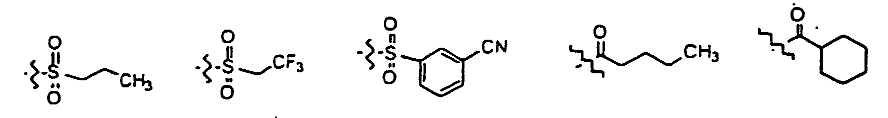 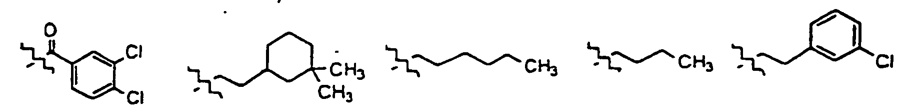 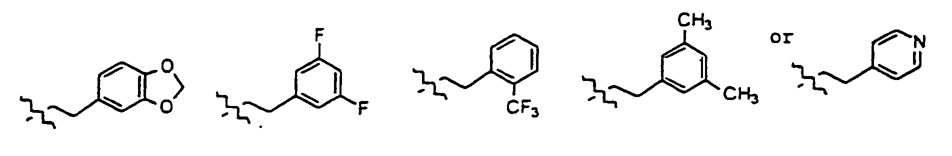
the compound of 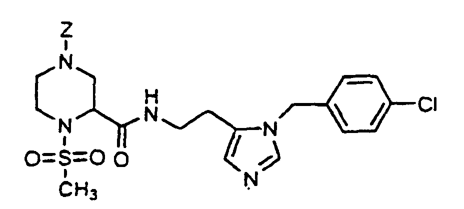 wherein Z is selected from: 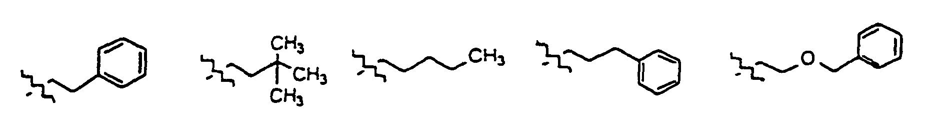 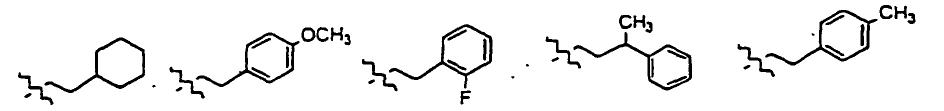 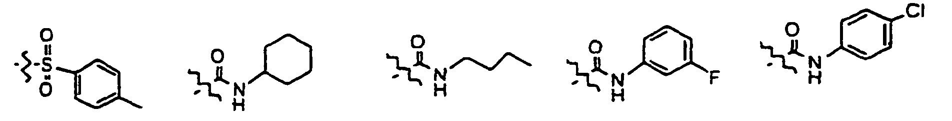 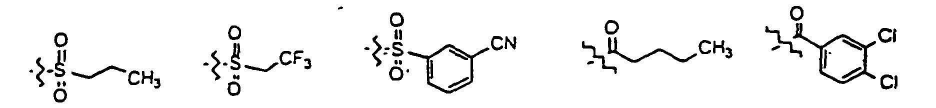 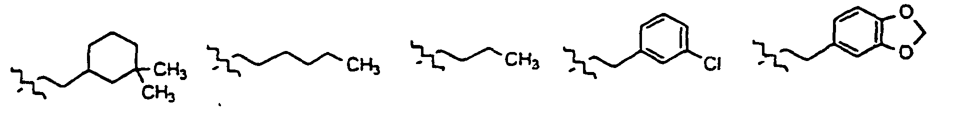 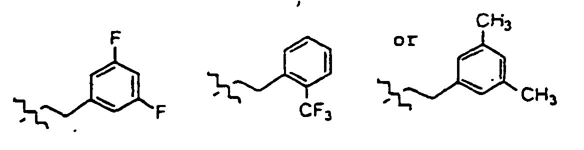
the compound of 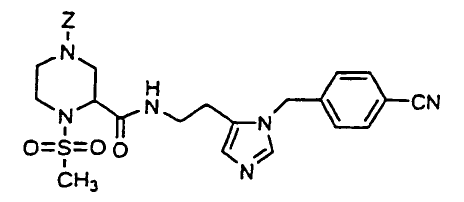 wherein Z is selected from:

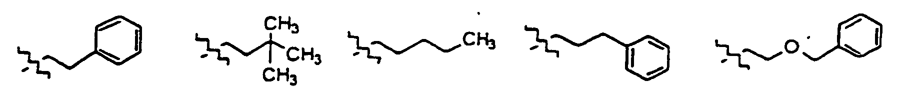

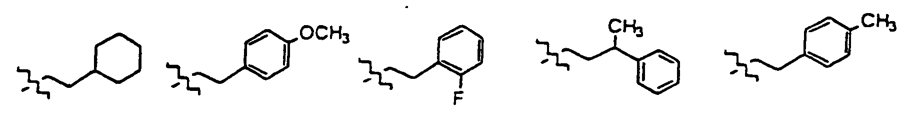

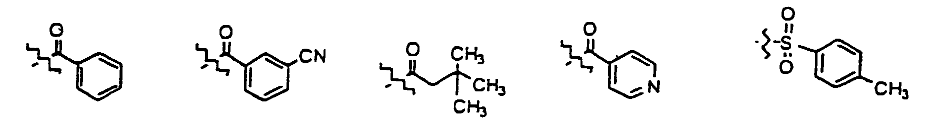

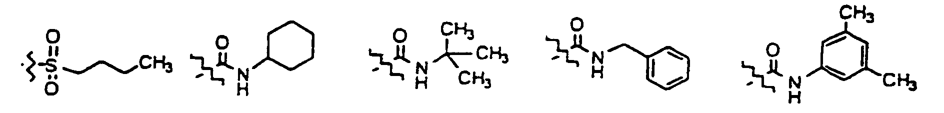

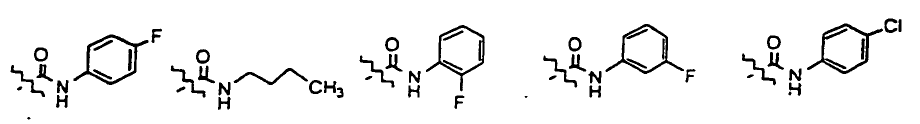

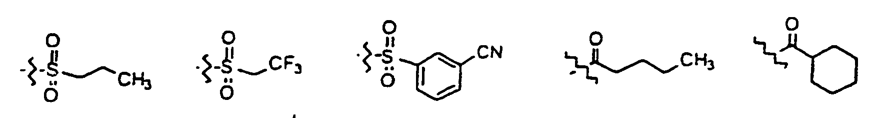

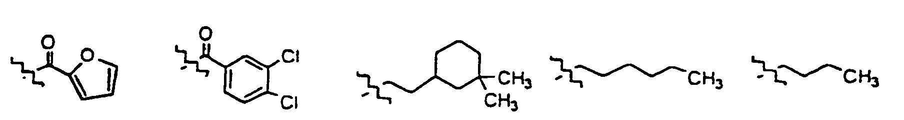

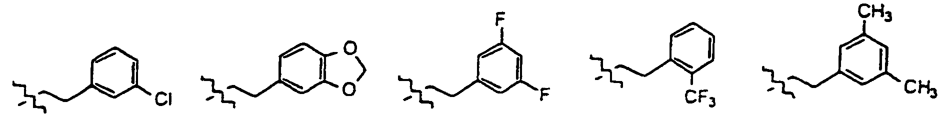

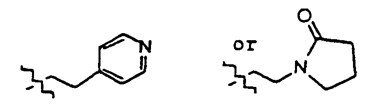

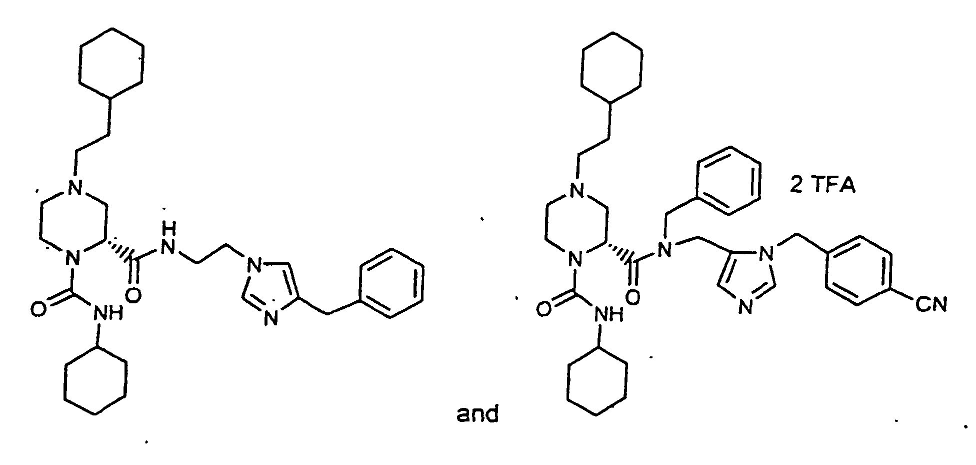

Preferably, the compounds are selected from the group consisting of: 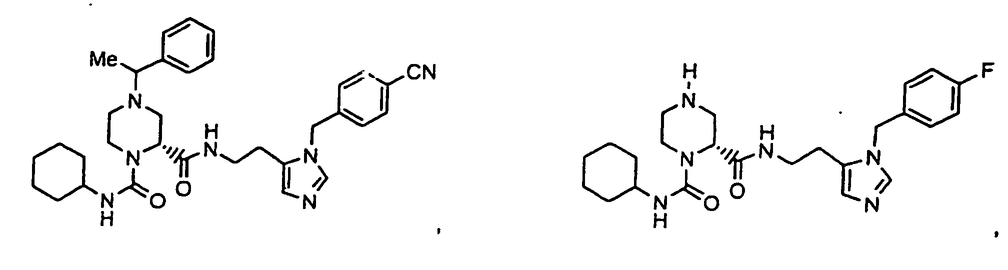 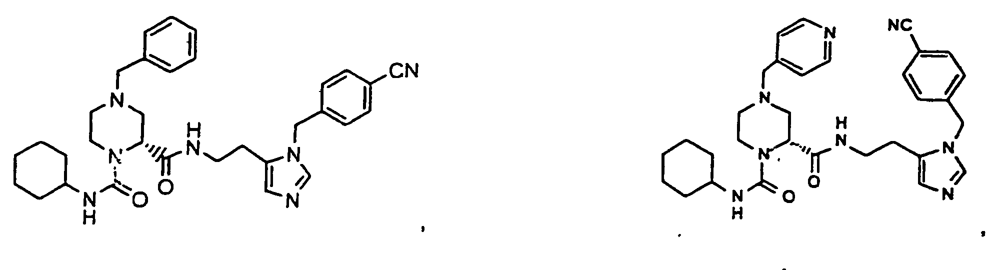 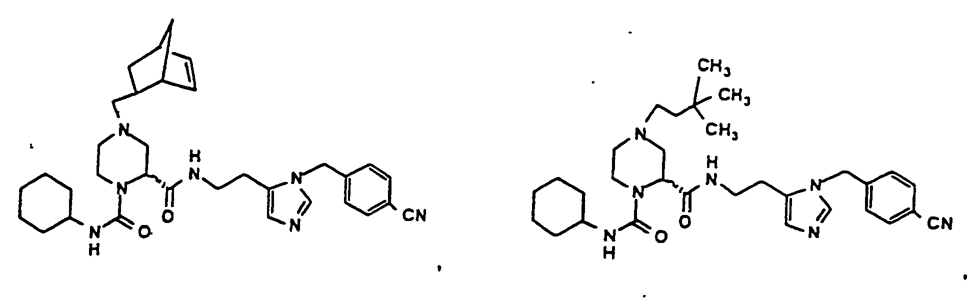 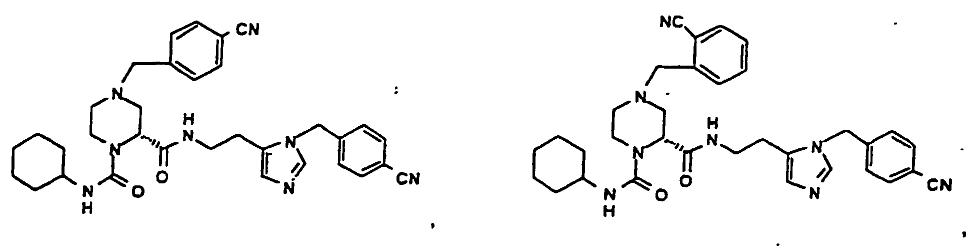 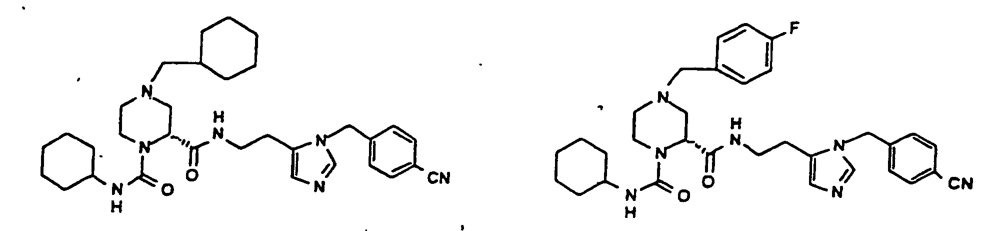 and

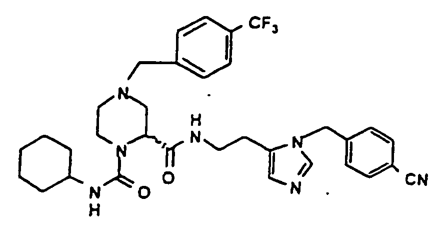

In another embodiment, the present invention is directed toward a pharmaceutical composition comprising an effective amount of a compound according to the invention in combination with a pharmaceutically acceptable carrier.

In another embodiment, the present invention is directed toward the use of a compound for the manufacture of a medicament for inhibiting the abnormal growth of cells, including transformed cells, comprising administering an effective amount of a compound according to the invention to a mammal (e.g., a human) in need of such treatment. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; (3) benign and malignant cells of other proliferative diseases in which aberrant Ras activation occurs, and (4) benign or malignant cells that are activated by mechanisms other than the Ras protein. Without wishing to be bound by theory, it is believed that these compounds may function either through the inhibition of G-protein function, such as ras p21, by blocking G-protein isoprenylation, thus making them useful in the treatment of proliferative diseases such as tumor growth and cancer, or through inhibition of ras famesyl protein transferase, thus making them useful for their antiproliferative activity against ras transformed cells.

The cells to be inhibited can be tumor cells expressing an activated ras oncogene. For example, the types of cells that may be inhibited include pancreatic tumor cells, lung cancer cells, myeloid leukemia tumor cells, thyroid follicular tumor cells, myelodysplastic tumor cells, epidermal carcinoma tumor cells, bladder carcinoma tumor cells, prostate tumor cells, breast tumor cells or colon tumors cells. Also, the inhibition of the abnormal growth of cells by the treatment with a compound according to the invention may be by inhibiting ras famesyl protein transferase. The inhibition may be of tumor cells wherein the Ras protein is activated as a result of oncogenic mutation in genes other than the Ras gene. Alternatively, compounds according to the invention may inhibit tumor cells activated by a protein other than the Ras protein.

This invention also provides the use of a compound according to the invention for the manufacture of a medicament for the inhibition of pancreatic tumor cells, lung cancer cells, myeloid leukemia tumor cells, thyroid follicular tumor cells, myelodysplastic tumor cells, epidermal: carcinoma tumor cells, bladder carcinoma tumor cells or prostate tumor cells, melanoma tumor cells, breast tumor cells or colon tumor cells.

In another embodiment, the present invention is directed toward the use of a compound according to the invention for the manufacture of a medicament for the inhibition of ras farnesyl protein transferase.

### DETAILED DESCRIPTION OF THE INVENTION

As used herein, the following terms are used as defined below unless otherwise indicated:
BOC - represents tert-butoxycarbonyl;
BOC-ON - represents [2-(tert-butoxycarbonyloxyimino)-2-phenylacetonitrile];
C - represents carbon;
CBZ - represents benzyloxycarbonyl;
ClCO₂Et - ethyl chloroformate;
CPh₃ - represents triphenylmethyl;
cycloalkyl-represents a saturated carbocyclic nng, branched or unbranched, of from 3 to 20 carbon atoms, preferably 3 to 7 carbon atoms;
DBU - represents 1,8-diazabicyclo[5.4.0]undec-7-ene;
DCC - represents dicyclohexylcarbodiimide;
DCM - represents dichloromethane;
DIC - represents diisopropylcarbodiimide;
DIPEA - diiosopropyl ethylamine
DMAP - represents 4-dimethylaminopyridine;
DMF - represents N,N-dimethylformamide;
EDC (also DEC) - represents 1-(3-dimethylaminopropyl)-3-ethylcarbodiimide hydrochloride or 1-ethyl-3-(3-dimethylaminopropyl)carbodiimide hydrochloride;
EtOAc - ethyl acetate;
EtOH - ethanol;
Et₃N - triethylamine;
FMOC - represents 9-fluorenylmethyoxycarbonyl;
FMOC-Cl - represents 9-fluoroenylmethyl chloroformate;
HATU - represents [O-(7-azabenzotriazol-1-yl)-1,1,3,3-tetramethyluronium hexafluorophosphate],
HOAc or AcOH - acetic acid;
HOBT - hydroxybenzotriazole;
Lutidine - 2,6-lutidine;
MCPBA - represents m-chloroperbenzoic acid;
Me - methyl;
MeOH - methanol;
NaBH⁵CN - sodium cyano borohydride
Ph - represents phenyl;
TBAF - represents tetrabutylammonium fluoride;
TFA - represents trifluoroacetic acid;
TFAA - trifluoroacetic anhydride:
THF - represents tetrahydrofuran.
M⁺ -represents the molecular ion of the molecule in the mass spectrum;
MH⁺ -represents the molecular ion plus hydrogen of the molecule in the mass spectrum;
Bu-represents butyl;
Et-represents ethyl;
Me-represents methyl;
Ph-represents phenyl;
benzotriazol-1-yloxy represents 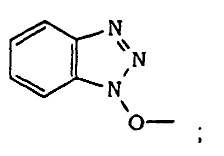
1-methyl-tetrazol-5-ylthio represents 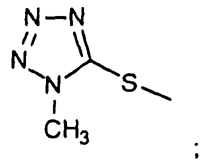
alkyl-(including the alkyl portions of alkoxy, alkylamino and dialkylamino)-represents straight and branched carbon chains and contains from one to twenty carbon atoms, preferably one to ten carbon atoms, also preferably one to six carbon atoms; for example methyl, ethyl, propyl, isopropyl, n-butyl, t-butyl, n-pentyl, isopentyl, hexyl, isononyl and the like; wherein said alkyl group may be optionally and independently substituted with one, two, three or more of the following: halo, alkyl, aryl, cycloalkyl, cyano (-CN), -CF₃, oxy (=O), aryloxy, -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² can independently represent hydrogen, alkyl, alkoxy, aryl, arylalkyl, heteroaryl, heteroarylalkyl, cycloalkyl, cycloalkylalkyl, heterocycloalkyl or heterocycloalkylalkyl;
alkenyl-represents straight and branched carbon chains having at least one carbon to carbon double bond and containing from 2 to 12 carbon atoms, preferably from 2 to 6 carbon atoms and most preferably from 3 to 6 carbon atoms; wherein said alkenyl group may be optionally and independently substituted with one, two, three or more of the following: halo, alkyl, aryl, alkoxy, amino, alkylamino, cyano (-CN), -CF₃, dialkylamino, hydroxy, oxy, phenoxy, -OCF₃, heterocycloalkyl, -SO₂NH₂, -NHSO₂R¹⁰, -SO₂NHR¹⁰, -SO₂R¹⁰, -SOR¹⁰, -SR¹⁰, -NHSO₂, -NO₂, -CONR¹⁰, -NCOR¹⁰ or -COOR¹⁰;
alkoxy-an alkyl moiety of one to 20 carbon atoms covalently bonded to an adjacent structural element through an oxygen atom, for example, methoxy, ethoxy, propoxy, butoxy, pentoxy, hexoxy and the like; wherein said alkoxy group may be optionally and independently substituted with one, two, three or more of the following: halo, alkyl, aryl, cycloalkyl, cyano (-CN), -CF₃, oxy (=O), aryloxy, -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
aryl (including the aryl portion of arylalkyl) - represents a carbocyclic group of 6 to 15 carbon atoms containing one or two aromatic rings (e.g., aryl is phenyl); wherein optionally, said aryl group can be fused with one other aryl, cycloalkyl, heteroaryl or heterocycloalkyl ring provided that when the moiety "Z" in compound (1.0) is aryl, the fused aryl is a bicyclic ring (e.g napthalene) which is not a tricyclic or greater fused ring system; and wherein any of the available substitutable carbon and nitrogen atoms in said aromatic rings and/or said fused rings may be optionally and independently substituted with one, two, three or more of the following: halo, alkyl, aryl, cycloalkyl, cyano (-CN), -CF₃, oxy (=O), aryloxy, -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
arylalkyl - represents an alkyl group, as defined above, wherein one or more hydrogen atoms of the alkyl moiety have been substituted with one or more aryl groups, as defined above (e.g. arylalkyl is benzyl or phenylethyl); wherein optionally, said arylalkyl group can be fused with one other aryl, cycloalkyl, heteroaryl or heterocycloalkyl ring provided that when the moiety "Z" in compound (1.0) is arylalkyl, the fused arylalkyl is a bicyclic ring (e.g napthalenyl) which is not a tricyclic or greater fused ring system; wherein said arylalkyl group may be optionally and independently substituted with one, two, three or more of the following: halo, alkyl, aryl, cycloalkyl, cyano (-CN), -CF₃, oxy (=O), -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
cycloalkyl-represents saturated carbocyclic rings branched or unbranched of from 3 to 20 carbon atoms, preferably 3 to 7 carbon atoms (e.g. cycloalkyl is cyclopropyl, cyclobutyl, cyclopentyl, cyclohexyl and the like); wherein said cycloalkyl ring optionally can be fused with one other cycloalkyl, cycloalkenyl or heterocycloalkyl ring to form a bicyclic ring which is not a tricyclic or greater fused ring system; wherein any of the available substitutable carbon and nitrogen atoms in said cycloalkyl ring and/or said fused ring may be optionally and independently substituted with one, two, three or more of the following: halo, alkyl, aryl, cycloalkyl, cyano (-CN), -CF₃, oxy (=O), aryloxy, -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
cycloalkylalkyl - represents an alkyl group, as defined above, wherein one or more hydrogen atoms of :he alkyl moiety have been substituted with one or more cycloalkyl rings as defined above; wherein said cycloalkylalkyl group may be optionally and independently substituted with one, two, three or more of the following: halo, alkyl, aryl, cycloalkyl, cyano (-CN), -CF₃, oxy (=O), -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
cycloalkenyl - represents a carbocyclic ring having one or two unsaturated bonds (i.e. carbon to carbon double bonds) and containing from 3 to 20 carbon atoms, preferably 3 to 7 carbon atoms wherein said one or two unsaturated bonds do not impart aromatic character to the cycloalkenyl ring; wherein said cycloalkenyl ring optionally can be fused with one other cycloalkyl, cycloalkenyl or heterocycloalkyl ring to form a bicyclic ring (e.g. norbornenyl) which is not a tricyclic or greater fused ring system; wherein any of the available substitutable carbon and nitrogen atoms in said cycloalkenyl ring and/or said fused ring may be optionally and independently substituted with one, two, three or more of the following: halo, alkyl, aryl, cycloalkyl, cyano (-CN), -CF₃, oxy (=O), -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
cycloalkenylalkyl - represents an alkyl group, as defined above, wherein one or more hydrogen atoms of the alkyl moiety have been substituted with one or more cycloalkenyl rings as defined above; wherein said cycloalkenylalkyl group optionally can be fused with one other cycloalkyl, cycloalkenyl or heterocycloalkyl ring to form a bicyclic ring (e.g. norbomylmethyl); wherein any of the available substitutable carbon and nitrogen atoms in said cycloalkenylalkyl group and/or said fused ring may be optionally and independently substituted with one, two, three or more of the following: halo, alkyl, aryl, cycloalkyl, cyano (-CN), -CF₃, oxy (=O), -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
halo-represents fluoro, chloro, bromo and iodo;
heteroalkyl-represents straight and branched carbon chains containing from one to twenty carbon atoms, preferably one to six carbon atoms interrupted by 1 to 3 heteroatoms selected from -O-, -S- and -N-; wherein any of the available substitutable carbon and nitrogen atoms in said heteroalkyl chain may be optionally and independently substituted with one, two, three or more of the following: halo, alkyl, aryl, cycloalkyl, cyano (-CN), -CF₃, oxy (=O), -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove;
heteroaryl-represents cyclic groups having at least one heteroatom selected from O, S and N, said heteroatom(s) interrupting a carbocyclic ring structure and having a sufficient number of delocalized pi electrons to provide aromatic character, with the aromatic heterocyclic groups containing from 2 to 14 carbon atoms (e.g. heteroaryl is imidazoyl); wherein said heteroaryl group optionally can be fused with one aryl, cycloalkyl, heteroaryl or heterocycloalkyl ring to form a bicyclic ring which is not a tricyclic or greater fused ring system; and wherein any of the available substitutable carbon or nitrogen atoms in said heteroaryl group and/or said fused ring may be optionally and independently substituted with one, two, three or more of the following: halo, alkyl, aryl, arylalkyl, cycloalkyl, cyano (-CN), -CF₃, oxy (=O), -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove. Representative heteroaryl groups can include, for example, furanyl, imidazoyl, pyrimidinyl, triazolyl, 2-, 3- or 4-pyridyl or 2-, 3- or 4-pyndyl N-oxide wherein pyridyl N-oxide can be represented as: 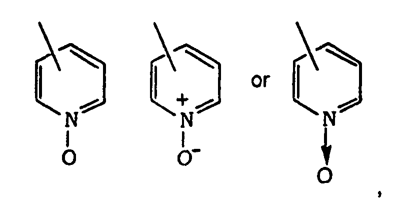
heteroarylalkyl - represents an alkyl group, as defined above, wherein one or more hydrogen atoms have been replaced by one or more heteroaryl groups; wherein said heteroaryl group optionally can be fused with one aryl, cycloalkyl, heteroaryl or heterocycloalkyl ring to form a bicyclic ring which is not a tricyclic or greater fused ring system; and wherein any of the available substitutable carbon or nitrogen atoms in said heteroaryl group and/or said fused ring may be optionally and independently substituted with one, two, three or more of the following: wherein said heteroarylalkyl group may be optionally and independently substituted with one, two, three or more of the following: halo, alkyl, aryl, cycloalkyl, cyano (-CN), -CF₃, oxy (=O), -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -CH₂NR¹²COR¹⁰, -NR¹²COR¹⁰, -COR¹⁰, -OCOR¹⁰, -OCO₂R¹⁰ or -COOR¹⁰, wherein R¹⁰ and R¹² are as defined hereinabove;
heterocycloalkyl-represents a saturated, branched or unbranched carbocylic ring containing from 3 to 15 carbon atoms, preferably from 4 to 6 carbon atoms, which carbocyclic ring is interrupted by 1 to 3 heteroatoms selected from -O-, -S- and -N- , wherein optionally, said ring may contain one or two unsaturated bonds which do not impart aromatic character to the ring; wherein said heterocycloalkyl group optionally can be fused with one aryl, cycloalkyl, heteroaryl or heterocycloalkyl ring to form a bicyclic ring which is not a tricyclic or greater fused ring system; and wherein any of the available substitutable carbon or nitrogen atoms in said heterocycloalkyl group and/or said fused nng may be optionally and independently and wherein any of the available substitutable carbon and nitrogen atoms in the ring may be optionally and independently substituted with one, two, three or more of the following: halo, alkyl, aryl, cycloalkyl, cyano (-CN), -CF₃, oxy (=O), aryloxy, -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove. Representative heterocycloalkyl groups can include 2- or 3-tetrahydrofuranyl, 2- or 3- tetrahydrothienyl, 1-, 2-, 3- or 4-piperidinyl, 2- or 3-pyrrolidinyl, 1-, 2- or 3-pipenzinyl, 2- or 4-dioxanyl, morpholinyl, 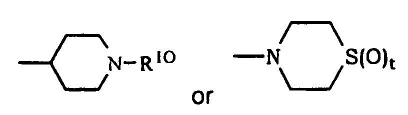 wherein R¹⁰ is defined hereinbefore and t is 0, 1 or 2.
heterocycloalkalkyl- represents an alkyl group, as defined above, wherein one or more hydrogen atoms have been replaced by one or more heterocycloalkyl groups; wherein optionally, said ring may contain one or two unsaturated bonds which do not impart aromatic character to the nng; and wherein said heterocycloalkylalkyl group may be optionally and independently substituted with one, two, three or more of the following: halo, alkyl, aryl, cycloalkyl, cyano (-CN), -CF₃, oxy (=O), aryloxy, -OR¹⁰, -OCF₃, heterocycloalkyl, heteroaryl, -NR¹⁰R¹², -NHSO₂R¹⁰, -SO₂NH₂, -SO₂NHR¹⁰, -SO₂R¹⁰, -SOR¹⁰, -SR¹⁰, -NHSO₂, -NO₂, -CONR¹⁰R¹², -NR¹²COR¹⁰, -COR¹⁰, -OCOR¹⁰, -OCO₂R¹⁰ or -COOR¹⁰, wherein R¹⁰ and R¹² are as defined hereinabove.

Certain compounds of the invention may exist in different stereoisomeric forms (e.g., enantiomers, diastereoisomers and atropisomers). The invention contemplates all such stereoisomers both in pure form and in mixture, including racemic mixtures.

Certain compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those skilled in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purpopses of the invention.

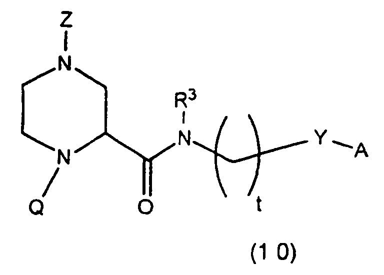

Compounds of the present invention can be prepared according to the following Schemes.

Library Preparation. A library of compounds is prepared by parallel synthesis. A generic structure of these compounds is shown in Figure 1. The "A" group on the side chain histamine along with "Z" group on N-4 of the piperazine are varied in the library. Every member of the library contains methyl sulfonate at position N-1 of the piperazine core (Figure 1).

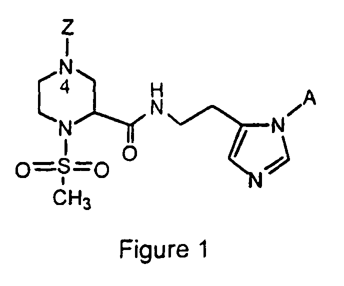

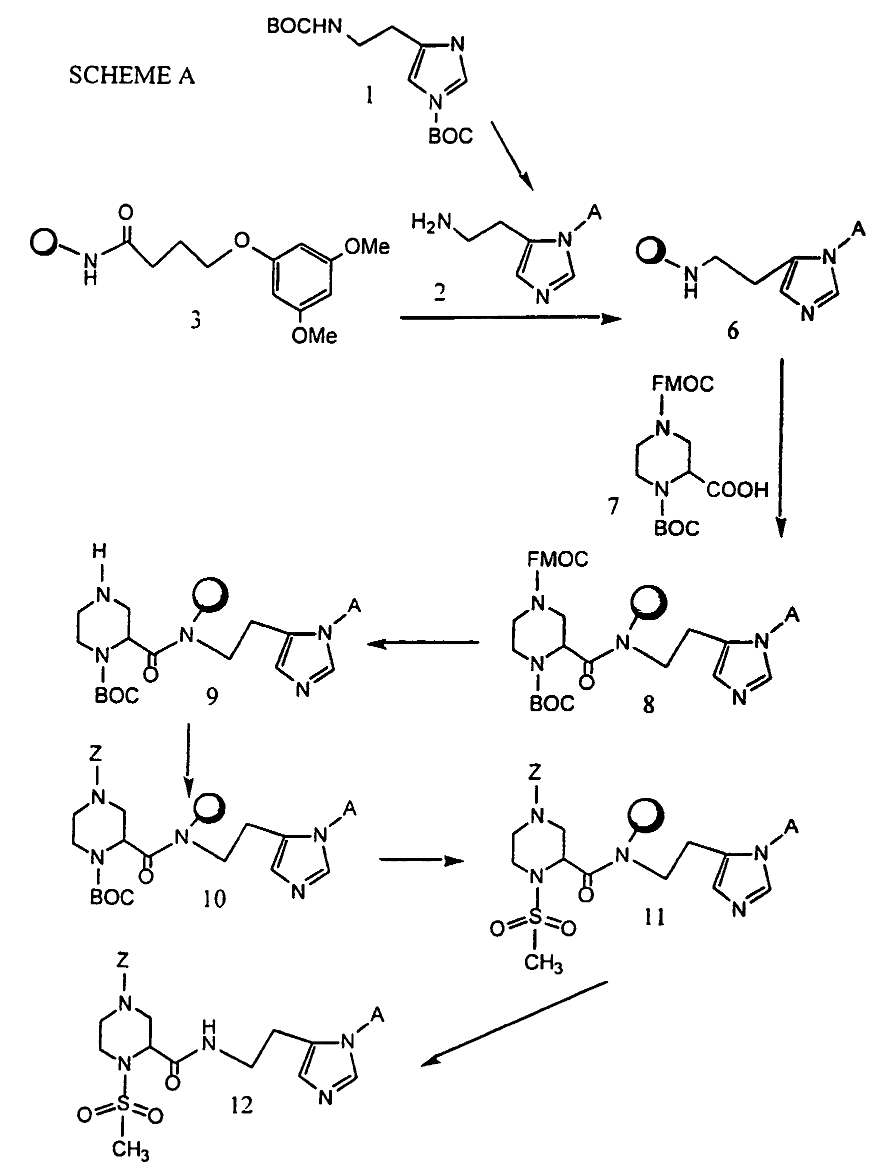

Referring to Scheme A, the side chain amines 2 were prepared by treating bis-Boc histamine 1 with the corresponding tnflate, and subsequent removal of the Boc group. Library was prepared on TentaGel® (trademark of Rapp Inc., Germany) resin 3 functionalized with 4-(4-formyl-3-methoxyphenoxy)butyric acid 4 (shown in Scheme 1) to give functionalized resin 5. TentaGel resin is a composite of low-cross-linked polystrrene and polyethylene glycol, which has been terminally ammo functionalized. Synthesis was initiated in Merrifield shaker vessels with reductive amination of the side chain amines 2 using the aldehyde of the acid cleavable linker of the functionalized resin 5 to give 6. This was followed by coupling N-4-Fmoc N-1-Boc piperazine carboxylic acid 7 with 6 to give 8. Removal of the Fmoc group of 8 gives 9. Resin 9 was dried and loaded into Robbins FlexChem block (96 wells) and subsequent reactions were performed in the block. Resin 9 was reductively alkylated with a number of corresponding aldehydes with NaBH₃CN in 2% HOAc in DMF or acylated with corresponding acid chlorides with lutidine in CH₂Cl₂ or sulfonyl chlorides with lutidine in CH₂Cl₂, or treated with isocyanates and DIPEA in CH₂Cl₂. Treatment of resin 10 with 10% HCl/MeOH followed by methanesulfonyl chloride gives resin 11. The product 12 is cleaved from resin 1 using trifluoroacetic acid.

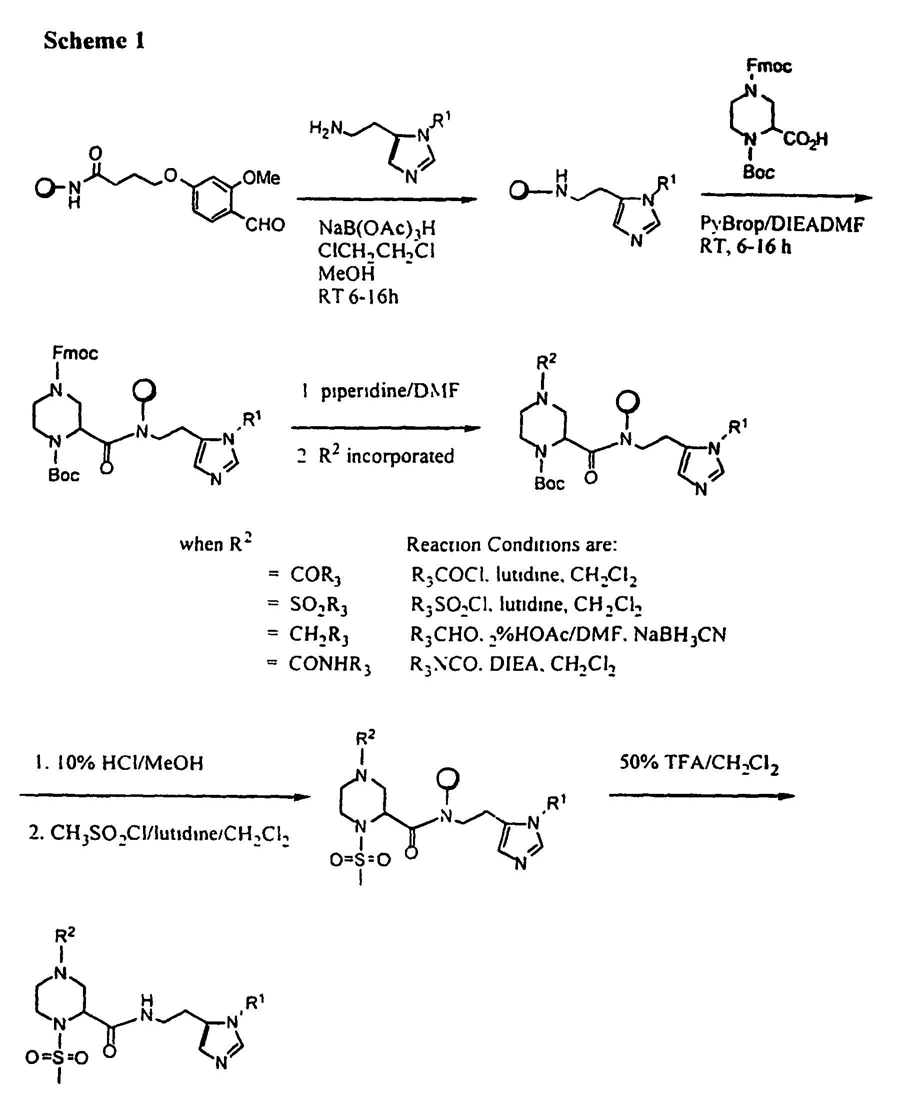

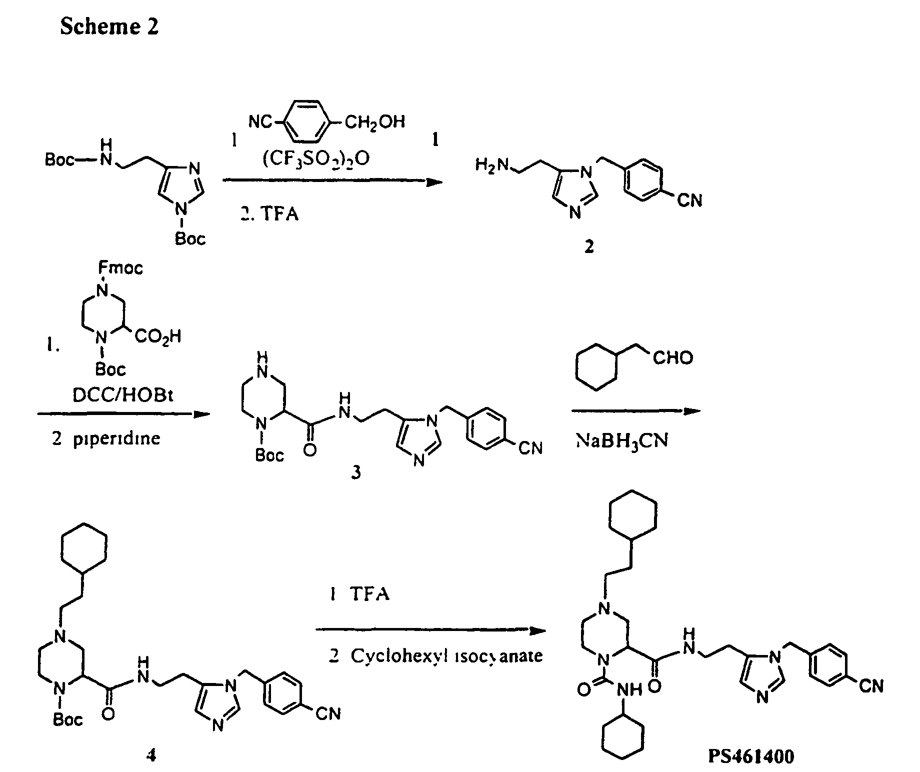

### Preparation procedure for PS 461400 (Scheme 2)

1. Preparation of compound **2**. To a stirred solution of triflic anhydride (1.9 mL, 11.3 mmole) in CH₂Cl₂ (35 mL) under Ar at -75 °C was added a solution of 4-cyanobenzyl alcohol (1)(1.37g, 10.3 mmole) and diisopropylethylamine (1.97 mL, 11.3 mmole) in CH₂Cl₂ (12 mL) dropwise. Stirring at -78 °C was continued for 20 min. and a solution of N-Bis-Boc-histamine (3.2 g, 10.3 mmole) in CH₂Cl₂ (35 mL) was added. The reaction was allowed to warm to room temperature slowly and stirred overnight. The reaction mixture was washed with sat. Na₂CO₃ solution (30 mL). The organic phase was dried (Na₂SO₄) and solvent removed in vacuo. The residue was purified by column chromatography with 5 % MeOH in CH₂Cl₂ to give 1.38g product in 41 % yield. MS: m/z 327 (MH⁺). This compound was treated with 25% trifluoroacetic acid in CH₂Cl₂ (20 mL) for 1 h. Solvent was removed in vacuo. The residue was dissolved in 5% HCl (15 mL) and washed with CH₂Cl₂ (10 mLx2). The aqueous layer was basified with NaOH to PH=9 and extracted with ethyl acetate (30 mLx 4). The combined organic phase was dried (Na₂SO₄) and solvent removed in vacuo to give 600 mg desired amine **2** in 66% yield. MS: m/z 227 (MH⁺).
2. Preparation of compound **3**. To a stirred solution of the amine (**2**) (600 mg, 2.65 mmole) and N-4-Fmoc-N-1-Boc-piperazine carboxylic acid (1 g, 2.21 mmole) in CH₂Cl₂ (20 mL) was added DCC (547 mg, 2.65 mmole) and HOBt (358 mg, 2.65 mmole). The reaction was stirred overnight. The reaction mixture was filtered. The filtrate was washed with water (20 mL). The organic phase was dried (Na₂SO₄) and solvent removed in vacuo. The residue was purified by flash column chromatography with 5% MeOH in CH₂Cl₂ to give the amide. The amide was treated with 20% piperidine in CHCl₃ (10 mL) for 2 h. Solvent was removed in vacuo. The residue was purified by column chromatography with 5 - 10% MeOH in CH₂Cl₂ to give 713 mg desired amine in 74% yield. MS: m/z 439 (MH⁺).
3. Preparation of compound **4**. Amine **3** (40 mg, 0.09 mmole) was dissolved in MeOH (3 mL), cyclohexylacetaldehyde (23 µL, 0.18 mmole) and sodium cyanoborohydride (182 uL 1.0 M solution in THF, 0.18 mmole) was added. The reaction was stirred overnight. Solvent was removed in vacuo and the residue was purified by flash column chromatography with 3 - 5 % MeOH in CH₂Cl₂ to give 22 mg of desired product in 44% yield. MS: m/z 549 (MH⁺).
4. Preparation of PS461400. To a stirred solution of compound 4 (22 mg, 0.04 mmole) in CH₂Cl₂ (2 mL) was added trifluoroacetic acid (0.5 mL). The mixture was stirred for 1 h and solvent was removed in vacuo. The residue was pumped on a high vacuum line for 2 h and dissolved in CH₂Cl₂ (2 mL). To this solution was added diisopropylethyl amine (35 µL, 0.2 mmole) and the cyclohexyl isocyanate (28.5 uL, 0 2 mmole). The reaction was stirred overnight, dissolved in water (10 mL) and extracted with CH₂Cl₂ (15 mL x 2). The organic layer was dried (Na₂SO₄) and solvent was removed in vacuo. The residue was purified by flash column chromatography with 4% MeOH in CH₂Cl₂ to give 11 mg desired product in 48% yield. MS: m/z 574 (MH⁺).

Using substantially the same reaction scheme of the above example, with appropriate alcohol 1, the following compounds were prepared:

| | | | | |
|---|---|---|---|---|
| PS287238 | PS812520 | PS465781 | PS159773 | PS372802 |
| PS793961 | PS478500 | PS783003 | PS593455 | PS320451 |
| PS477090 | PS516972 | PS410892 | PS241936 | PS409643 |
| PS725556 | PS769295 | PS075114 | PS990951 | PS192638 |
| PS354164 | PS395570 | PS956973 | PS859989 | PS467023 |

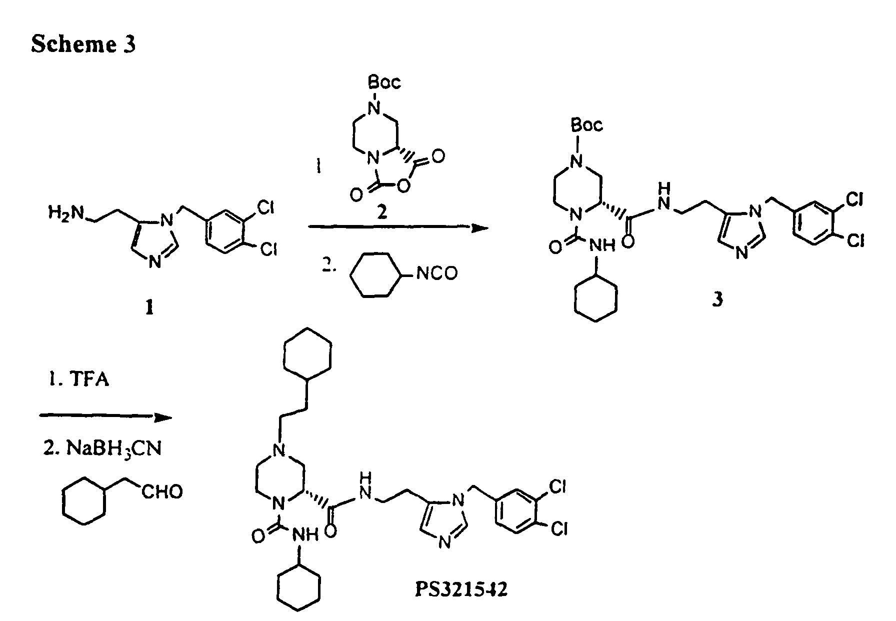

### Preparation procedure for PS 321542 (Scheme 3)

1. Preparation of compound **3** To a stirred solution of the anhydride **1** (100 mg, 0.39 mmole) in CH₂Cl₂ (4 mL) was added a solution of the amine **1** (105 mg, 0.39 mmole) in a mixture of MeOH/CH₂Cl₂(0.5 mL/2 mL). The reaction was stirred for 1 h. The reaction was cooled to 0 °C and cyclohexyl isocyanate (111 µL, 0.78 mmole) was added. The reaction was stirred overnight. The reaction was partitioned between CH₂Cl₂ (40 mL) and sat. NaCl solution (20 mL). The organic layer was dried (Na₂SO₄) and solvent removed in vacuo to give 200 mg desired product in 85% yield. MS: m/z 607 (MH⁺).
2. Preparation of compound PS321542. To a stirred solution of compound 3 (137 mg, 0.226 mmole) in CH₂Cl₂ (3 mL) was added trifluoroacetic acid (1 mL). The mixture was stirred for 1 h and solvent was removed in vacuo. The residue partitioned between 1 N NaOH (20 mL) and CH₂Cl₂ (20 mL). The aqueous layer was extracted with CH₂Cl₂ and EtOAc. The combined organic phase was dried (Na₂SO₄) and solvent was removed in vacuo. The amine was dissolved in MeOH (3 mL). To this solution was added cyclohexyl acetaldehyde (57 mg, 0.45 mmole) and sodium cyanoborohydride (1.0 M solution in THF, 452 µL, 0.45 mmole). The reaction was stirred overnight. Solvent was removed in vacuo and the residue partitioned between 1 N NaOH (20 mL) and CH₂Cl₂ (20 mL). The organic layer was dried (Na₂SO₄) and evaporated. The residue was purified by flash column chromatography with 4% MeOH in CH₂Cl₂ to give 52 mg product in 37% yield. MS: m/z 617 (M⁺).

Using substantially the same reaction scheme of the above example, with racemic anhydride 2 when appropriate, the following compounds were prepared:

| | | | | |
|---|---|---|---|---|
| PS 815156 | PS288326 | PS130057 | PS064691 | PS028348 |
| PS813558 | PS319448 | PS420083 | PS259236 | PS437810 |
| PS381385 | PS201633 | | | |

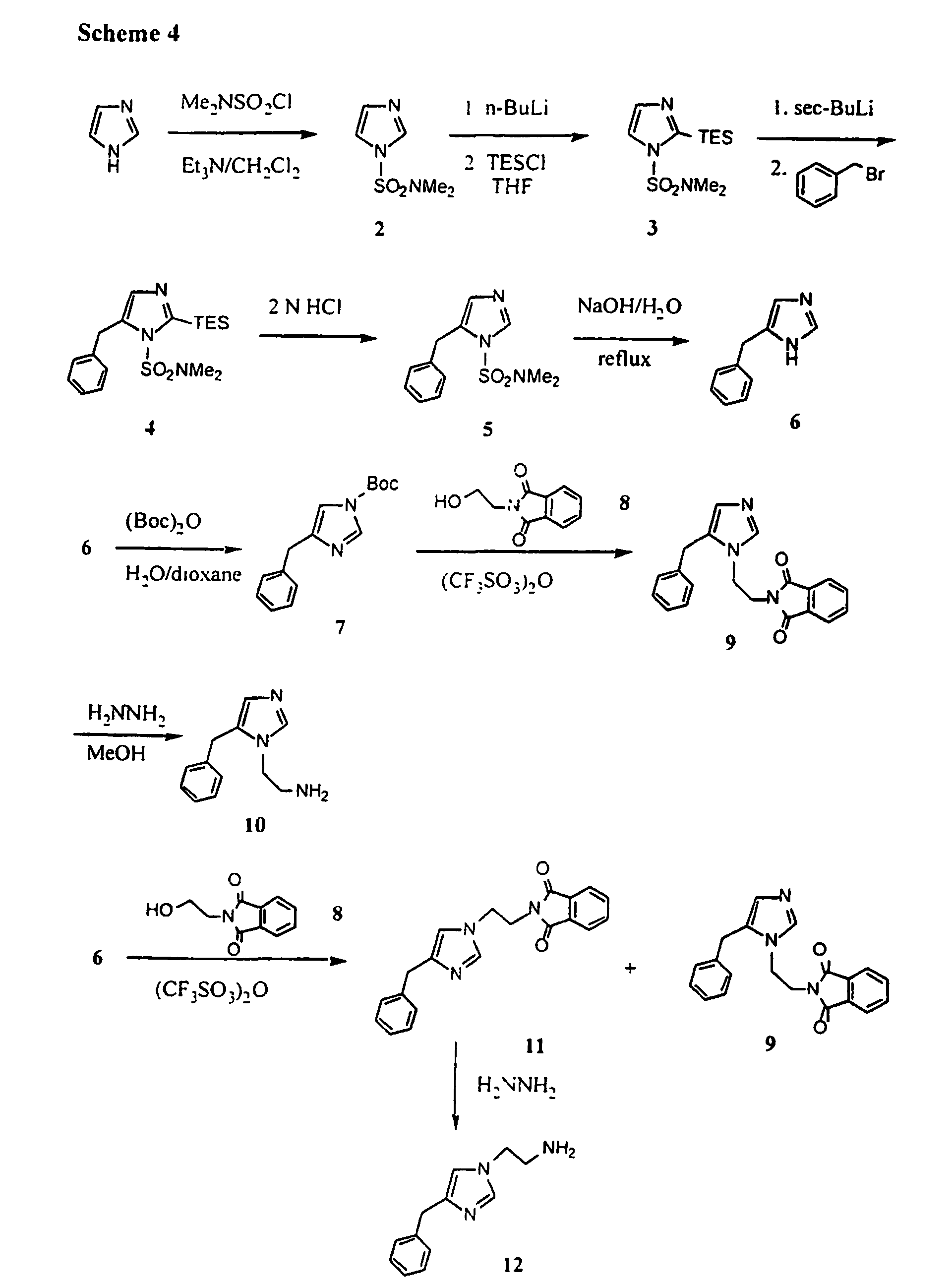

### Preparation procedure for 1-(2-Aminoethyl)-5-benzylimidazole (10) and 1-(2-Aminoethyl)-4-benzylimidazole (12) (Scheme 4)

1. Preparation of compound **2**. To a stirred solution of imidazole (5g, 73.4 mmole) in CH₂Cl₂ (30 mL) at 0 °C was added triethylamine (15.4 mL, 110 mmole) and dimethylsulfamoyl chloride (8 3 mL, 77.1 mmole) dropwise. The reaction was allowed to warm to room temperature slowly and stirred overnight. The reaction was quenched with water (30 mL) and extracted with CH₂Cl₂ (30 mLx2). The organic phase was dried (Na₂SO₄) and solvent removed in vacuo to give compound **2** in 89% yield. MS: m/z 176 (MH⁺).
2. Preparation of compound **5**. To a stirred solution of compound **2** (1.0g, 5.72 mmole) in THF (20 mL) at -78 °C under Ar was added n-BuLi in hexane (2.5 M solution, 2.44 mL, 6.12 mmole). The reaction mixture was stirred at at -78 °C for 30 min., and chlorotnethylsilane (1.92 mL, 11.44 mmole) was added. The reaction was stirred at room temperature for 5 h, then solvent and excess chlorosilane was removed under reduced pressure by gentle heating to give intermediate **3**. THF (20 mL) was added to intermediate **3** and the solution was cooled to -78 °C. Sec-butyllithium in cyclohexane (1.3 M solution, 8.8 mL, 11.44 mmole) was added and the mixture stirred at -78 °C for 30 min., then benzyl bromide (2.04 mL, 17.16 mmole) was added. Stirring was continued at -78 °C for 30 min., and at room temperature overnight. Solvent was removed in vacuo to give intermediate **4**. Intermediate **4** was stirred with 2 N HCl (50 mL) for 3 h and the mixture was washed with ether (20 mLx2). The aqueous phase was basified with NaOH (40% w/w) to pH=11. then extracted with ether (50 mLx3). Combined organic phase was dried (Na₂SO₄) and solvent removed in vacuo. The residue was purified by flash column chromatography with 35% hexane in EtOAc to give 560 mg compound 5 in 37% yield. MS: m/z 266 (MH⁺).
3. Preparation of compound **6**. Compound **5** (560 mg, 2 11 mmole) was refluxed in 4% NaOH (w/w, 100 mL) overnight. Solvent was removed in vacuo. The residue was triturated with THF, filtered and dried (Na₂SO₄).
   Solvent was removed under reduced pressure to give 240 mg 4(5)-benzylimidazole (**6**) in 72% yield. MS: m/z 159 (MH⁺).
4. Preparation of 1-(2-Aminoethyl)-5-benzylimidazole **(10)**. To a stirred solution of compound **6** (230 mg, 1.45 mmole) in H₂O/dioxane (1:1, 20 mL) was added sodium carbonate (31 mg, 0.29 mmole) and di-t-butyldicarbonate (400 mg, 1.74 mmole). The mixture was stirred overnight, then extracted with CH₂Cl₂ (40 mL x 2). Combined organic layer was washed with water (20 mL), dried (Na₂SO₄) and solvent was removed in vacuo to give 305 mg compound **7** in 82% yield. MS: m/z 259 (MH⁺). To a stirred solution of triflic anhydride (219 uL, 1.3 mmole) in CH₂Cl₂ (5 mL) under Ar at -78 °C was added a solution of compound **8** (249 mg, 1.3 mmole) and DIEA in CH₂Cl₂ (5 mL). Stirring at -78°C was continued for 20 min., a solution of compound **7** in CH₂Cl₂ (5 mL) was added dropwise. The reaction was allowed to gradually warm to room temperature overnight. To the reaction was added sat. NaHCO₃ (10 mL). The mixture was extracted with CH₂Cl₂ (20 mL x 2). Organic layer was washed with water (10 mL), dried (Na₂SO₄) and solvent removed in vacuo. The residue was purified by column chromatography with 1-5 % MeOH in CH₂Cl₂ to give 60 mg compound **9.** MS: m/z 332 (MH⁺). Compound **9** was dissolved in MeOH (2 mL) and hydrazine (10 µL) was added. The reaction was stirred overnight and solvent was removed in vacuo. The residue was dissolved in 0.1 N HCl (10 mL) and washed with EtOAc (10 mL). The aqueous phase was basified with NaOH until pH=11, and extracted with EtOAc (20 mLx3). Combined organic layer was dried (Na₂SO₄) and solvent removed in vacuo to give the title compound 10. MS: m/z 202 (MH⁺).
5. Preparation of 1-(2-Aminoethyl)-4-benzylimidazole **(12).** To a stirred solution of triflic anhydride (245 µL, 1.46 mmole) in CH₂Cl₂ (5 mL) under Ar at -78 °C was added a solution of compound **8** (278 mg, 1.46 mmole) and DIEA in CH₂Cl₂ (5 mL) Stirring at -78°C was continued for 20 min., a solution of compound **6** in CH₂Cl₂ (5 mL) was added dropwise. The reaction was allowed to gradually warm to room temperature overnight. To the reaction was added sat. NaHCO₃ (10 mL). The mixture was extracted with CH₂Cl₂ (20 mL x 2). Organic layer was washed with water (10 mL), dried (Na₂SO₄) and solvent removed in vacuo. The residue was purified by column chromatography with 1-5 % MeOH in CH₂Cl₂ to give 112 mg compound **11.** MS: m/z 332 (MH⁺). Compound **11** was dissolved in MeOH (2 mL) and hydrazine (10 µL) was added. The reaction was stirred overnight and solvent was removed in vacuo. The residue was dissolved in 0.1 N HCl (10 mL) and washed with EtOAc (10 mL). The aqueous phase was basified with NaOH until pH=11, and extracted with EtOAc (20 mLx3). Combined organic layer was dried (Na₂SO₄) and solvent removed in vacuo to give the title compound 12. MS: m/z 202 (MH⁺).
Compound **10** was used to prepare PS319448 using procedure described in **scheme 2.** Compound **12** was used to prepare PS204446 using procedure descnbed in **scheme 2.**

Reagents and reaction conditions for protecting and deprotecting compounds is well known, as described, for example, in T.W. Greene and P. Wuts, Protective Groups in Organic Synthesis, 2nd Ed., Wiley Interscience, N.Y. 1991, 473 pages.

Compounds of the present invention and preparative starting materials therof, are exemplified by the following examples, which should not be construed as limiting the scope of the disclosure. Alternative mechanistic pathways and analogous structures within the scope of the invention may be apparent to those skilled in the art, such as by the methods described in WO95/10516.

Compounds of formula (1.0) can be isolated from the reaction mixture using conventional procedures, such as, for example, extraction of the reaction mixture from water with organic solvents, evaporation of the organic solvents, followed by chromatography on silica gel or other suitable chromatographic media Alternatively, compounds (1.0) can be dissolved in a water-miscible solvent, such as methanol, the methanol solution is added to water to precipitate the compound, and the precipitate is isolated by filtration or centrifugation.

Compounds of the present invention and preparative starting materials thereof, are exemplified by the following examples, which should not be construed as limiting the scope of the disclosure.

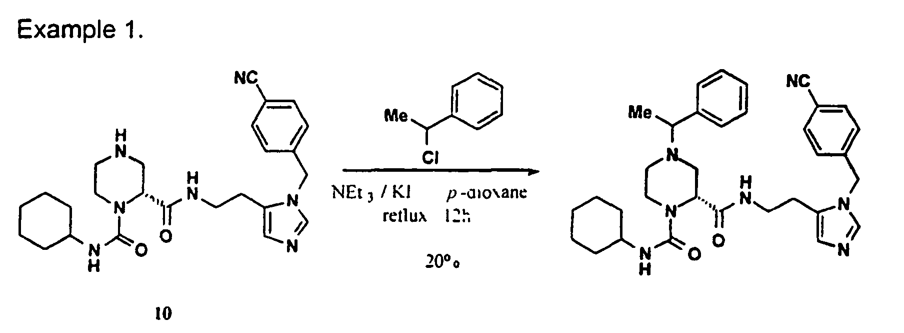

A solution of **10** (50 mg, 0.108 mmol, 1.0 eq.), 1-chloro-1-phenylethane (170 µl, 1.29 mmol, 12.0 eq.), and triethylamine (180 µl, 1.29 mmol, 12.0 eq.) in anhydrous *p*-dioxane (10 ml) is gently refluxed at 105 °C under a nitrogen atmosphere for 12h. The solution is cooled to room temperature and the volatiles are removed under vacuum at 30 °C. The residue is taken up in distilled water (10 ml) and extracted with dichloromethane (5 x 5 ml). The combined organic extracts are washed with brine (5 ml), dried over Na₂SO₄, filtered, and concentrated under vacuum at 30 °C. *N2-[2-[1-[(4-cyanophenyl)methyl]-1H-imidazol-5-yl]ethyl]-N1-cyclohexyl-4-(1-phenylethyl)-1,2(R)-piperazinedicarboxamide* (12 mg, 0.021 mmol, 20%) is obtained as a light-yellow semi-solid after preparative scale thin layer chromatography (CH₃CN : 2 *N* NH₃/MeOH = 90 : 10 v/v as eluent) over silica gel.
HR-MS (FAB):
Calculated for C₃₃H₄₂N₇O₂ ([M+H]⁺): 568.3400. Found: 568.3407.

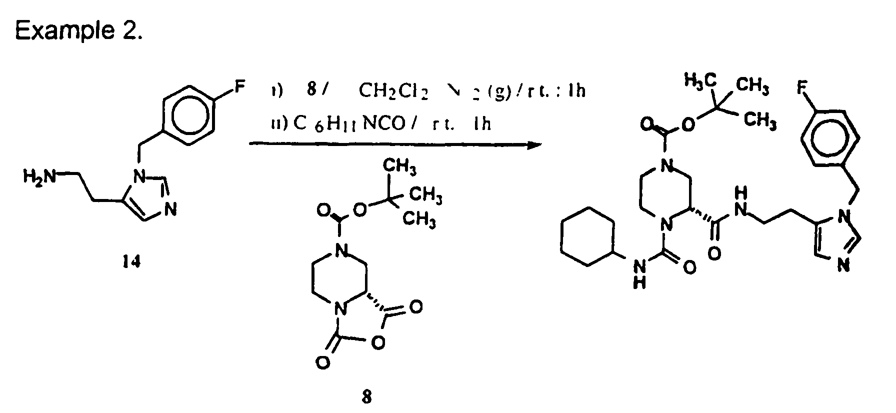

A solution of **14** (500 mg, 2.28 mmol, 1.0 eq.) in anhydrous dichloromethane (5 ml) was added dropwise over a period of 5 minutes to a stirred solution of anhydride 8 (701 mg, 2.74 mmol, 1.2 eq.) in anhydrous dichloromethane (10 ml) at room temperature. A stream of nitrogen was bubbled through the solution to expel evolved carbon dioxide. The colorless solution was stirred for one hour amid nitrogen bubbling. Bubbling was terminated and cyclohexyl isocyanate (594 ml, 4.56 mmol, 2.0 eq.) was added dropwise over a period of 5 minutes. The light-yellow solution was stirred at room temperature for one hour. The volatiles were removed under vacuum at 30 °C. The resulting viscous oil was partitioned between dichloromethane (25 ml) and 1 *N* aqueous NaOH solution (25 ml). The aqueous layer was extracted with dichloromethane (3 x 10 ml). The combined organic extracts were washed with brine (5 ml), dried over Na₂SO₄, filtered, and concentrated under vacuum at 30°C. Preparative scale thin layer chromatography (CH₂Cl₂ . 2 N NH₃/ MeOH = 90 : 10 v/v) over silica gel afforded *1,1-dimethylethyl-1-[(cyclohexylamino)carbonyl]-2(R)-[[[2-[1-(4-fluorophenyl)methyl]-1H-imidazol-5-yl]ethyl]amino]carbonyl]-4-piperazinecarboxylate* (190 mg, 0 34 mmol, 15%) as an off-white foam. Melting Point: 72 °C (decomposition).
MS (FAB+): m/e 557 ([M+H]⁺).
HR-MS (FAB):
Calculated for C₂₉H₄₂FN₆O₄ ([M+H]⁺) 557.3252. Found: 557.3240.

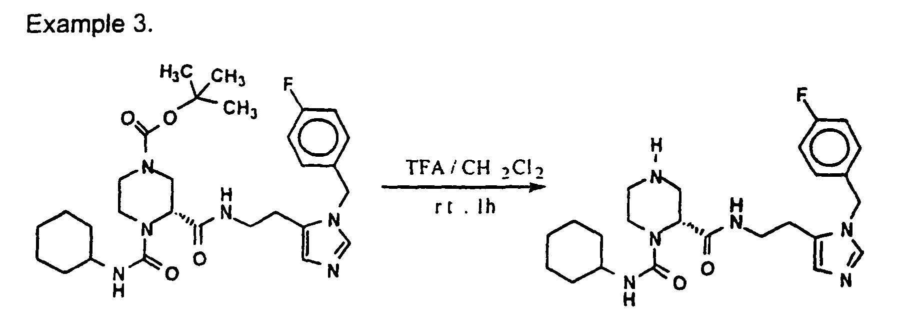

The title compound of Example 2 (190 mg, 0.34 mmol, 1.0 eq.) was dissolved in a mixture of anhydrous dichloromethane (10 ml) and trifluoroacetic acid (10 ml). The resulting yellow solution was stirred at ambient temperature under a nitrogen atmosphere for one hour. The volatiles were evaporated under vacuum at 30 °C and the remaining viscous oil was directly flash chromatographed (CH₂Cl₂ : 2 *N* NH₃/ MeOH = 90 : 10 v/v) over silica gel to give *N2-[2-[1-[(4-fluorophenyl)methyl]-1H-imidazol-5-yl]ethyl]-N1-cyclohexyl-1,2(R)-piperazinecarboxamide* (121 mg, 0.27 mmol, 78%) as an off-white solid.

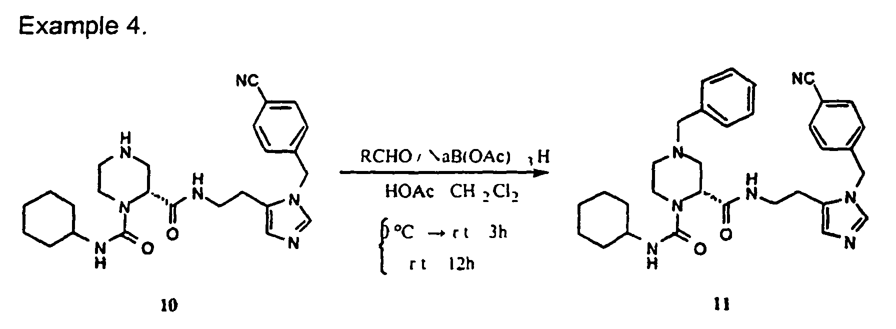

Sodium tnacetoxyborohydride (75 mg, 0.336 mmol, 3.1 eq.) is added portionwise (3 x 25 mg) to a stirred solution of **10** (50 mg, 0.108 mmol, 1.0 eq.) and benzoic aldehyde (R=phenyl) (0.336 mmol, 3.1 eq.) in a mixture of glacial acetic acid (0.5 ml) and anhydrous dichloromethane (10 ml) at 0 °C under a nitrogen atmosphere The mixture is slowly (3h) warmed to room temperature and stirred for another 12h. The volatiles are removed under vacuum at 30 °C. The residue is taken up in 1 *N* aqueous NaOH solution (10 ml) and extracted with dichloromethane (5 x 5 ml). The combined organic extracts are washed with brine (5 ml), dried over Na₂SO₄, filtered, and concentrated under vacuum at 25°C. The product is obtained after preparative scale thin layer chromatography (using either CH₂Cl₂ : 2 N NH₃/ MeOH = 90 : 10 v/v or CH₃CN : 2 N NH₃/ MeOH = 90 : 10 v/v as eluent) over silica gel.

In Examples 5-28 by following essentially the same procedure as described in Example 4, except that the corresponding aldehyde is substituted for benzoic aldehyde, the title compound is obtained. 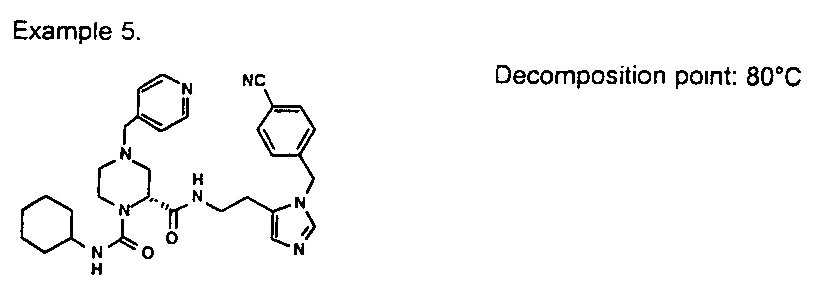 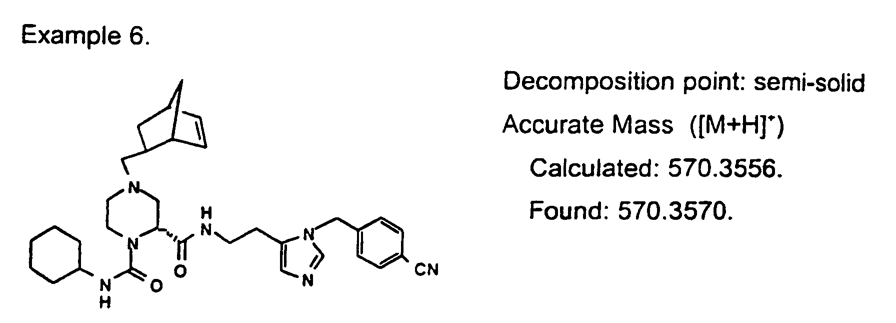 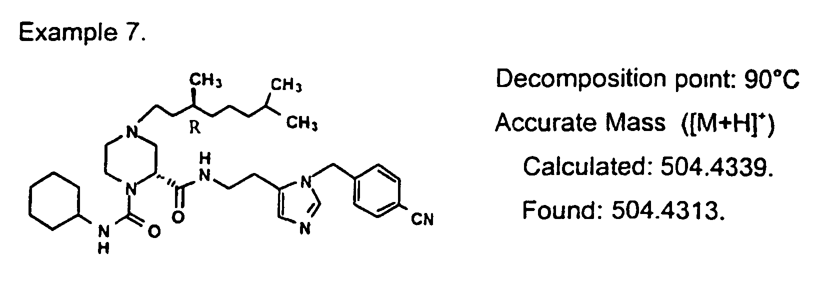 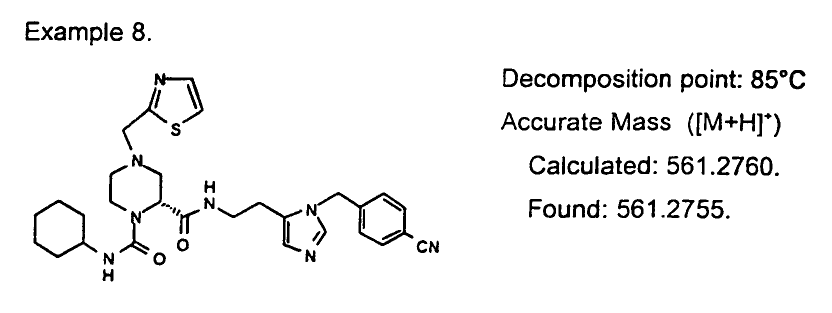 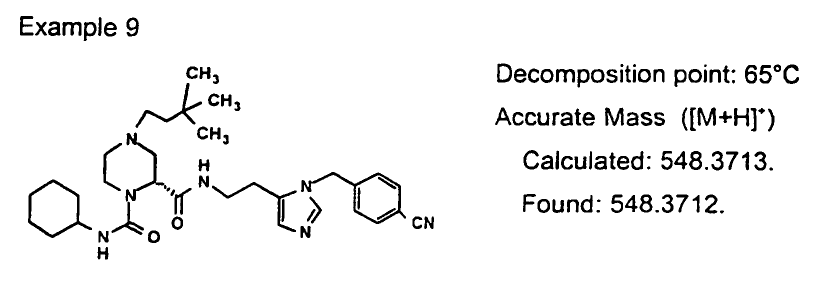 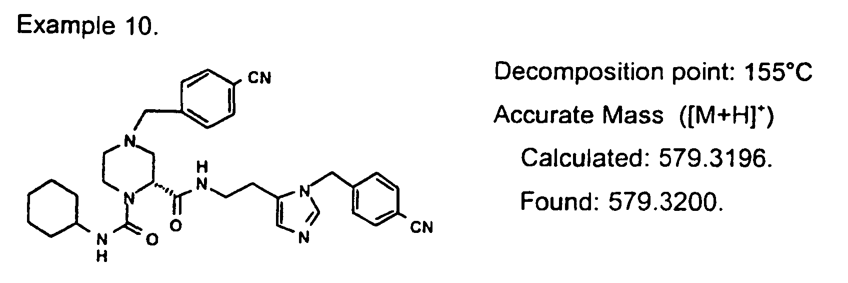 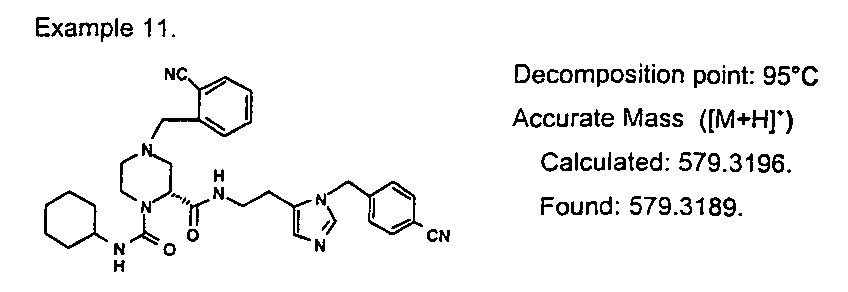 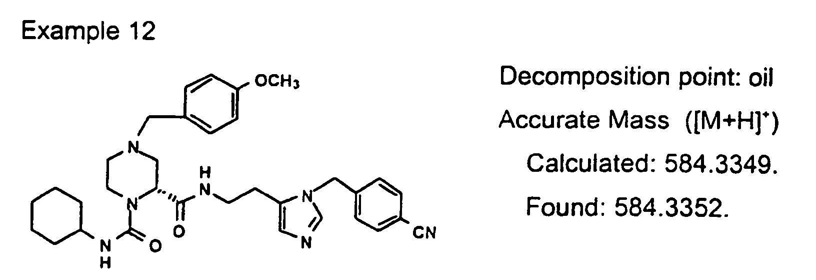 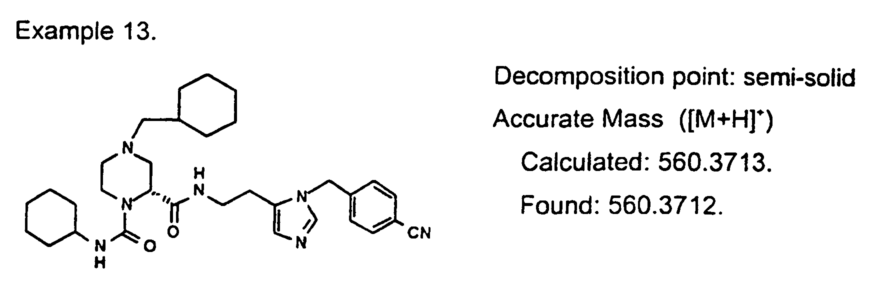 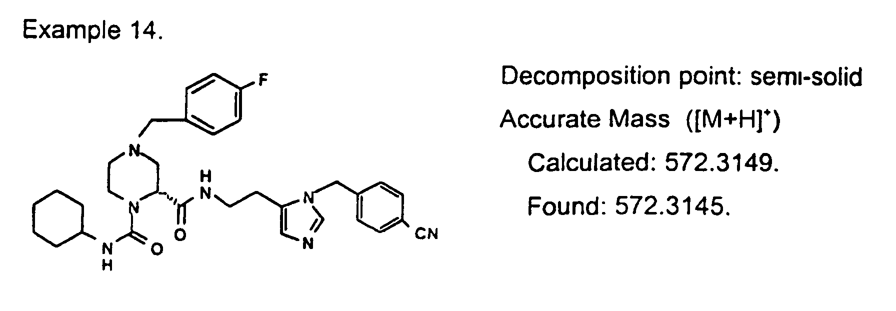 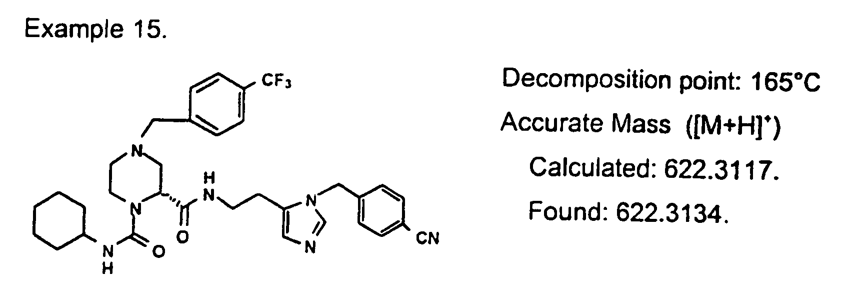 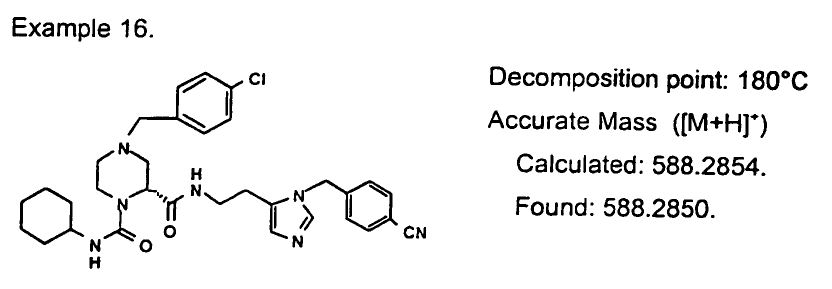 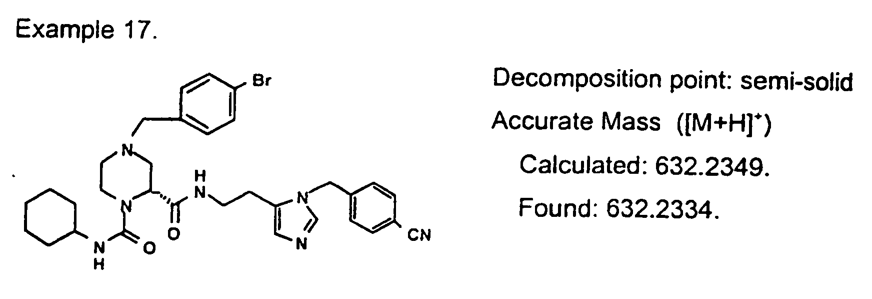 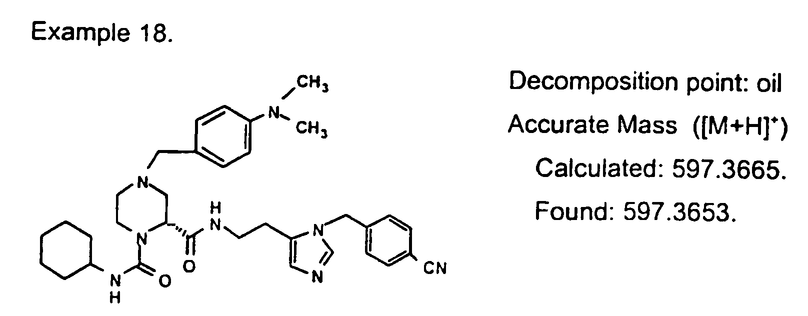 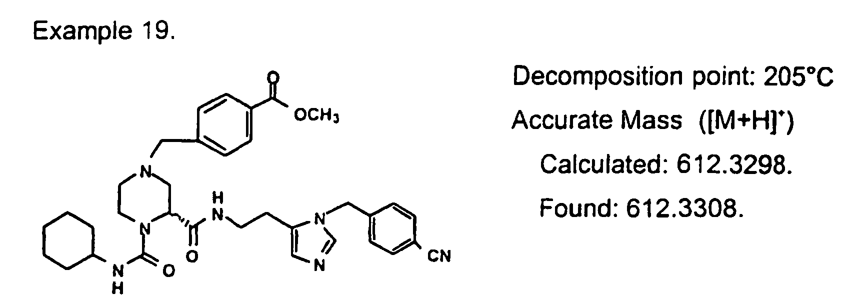 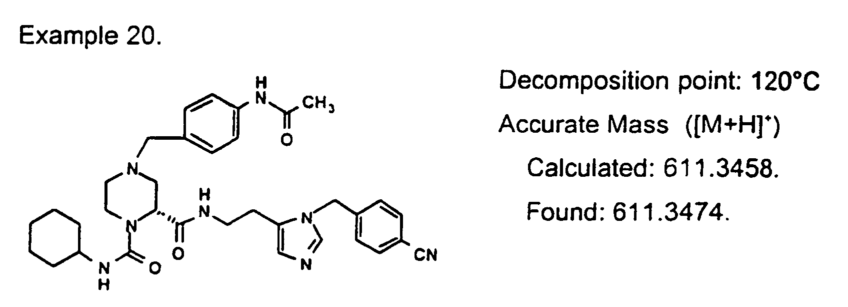 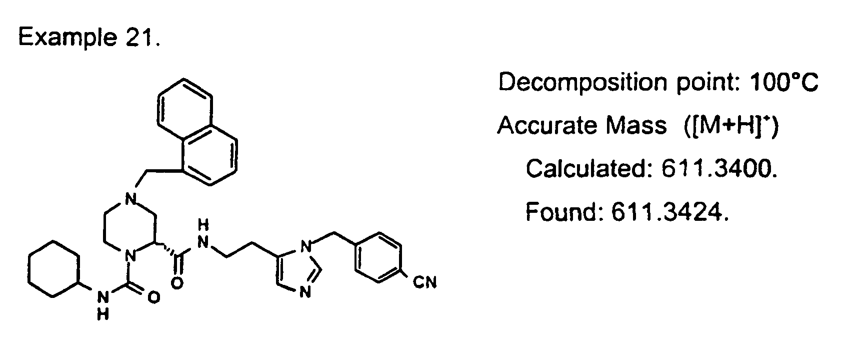 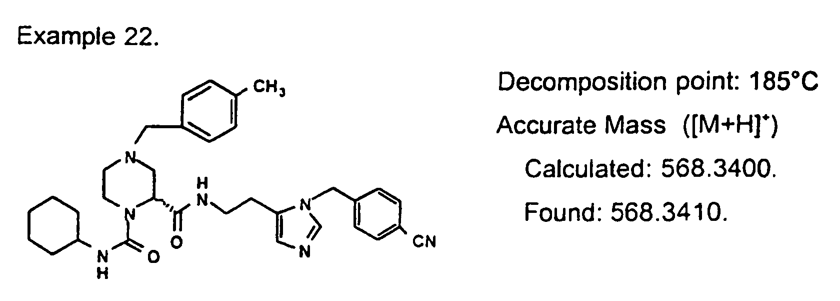 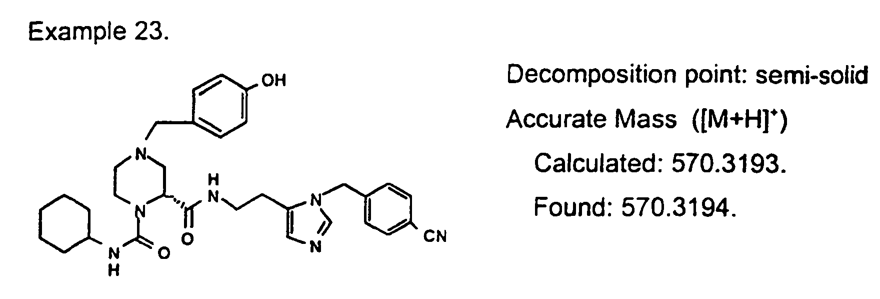 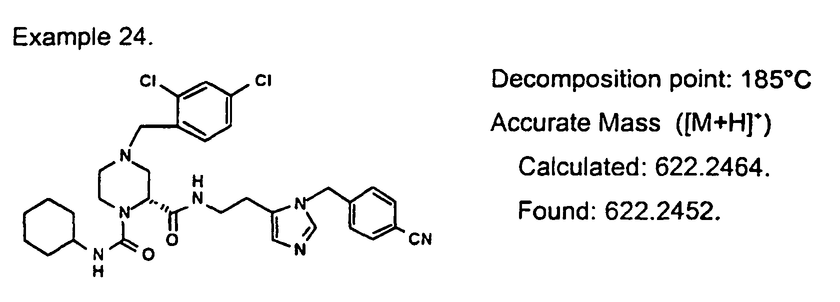 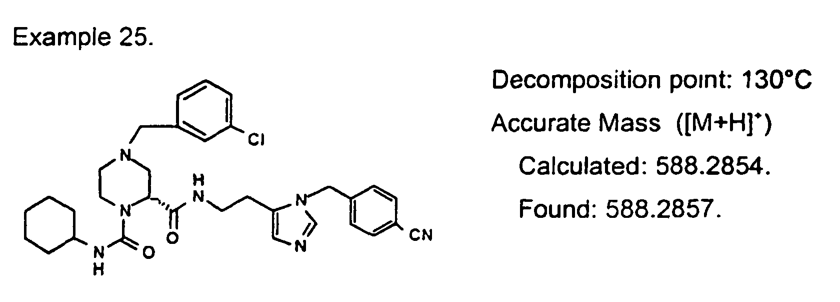 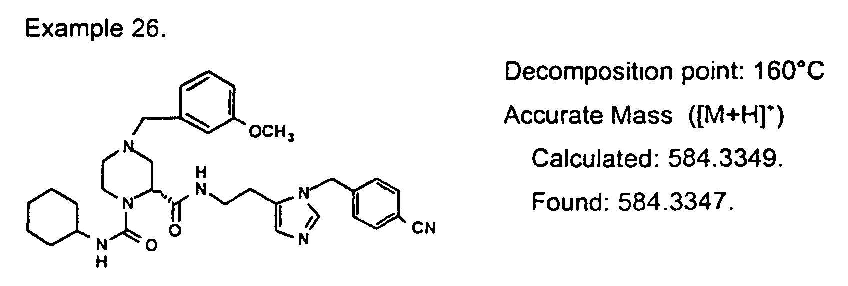 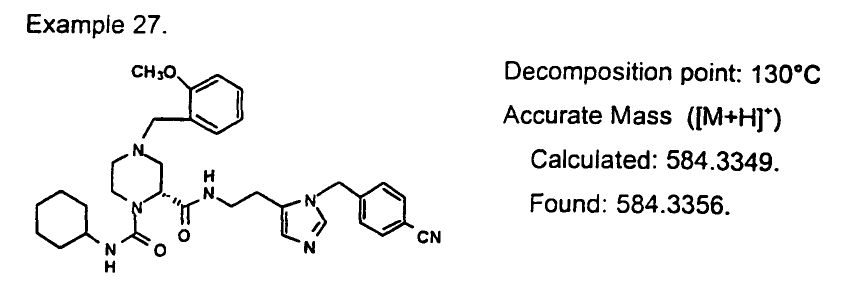 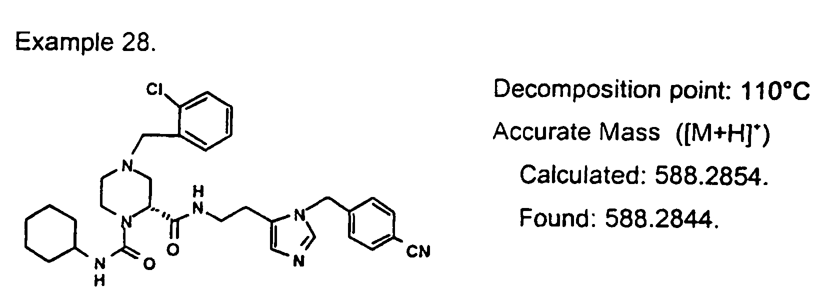 By using the title compounds of Example 3 and by following essentially the same procedure as described in Example 4, the following compounds are prepared.

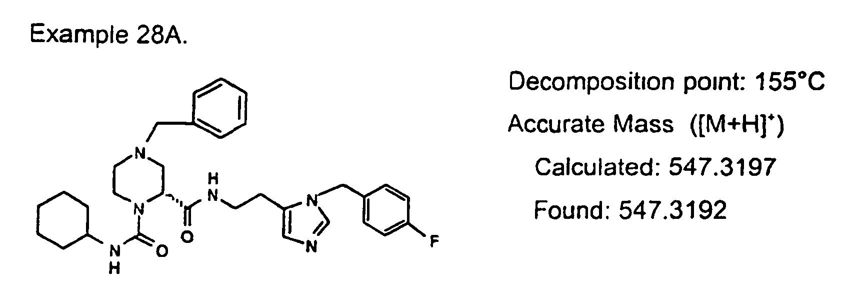

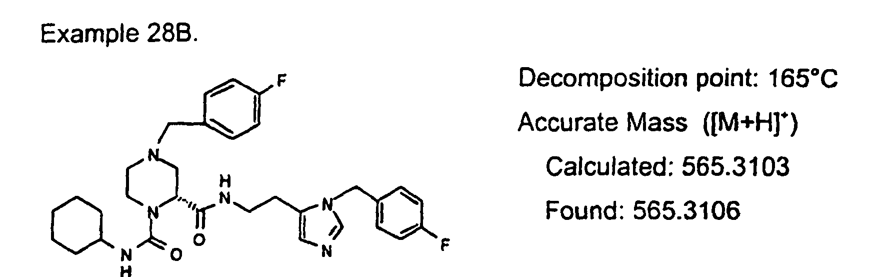

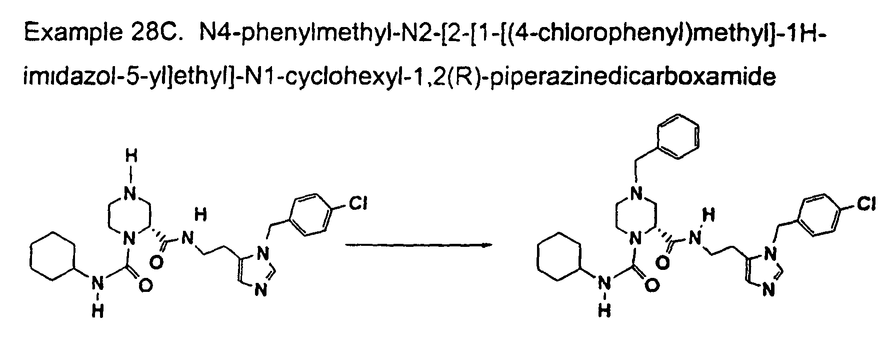

To the title compound from Preparative Example 12 (75 mg, 0.16 mmol) and benzaldehyde (0.05 mL, 0 5 mmol) dissolved in glacial acetic acid (0.5 mL) and anhydrous dichloromethane (10 ml) at 0°C is added sodium triacetoxyborohydride (102 mg, 0.5 mmol) and the resulting mixture is warmed to room temperature and stirred for an additional 12 hours. The reaction mixture is concentrated *in vacuo*, diluted with dichloromethane and washed with 1*N* NaOH (aq). The organic phase is dried over anhydrous MgSO₄, filtered, concentrated *in vacuo,* and purified by preparative plate chromatography (silica gel) using 7% MeOH (saturated with ammonia 2*M*)-CH₃CN as eluent to afford the title compound as a white solid (60 mg, MH⁺ = 563, mp = 116.2°C).

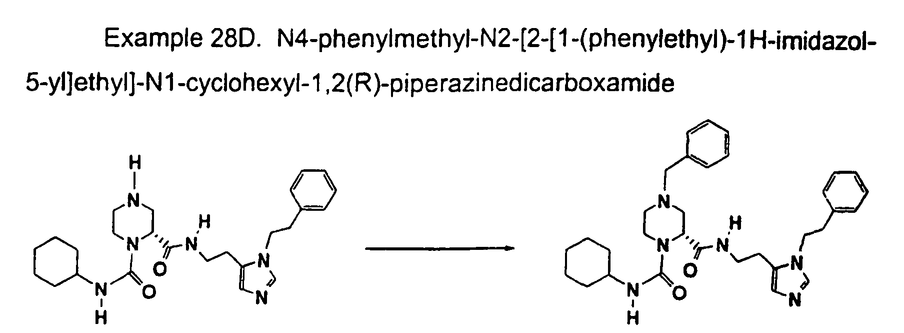

To the title compound from Preparative Example 12A (75 mg, 0.17 mmol) and benzaldehyde (0.05 mL, 0.5 mmol) dissolved in glacial acetic acid (0.5 mL) and anhydrous dichloromethane (10 ml) at 0°C is added sodium triacetoxyborohydride (108 mg, 0.5 mmol) and the resulting mixture is warmed to room temperature and stirred for an additional 12 h. The reaction mixture is concentrated *in vacuo*, diluted with dichloromethane and washed with 1 N NaOH (aq). The organic phase is dried over anhydrous MgSO₄, filtered, concentrated *in vacuo*, and purified by preparative plate chromatography (silica gel) using 7% MeOH (saturated with ammonia 2*M*)-CH₃CN as eluent to afford the title compound as a white solid (61 mg, MH⁺ = 543, mp = 64.4°C).

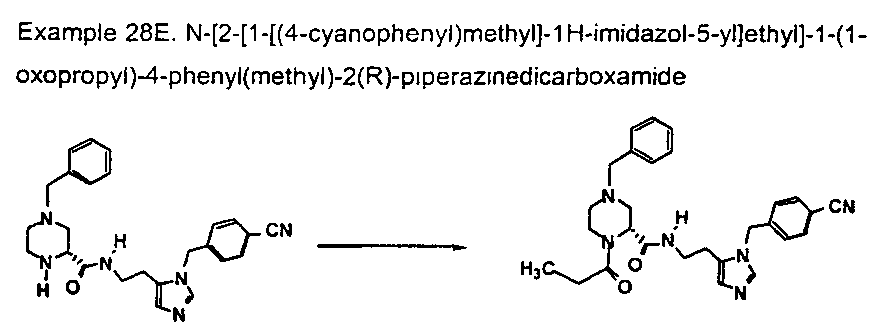

To a solution of the title compound from Preparative Example 11 (50 mg, 0.12 mmol, 1.0 eq) and triethylamine (33 mL, 0.24 mmol) in anhydrous dichloromethane (2 ml) is added propionyl chloride (15.2 mL, 0.18 mmol). The resulting mixture is stirred at room temperature under N₂ for 48 hrs and then quenched with saturated NaHCO₃(aq) solution The mixture is extracted with dichloromethane and dried over anhydrous Na₂SO₄. The organic phase is filtered, concentrated in vacuo, and the residue purified by preparative plate chromatography (silica gel) using 6% methanol - dichloromethane saturated with ammonium hydroxide to afford the title compound as an off-white solid (38.1 mg, mp = 88.2-168.6°C, MH+ = 485).

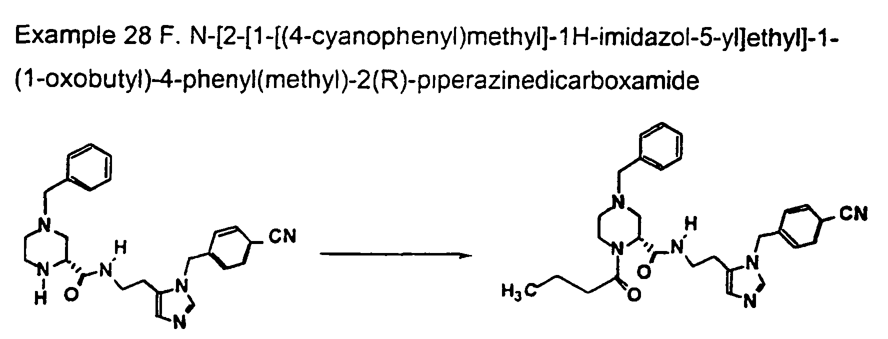

In a similar manner as is described in Example 28E, but using butyryl chloride instead of propionyl chloride, the title compound is prepared as an off-white solid (34.3 mg, mp = 82.32-142.5°C, MH+ = 499).

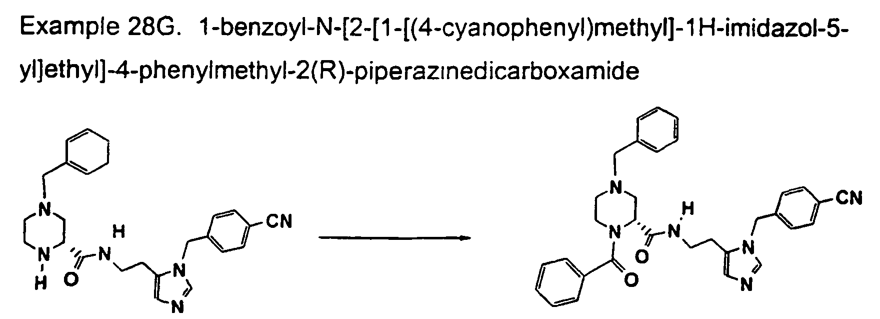

In a similar manner as is described in Example 28E, but using benzoyl chloride instead of propionyl chloride, the title compound is prepared as an off-white solid (33.2 mg, mp = 115.3-170.3°C, MH+ = 533).

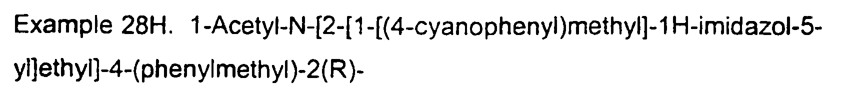

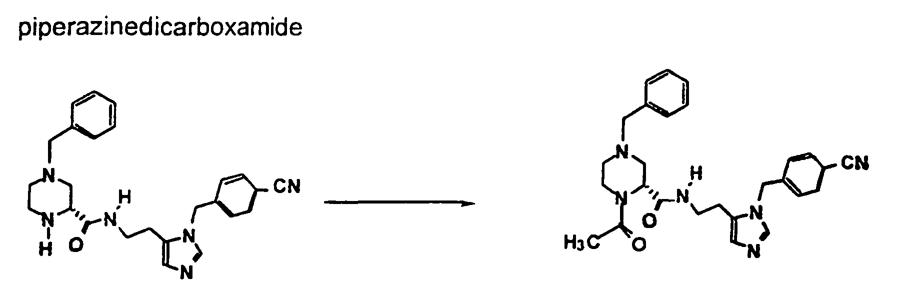

In a similar manner as is described in Example 28E, but using acetyl chloride instead of propionyl chloride, the title compound is prepared as an off-white solid (44.0 mg, mp = 93.0-174.9°C, MH+ = 471).

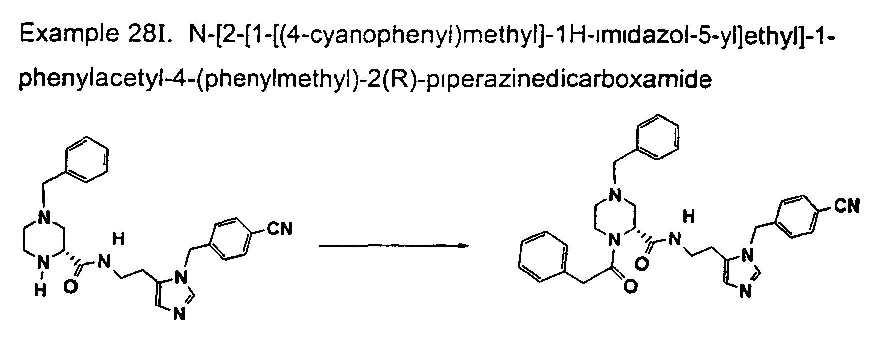

In a similar manner as is described in Example 28, but using phenylacetyl chloride instead of propionyl chloride, the title compound was prepared as an off-white solid (57.7 mg, 45%, mp = 85.6-96.7°C, MH+ = 547).

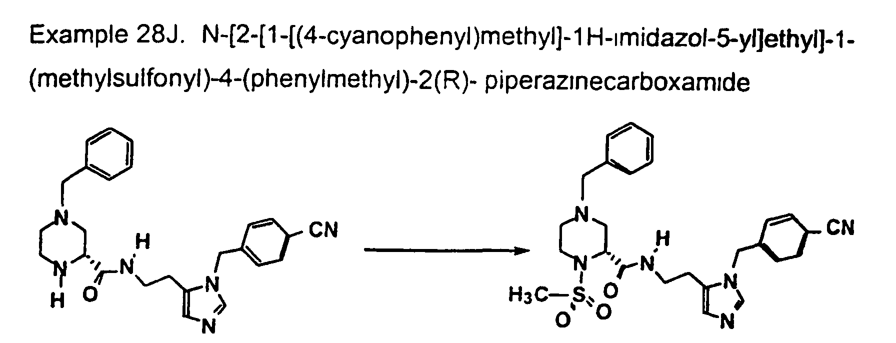

In a similar manner as described in Example 28E, but using methanesulfonyl chloride instead of propionyl chloride, the title compound is prepared as an off-white solid (52.4 mg, mp = 96.5-161.7°C, MH+ = 507).

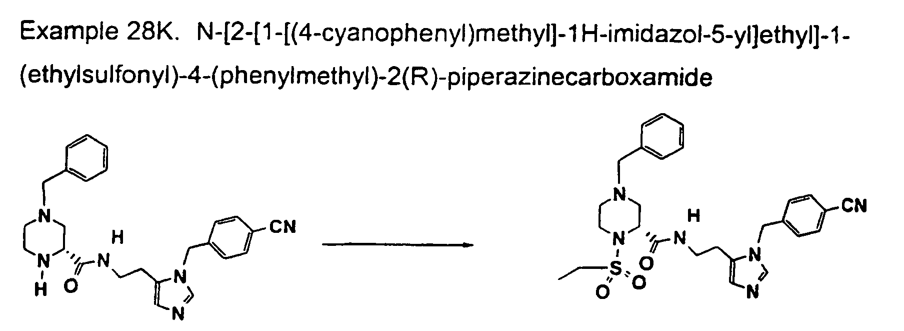

In a similar manner as is described in Example 28E, but using ethanesulfonyl chloride instead of propionyl chloride, the title compound is prepared as an off-white solid (40.4 mg, mp = 97.3-150.2°C, MH+ = 521).

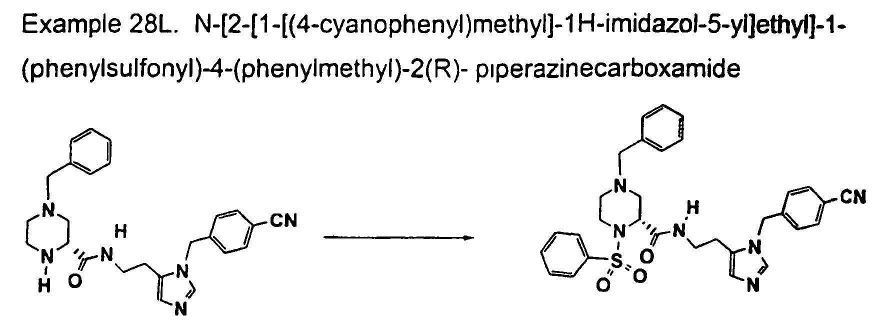

In a similar manner as is described in Example 28E, but using benzenesulfonyl chloride instead of propionyl chloride, the title compound is prepared as an off-white solid (50 mg, 75%, mp = 105.7-166.5°C, MH+ = 569).

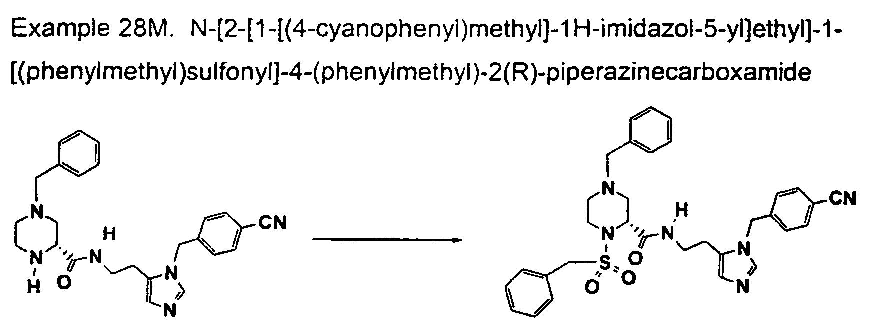

In a similar manner as is described in Example 28E, but using benzylsulfonyl chloride instead of propionyl chloride, the title compound is prepared as an off-white solid (26.8 mg, mp = 118.1-180.5°C, MH+ = 583).

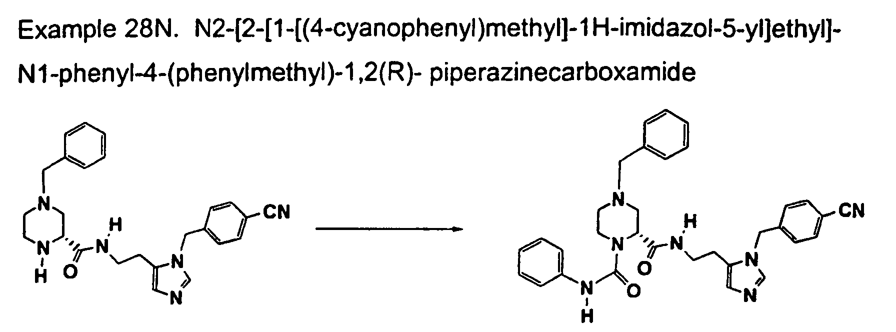

In a similar manner as is described in Preparative Example 12, but using the title compound from Preparative Example 11 and phenylisocyanate instead of cyclohexylisocyanate, the title compound is prepared as an off-white solid (28.8 mg, mp = 128.0-139.2°C, MH+ = 548).

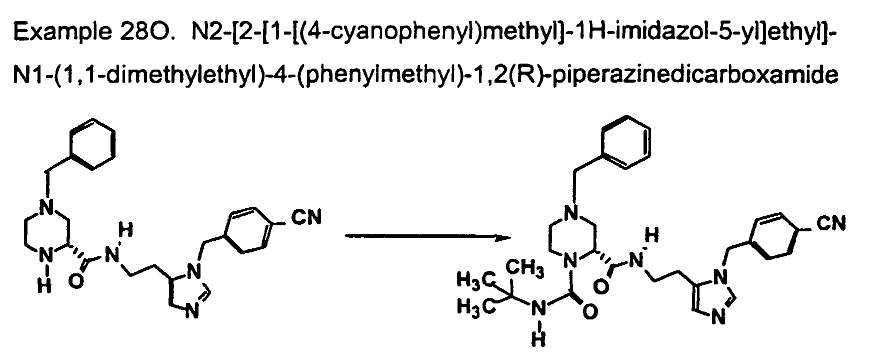

In a similar manner as is described in Preparative Example 12, but using the title compound from Preparative 11 and t-butylisocyanate instead of cyclohexylisocyanate, the title compound is prepared as an off-white solid (66.8 mg, mp = 97.8°C, MH+ = 528).

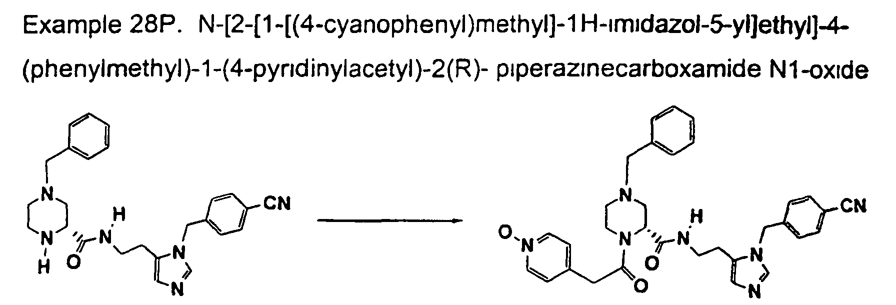

To the title compound from Preparative Example 11 (100 mg, 0.23 mmol) are added HOBt (47 mg, 0.35 mmol), DEC (67 mg, 0.35 mmol), pyridylacetic acid N-oxide (53 mg, 0.35 mmol), NMM (39 mL, 0.35 mmol) and anhydrous DMF (10 mL). The mixture is stirred at room temperature under N₂ overnight. The mixture is concentrated *in vacuo*, diluted with CH₂Cl₂ and washed with a saturated aqueous solution of NaHCO₃. The organic phase is dried over anhydrous MgSO₄, filtered and concentrated *in vacuo*. The residue is purified by preparative plate chromatography (silica gel) using 6% MeOH-98% CH₂Cl₂ saturated with aqueous ammonium hydroxide to give the title compound as an off-white solid (54 mg, MH⁺ = 564).

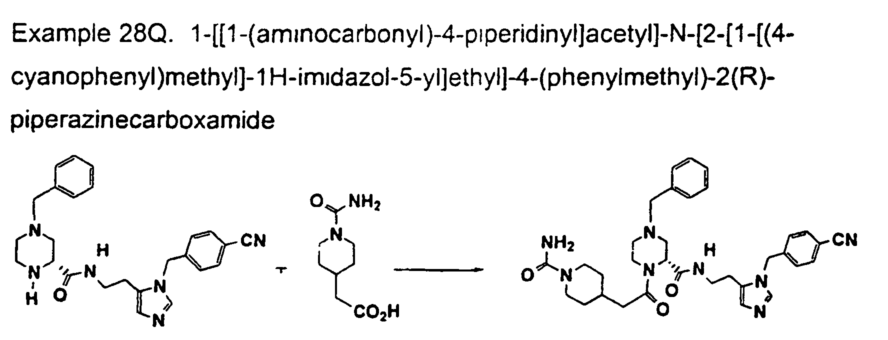

In a similar manner as is described in Example 28P, but using the title compound from Preparative Example 11 and the piperidylacetic acid from Preparative Example instead of pyndylacetic acid N-oxide, the title compound is prepared as an off-white solid (76 mg, 55%, mp = 118.5°C, MH+ = 597).

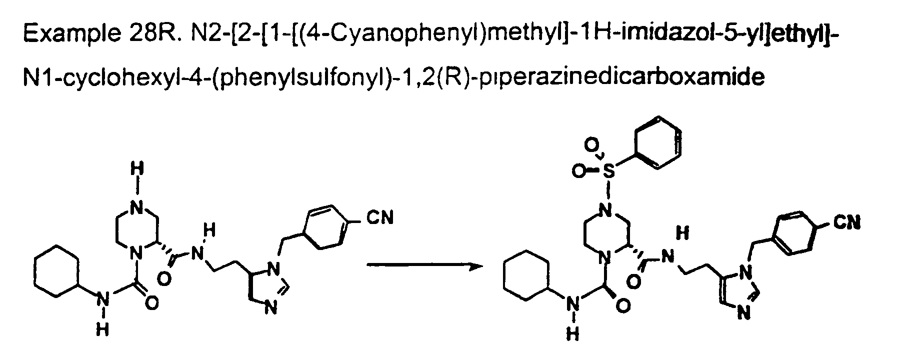

To a solution of the title compound from Prepartive Example 8 and 9 (50 mg, 0.12 mmol, 1.0 eq) and triethylamine (0.05 mL, 0.36 mmol) in anhydrous dichloromethane (3 ml) is added benzenesulfonyl chloride (0.02 mL, 0.12 mmol). The resulting mixture is stirred at room temperature under N₂ for 72 hrs, then diluted with additional dichloromethane, washed with aqueous sodium hydroxide (1M) and dried over anhydrous MgS04. The organic phase is filtered, concentrated in vacuo, and the residue purified by preparative plate chromatography (silica gel) using 10% methanol (saturated with ammonia)-90% acetonitrile, and repurified using 5% methanol-dichloromethane saturated with aqueous ammonium hydroxide to afford the title compound as an orange solid (5 mg, mp = 143.6°C, MH+ = 604).

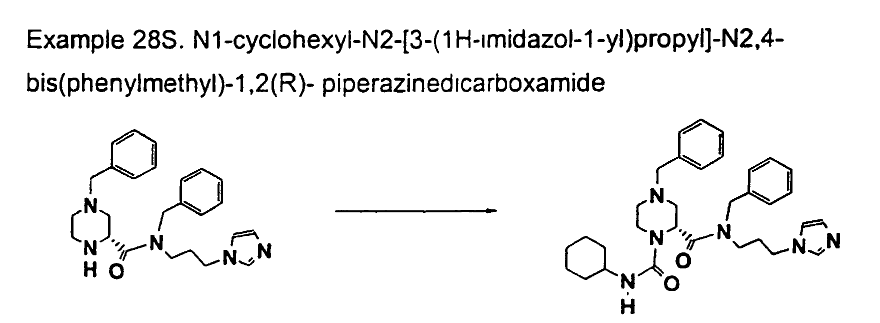

To a solution of the title compound from Example 28Y (0.13 g, 0.3 mmol) dissolved in anhydrous dichloromethane (3 ml) is added cyclohexylisocyanate (0.054 mL, 0.43 mmol) and the resulting solution is stirred at room temperature overnight, then concentrated *in vacuo*. The residue is purified by preparative plate chromatography (silica gel) using 5% MeOH-CH₂Cl₂ saturated with aqueous ammonium hydroxide to give the title compound as a white solid (164 mg, mp = 89.1°C, MH⁺ = 543).

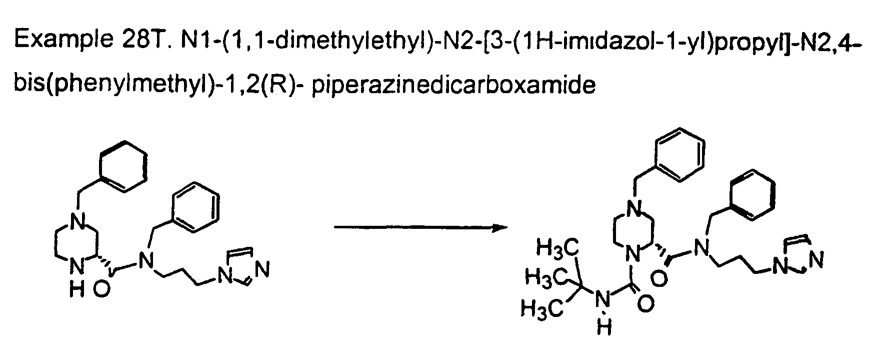

To a solution of the title compound from Example 28Y (0.13 g, 0.3 mmol) dissolved in anhydrous dichloromethane (3 ml) is added t-butylisocyanate (0.05 mL, 0.43 mmol) and the resulting solution is stirred at room temperature overnight, then concentrated *in vacuo*. The residue is purified by preparative plate chromatography (silica gel) using 5% MeOH-CH₂Cl₂ saturated with aqueous ammonium hydroxide to give the title compound as a white solid (131 mg, mp = 57.8°C, MH⁺ = 517).

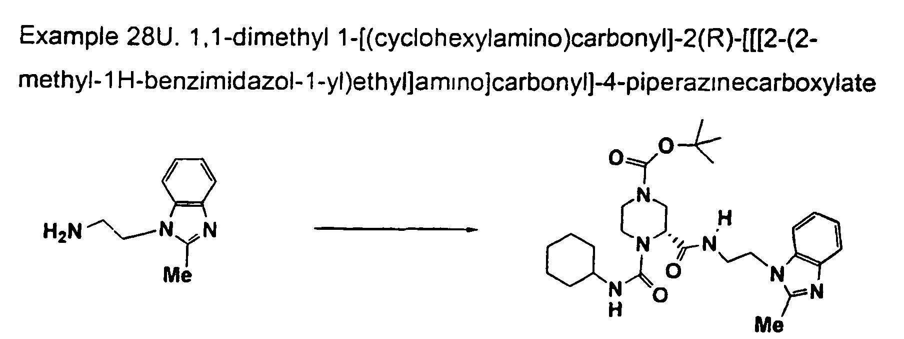

A solution of the title compound from Preparative Example 14 (0.9 g, 5.14 mmol) and the anhydride from Preparative Example (1.38 g, 1.05 eq) dissolved in anhydrous dichloromethane (10 ml) are stirred at room temperature overnight. Additional anhydride (0.105 g) is added and after 1 hr cyclohexylisocyanate (0.98 mL, 7.71 mmol) is added to the reaction mixture which is stirred for an additional 1 5 hrs. Concentration *in vacuo* and purification by flash column chromatography (silica gel) using 1-3% MeOH-CH₂Cl₂ saturated with ammonium hydroxide as eluent affords the title compound as a white solid (1.82 g, mp = 126.9-128.9 °C, MH⁺ = 513).

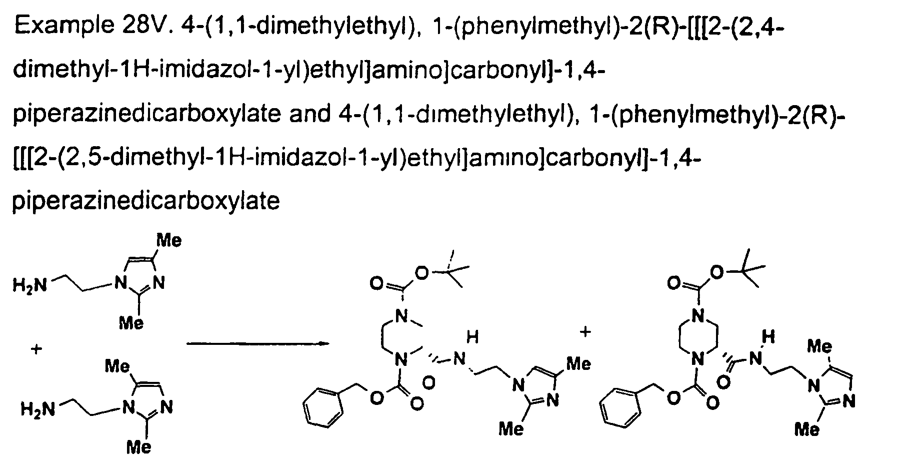

A solution of the title compound from Preparative Example 15 (2.12 g, 15.2 mmol), triethylamine (30.4 mmol) and the anhydride from Preparative Example (3.89 g, 15.2 mmol) dissolved in anhydrous dichloromethane (30 ml) is stirred at room for 30 min. Benzyloxycarbonylsuccinamide (4.17 g, 16.7 mmol) is added and the resulting mixture is stirred at room temperature overnight. Concentration *in vacuo* and purification by flash column chromatography (silica gel) using 2% MeOH-CH₂Cl₂ saturated with ammonium hydroxide as eluent affords the title compound as a (2.57 g). The regioisomers are separated by HPLC (Chiracel AD column) using 5% isopropanol-95% hexane-0.2% diethylamine to give the 2,4-dimethyl isomer (mp = 64.2 °C, MH⁺ = 486) and the 2,5-dimethyl isomer (mp = 71.5 °C, MH⁺ = 486).

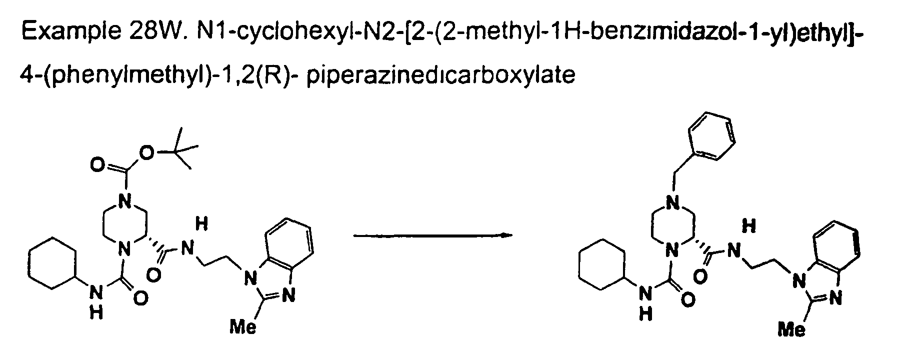

A solution of the title compound from Example 28U (101) (0.49 g, 0.89 mmol) dissolved in anhydrous dichloromethane (10 ml) and trifluoroacetic acid (2 ml) are stirred at room temperature for 3 hrs. The resulting solution is concentrated *in vacuo*, then the residue is combined with anhydrous dichloromethane (10 ml), benzaldehyde (0.28 mL, 2.68 mmol), glacial acetic acid (1 mL) and sodium triacetoxyborohydride (0.76 g, 3.6 mmol) and stirred at room temperature for 48 h. The reaction mixture is concentrated *in vacuo*, diluted with dichloromethane and washed with 1*N* NaOH (aq). The organic phase is dried over anhydrous MgSO₄, filtered, concentrated *in vacuo*, and purified by flash column chromatography (silica gel) using 1-3% MeOH-CH₂Cl₂ saturated with ammonium hydroxide as eluent to afford the title compound as a white solid (0.40 g, mp = 192.9-194.9 °C, MH⁺ = 503).

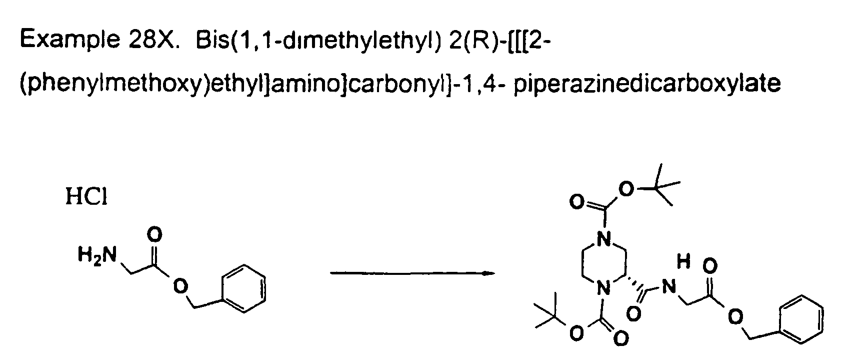

A solution of benzyl 2-aminoacetate hydrochloride (1.3 g, 7.8 mmol), triethylamine (3.26 mL, 23.4 mmol) and the anhydride from Preparative Example (2 g, 7.8 mmol) dissolved in anhydrous dichloromethane (40 ml) are stirred at room temperature overnight. The reaction mixture is diluted with dichloromethane and washed with 1*N* NaOH (aq) and brine. The organic phase is dried over anhydrous MgSO₄, filtered and concentrated *in vacuo* to afford the title compound as a white solid (1.03 g, MH⁺ = 478 (35%), 322(100%)).

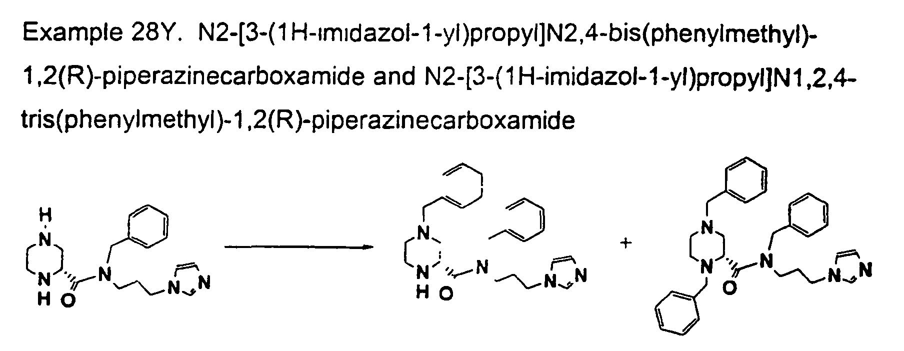

To the title compound from Preparative Example 17 (381 mg, 1.16 mmol) and benzaldehyde (0.12 mL, 1.16 mmol) dissolved in glacial acetic acid (1 mL) and anhydrous dichloromethane (15 ml) at 0°C are added sodium triacetoxyborohydride (740 mg, 3.5 mmol) and the resulting mixture is warmed to room temperature and stirred for an additional 12 h. The reaction mixture is washed with 1*N* NaOH (aq) and the organic phase is dried over anhydrous MgSO₄, filtered and concentrated *in vacuo*. The residue is purified by preparative plate chromatography (silica gel) using 2-5% MeOH-CH₂Cl₂ saturated with aqueous ammonium hydroxide to give the title dibenzyl compound (256 mg, MH⁺ = 418) and the title tribenzyl compound (78.4 mg, MH⁺ = 508).

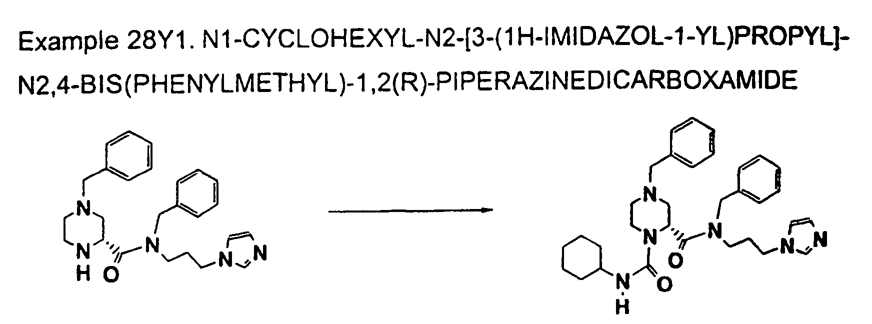

To a solution of the title compound from Example 28Y (0.13 g, 0.3 mmol) dissolved in anhydrous dichloromethane (3 ml) was added cyclohexylisocyanate (0.054 mL, 0.43 mmol) and the resulting solution was stirred at room temperature overnight, then concentrated in vacuo. The residue was purified by preparative plate chromatography (silica gel) using 5% MeOH-CH₂Cl₂ saturated with aqueous ammonium hydroxide to give the title compound as a white solid (164 mg, 99%, mp = 89.1 °C, MH⁺ = 543). 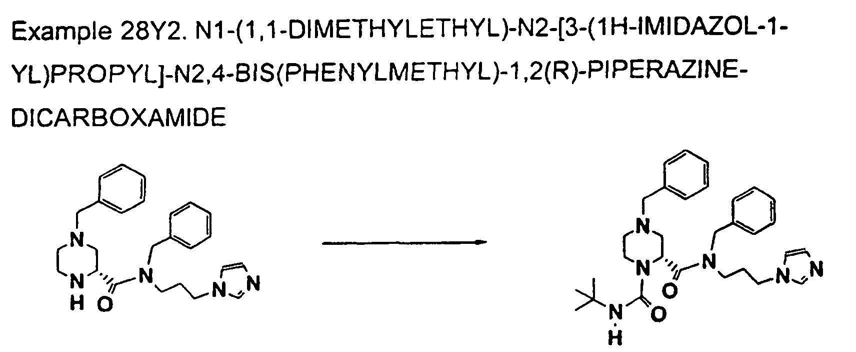 To a solution of the title compound from Example 28Y (0.13 g, 0.3 mmol) dissolved in anhydrous dichloromethane (3 ml) was added t-butylisocyanate (0.05 mL, 0 43 mmol) and the resulting solution was stirred at room temperature overnight, then concentrated in vacuo. The residue was purified by preparative plate chromatography (silica gel) using 5% MeOH-CH₂Cl₂ saturated with aqueous ammonium hydroxide to give the title compound as a white solid (131 mg, 83%, mp = 57.8°C, MH⁺ = 517). 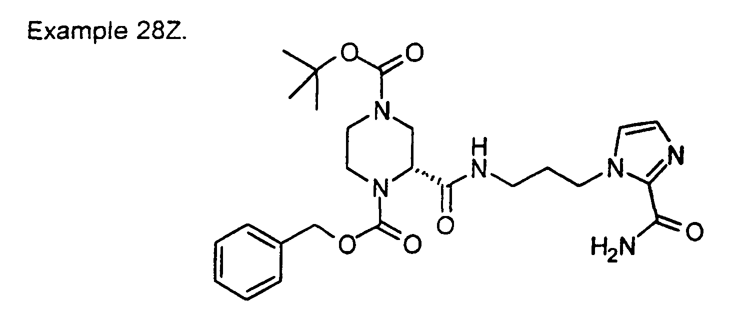 A solution of the title compound from Step B of Preparative Example 23 (0.23 g, 0.86 mmol) is stirred in 4M HCl in dioxane (4 mL) at room temperature overnight. The solution is concentrated and stirred with CH₂Cl₂ (10 mL), DMF (10 mL) and TEA (0.47 mL, 5 eq.) before adding piperazine anhydride (0.26g, 1.2 eq.) portionwise. The resulting solution is stirred at room temperature 1.5 hours and additional anhydride added (0.043g, 0.2 eq.). The reaction mixture is stirred 0.5 hours before adding CBZ-OSuc (0.28g, 1.2 eq.). The resulting solution is stirred 3 hours and quenched by the addition of NaHCO₃ (10 mL), diluted with H₂O (20 mL) and extracted with CH₂Cl₂ (3 X 50 mL). The combined organics are dried over Na₂SO₄, filtered, and concentrated *in vacuo*. The crude product is purified by flash chromatography using a 5% MeOH in CH₂Cl₂ solution as eluent to give a white solid (0.32 g): mp= 71-75 °C; LCMS: MH⁺= 515.

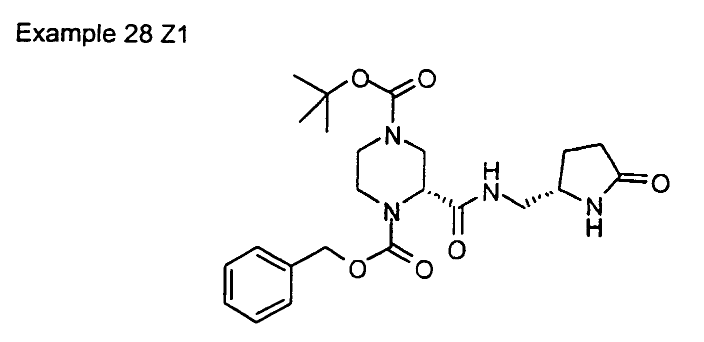

By essentially the same procedure as set forth in Example 28 Z, except using the title compound from Step B of Preparative Example 24, the title compound is prepared (1 66 g): mp= 77-79 °C; LCMS: MH⁺= 461. 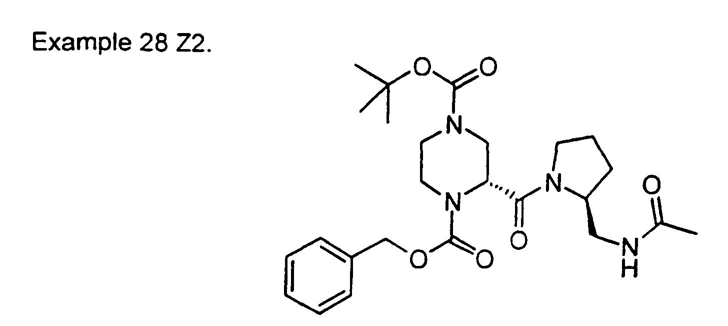 By essentially the same procedure set forth in Example 28 Z except using the title compound from Step C of Preparative Example 25 (0.17g, 0.950 mmol), the title compound is prepared (0.37 g): mp= 58-60 °C; LCMS: MH⁺= 489.

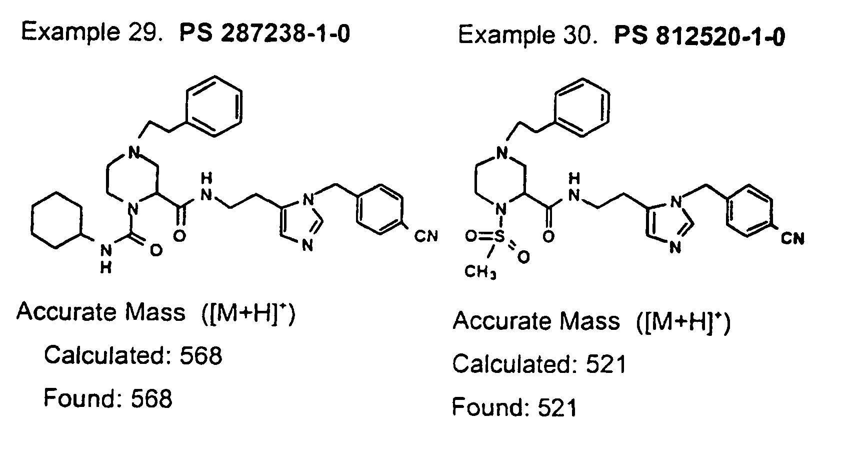

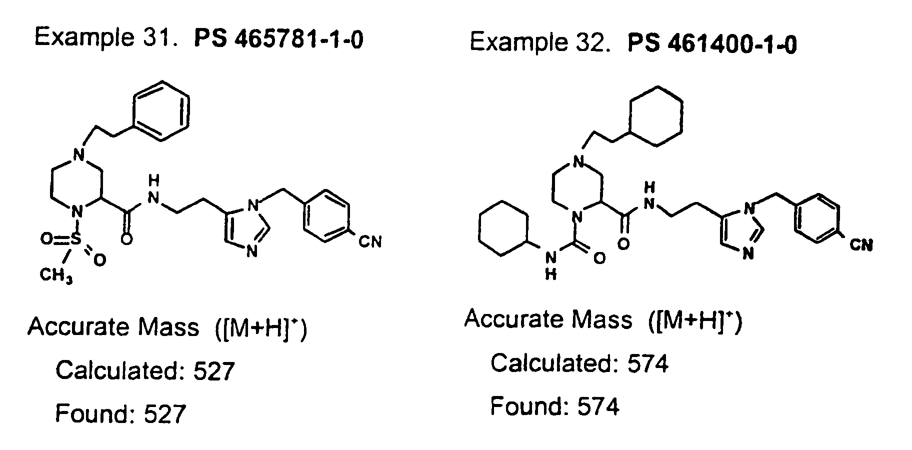

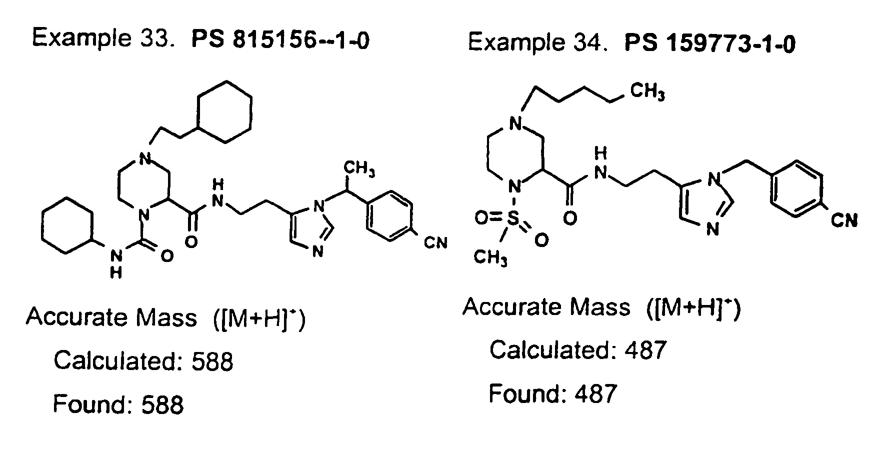

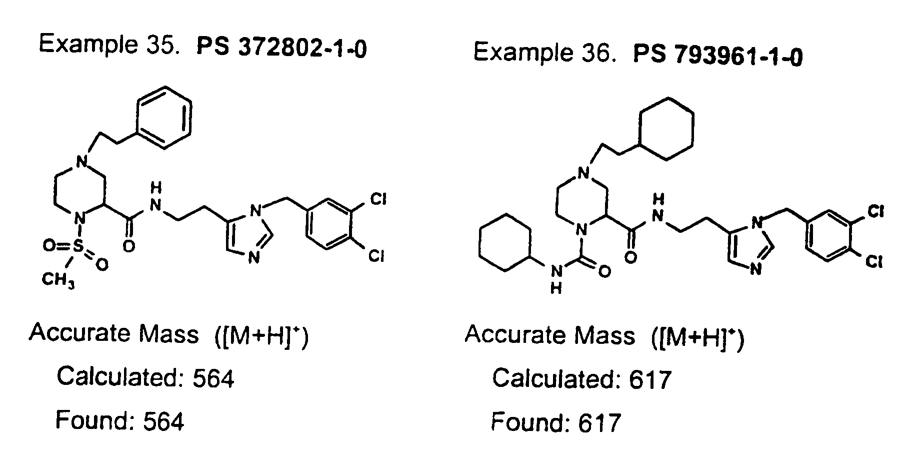

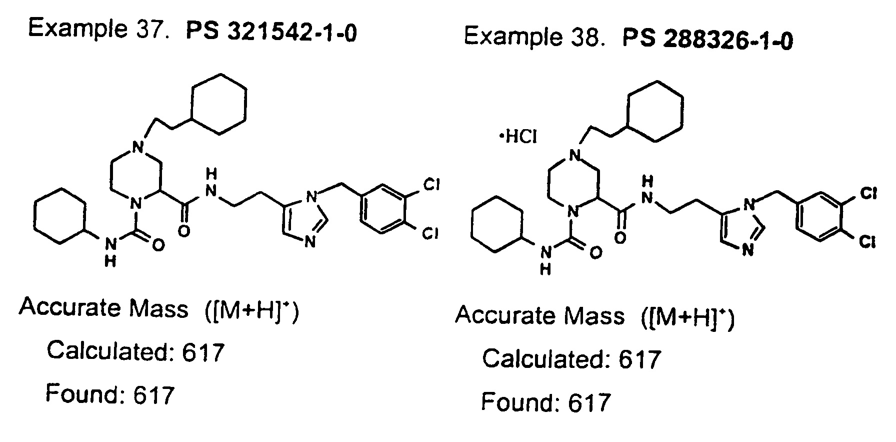

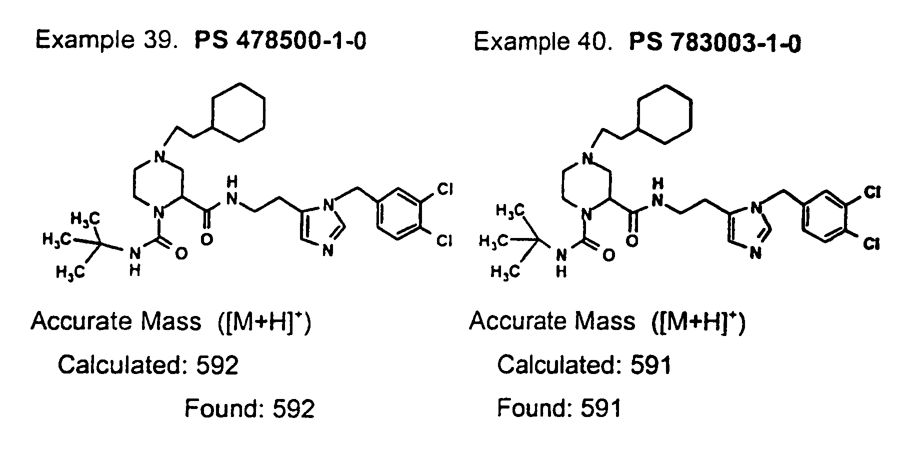

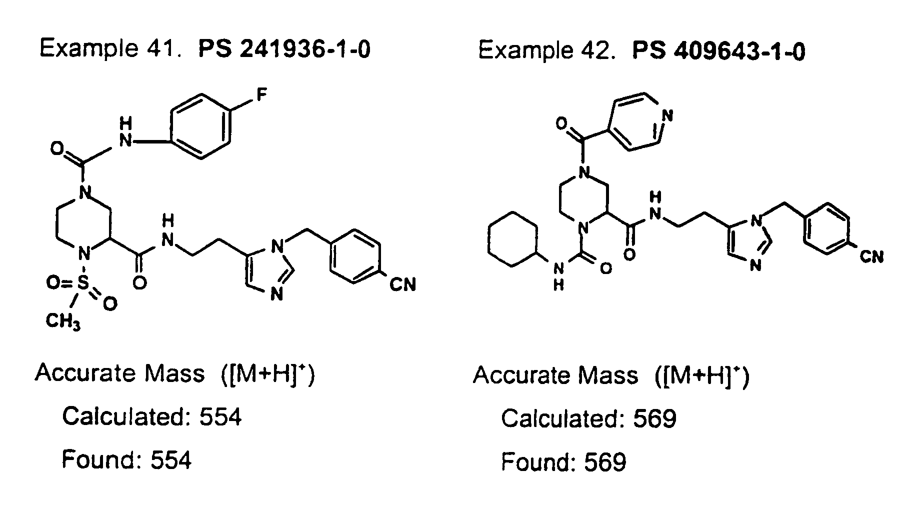

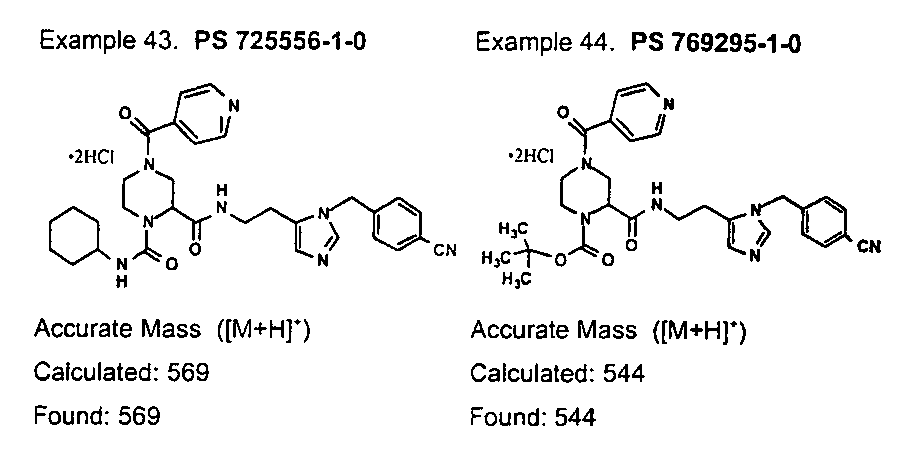

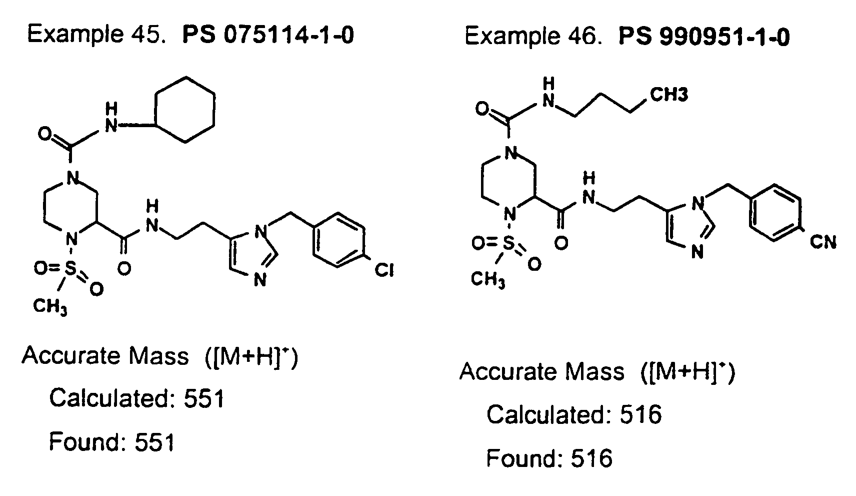

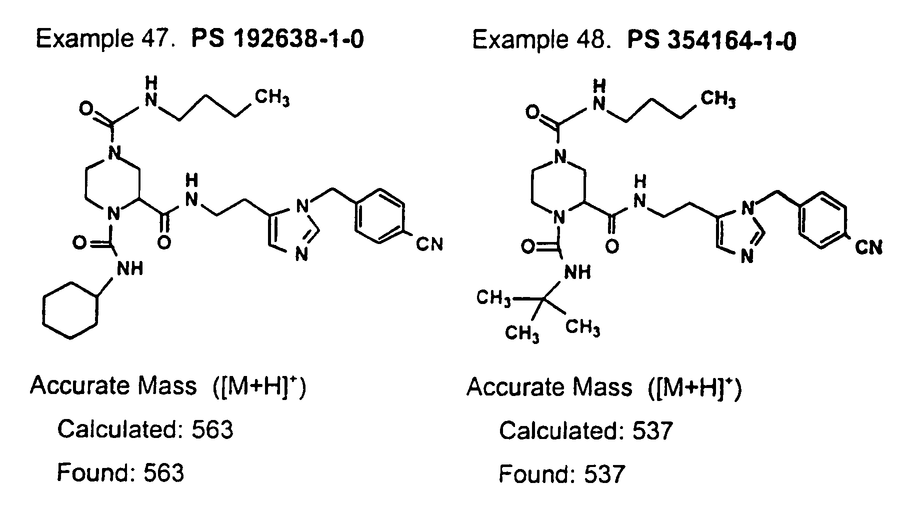

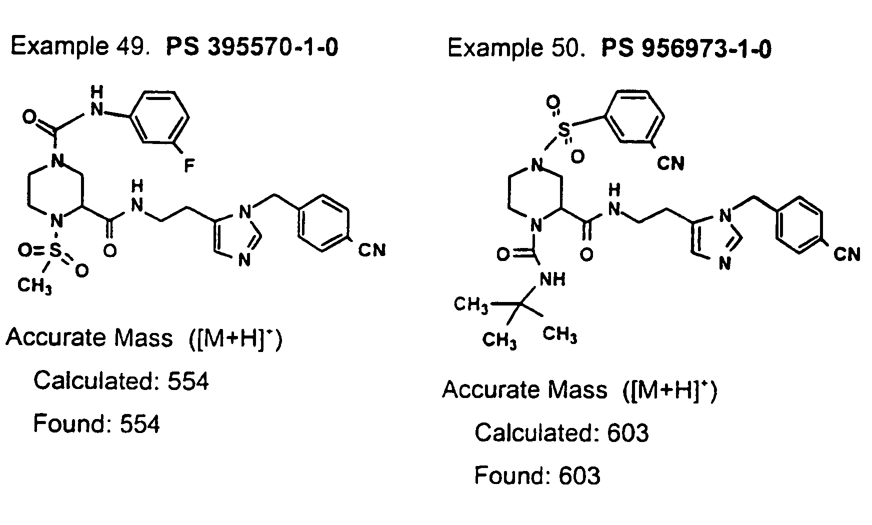

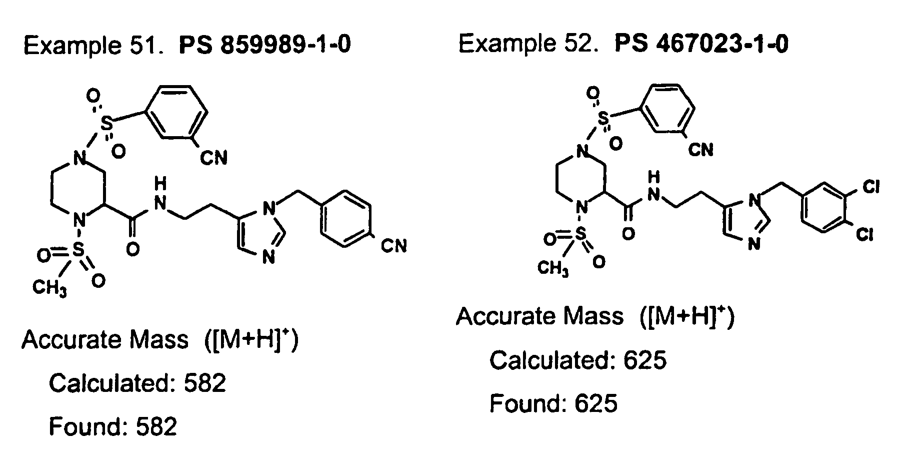

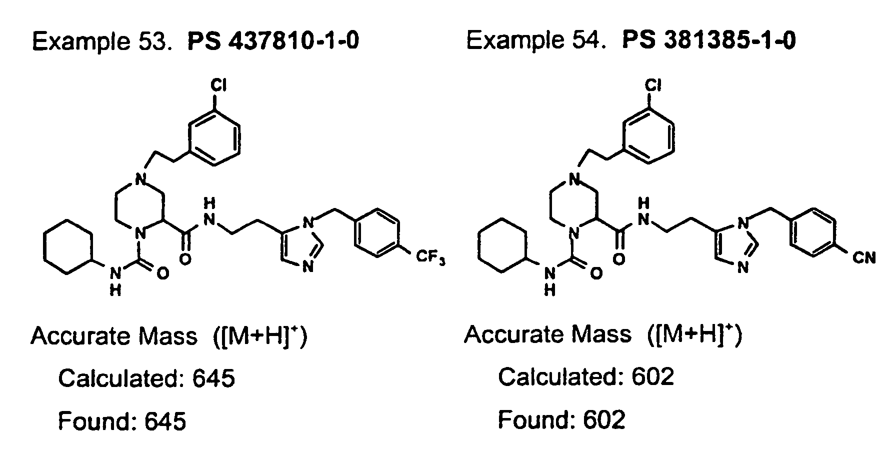

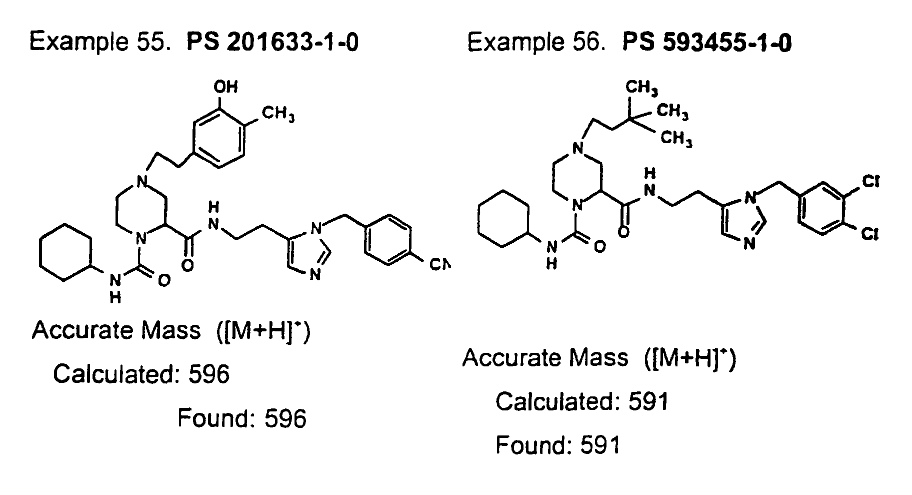

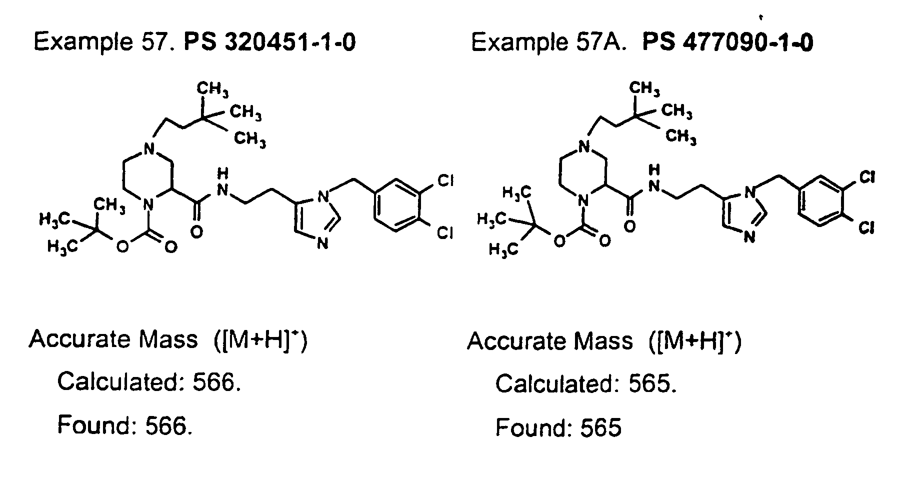

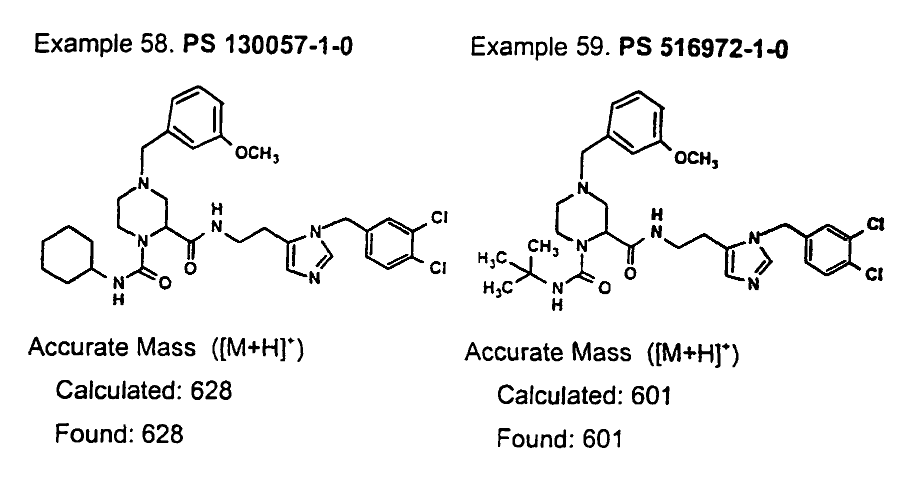

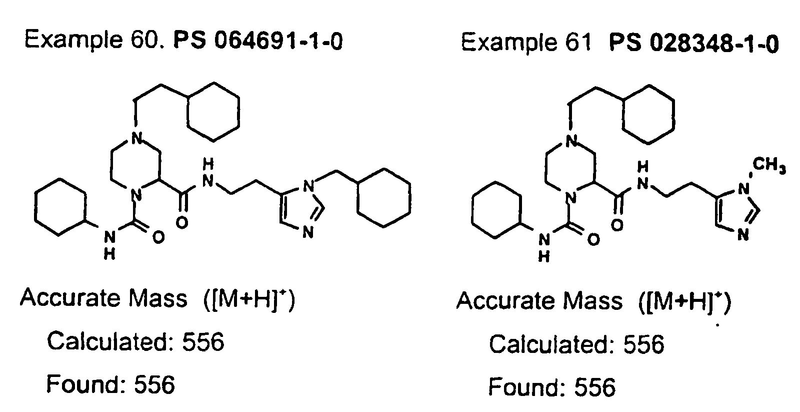

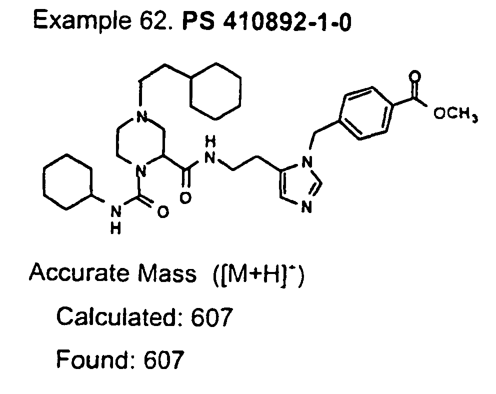

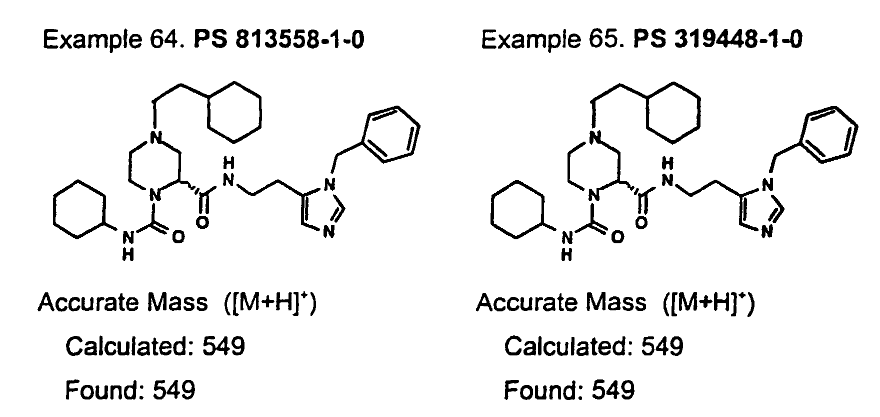

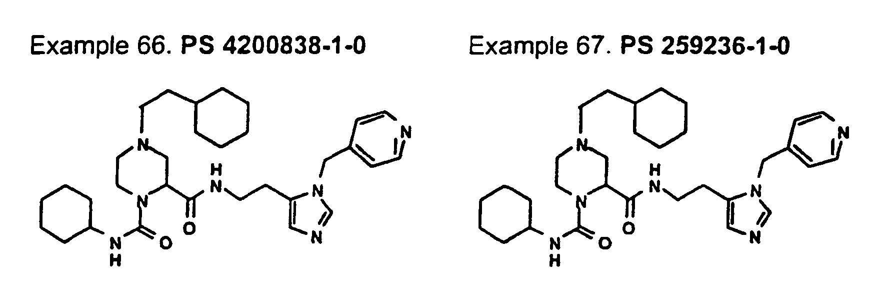

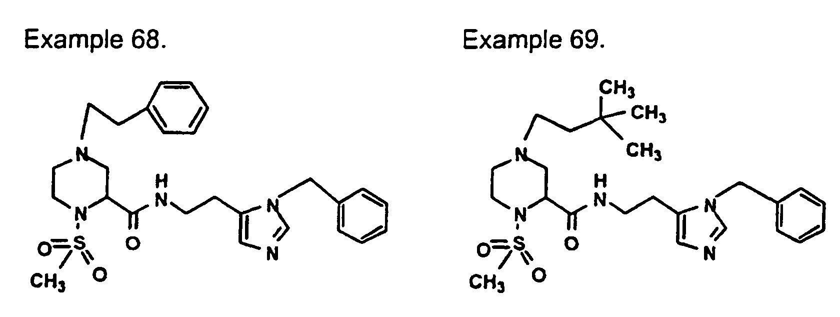

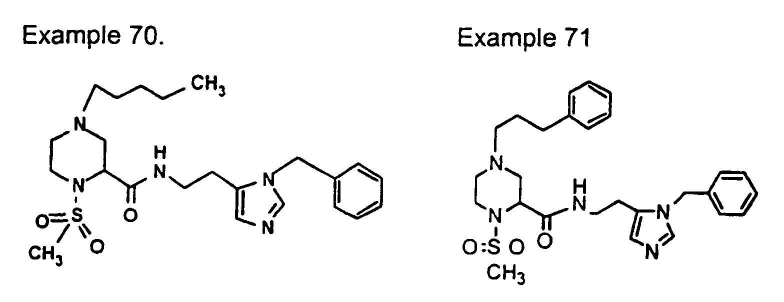

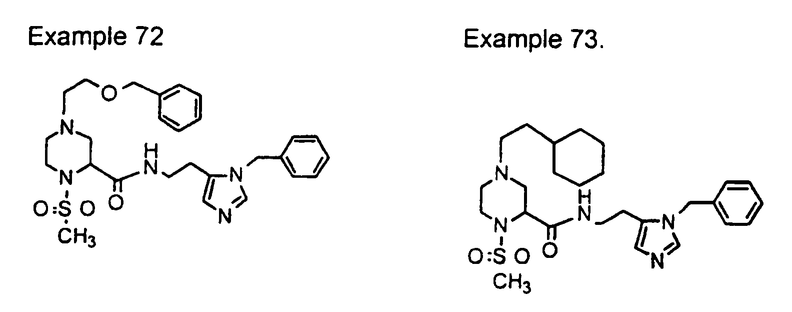

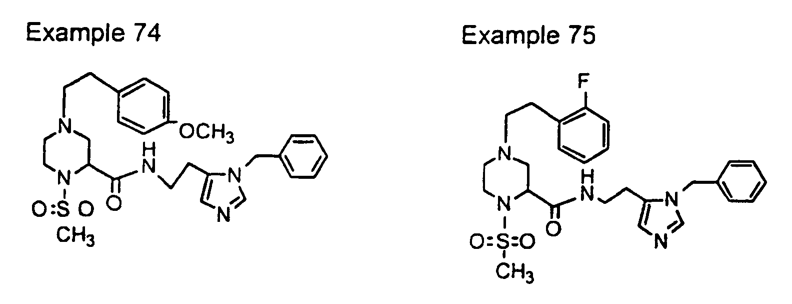

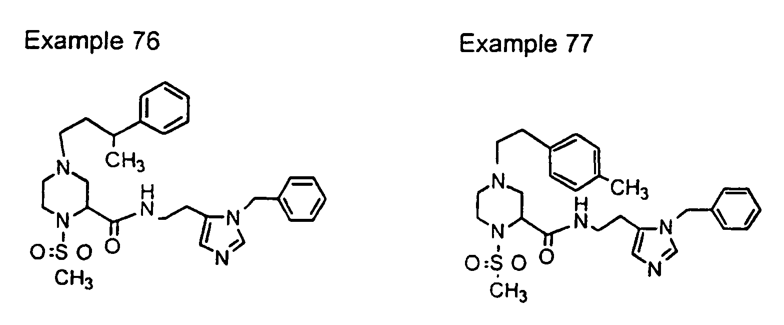

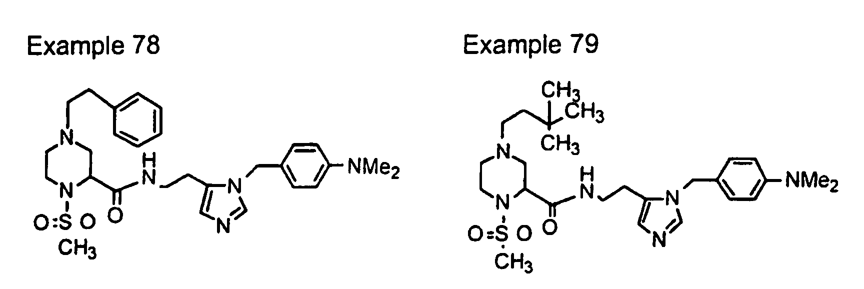

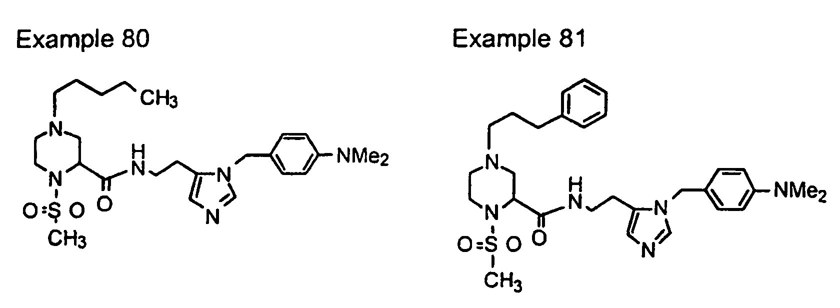

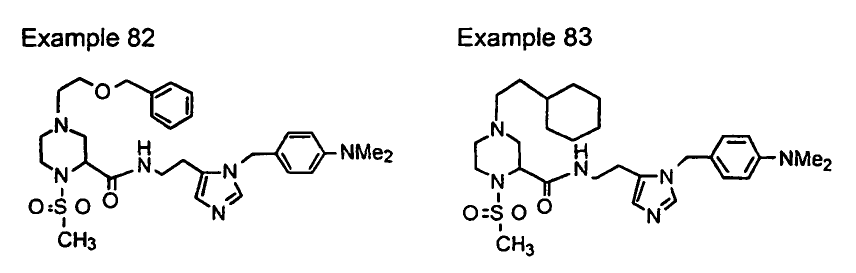

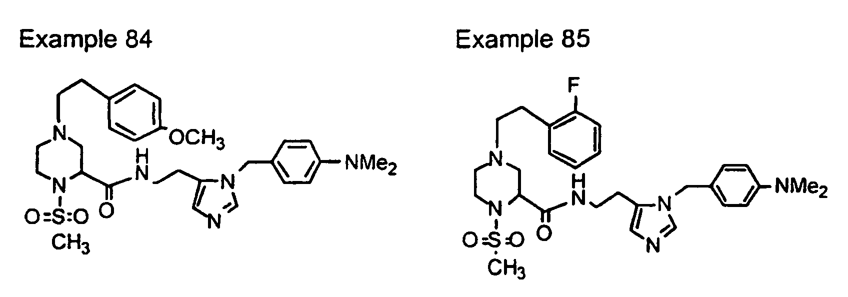

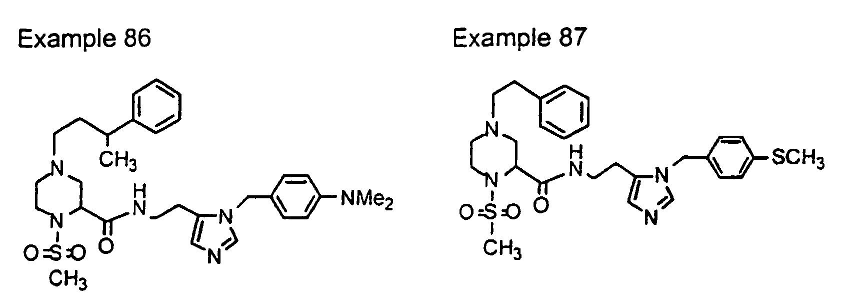

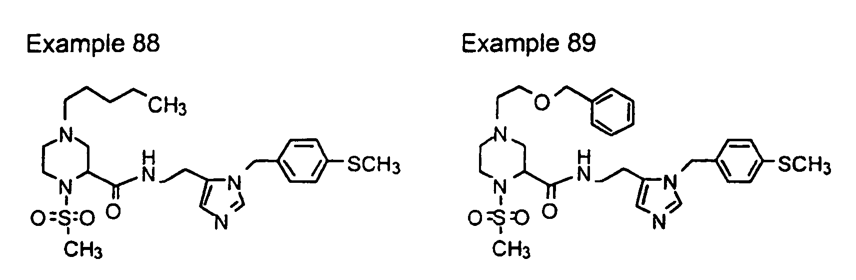

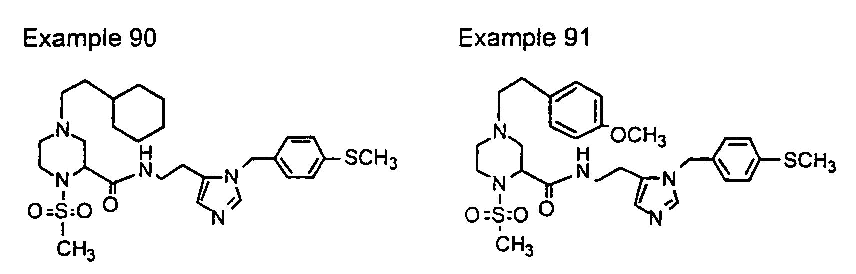

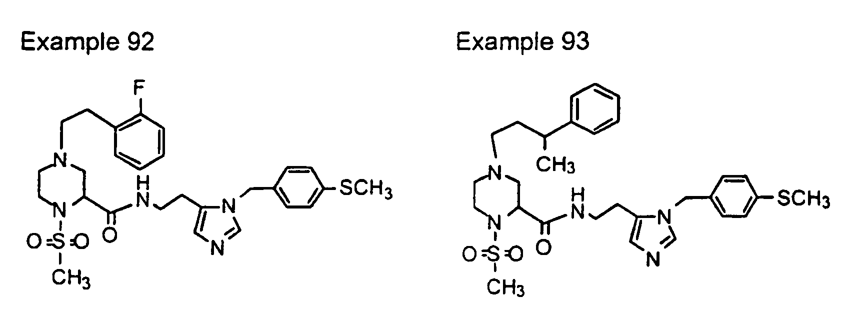

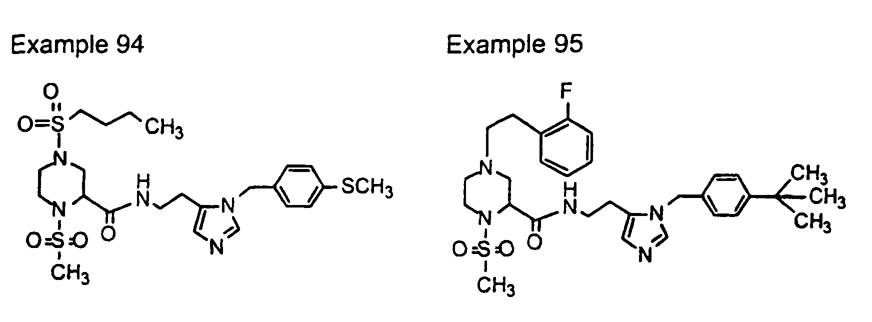

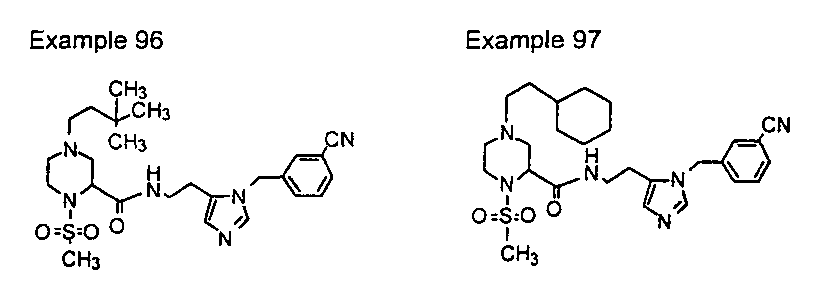

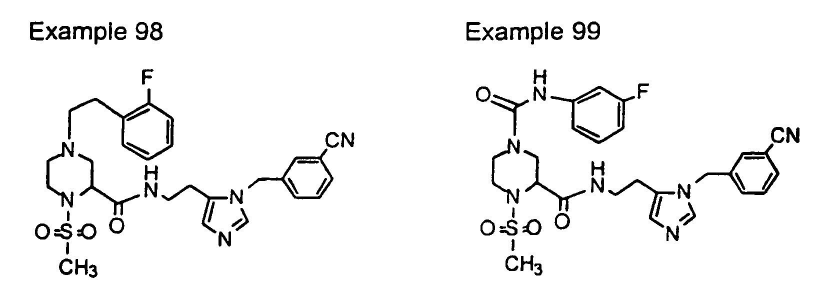

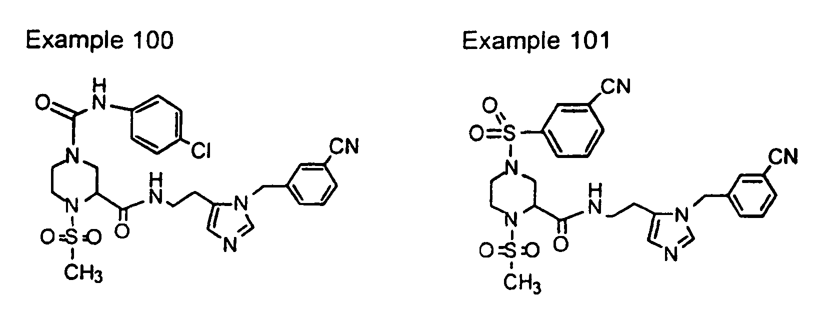

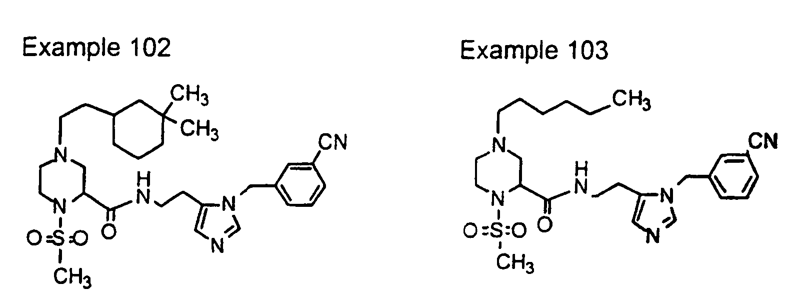

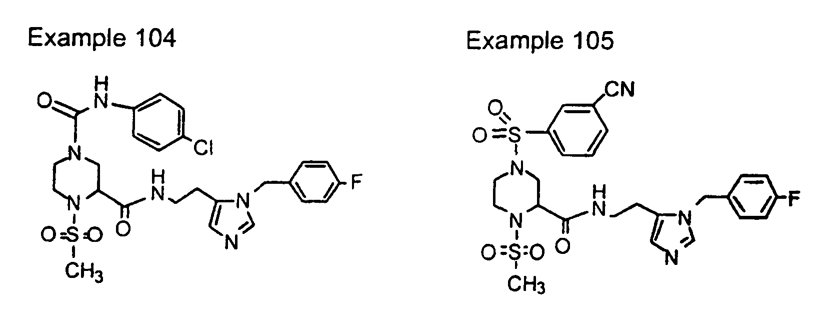

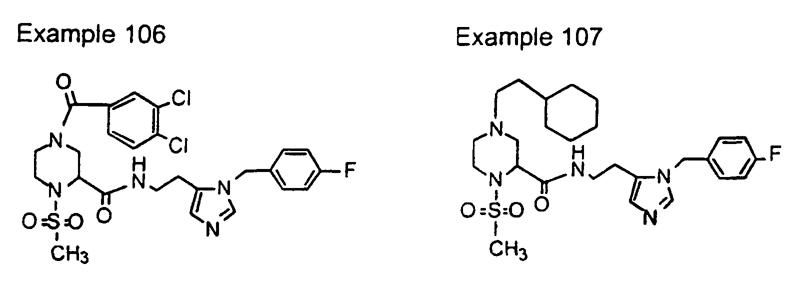

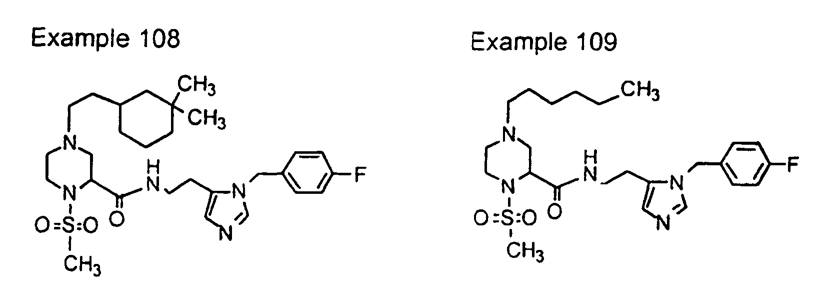

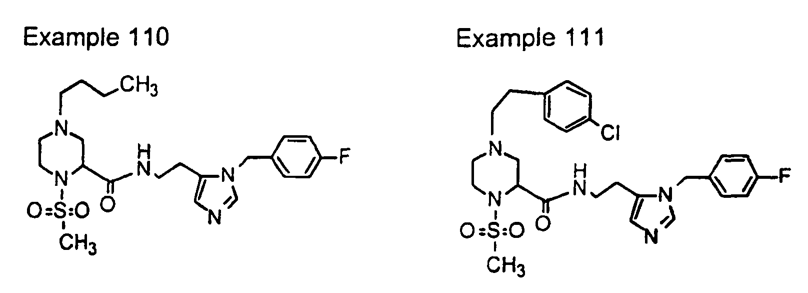

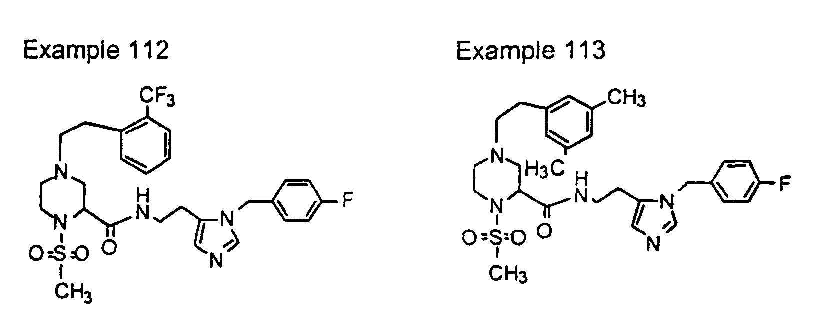

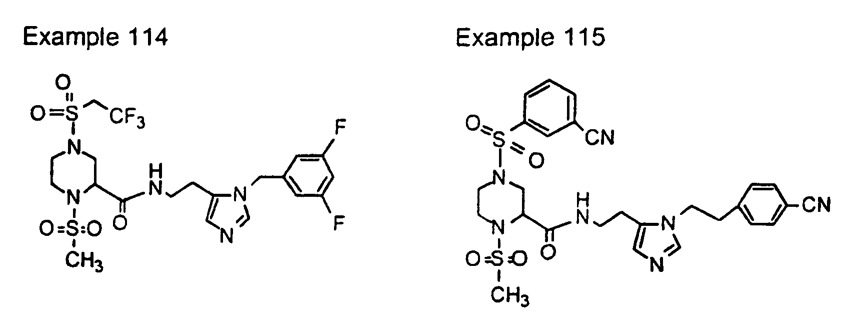

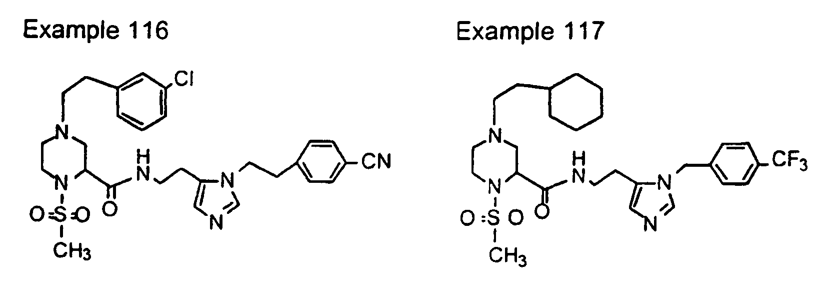

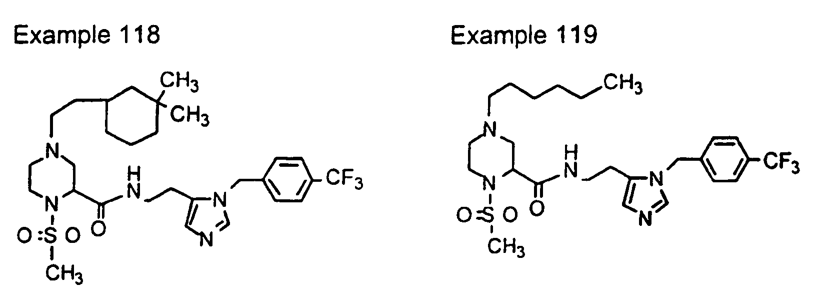

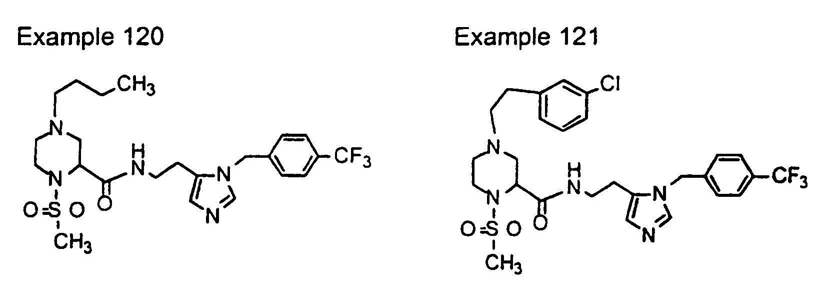

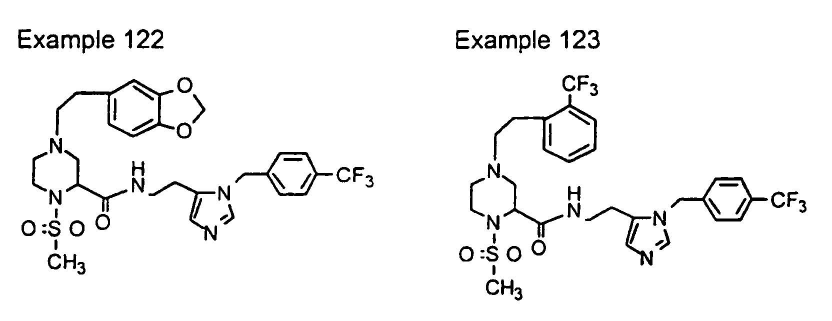

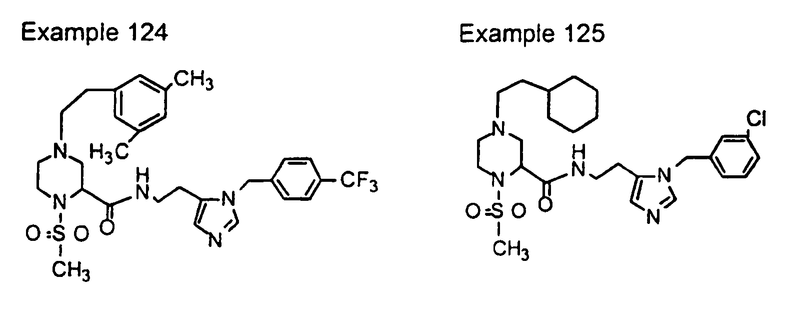

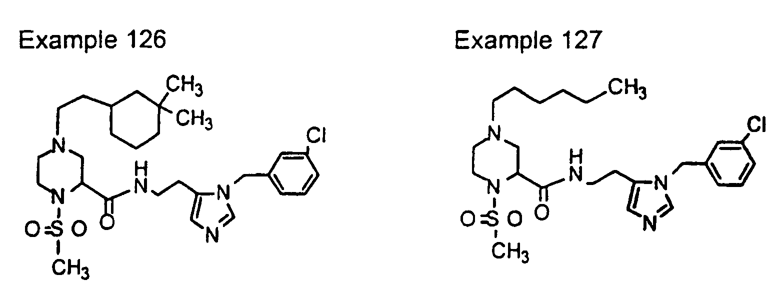

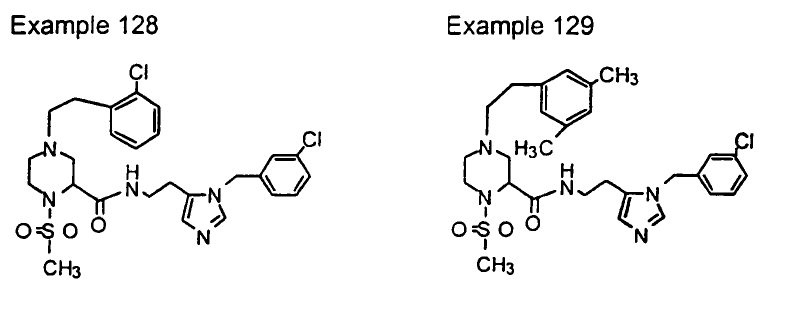

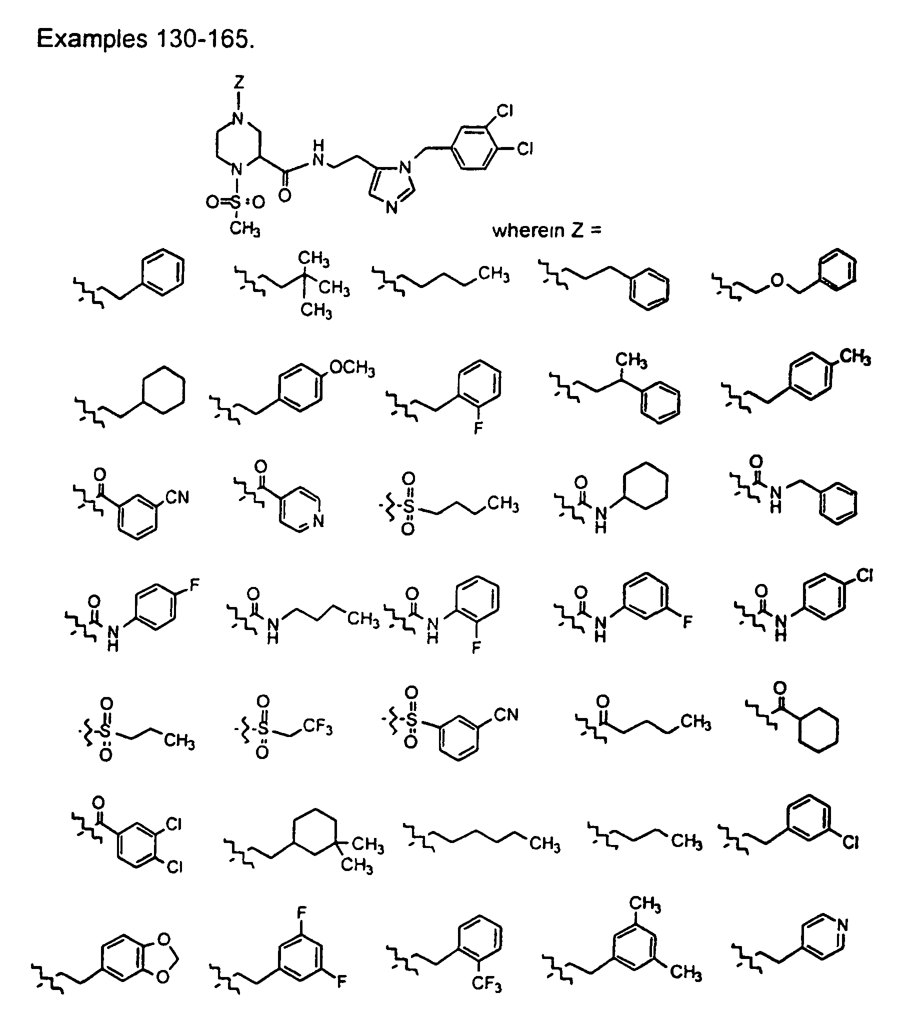

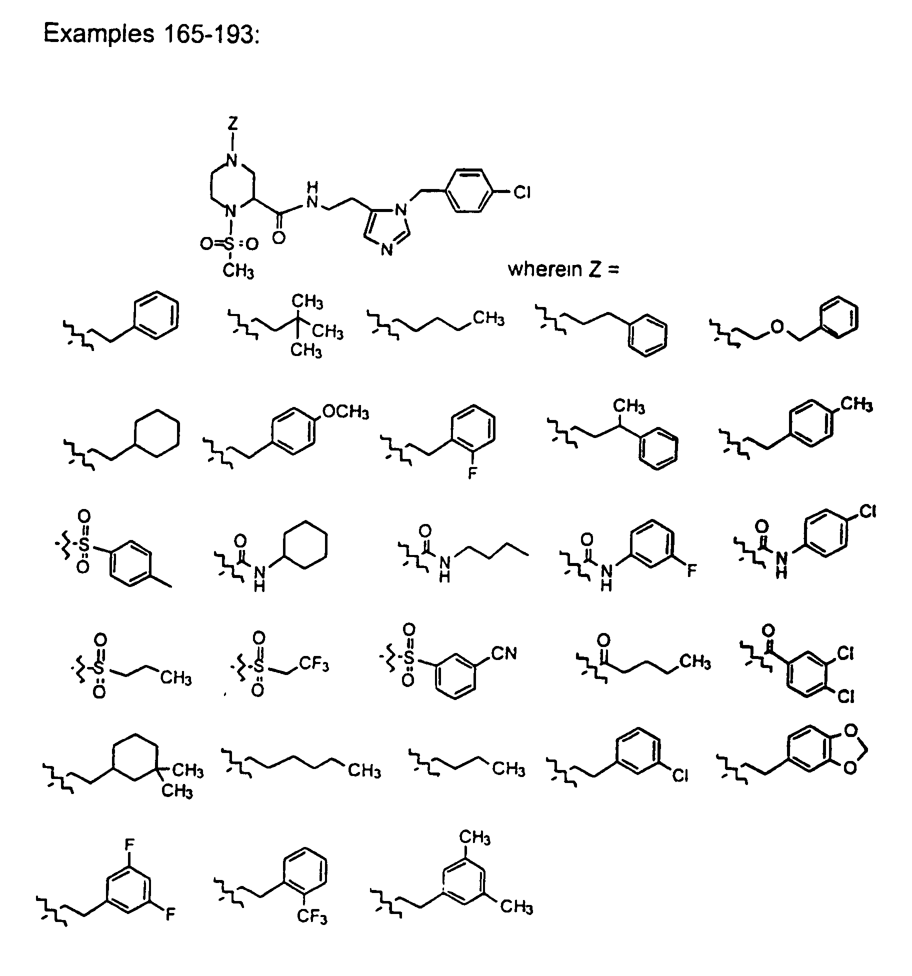

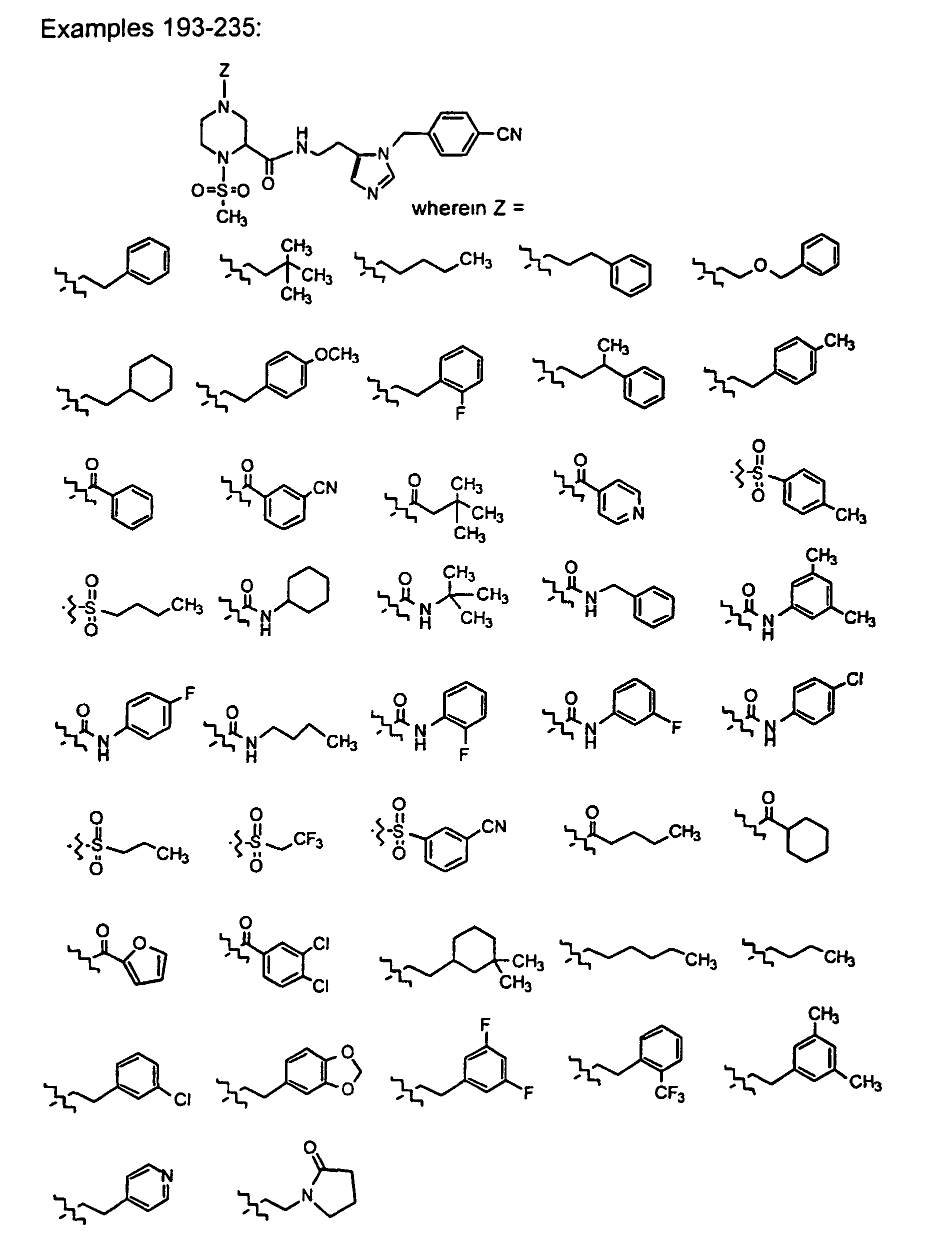

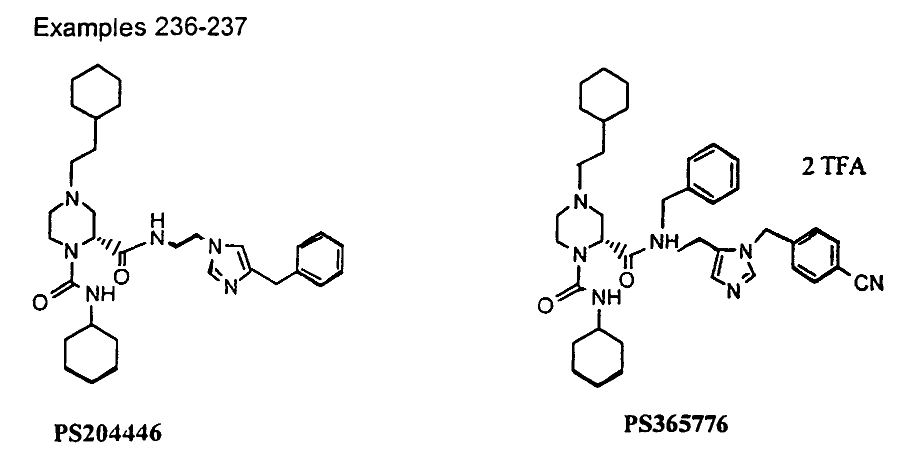

### PREPARATION OF STARTING MATERIALS

Starting materials useful in preparing the compounds of the present invention are exemplified by the following preparative examples, which should not be construed to limit the scope of the disclosure. Methods for preparing various starting materials can also be found in references such as, for example, Emmett, J. C., Holloway, F. H., Turner, J. L. J. Chem. Soc., Perkin Trans. 1 1979, 1341-1344 and Abdel-Magid, A. F., Maryanoff, C. A., Carson, K. G. *Tetrahedron Lett*. 1990, 31, 5595.

### Preparative Example 1.

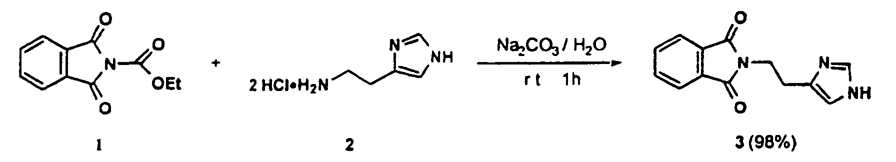

N-Carbethoxyphthalimide **1** (62.8 g, 0.275 mol, 1.1 eq.) is added portionwise over a period of 30 minutes to a stirred solution of histamine dihydrochloride **2** (46.7 g, 0.250 mol, 1 0 eq.) and sodium carbonate (54.3 g, 0.513 mol, 2.05 eq.) in distilled water (1250 ml) at room temperature. The resulting snow-white suspension is stirred vigorously at room temperature for 90 minutes. The solid is filtered off and thoroughly washed with ice-cold distilled water (4 x 50 ml) The solid is collected and dried under vacuum over P₂O₅ at 60 °C for 12h to give N^{a}-phthaloylhistamine **3** (59.2 g, 0.245 mol, 98%) in high punty (>95% by ¹H NMR). The snow-white solid is used directly without further purification.

### Preparative Example 2.

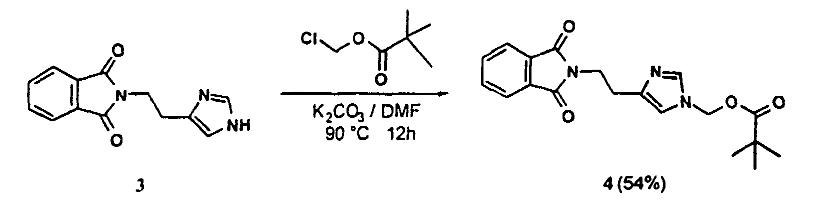

A solution of chloromethyl pivalate (18.5 ml, 0.125 mol, 1.2 eq.) in anhydrous *N*,*N*-dimethylformamide (DMF, 100 ml) is added dropwise over a period of one hour to a stirred mixture of 3 (25.0 g, 0.104 mol, 1.0 eq.) and potassium carbonate (17.2 g, 0.125 mol, 1 2 eq.) in anhydrous DMF (500 ml) at 90 °C under a nitrogen atmosphere. The mixture is stirred at 90 °C for 12h. The volatiles are removed under vacuum at 50 °C. The residue is taken up in brine (100 ml) and extracted with ethyl acetate (4 x 25 ml). The combined organic extracts are dried over Na₂SO₄, filtered, and concentrated under vacuum at 30 °C. The residual off-white solid is flash-chromatographed (hexanes . acetone = 6 : 4 v/v) over silica gel to give *N*^{*t*}*-pivaloyloxymethyl-N*^{a}*-phthaloylhistamine* **4** (20 g, 0.056 mol) as a crystalline, white solid of high purity

### Preparative Example 3.

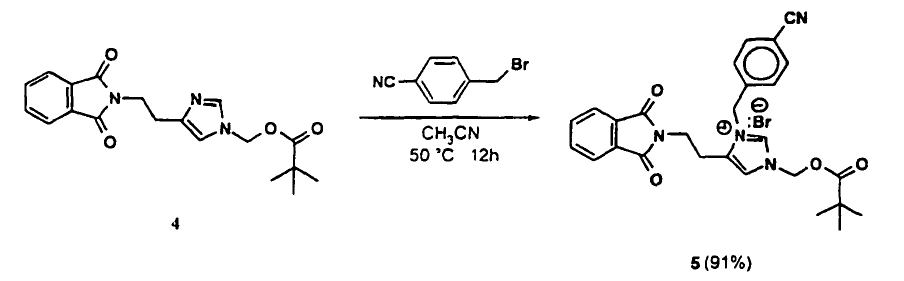

A solution of **4** (10 2 g, 28.7 mmol, 1.0 eq.) and *a*-bromo-*p*-tolunitrile (11.4 g, 57.4 mmol, 2.0 eq.) is stirred in anhydrous acetonitrile (150 ml) at 50°C under a nitrogen atmosphere for 12h. The resulting snow-white suspension is cooled to room temperature and chilled in a refrigerator at - 20 °C for one hour. The precipitate is filtered off and thoroughly washed with ice-cold ethyl acetate (4 x 50 ml). The solid is collected and dried under vacuum over P₂O₅ at 50 °C for 12h to give *1-pivaloyloxymethyl-3-(4-cyanobenzyl)-4-(2-phthalimidoethyl)imidazolium bromide 5* (14.4 g, 26.2 mmol).

### Preparative Example 3A. 1-Pivaloyloxymethyl-3-(4-chlorobenzyl)-4-(2-phthalimidoethyl)imidazolium chloride

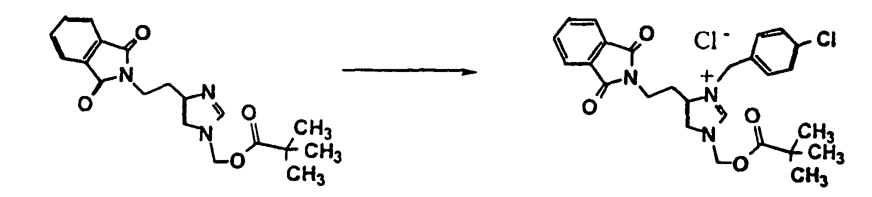

A solution of the title compound from Preparative Example 2 (5 g, 14.1 mmol) and 4-chlorobenzylchloride (2.5 g, 15.5 mmol) was stirred in anhydrous acetonitrile (60 ml) at reflux under a nitrogen atmosphere for 48 h. The mixture was concentrated in vacuo and recrystallized from ethyl acetate-hexane to give the title compound as a solid (3.2 g, MH+ = 480), and the filtrate which was concentrated to give additional product (3.6 g).

### Preparative Example 3B. 1-Pivaloyloxymethyl-3-(4-phenylethyl)-4-(2-phthalimidoethyl)imidazolium bromide

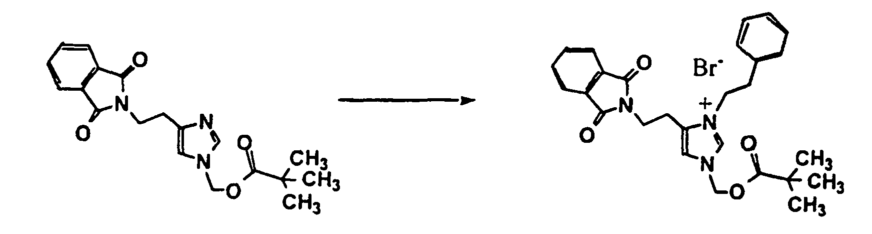

A solution of the title compound from Preparative Example 2 (5 g, 14.1 mmol) and 2-bromoethylbenzene (15.5 mmol, 1 1 eq) is stirred in anhydrous acetonitrile (50 ml) at reflux under a nitrogen atmosphere for 72 h. The mixture is concentrated in vacuo to give the title compound as a sticky oil (100%, MH+ = 460).

### Preparative Example 3C.

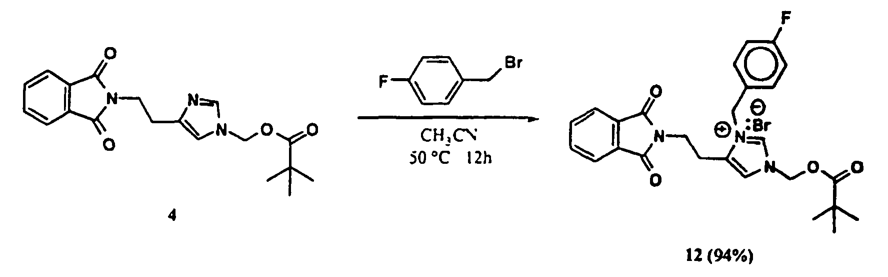

A solution of **4** (3.0 g, 8.4 mmol, 1.0 eq.) and 4-fluorobenzylbromide (2.2 ml, 16.9 mmol, 2.0 eq.) is stirred in anhydrous acetonitrile (50 ml) at 50 °C under a nitrogen atmosphere for 12h. The resulting snow-white suspension is cooled to room temperature and chilled in a refrigerator at - 20 °C for one hour. The precipitate is filtered off and thoroughly washed with ice-cold ethyl acetate (4 x 25 ml) The solid is collected and dried under vacuum over P₂O₅ at 50 °C for 12h to give *1-pivaloyloxymethyl-3-(4-fluorobenzyl)-4-(2-phthalimidoethyl)imidazolium bromide* **12** (4.32 g, 7.93 mmol) in 94% yield.

### Preparative Example 4.

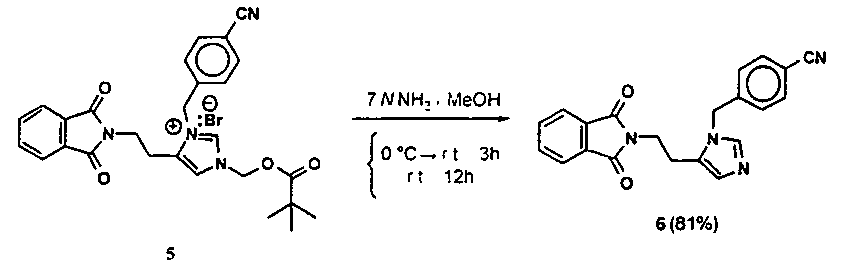

A 7 *N* solution of ammonia in methanol (75 ml, 0.525 mol, 7.25 eq.) is added dropwise over a period of 75 minutes to a stirred solution of 5 (40 g, 0.073 mol, 1.00 eq.) in anhydrous methanol (1000 ml) at 0 °C under a nitrogen atmosphere. The mixture is slowly (3h) warmed to ambient temperature and stirred for another 12h. The volatiles are evaporated under vacuum at 30 °C and the residual white solid is flash-chromatographed (CH₂Cl₂ : 2 *N* NH₃/ MeOH = 90 : 10 v/v) over silica gel to give *N*^{*p*}*-(4-cyanobenzyl)-N*^{*a*}*-phthaloylhistamine* **6** (21 g, 0.059 mol).

### Preparative Example 4A. N-[(4-chlorophenyl)methyl]-N-phthaloylhistamine

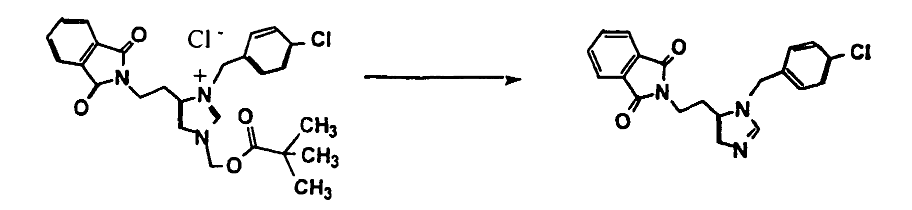

A 7 *N* solution of ammonia in methanol (10 ml, 0.07 mol) is added slowly to a stirred solution of the title compound from Preparative Example 3A (3.2 g, 6.6 mmol) diluted with MeOH (10 mL) at -20°C. The resulting mixture is warmed to room temperature and stirred for another 12 h, then concentrated *in vacuo* and purified by flash column chromatography (silica gel) using 3% MeOH-CH₂Cl₂ saturated with ammonium hydroxide as eluent to afford the title compound as a sticky solid (1.2 g, 51%, MH⁺ = 366).

### Preparative Example 4B. N-(4-phenylethyl)-N-phthaloylhistamine

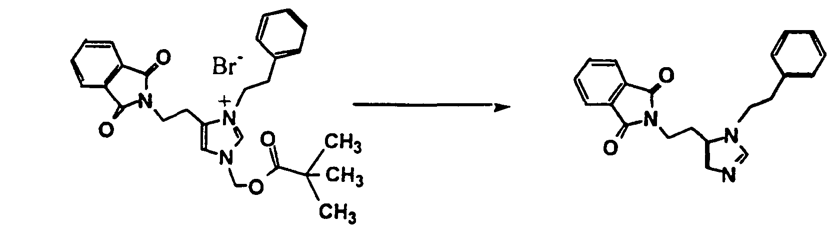

A 7 N solution of ammonia in methanol (23 ml, 0.16 mol) is added slowly to a stirred solution of the title compound from Preparative Example 3B (15.8 mmol) diluted with MeOH (60 mL) at -20°C. The resulting mixture is warmed to room temperature and stirred for another 96 h, then concentrated *in vacuo* and purified by flash column chromatography (silica gel) using 3% MeOH-CH₂Cl₂ saturated with ammonium hydroxide as eluent to afford the title compound (1 6 g, MH⁺ = 346).

### Preparative Example 4C.

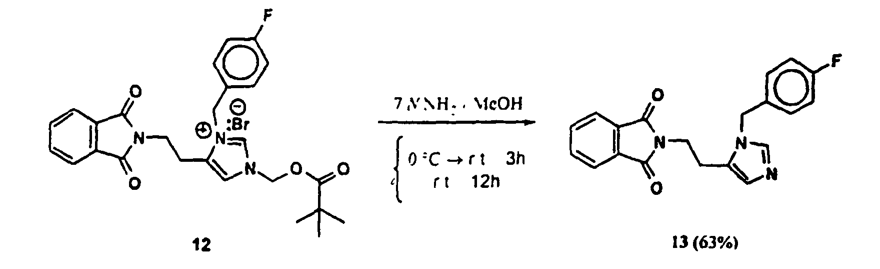

A 7 *N* solution of ammonia in methanol (8.0 ml, 55.10 mmol, 7.25 eq.) is added dropwise over a period of 10 minutes to a stirred solution of 12 (4.0 g, 7.35 mmol, 1.00 eq.) in anhydrous methanol (50 ml) at 0 °C under a nitrogen atmosphere. The mixture is slowly (3h) warmed to ambient temperature and stirred for another 12h. The volatiles are evaporated under vacuum at 30°C and the residual white solid is flash-chromatographed (CH₂Cl₂ : 2 *N* NH₂, MeOH = 97 : 3 v/v) over silica gel to give *N*^{*p*}*-(4-fluorobenzyl)-N*^{*a*}*-phthaloyihistamine* 13 (1.62 g, 4.63 mmol, 63%) as a snow-white solid.

### Preparative Example 5.

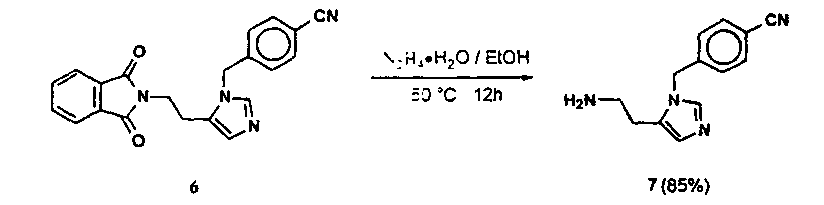

A solution of **6** (21 g, 0.059 mol, 1.0 eq.) and hydrazine monohydrate (15 ml, 0.884 mol, 15 0 eq.) in absolute ethanol (250 ml) is stirred at 50°C under a nitrogen atmosphere for 12h. The snow-white suspension is cooled to room temperature and chilled in a refrigerator at - 20 °C for one hour. The precipitate (phthalyl hydrazide) is filtered off and thoroughly washed with ice-cold ethanol (190 proof, 500 ml). The filtrates are combined and concentrated under vacuum at 30°C. The residue is subjected to flash column chromatography (CH₂Cl₂ : 2 *N* NH₃/ MeOH = 90 : 10 v/v) over silica gel to give *N*^{*p*}*-(4-cyanobenzyl)histamine* 7 (11.4 g, 0.050 mol, 85%) as a thick, light-brown oil.

### Preparative Example 5A. N-[(4-chlorophenyl)methyl]-histamine

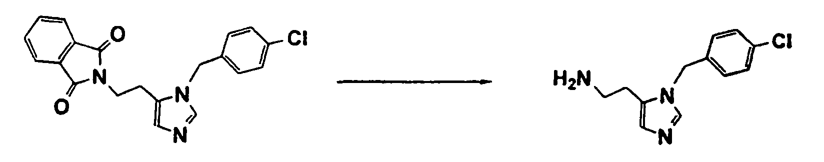

A solution of the title compound from Preparative Example 4A (1.21 g, 3.3 mmol) and hydrazine monohydrate (1.7 ml, 0.033 mol, 10 eq.) in absolute ethanol (20 ml) is stirred at 50 °C under a nitrogen atmosphere for 20 min. The resulting suspension is diluted with ethanol and dichloromethane and filtered. The filtrate is concentrated *in vacuo* to afford the title compound as a yellow oily solid (0.7 g, MH⁺ = 236).

### Preparative Example 5B. N-(phenylethyl)histamine

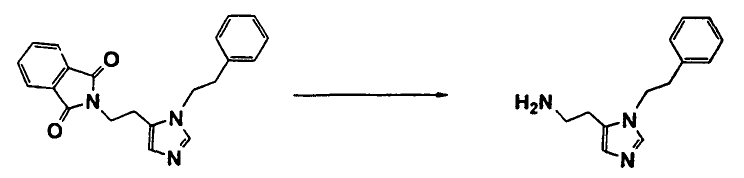

A solution of the title compound from Preparative Example 4B (1.54 g, 4.4 mmol) and hydrazine monohydrate (2.2 ml, 0.044 mol, 10 eq.) in absolute ethanol (25 ml) is stirred at 50 °C under a nitrogen atmosphere for 20 min, then at room temperature overnight. The resulting suspension is diluted with ethanol and dichloromethane and filtered. The filtrate is concentrated *in vacuo* to afford the title compound as an oily solid (0.88 g, MH⁺ = 216).

### Preparative Example 5C.

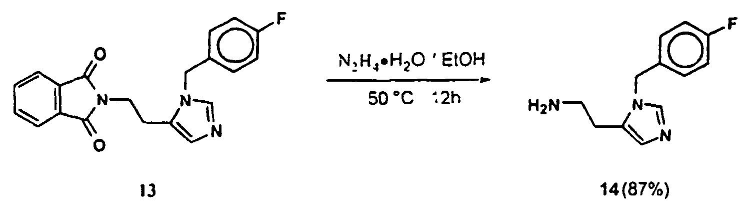

A solution of 13 (1.6 g, 4.6 mmol, 1.0 eq.) and hydrazine monohydrate (1.1 ml, 22.9 mmol, 5.0 eq) in absolute ethanol (25 ml) was stirred at 50 °C under a nitrogen atmosphere for 12h. The snow-white suspension was cooled to room temperature and chilled in a refrigerator at -20 °C for one hour. The precipitate (phthalyl hydrazide) was filtered off and thoroughly washed with ice-cold ethanol (190 proof, 50 ml). The filtrates were combined and concentrated under vacuum at 30 °C. The residue was flash column chromatographed (CH₂Cl₂ : 2 N NH₃/ MeOH = 90 : 10 v/v) over silica gel to give *N*^{*p*}*-(4-fluorobenzyl)histamine* **14** (870 mg, 3.97 mmol, 87%) as a brown oil.

### Preparative Examples 8 and 9.

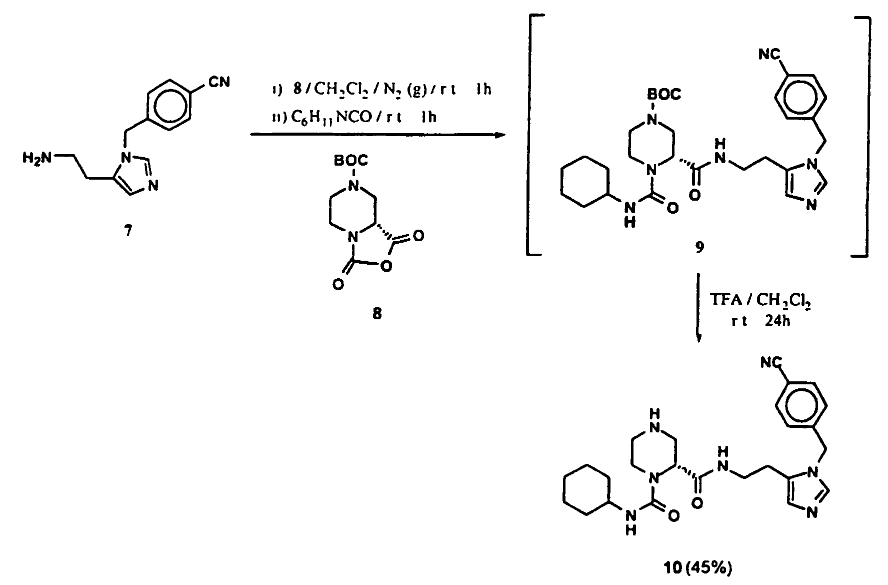

A solution of 7 (1.50 g, 6.63 mmol, 1.0 eq.) in anhydrous dichloromethane (30 ml) is added dropwise over a period of 30 minutes to a stirred solution of anhydride **8** (2.04 g, 7.95 mmol, 1.2 eq.) in anhydrous dichloromethane (30 ml) at room temperature. A stream of nitrogen is bubbled through the solution to expel evolved carbon dioxide. The colorless solution is stirred for one hour amid nitrogen bubbling. Bubbling is terminated and cyclohexyl isocyanate (1.75 ml, 13.26 mmol, 2.0 eq.) is added dropwise over a period of 5 minutes. The brown solution is stirred at room temperature for one hour to give **9** (confirmed by ¹H NMR). The volatiles are removed under vacuum at 30 °C. The residue is taken up in a mixture of trifluoroacetic acid (TFA, 30 ml) and anhydrous dichloromethane (30 ml) and is stirred at ambient temperature under a nitrogen atmosphere for 24h. The mixture is concentrated under vacuum at 30 °C. The residual light-brown oil is taken up in 1 *N* aqueous NaOH solution (100 ml) and extracted with dichloromethane (4 x 25 ml). The combined organic extracts are washed with brine (25 ml), dried over Na₂SO₄, filtered, and concentrated under vacuum at 30 °C. The resulting oil is flash-chromatographed (CH₂Cl₂ : 2 *N* NH₃/ MeOH = 90 : 10 v/v) over silica gel to give *N2-[2-[1-[(4-cyanophenyl)methyl]-1H-imidazol-5-yl]ethyl]-N1-cyclohexyl-1,2(R)-piperazinedicarboxamide* **10** (1.34 g, 2.95 mmol) as a light-yellow foam.

### Preparative Example 10A N4-(1,1-dimethylethyloxycarbonyl)-N2-[2-[1-[(4-chlorophenyl)methyl]-1H-imidazol-5-yl]ethyl]-1,2(R)-piperazinecarboxamide

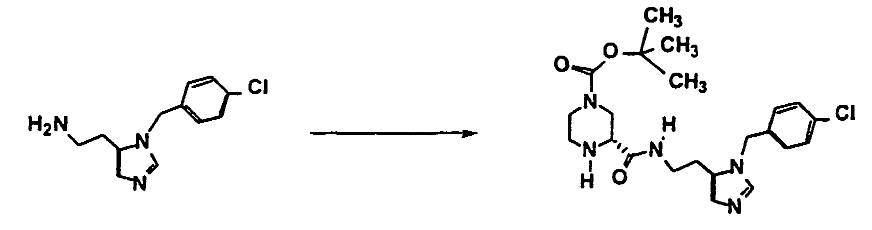

A solution of the title compound from Preparative Example 5A (0.695 g, 2.94 mmol) and the anhydride from Preparative Example (0.75 g, 2.94 mmol) dissolved in anhydrous dichloromethane (10 ml) is stirred at room temperature overnight. Additional anhydride (0.1 g) is added and after 1 hr the reaction mixture is diluted with CH₂Cl₂ and extracted with 1 M HCl (aq). The aqueous phase is basified with 1 *N* NaOH (aq), extracted with CH₂Cl₂ and the organic phase dried over anhydrous MgSO₄. After filtration, the organic phase is concentrated *in vacuo* to afford a white foam (0.744 g, MH⁺ = 448).

### Preparative Example 10B. N4-(1,1-dimethylethyloxycarbonyl)-N2-[2-[1-(phenylethyl)-1 H-imidazol-5-yl]ethyl]-1,2(R)-piperazinecarboxamide

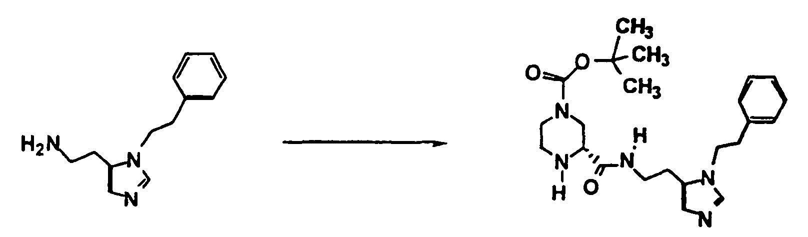

A solution of the title compound from Preparative Example 5B (0.874 g, 4.04 mmol) and the anhydride from Preparative Example (1.04 g, 4.04 mmol) dissolved in anhydrous dichloromethane (10 ml) is stirred at room temperature overnight. Additional anhydride (0.23 g) is added and after 1 hr the reaction mixture is diluted with CH₂Cl₂ and extracted with 1*M* HCl (aq). The aqueous phase is basified with 1*N* NaOH (aq), extracted with CH₂Cl₂ and the organic phase dried over anhydrous MgSO₄. After filtration, the organic phase is concentrated *in vacuo* to afford a white foam (1.22 g, 71 %, MH⁺ = 428).

### Preparative Example 10. N4-(1,1-dimethylethyloxycarbonyl)-N2-[2-[1-[(4-cyanophenyl)methyl]-1H-imidazol-5-yl]ethyl]-1,2(R)-piperazinecarboxamide

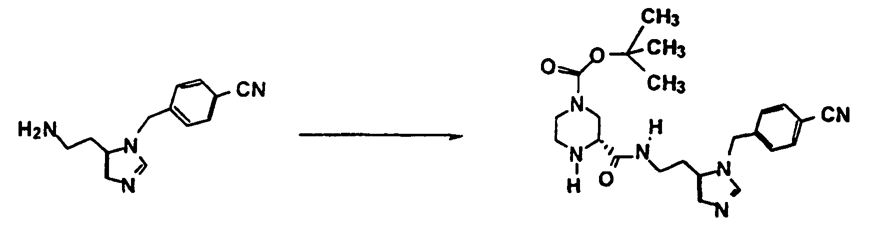

A solution of the title compound from Preparative Example 5 (7.8 g, 34.5 mmol), triethylamine (10 mL) and the anhydride from Preparative Example (10 g, 39.0 mmol) dissolved in anhydrous dichloromethane (300 ml) is stirred at room temperature overnight. The reaction mixture is concentrated *in vacuo*, diluted with CH₂Cl₂ and extracted with 1 M HCl (aq). The aqueous phase is basified with 1 N NaOH (aq), extracted with CH₂Cl₂ and the organic phase dried over anhydrous MgSO₄. After filtration, the organic phase is concentrated *in vacuo* to afford the title compound (10.0 g, MH⁺ = 448).

### Preparative Example 11. N2-[2-[1-[(4-cyanophenyl)methyl]-1H-imidazol-5-yl]ethyl]-N4-phenyl-1,2(R)-piperazinecarboxamide

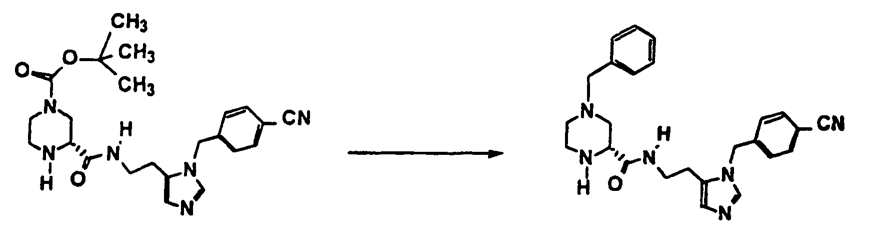

A solution of the title compound from Preparative Example 10 (5 g, 1.1.4 mmol) dissolved in anhydrous dichloromethane (50 ml) and trifluoroacetic acid (10 ml) is stirred at room temperature for 1 hr, then concentrated *in vacuo*. The residue is combined with benzaldehyde (1.4 mmol) and diluted with glacial acetic acid (25 mL) and anhydrous dichloromethane (100 ml), then cooled to 0°C. Sodium triacetoxyborohydride (4.83 g, 22.8 mmol) is added and the resulting mixture is stirred at 0°C for 2 h, then at room temperature overnight. The reaction mixture is concentrated *in vacuo*, diluted with dichloromethane and extracted with 1*N* HCl (aq). The aqueous phase is basified with 1 N NaOH (aq) and extracted with dichloromethane, then dried over anhydrous Na₂SO₄, filtered, concentrated *in vacuo*. Flash column chromatography (silica gel) using 10% MeOH-CH₂Cl₂ saturated with ammonium hydroxide as eluent affords the title compound (1.5 g, MH⁺ = 428).

### Preparative Example 12. N2-[2-[1-[(4-chlorophenyl)methyl]-1H-imidazol-5-yl]ethyl]-N1-cyclohexyl-1,2(R)-piperazinecarboxamide

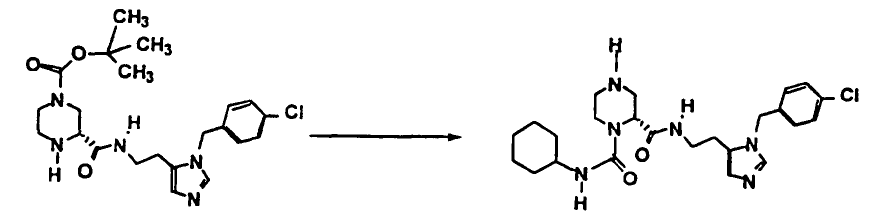

To a solution of the title compound from Preparative Example 10A (0.30 g, 0.67 mmol) dissolved in anhydrous dichloromethane (3 ml) is added cyclohexylisocyanate (0.09 mL. 0.7 mmol) and the resulting solution was stirred at room temperature for 30 mm, then concentrated *in vacuo*. The resulting residue is diluted with dichloromethane (3 ml) trifluoroacetic acid (3 ml). The solution is stirred at room temperature overnight, then concentrated *in vacuo*, diluted with dichloromethane and washed with 1*N* NaOH (aq). The organic phase is dried over anhydrous MgSO₄, filtered, and concentrated *in vacuo* to afford a yellow foam (0.319 g, MH⁺ = 473).

### Preparative Example 12A. N2-[2-[1-(phenylethyl)-1H-imidazol-5-YL]ethyl]-N1-cyclohexyl-1,2(R)-piperazinedicarboxamide

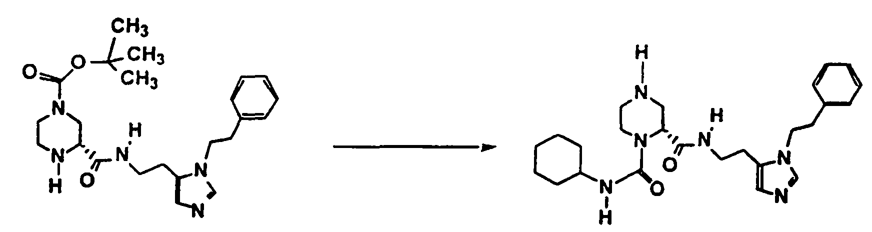

To a solution of the title compound from Preparative Example 10B (0.33 g, 0.77 mmol) dissolved in anhydrous dichloromethane (3 ml) is added cyclohexylisocyanate (0.11 mL, 0.9 mmol) and the resulting solution is stirred at room temperature for 30 min, then concentrated *in vacuo*. The resulting residue is diluted with dichloromethane (3 ml) trifluoroacetic acid (3 ml). The solution is stirred at room temperature overnight, then concentrated *in vacuo,* diluted with dichloromethane and washed with 1*N* NaOH (aq). The organic phase is dried over anhydrous MgSO₄, filtered, and concentrated *in vacuo* to afford a yellow foam (0.338 g, MH⁺ = 453).

### Preparative Example 13.

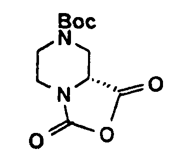

### Step a. 2-R-carboxyl-piperazine-di-(R)-(-)-camphorsulfonic

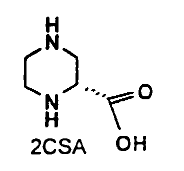

To 2.5 kg of (R)-(-)-camphorsulfonic acid stirring at 60°C in 1250 ml of distilled water are added a soution of the potassium salt of 2-carboxylpiperazine (565 gm, 3.35 mol). The mixture is allowed to stir at 95°C until completely dissolved. The solution is allowed to stand at ambient temperature for 48 hrs. The resulting precipitate is filtered to obtain 1444 gm of damp solid. The solids are then dissolved in 1200 ml of distilled water and heated on a steam bath until all solids dissolved. The hot solution is then set aside to cool slowly for 72 hrs. The crystalline solids are filtered to give 362 gm of pure product as a white crystalline solid. [a]_{D}=-14.9 °

### Step b. N,N-di-tert.butoxycarbonyl-2-R-carboxyl-piperazine

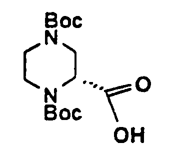

2-R-carboxyl-piperazine-di-(R)-(-)-camphorsulfonic (362 gm, 0.608 mol) from Step a. is dissolved in 1.4 L of distilled water and 1.4 L of methanol. 75 ml of 50% NaOH are dripped in to the stirred reaction mixture to obtain an about pH= 9.5 solution. To this solution is added di-tert.butyl-dicarbonate (336 gm, 1.54 mol) as a solid. The pH drops to about 7.0. The pH of the reaction mixture is maintained at 9.5 with 50% NaOH (total of 175 ml), and the reaction mixture is stirred for 2.5 hours to obtain a white precipitate. The reaction mixture is diluted to 9 L with ice/water followed by washing with 2 of ether. The ether is discarded and the pH of the aqueous layer isadjusted to pH 3.0 by the portionwise addition of solid citric acid. The acidified aqueous layer is then extracted with dichloromethane 3X with 2L. The organic layers are combined, dried over sodium sulfate, filtered and evaporated to obtain 201.6 gm of title compound as a white glassy solid. FABMS (M+1)=331

### Step c. "N-Boc-piperazine-2-amidoanhydride"

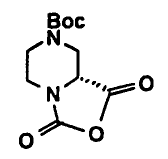

To an ice cold solution N,N-dimethylformamide (49.6 ml) is added, dropwise, thionylchloride (46.7 ml) over a period of 5 minutes in a 5 L round bottom flask under a nitrogen atmosphere The reaction mixture is allowed to stir for 5 min. and the ice bath removed and the reaction mixture allowed to stir at ambient temperature for 30 min. The reaction mixture is cooled again in an ice bath and a solution of of N,N-di-butoxycarbonyl-2-R-carboxyl-piperazine (201.6 gm, 0.61 mmol) from Step b. in 51.7 ml of pyridine and 1.9 L of acetonitrile are cannulated into the reaction mixture. The reaction mixture is allowed to warm to ambient to obtain a yellowish turbid solution. After stirring at ambient temperature for 18 hours, the reaction mixture is filtered and the filtrate poured into ice water (7L) and then extracted with 4X 2 L of ethylacetate, dried over sodium sulfate, filtered and evaporated to dryness under vacuo to obtain 115.6 gm of the title product as a white solid. FABMS (M+1)=257.

### Preparative Example 14. [2-(2-methyl-1 H-benzimidazol-1-yl)ethyl]amine

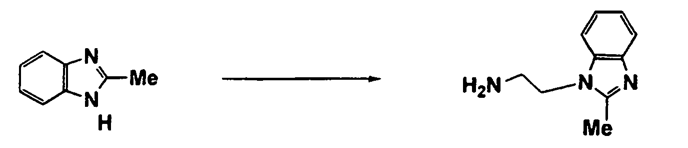

A mixture of 2-methylbenzimidazole (4.93 g, 37.3 mmol), 2-chloroethylamine hydrochloride (4.67 g, 40.3 mmol), tetrabutylammonium sulfate (0.51 g, 1.5 mmol), sodium hydroxide (5.37 g, 134.2 mmol) and acetonitrile (80 mL) are stirred at reflux for 48 hrs. The resulting mixture is cooled to room temperature, filtered and concentrated *in vacuo*. The residue is purified by flash column chromatography (silica gel) using 1-3% MeOH-CH₂Cl₂ saturated with ammonium hydroxide as eluent to afford the title compound (3.56 g, MH⁺ = 176).

### Preparative Example 15. [2-(2,4-dimethylimidazol-1-yl)ethyl]amine and [2-(2,5-dimethylimidazol-1-yl)ethyl]amine

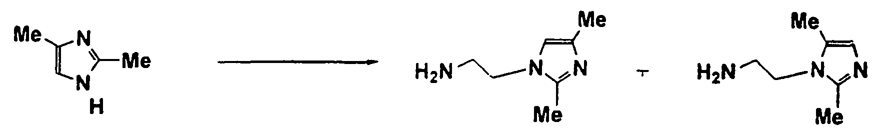

Following a similar procedure used in Preparative Example 14, but using 2,4-dimethylimidazole instead of 2-methylbenzimidazole, the title compounds were prepared as a mixture of regioisomers (10.7 g, MH⁺ =).

### Preparative Example 16. 3-(1H-imidazol-1-yl)propyl-phenylmethylamine

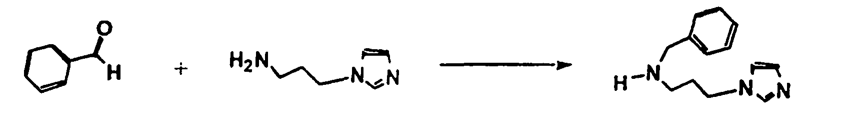

A mixture of 1-(3-aminopropyl)imidazole (Aldnch, 37.1 g, 297 mmol), benzaldehyde (30 g, 283 mmol), 3A molecular sieves (50 g), sodium acetate (24.1 g, 283 mmol) and anhydrous methanol (700 mL) are stirred at room temperature under N₂ overnight. The mixture is cooled to 0°C and sodium borohydride (10.9 g, 288 mmol) is added portionwise over 1 hour. The mixture is stirred at room temperature for 3 hours. The mixture is filtered through celite, washed with methanol, and concentrated *in vacuo* to give a residue which is diluted with dichloromethane and washed with 10% aqueous sodium hydroxide. The organic phases are washed with brine, dried over anhydrous magnesium sulfate, filtered and concentrated in vacuo to give the title compound as a pale yellow oil (56.3 g, MH⁺ = 216).

### Preparative Example 17. 2(R)-[[[3-(1H-imidazol-1-yl)propyl](phenylmethyl)amino]carbonyl]piperazine

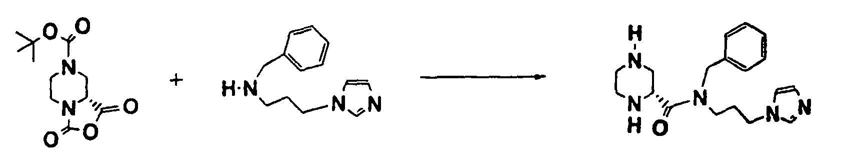

A mixture of the title compound from Preparative Example 16 (1.34 g, 6.2 mmol), the title compound from Preparative Example 13 (1.6 g, 6.2 mmol), triethyl amine (1.3 mL, 9.3 mmol) and anhydrous CH₂Cl₂ (10 mL) are stirred at room temperature for 48 hrs Trifluoroacetic acid (10 mL) is added and the resulting mixture is stirred for an additional 1.5 hrs. Aqueous NaOH (1N) is added dropwise to neutralize the reaction mixture and the resulting mixture is extracted with CH₂Cl₂. The organic phase is dried over anhydrous MgSO₄, filtered and concentrated *in vacuo* to give a residue which is purified by flash column chromatography (silica gel) using 1 % MeOH-99% CH₂Cl₂ saturated with aqueous ammonium hydroxide to give the title compound as an oil (520 mg, MH⁺ = 328).

### Preparative Example 18. Ethyl 4-piperidinyl acetate

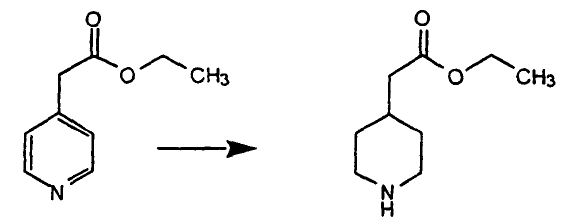

Ethyl 4-pyridyl acetate (4.5g, 27.24mmoles) is placed in a 500mL Parr bottle and dissolved in anhydrous EtOH (70mL). To the bottle is added 10% Palladium on charcoal (1 0 g). The bottle is put on a hydrogenator and the contents shaken under 55 psi hydrogen pressure at 25°C for 94h. The mixture is filtered through Celite® and washed with 4x40mL anhydrous EtOH. The filtrate is rotovapped down and the residue chromatographed on silica gel using 3% (10% conc. NH₄OH in methanol)dichloromethane as the eluant to give 2 944g of the title compound.

### Preparative Example 19. Ethyl 1-aminocarbonyl-4-piperidinyl acetate

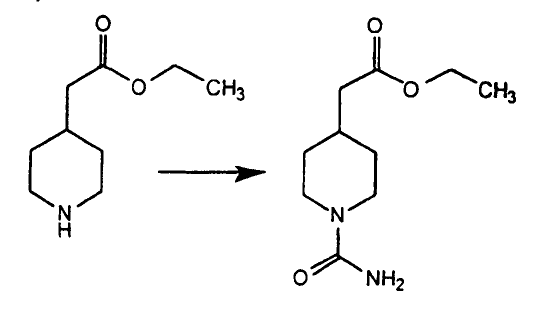

Ethyl 4-piperidinyl acetate (500mg; 2.92mmoles) is dissolved in anhydrous CH₂Cl₂ (25mL) To the stirring solution is added tnmethylsilyl isocyanate (5.9mL; 43.8mmoles) and the solution is stirred at 25°C for 17h. The solution is worked up in CH₂Cl₂-saturated NaHCO₃ and the product chromatographed on silica gel using 2→3%(conc. NH₄OH in methanol)dichloromethane as the eluant to give 622mg of the title compound.

### Preparative Example 20. 1-Aminocarbonyl-4-piperidinylacetic acid

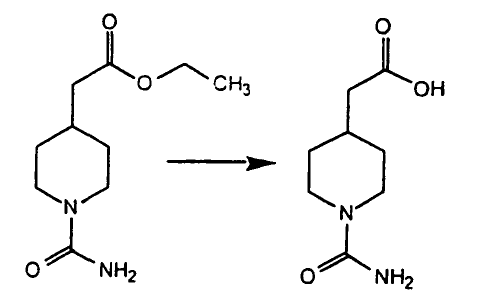

Ethyl 1-aminocarbonyl-4-piperidinyl acetate (153.6mg, 0.717mmoles) is dissolved in anhydrous CH₂Cl₂ (3.58mL) and EtOH (3.58mL). To the solution is added 1 0M LiOH (1.73mL, 1.73mmoles) and the mixture is stirred at 50°C for 5.5h. The mixture is cooled quickly to 25°C and 1.0N HCl (2.02mL, 2.02mmoles) is added and the mixture stirred for 5 minutes and then rotovapped to dryness to give the title compound which is used without further purification.

### Preparative Example 21.

### Step A:

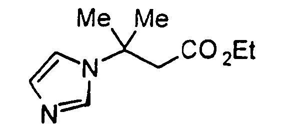

Ethyl 2,2-dimethyl acrylate (50.0g, 2.0 eq.) is stirred with imidazole (13.28g, 200 mmol) at 90° for 48 hours. The resulting solution is cooled, diluted with water (150 mL) and CH₂Cl₂ (150 mL) and separated. The aqueous layer is washed with CH₂Cl₂ (2 x 75 mL) and the combined organics are dried over Na₂SO₄ and concentrated *in vacuo*. The crude mixture is purified by flash chromatography using a 10% MeOH in CH₂Cl₂ solution as eluent to give pure product as a clear oil (11.27g).

### Step B:

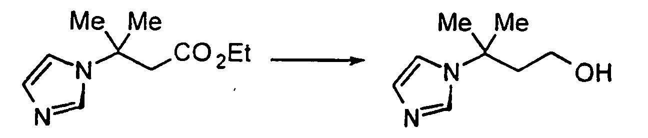

A solution of the title compound from Step A (10.0g, 50.96 mmol) is treated with LiAlH₄ (51 mL, 1 M solution in ether, 1.0 eq.). The reaction mixture is stirred one hour at room temperature before quenching by the dropwise addition of saturated Na₂SO₄ (~3.0 mL). The resulting slurry is dried with Na₂SO₄ (solid), diluted with EtOAc (100 mL) and filtered through a plug of Celite. The filtrate is concentrated to give a yellow oil (6.87, 87% yield) which is used without further purification.

### Step C:

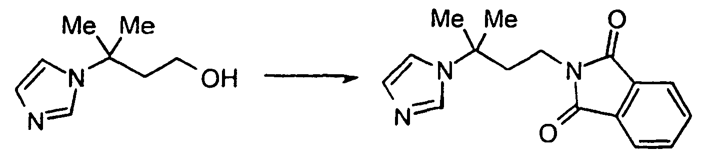

To a solution of the title compound Step B (6 85g, 44.42 mmol), phthalimide (7.19g, 1.1 eq.), and tnphenylphosphorous (Ph₃P) (12.82g, 1.1 eq.) in THF (200 mL) at 0° C is added DEAD(7.69 mL, 1.1 eq.) over 10 minutes. The resulting solution is warmed to room temperature and stirred 48 hours. The reaction mixture is concentrated under reduced pressure and the product isolated by crystallization from CH₂Cl₂/Et₂O to give a white solid (10.03 g).

### Step D:

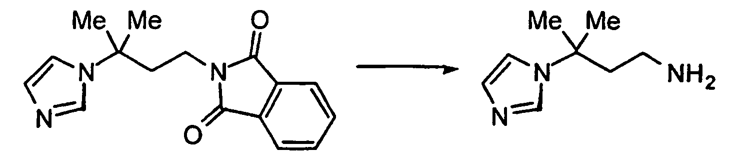

A solution of the title compound from Step C (9.50g, 33.53 mmol) and (N₂H₄) (1.25 mL, 1.2 eq.) in EtOH (100 mL) is heated at reflux 4 hours. The resulting slurry is cooled, filtered, and the filtrate concentrated under reduced pressure. The crude product is purified by flash chromatography using a 15% (10% NH₄OH in MeOH) solution in CH₂Cl₂ as eluent to give a pale yellow oil (2.80g).

### Preparative Example 22.

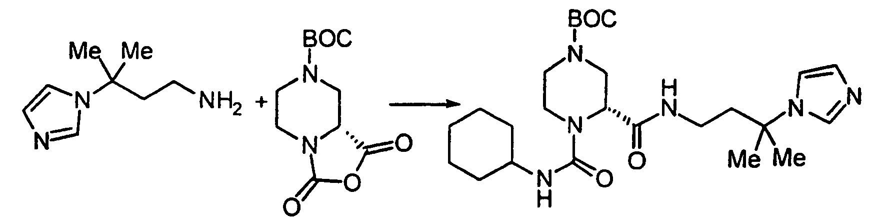

Piperazine anhydride (0.28g, 1.0 eq.) is added portionwise to a solution of the title compound from Step D of Preparative Example 21 (0.17g, 1.2 mmol) in CH₂Cl₂ (5.0 mL) and the resulting solution is stirred 10 minutes at room temperature before adding cyclohexyl isocyanate (0.21 mL, 1.5 eq.). After stirring at room temperature 15 minutes, the reaction mixture is quenched by the addition of MeOH (1 mL), concentrated *in vacuo*, and purified by flash chromatography using a 10% MeOH in CH₂Cl₂ solution as eluent to yield a white solid (0.46g), mp= 71-74 °C.

### Preparative Example 23.

### Step A:

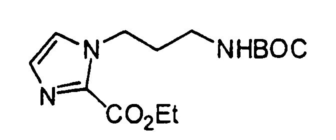

n-BuLi (2.79 mL; 1.75M in hexanes; 2.2 eq.) is added dropwise to a solution of N-BOC aminopropylimidazole (0.50 g, 2.22 mmol) in THF (15 mL) at -78 °C. The resulting solution is warmed to -20 °C and stirred two hours before adding TMSCI (0.65 mL, 2.3 eq.). The reaction mixture is warmed to room temperature, stirred one hour, recooled to -78 °C and treated with ClCO₂Et (0.25g, 1.2 eq.) The resulting solution is warmed to room temperature and stirred 60 hours. The reaction is quenched by the addition of water (10 mL) and extracted with CH₂Cl₂ (3 X 20 mL). The combined organics are dried over Na₂SO₄, filtered, and concentrated in *vacuo*. The crude product is purified by flash chromatography using a 5% MeOH in CH₂Cl₂ solution as eluent (0.35g, 53% yield).

### Step B:

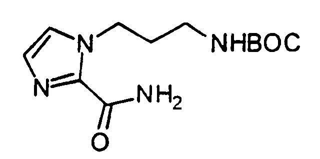

A solution of the title compound from Step A (0.35g, 1.18 mmol) is stirred in EtOH (10 mL) and 30% NH₄OH (2 mL) for 24 hours. The reaction mixture is concentrated under reduced pressure and purified by flash chromatography using neat EtOAc as eluent (0.23g).

### Preparative Example 24.

### Step A:

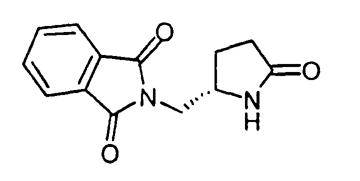

By using essentially the same procedure set forth in Step C of Preparative Example 21 except using S-(+)-5-hydroxymethyl-2-pyrrolidinone (1.0g, 8.69 mmol) in place of the title compound is prepared (1.50g).

### Step B:

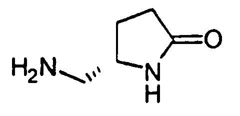

By using essentially the same procedure as set forth in Step D of Preparative Example 21 except using the title compound from Step A of Preparative Example 24 (1.50g, 6.14 mmol), the title compound is prepared (0.59 g).

### Preparative Example 25.

### Step A:

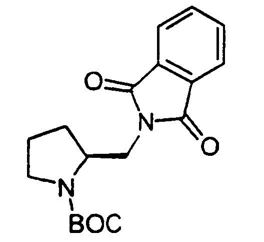

By essentially the same procedure set forth in Step C of Preparative Example 21 except using N-BOC-S-prolinol (0.50g, 2.48 mmol), the title compound is prepared (0.59 g).

### Step B:

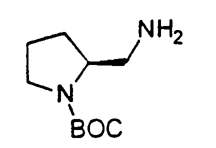

By essentially the same procedure set forth in Step D of Preparative Example 21 except using the title compound from Step A of Preparative Example 25 (0.59 g, 1.79 mmol), the title compound is prepared (0.36g).

### Step C:

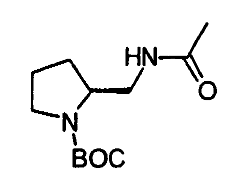

Ac₂O (0.20 mL, 1.2 eq.) is added to the title compound from Step B Preparative Example 25 (0.36 g, 1.80 mmol) and TEA (0.30 mL, 1.2 eq.) in CH₂Cl₂ (8.0 mL). The resulting solution is stirred 2 hours and quenched by the addition of saturated NaHCO₃ (10 mL). The resulting solution is extracted with CH₂Cl₂ (2 X 20 mL), dried over Na₂SO₄, and concentrated in *vacuo*. The crude product is purified by flash chromatography eluting first with neat EtOAc followed by 5% MeOH in EtOAc (0.23g).

### ASSAYS

1. In vitro enzyme assays: FPT IC₅₀ (inhibition of farnesyl protein transferase, in vitro enzyme assay) are determined by the methods disclosed in WO/10515 or WO 95/10516. The data demonstrate that the compounds of the invention are inhibitors of Ras-CVLS famesylation by partially purified rat brain farnesyl protein transferase (FPT). The data also show that there are compounds of the invention which can be considered as potent (IC₅₀ <10 µM) inhibitors of Ras-CVLS famesylation by partially purified rat brain FPT.

Compounds of Examples 1-3, 5-28, 28A-28X, 28Y1, 28Y2, 28Z, 28Z1, 28Z2, 29-57, 57A, 58-62, and 64-67 had an FPT IC₅₀ within the range of 0.18nM to 4000nM (nM represents nanomolar).

Compounds of Examples 1, 5, 6, 8-17, 20-28, 28C, 28E, 28F, 28G, 28I, 28M, 280, 28P, 28Q, 28R, 28S, 28T, 28V, 28Y1, 28Y2, 29, 31, 32, 34, 36-38, 42, 43, 47, 53-56, 58, 61 and 64 had an FPT IC₅₀ within the range of 0.18nM to 21nM.

Compounds 20, 25, 26, 27, 28, 280, 54 and 55 had an FPT IC₅₀ within the range of 0.18nM to 1.5nM.

Additional assays can be earned out by following essentially the same procedure as described above, but with substitution of alternative indicator tumor cell lines in place of the T24-BAG cells. The assays can be conducted using either DLD-1-BAG human colon carcinoma cells expressing an activated K-ras gene or SW620-BAG human colon carcinoma cells expressing an activated K-ras gene. Using other tumor cell lines known in the art, the activity of the compounds of this invention against other types of cancer cells could be demonstrated.

### Soft Agar Assay:

Anchorage-independent growth is a characteristic of tumorigenic cell lines. Human tumor cells can be suspended in growth medium containing 0.3% agarose and an indicated concentration of a farnesyl transferase inhibitor. The solution can be overlayed onto growth medium solidified with 0.6% agarose containing the same concentration of farnesyl transferase inhibitor as the top layer. After the top layer is solidified, plates can be incubated for 10-16 days at 37°C under 5% CO₂ to allow colony outgrowth. After incubation, the colonies can be stained by overlaying the agar with a solution of MTT (3-[4,5-dimethyl-thiazol-2-yl]-2,5-diphenyltetrazolium bromide, Thiazolyl blue) (1 mg/mL in PBS). Colonies can be counted and the IC₅₀'s can be determined.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation may be varied or adjusted from about 0.01 mg to about 1000 mg, preferably from about 0.01 mg to about 750 mg, more preferably from about 0.01 mg to about 500 mg and most preferably from about 0.01 mg to about 250 mg, according to the particular application.

The actual dosage employed may be varied depending upon the requirements of the patient and the seventy of the condition being treated. Determination of the proper dosage regimen for a particular situation is within the skill of the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 0.04 mg/day to about 4000 mg/day, in two to four divided doses.

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

1. A compound selected from the group consisting of:
the compound of 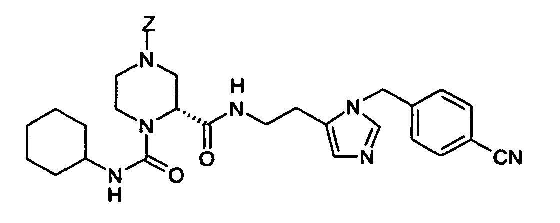 wherein Z is selected from: 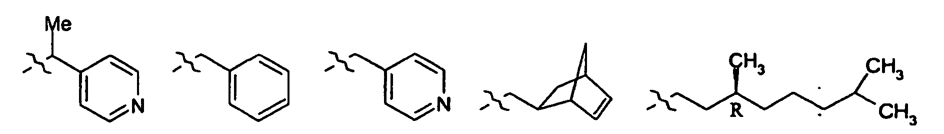 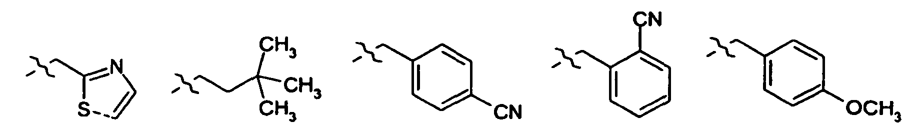 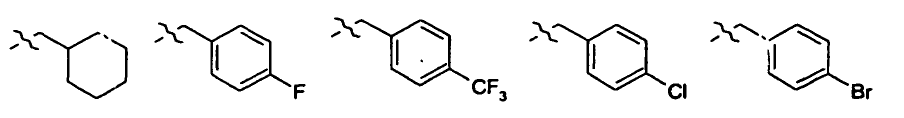 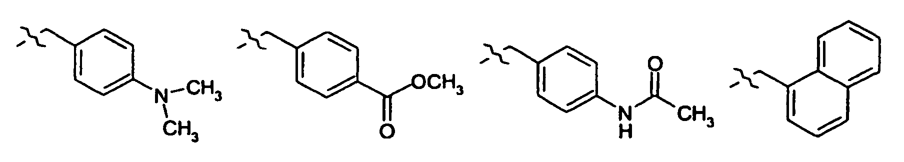 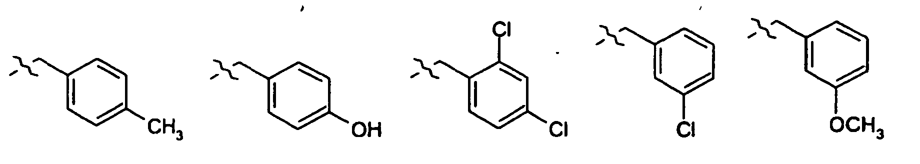 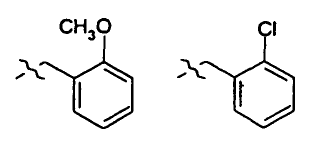 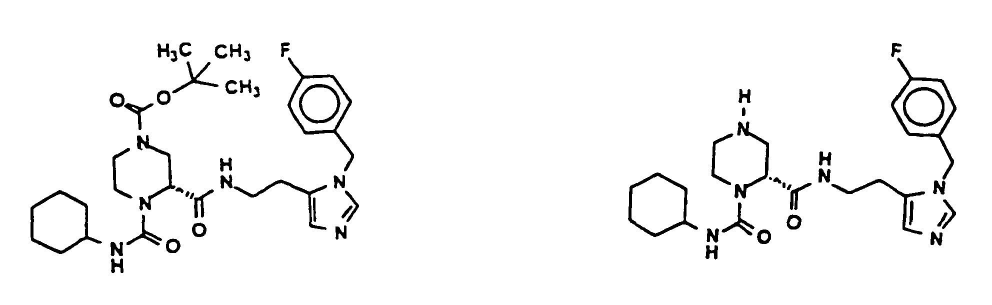 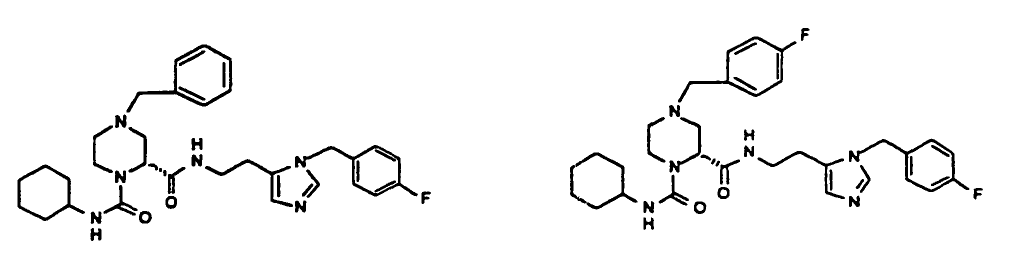 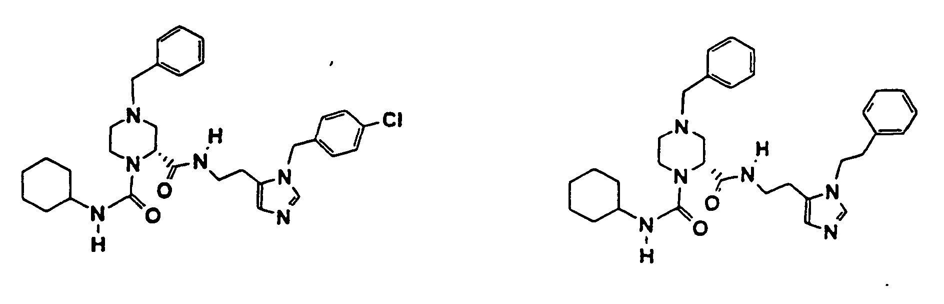
the compound of 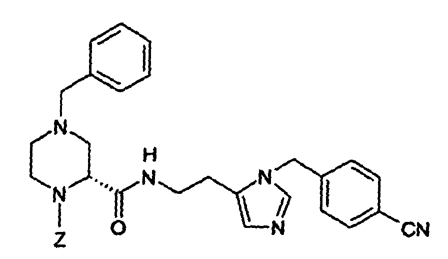 wherein Z is selected from: 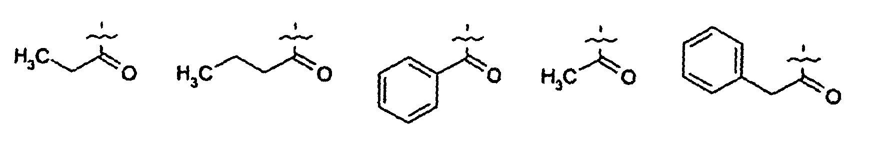 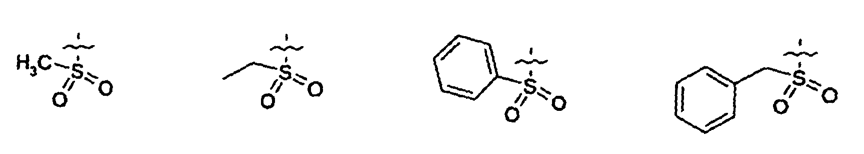 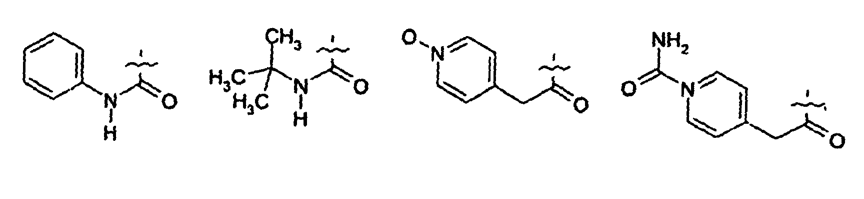 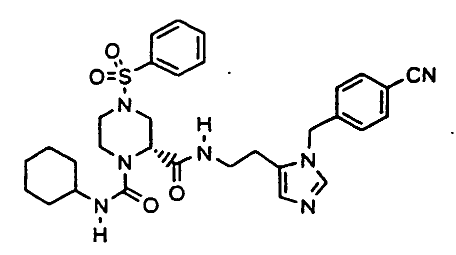 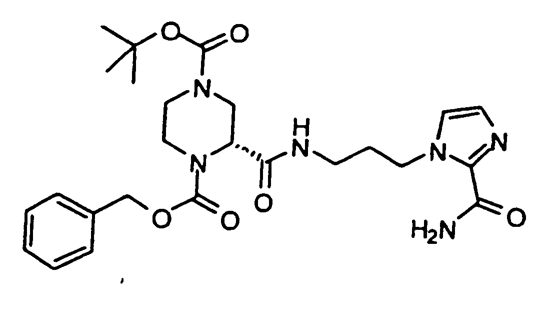 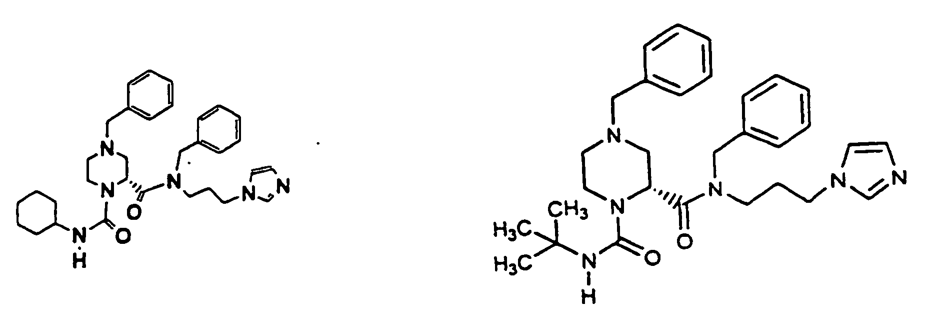 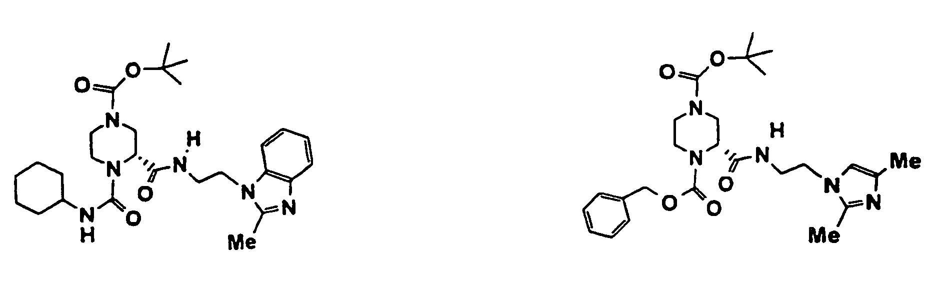 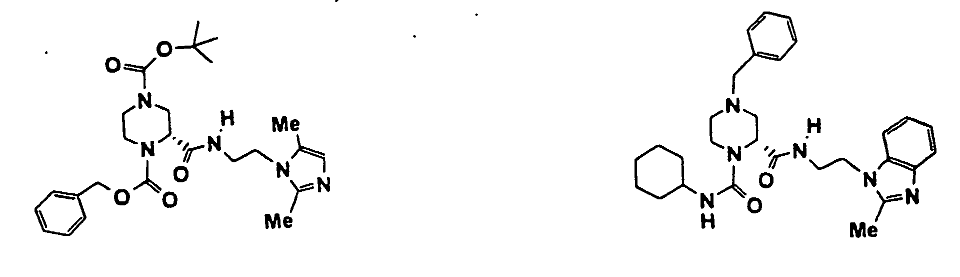 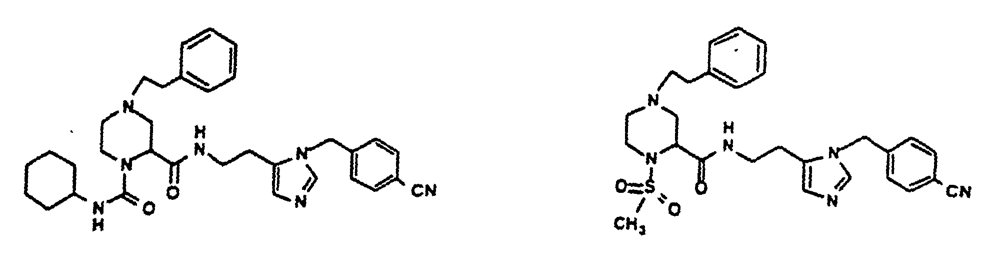 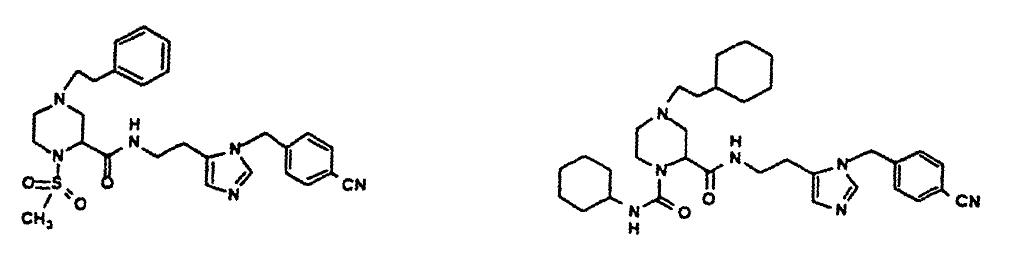 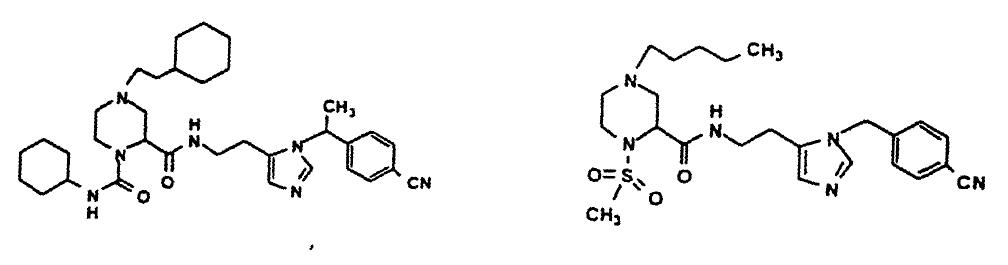 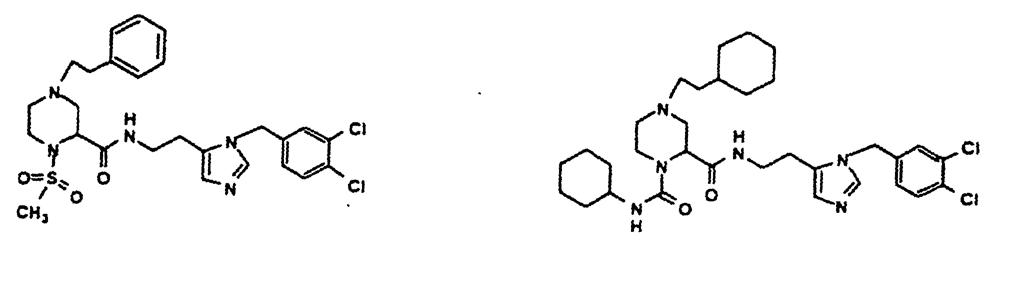 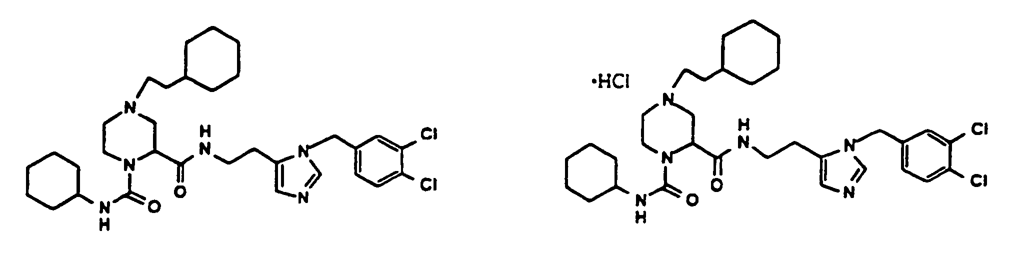 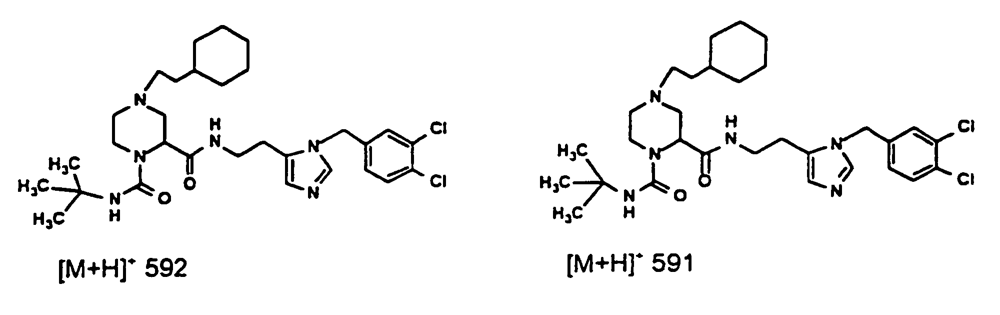 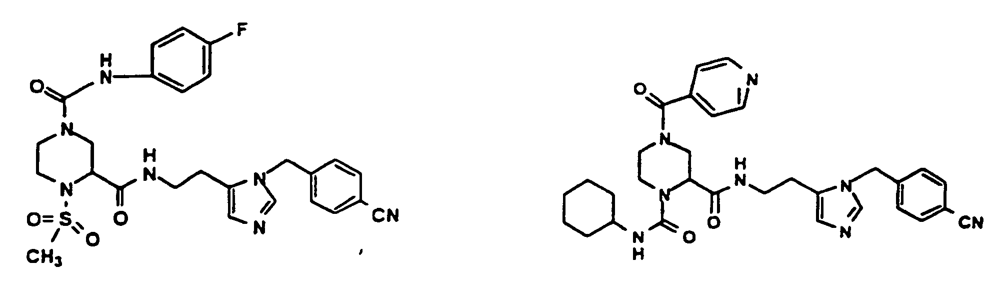 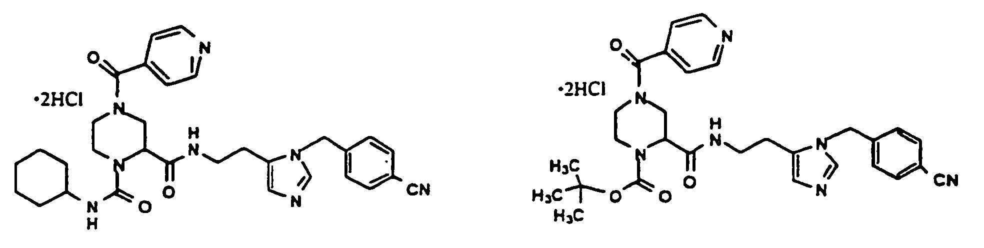 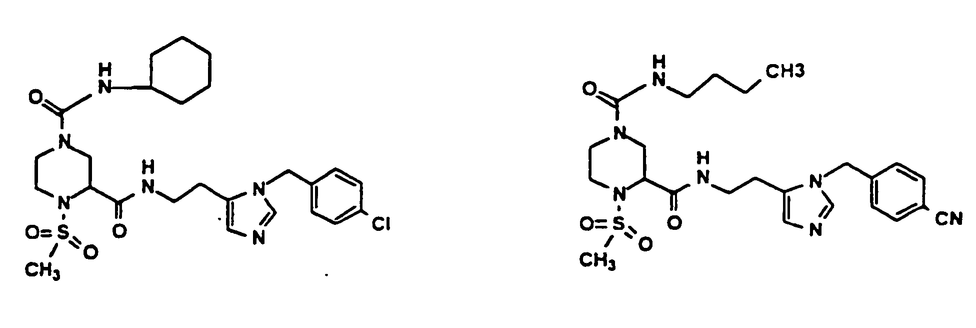 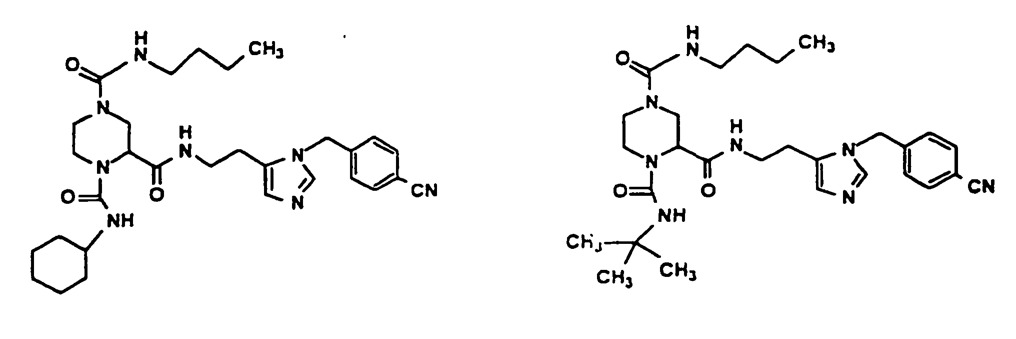 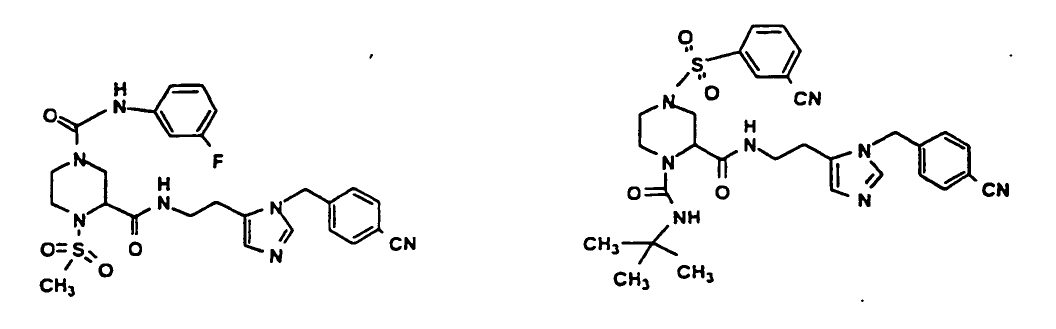 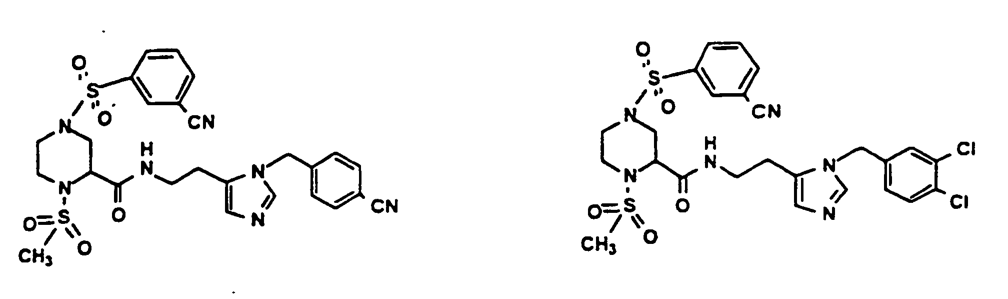 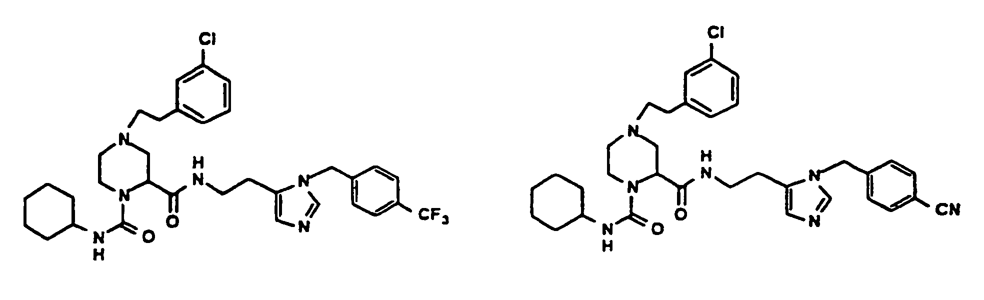 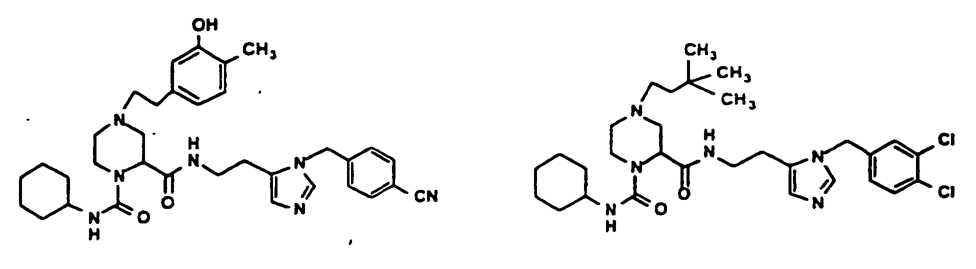 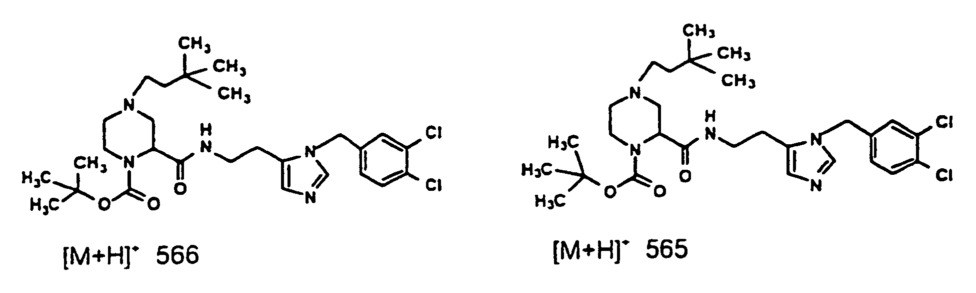 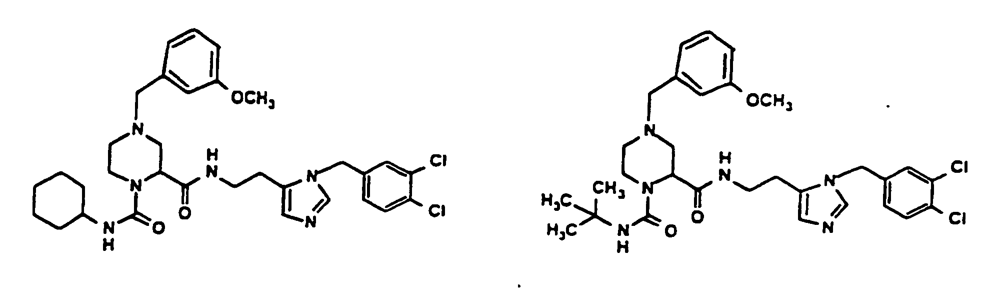 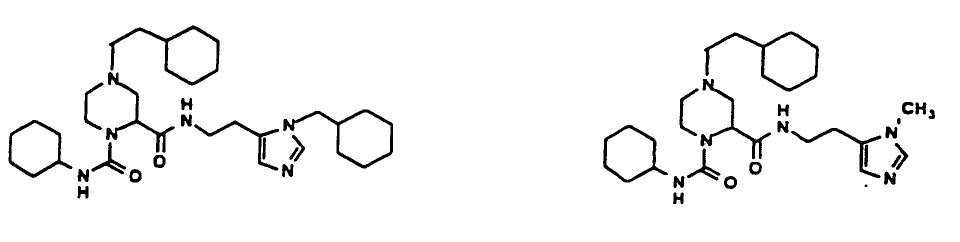 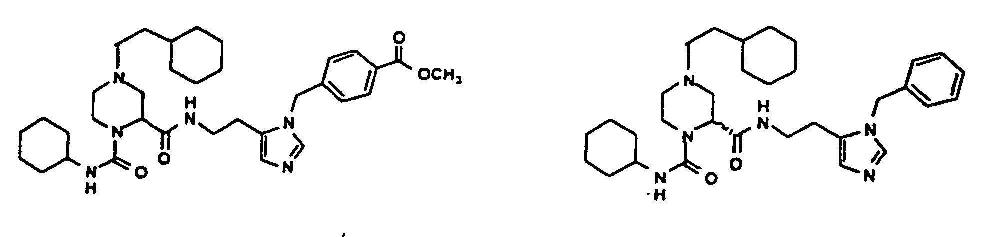 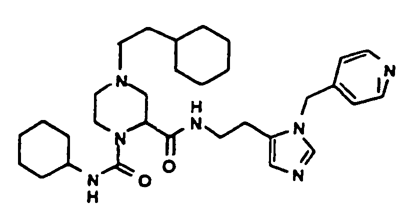
the compound of 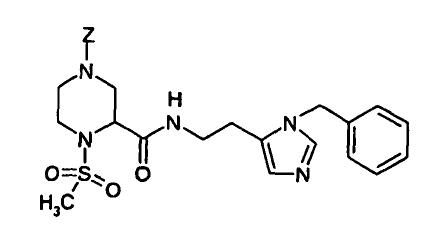 wherein Z is selected from 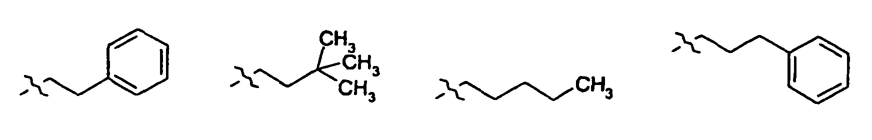 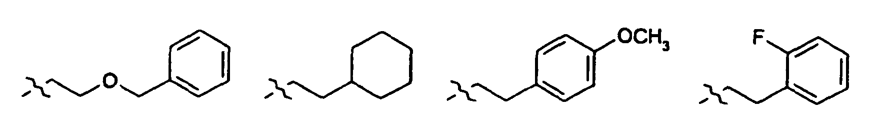 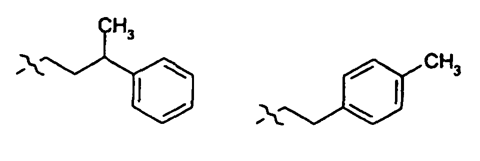
the compound of 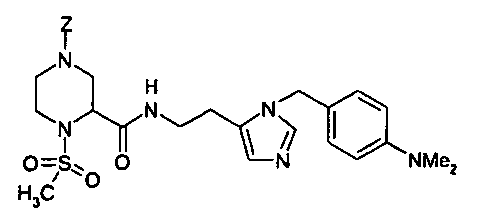 wherein Z is selected from 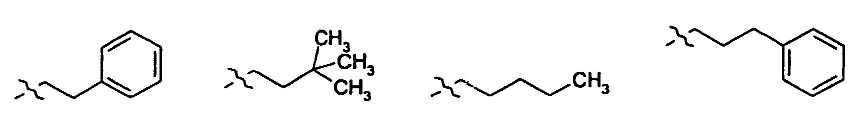 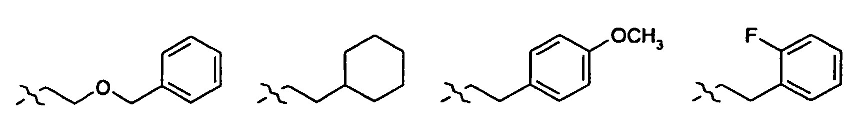 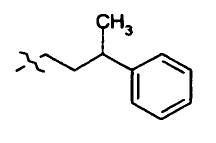
the compound of 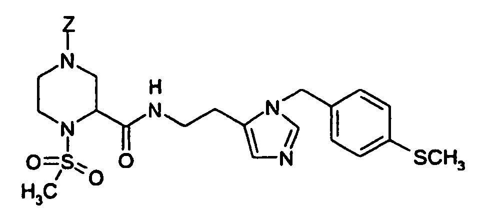 wherein Z is selected from 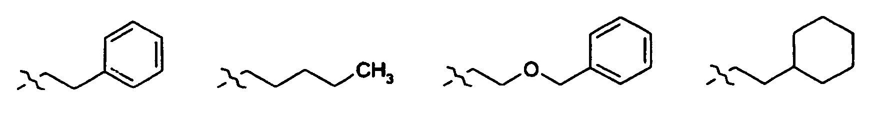 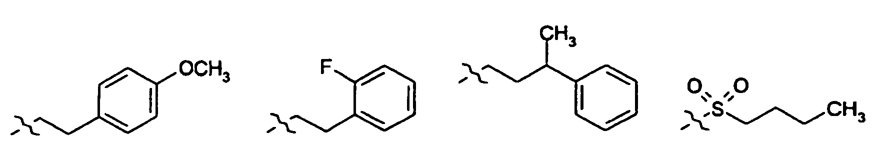 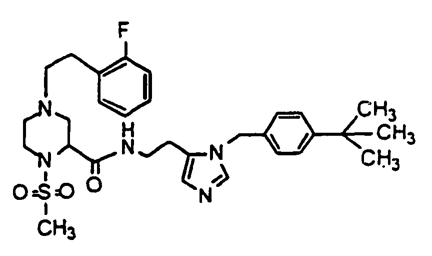
the compound of 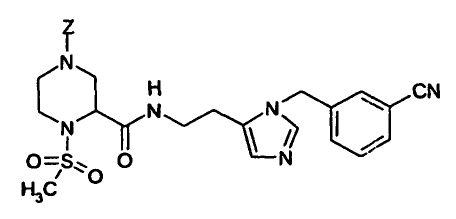 wherein Z is selected from 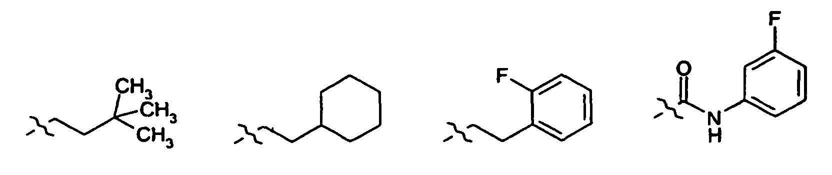 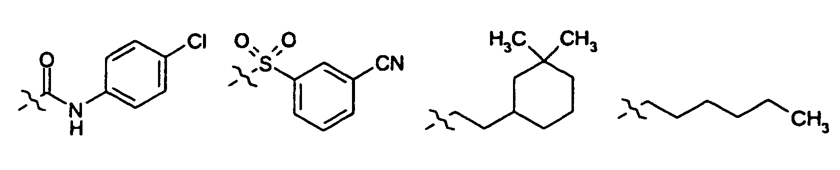
the compound of 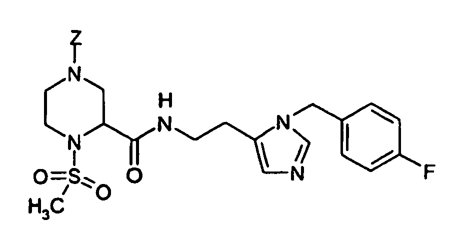 wherein Z is selected from 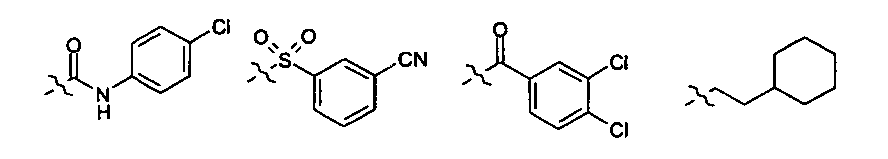 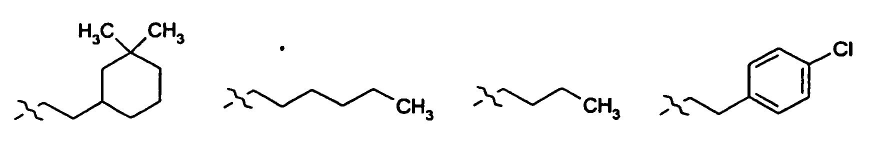 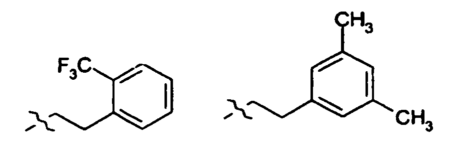 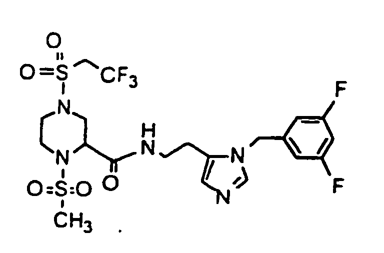 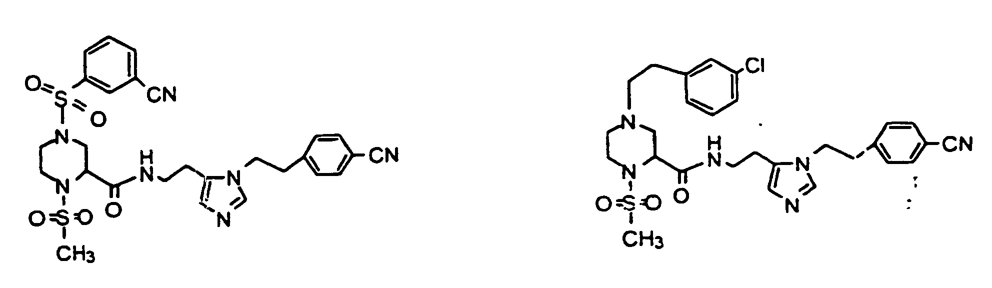
the compound of 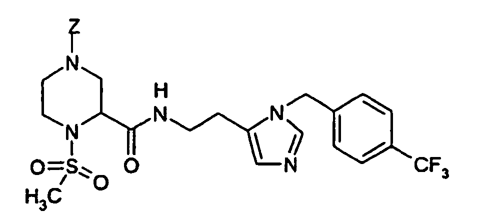 wherein Z is selected from 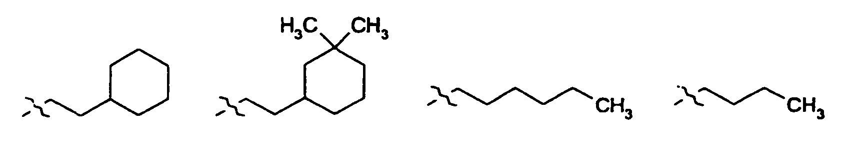 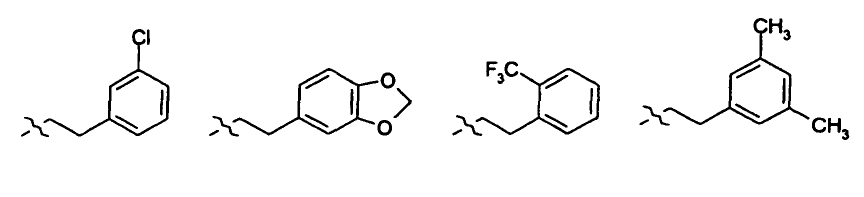
the compound of 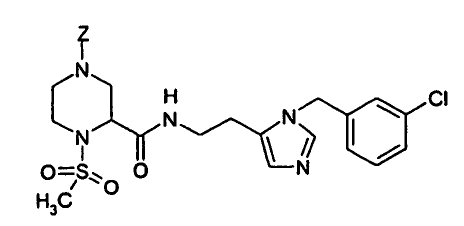 wherein Z is selected from 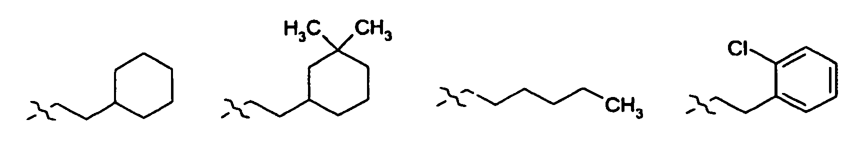 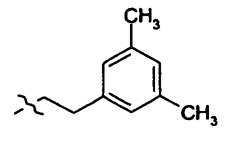
the compound of 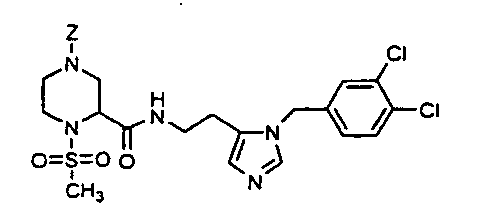 wherein Z is selected from: 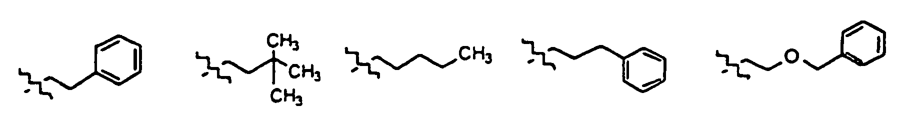 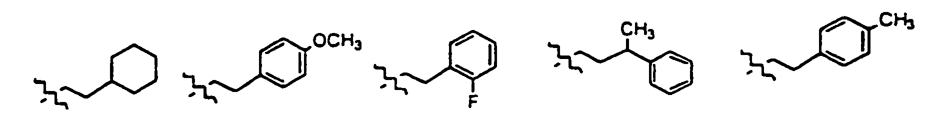 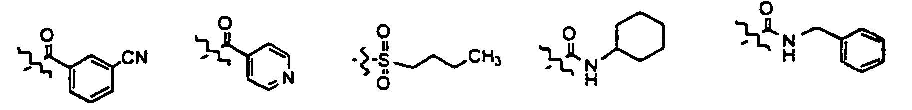 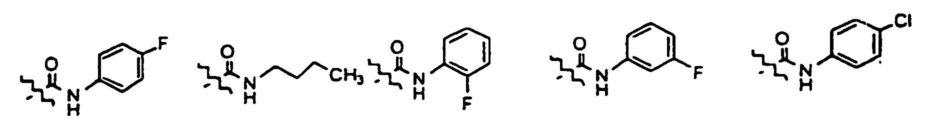 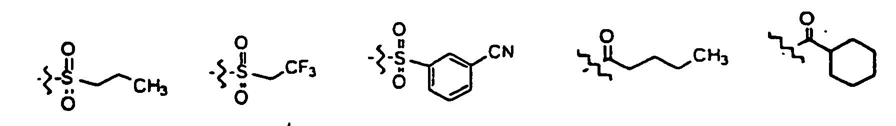 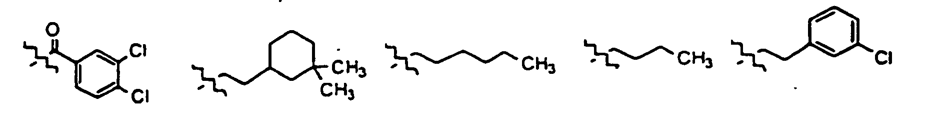 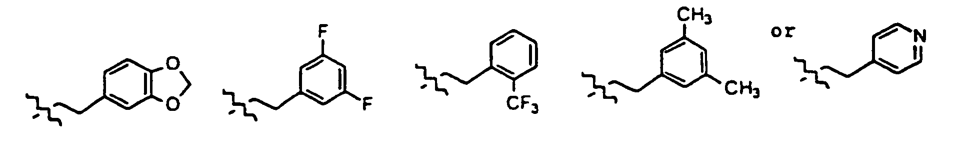
the compound of 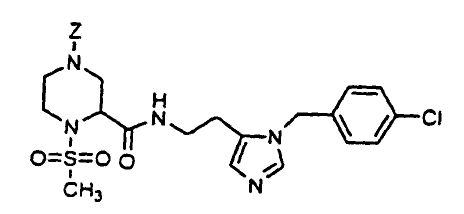 wherein Z is selected from: 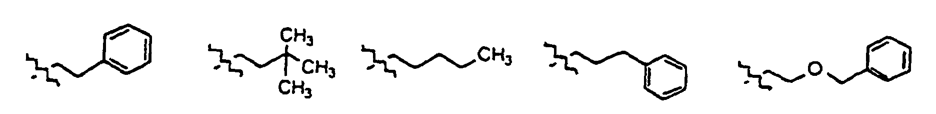 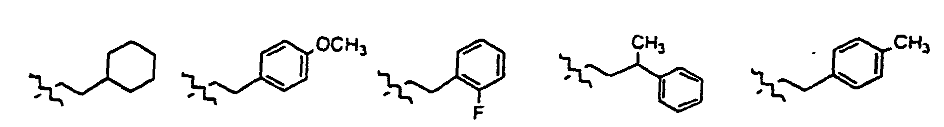 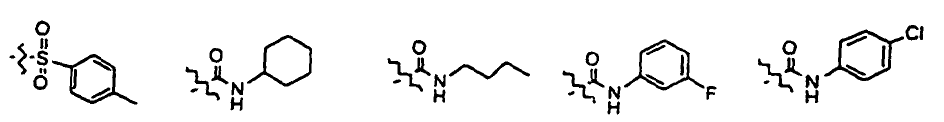 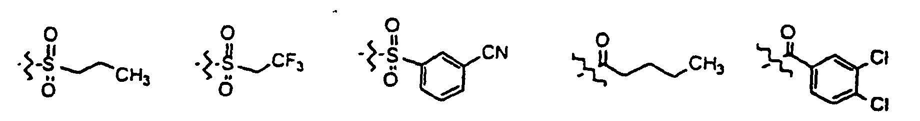 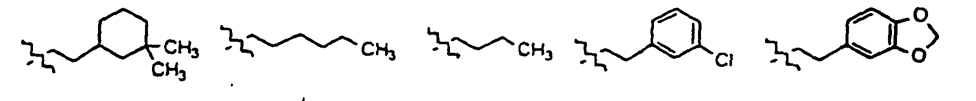 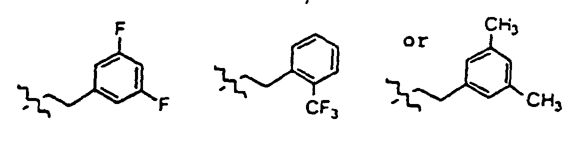
the compound of 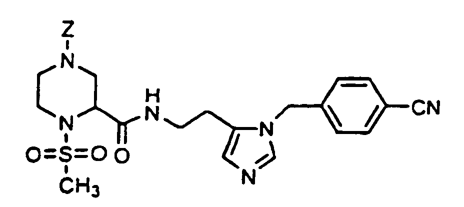 wherein Z is selected from:

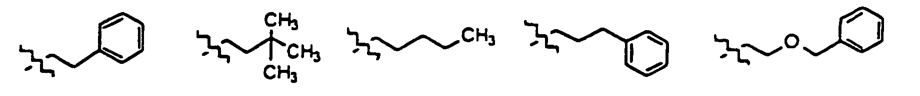

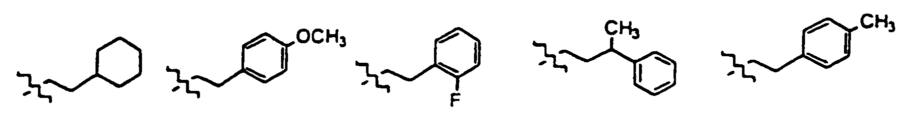

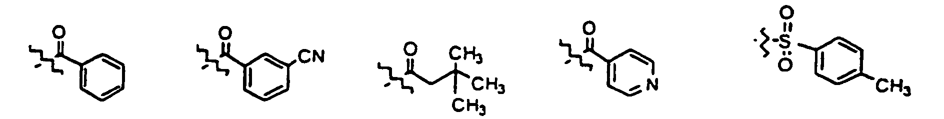

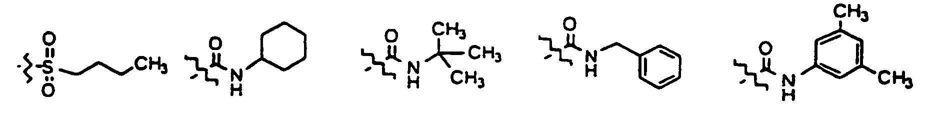

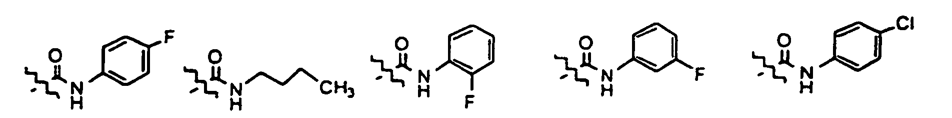

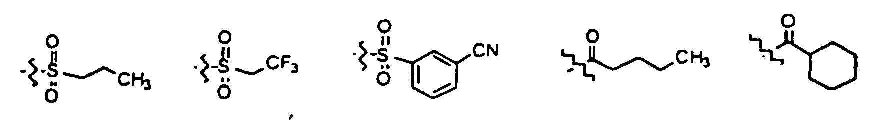

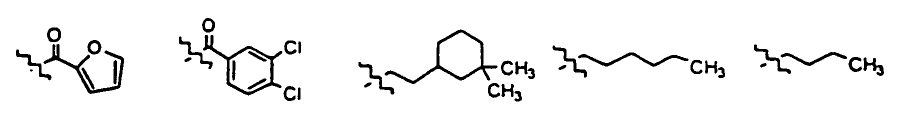

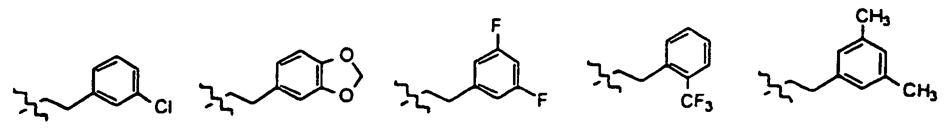

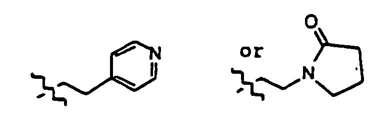

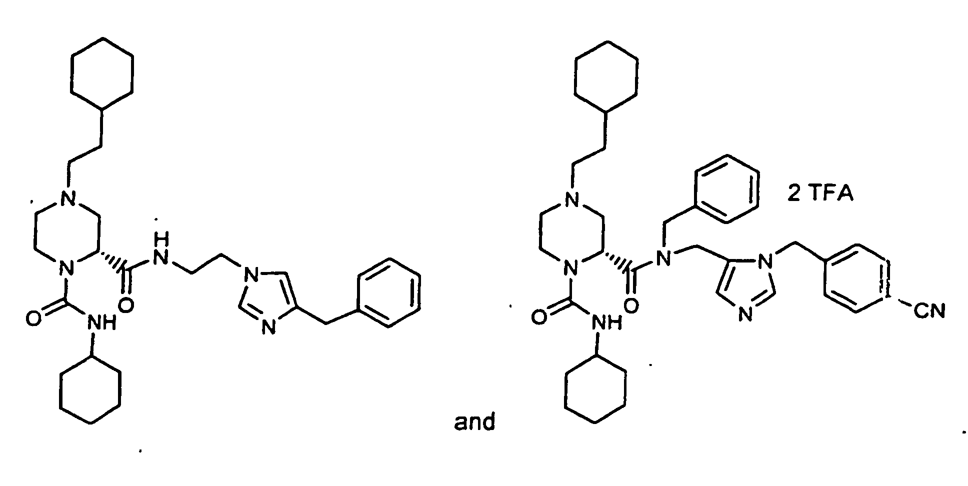

2. A compound selected from the group consisting of: 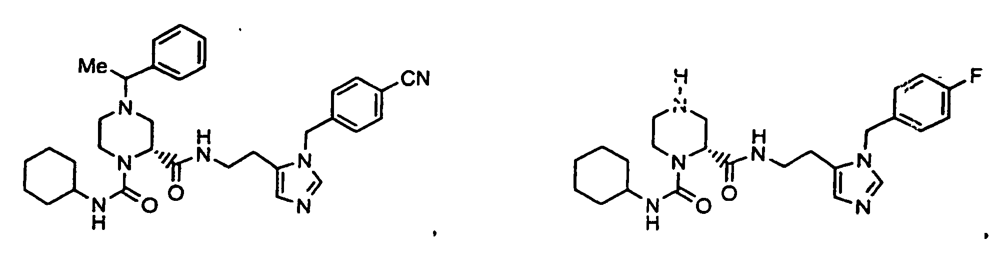 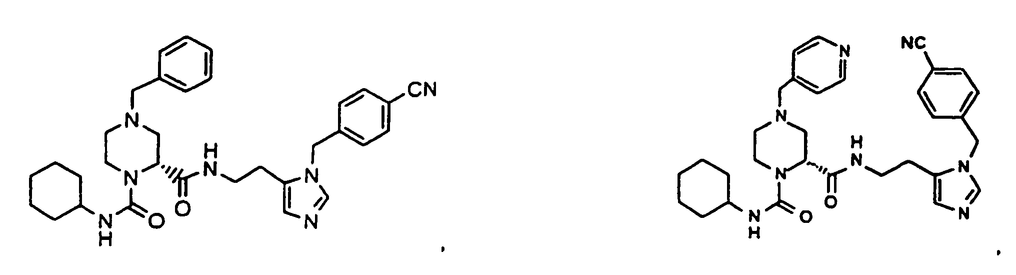 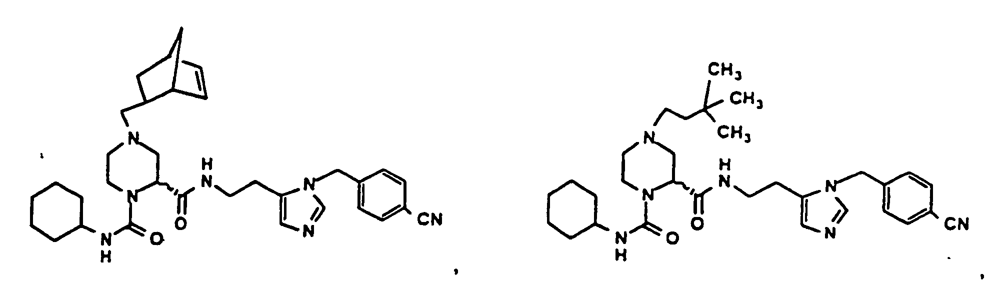 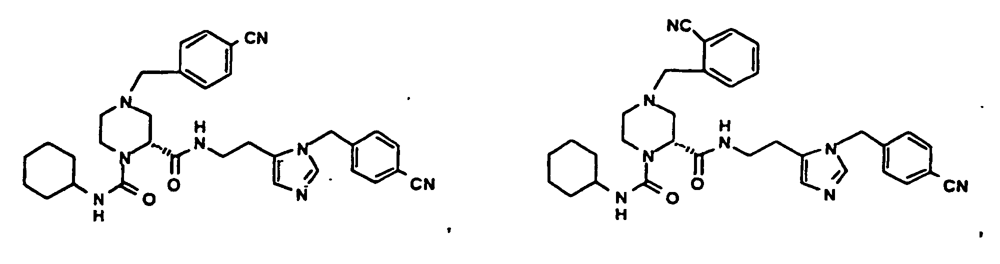 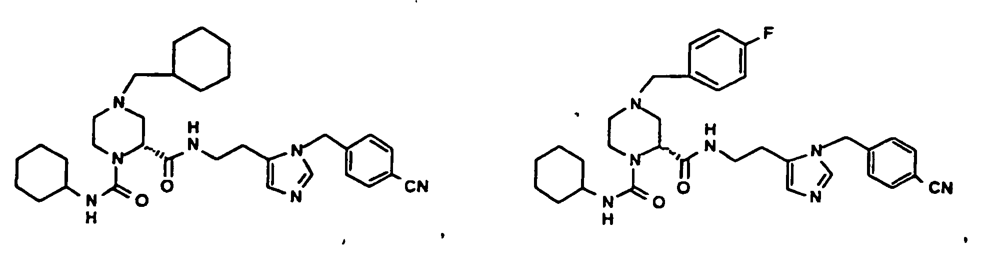 and

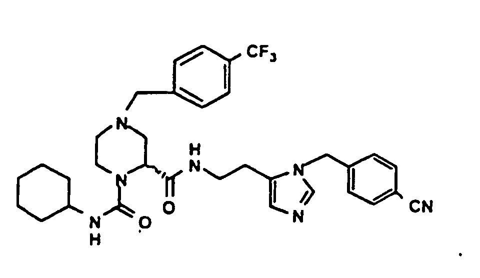

3. A pharmaceutical composition comprising an effictive amount of a compound of any of Claims 1 to 2 in combination with a pharmaceutically acceptable carrier.

4. The use of a compound of any of Claims 1 to 2 for the manufacture of a medicament for inhibiting the abnormal growth of cells.

5. The use of Claim 4 wherein the cells inhibited are tumor cells.

6. The use of Claim 4 wherein the cells inhibited are pancreatic tumor cells, lung cancer cells, myeloid leukemia tumor cells, thyroid follicular tumor cells, myelodysplastic tumor cells, epidermal carcinoma tumor cells, bladder carcinoma tumor cells or prostate tumor cells, melanoma tumor cells, breast tumor cells or colon tumor cells.

7. The use of Claim 4 wherein the inhibition of the abnormal growth of cells occurs by the inhibition of ras famesyl protein transferase.

8. The use of Claim 4 wherein the inhibition is of tumor cells wherein the Ras protein is activated as a result of oncogenic mutation in genes other than the Ras gene.

9. The use of a compound of any of Claims 1 to 2 for the manufacture of a medicament for the inhibition of pancreatic tumor cells, lung cancer cells, myeloid leukemia tumor cells, thyroid follicular tumor cells, myelodysplastic tumor cells, epidermal carcinoma tumor cells, bladder carcinoma tumor cells or prostate tumor cells, melanoma tumor cells, breast tumor cells or colon tumor cells.

10. The use of a compound of any of Claims 1 to 2 for the manufacture of a medicament for the inhibition of ras famesyl protein transferase.

## Patentansprüche

1. Verbindung ausgewahlt aus der Gruppe bestehend aus:
der Verbindung 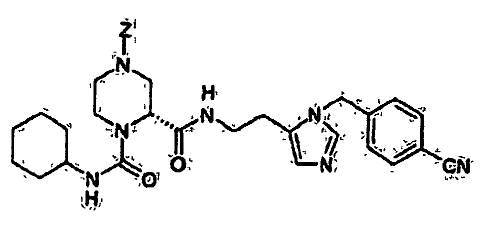 worin Z ausgewahlt ist aus: 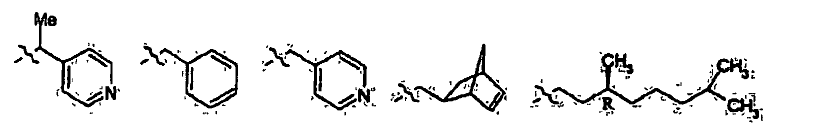 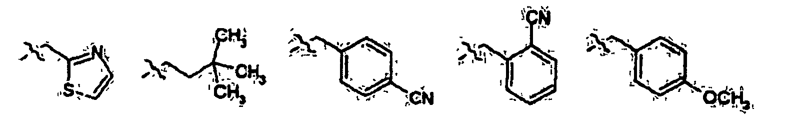 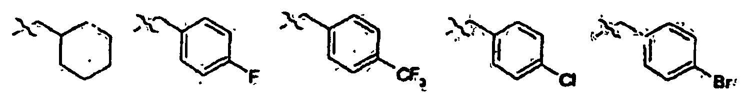 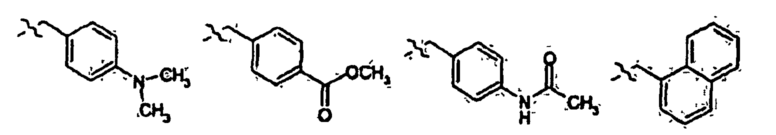 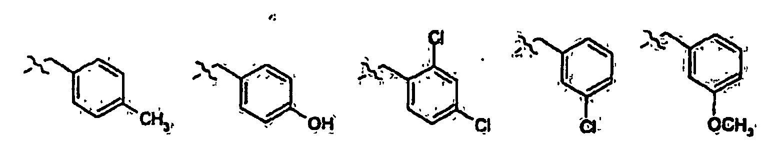 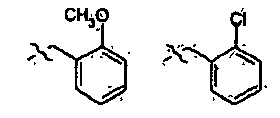 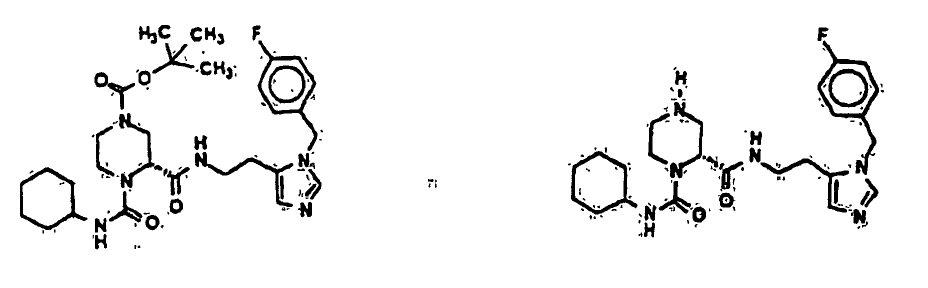 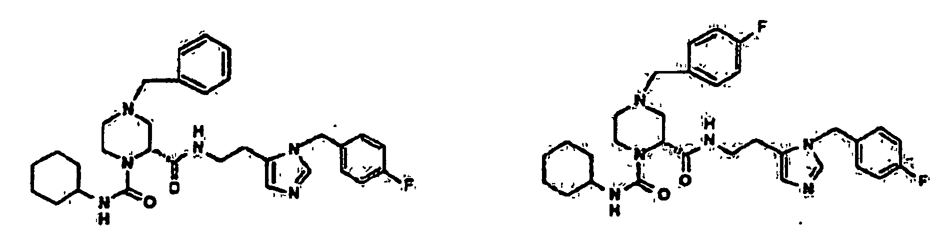 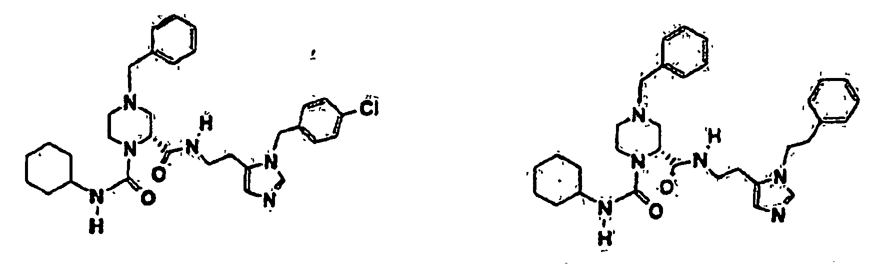
der Verbindung 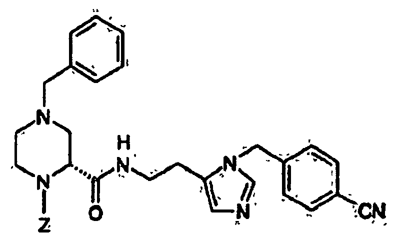 worin Z ausgewählt ist aus: 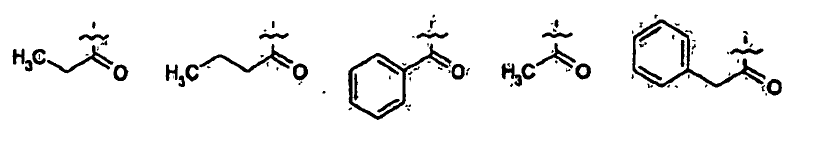 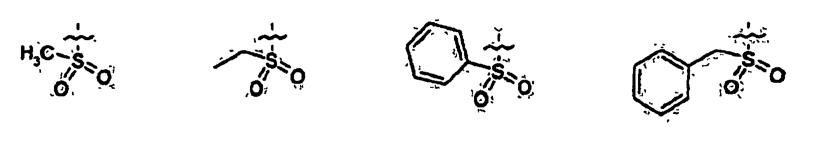 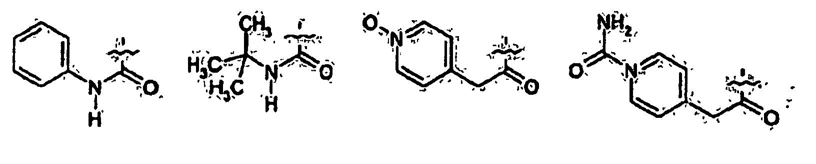 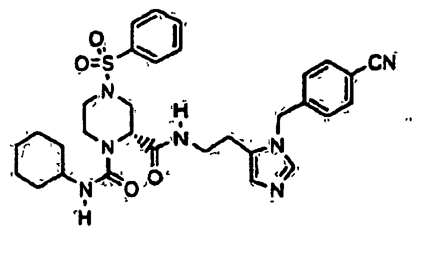 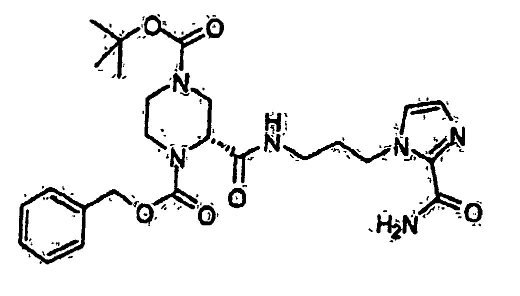 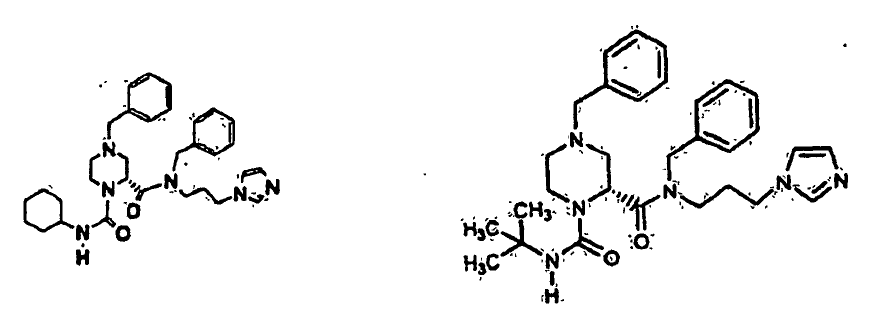 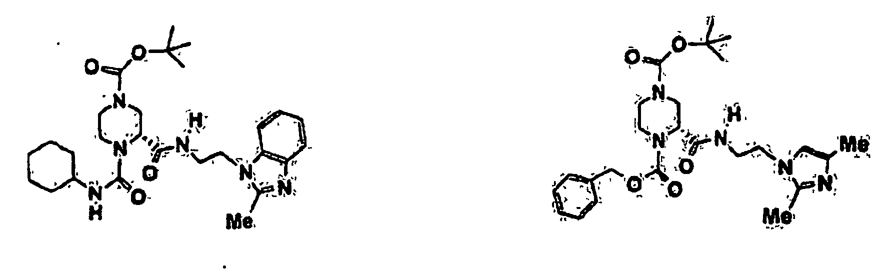 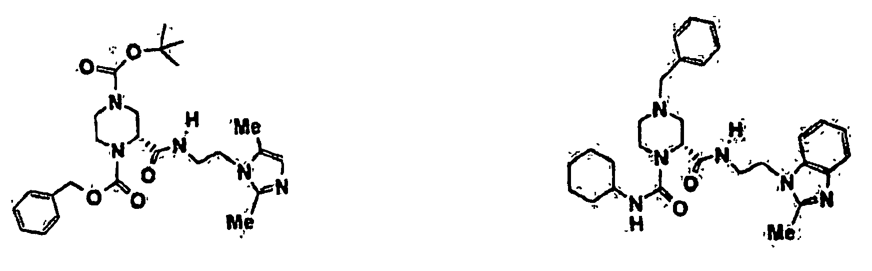 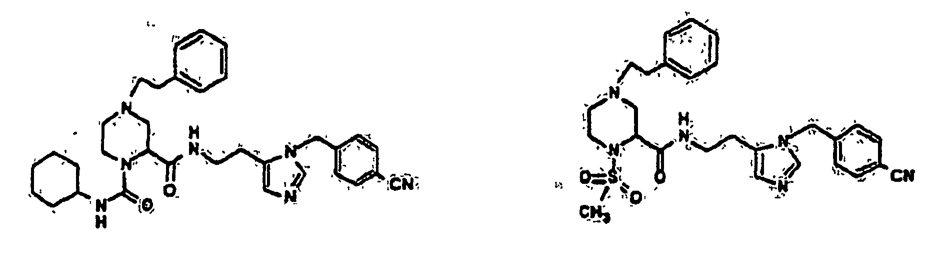 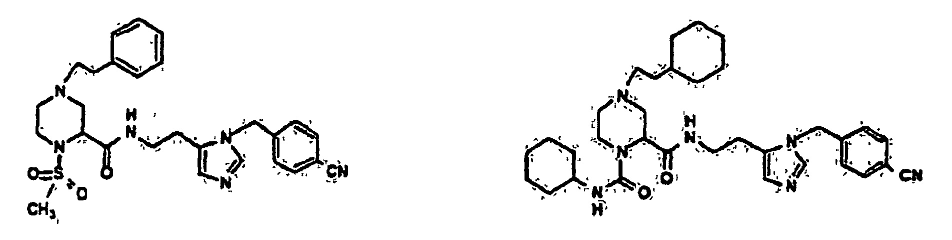 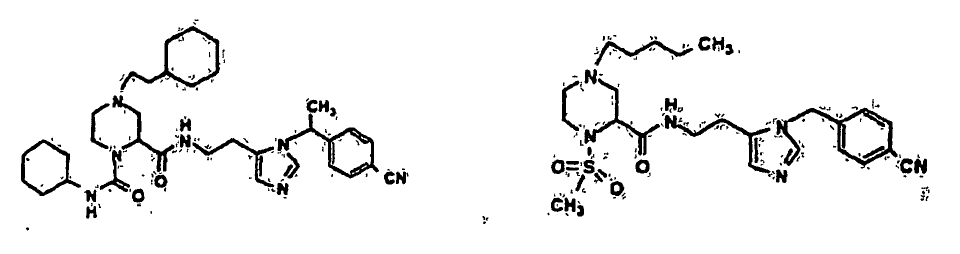 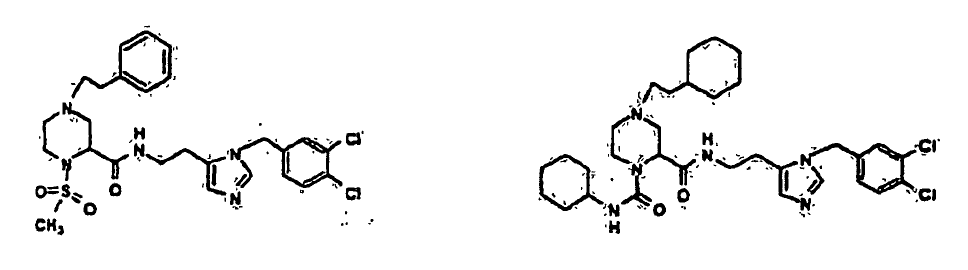 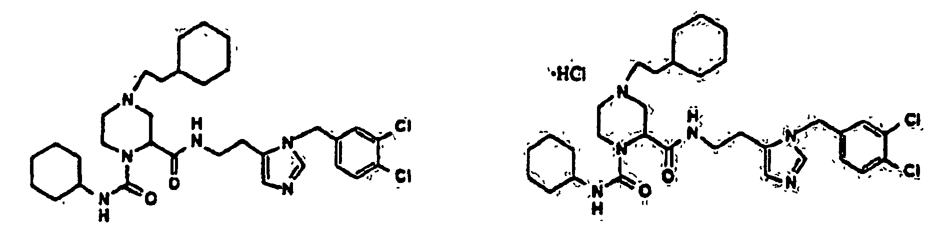 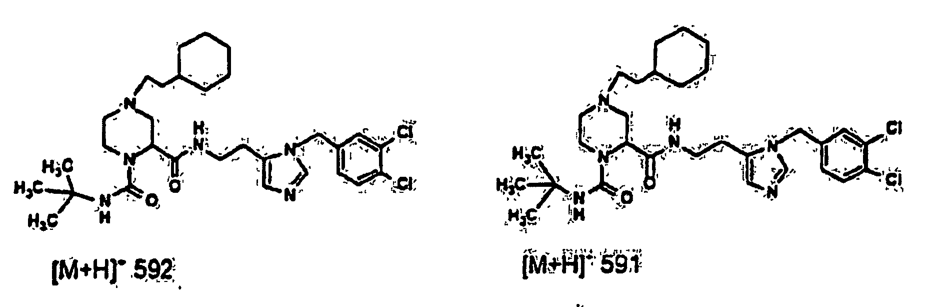 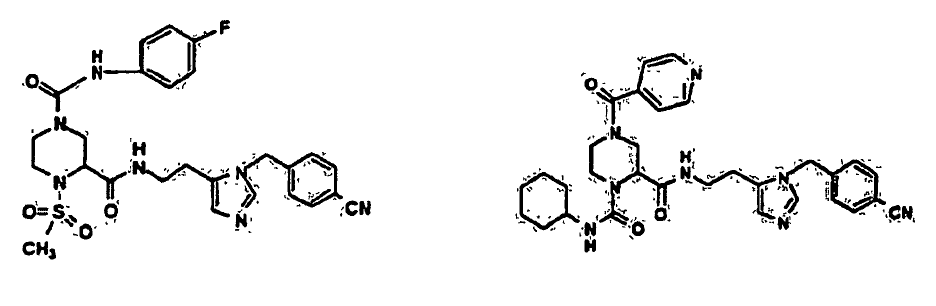 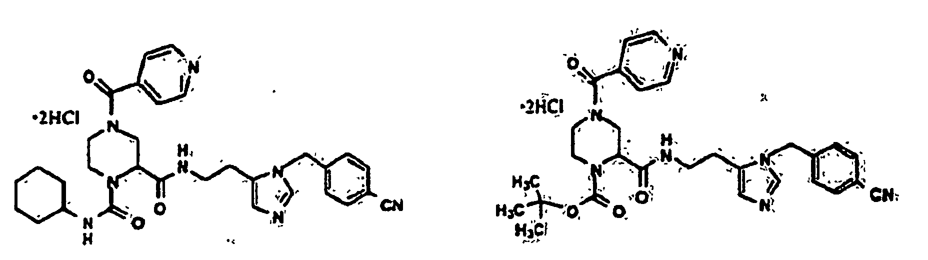 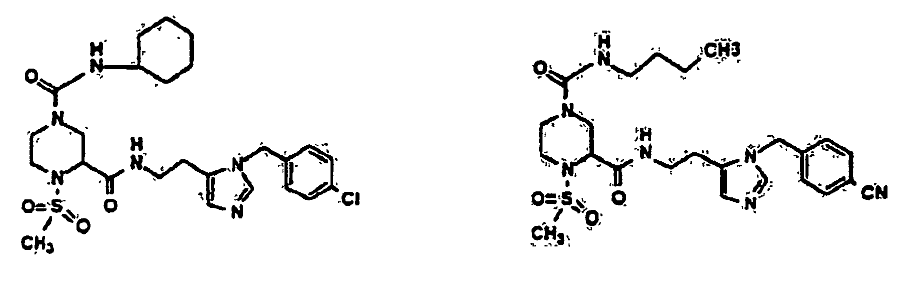 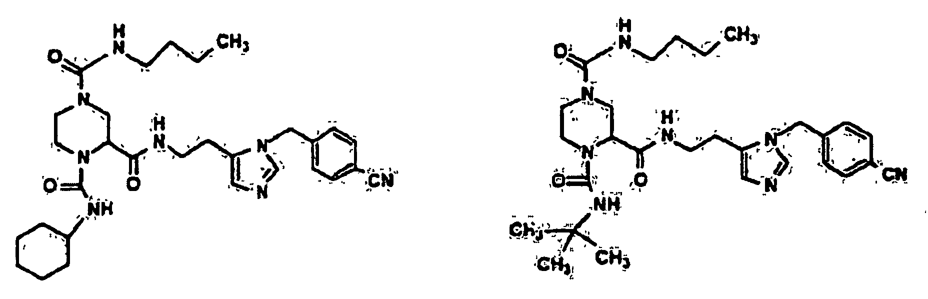 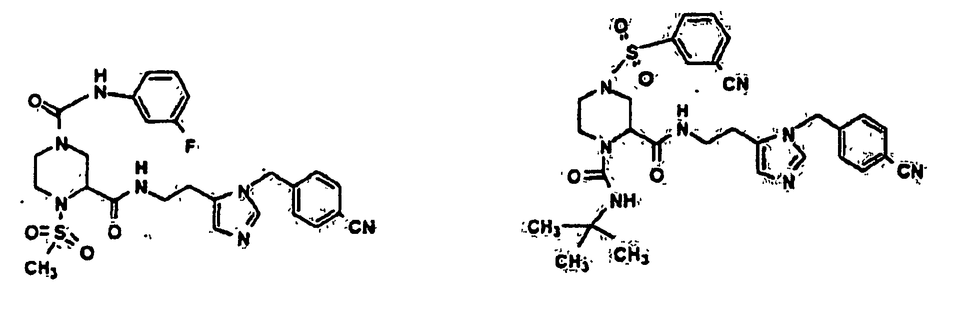 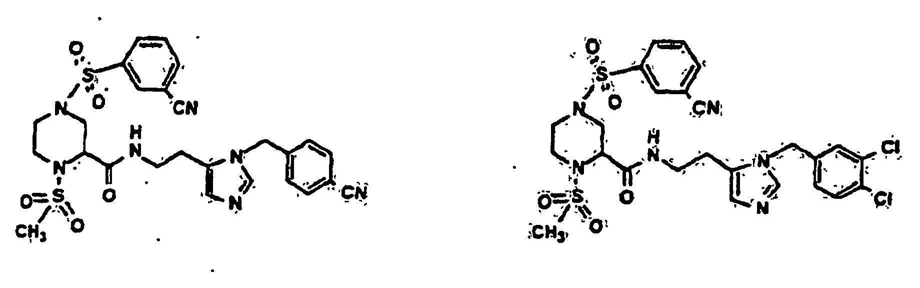 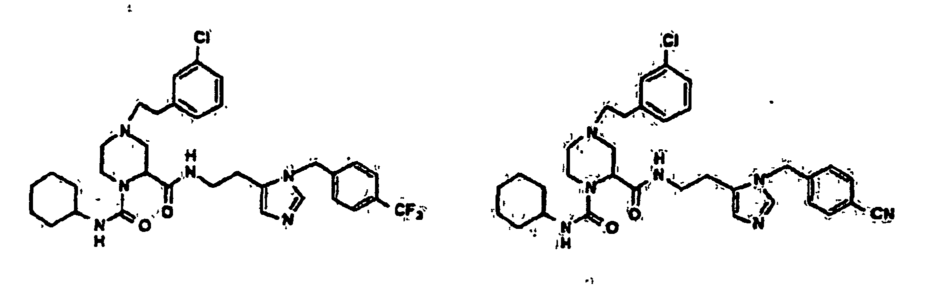 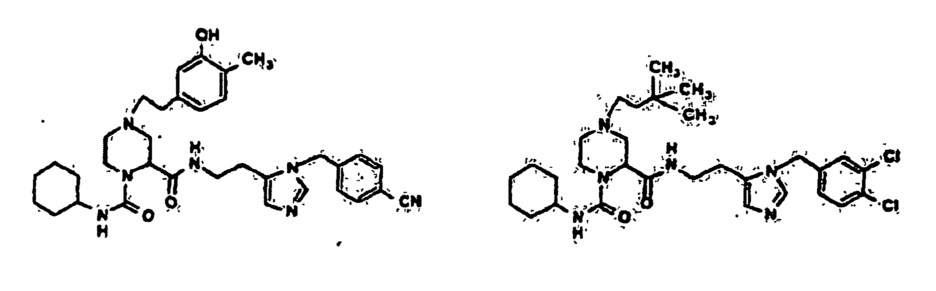 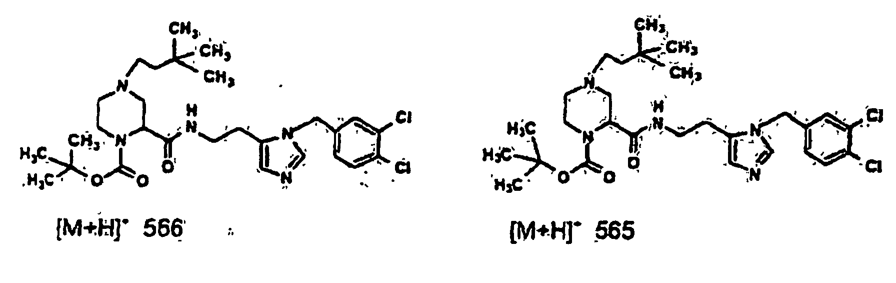 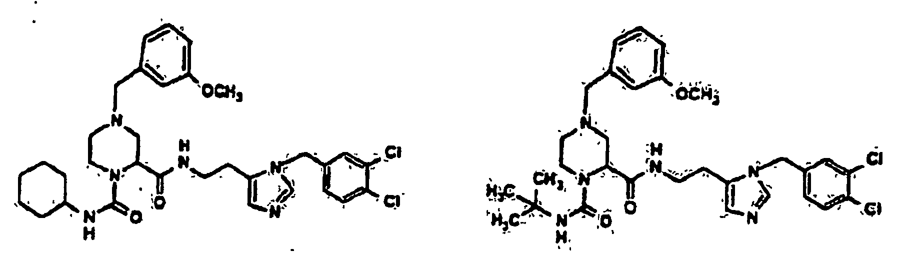 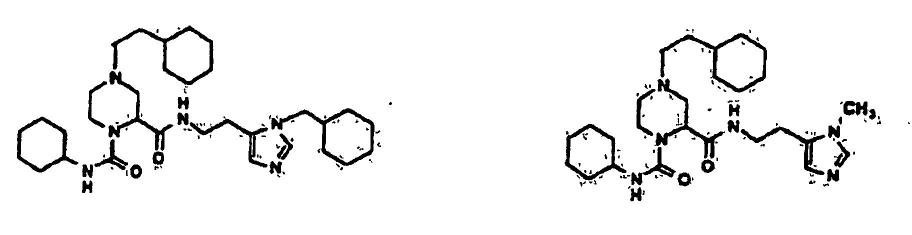 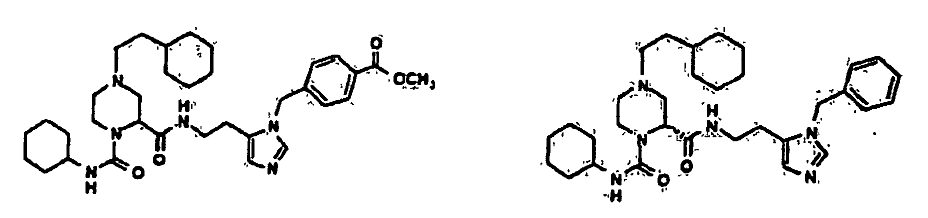 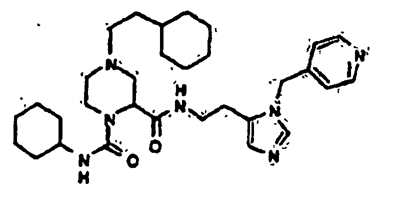
der Verbindung 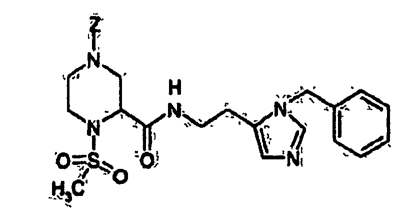 worin Z ausgewählt ist aus: 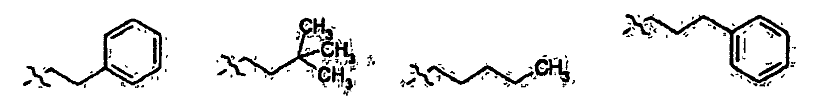 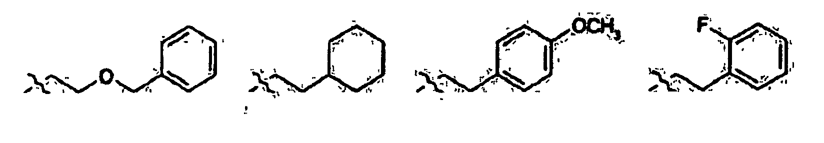 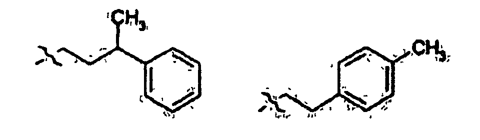
der Verbindung 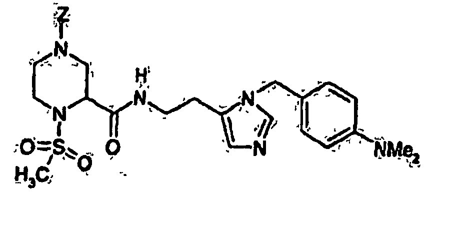 worin Z ausgewählt ist aus: 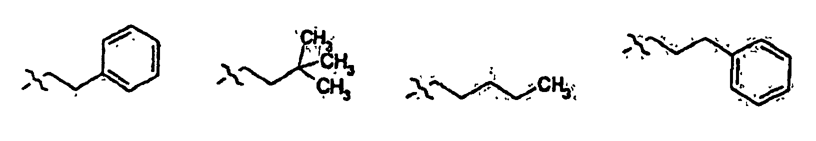 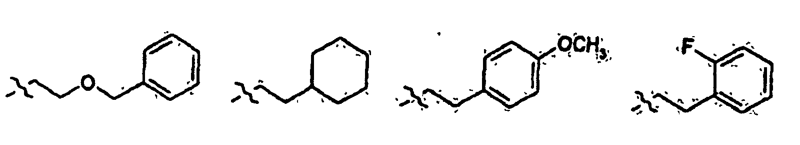 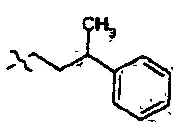
der Verbindung 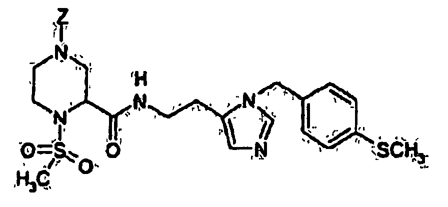 worin Z ausgewählt ist aus: 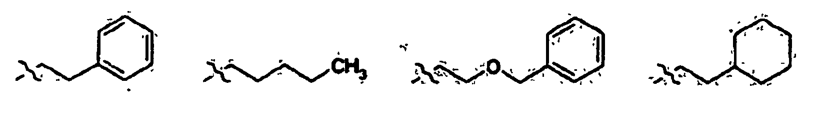 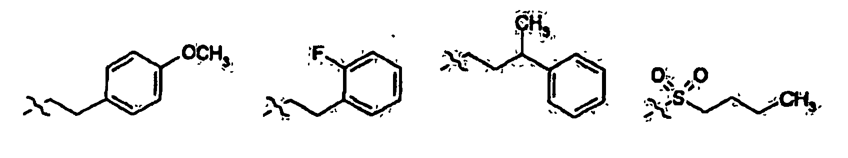 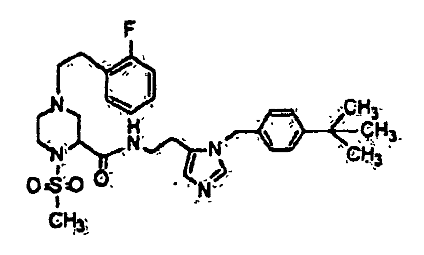
der Verbindung 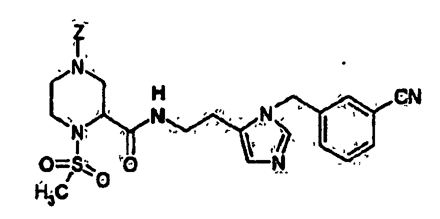 worin Z ausgewählt ist aus: 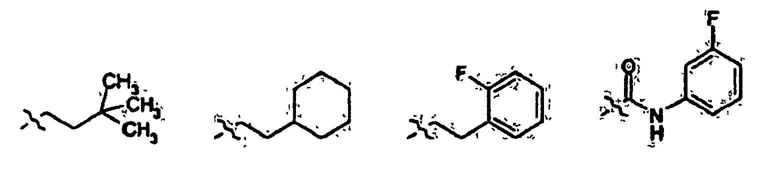 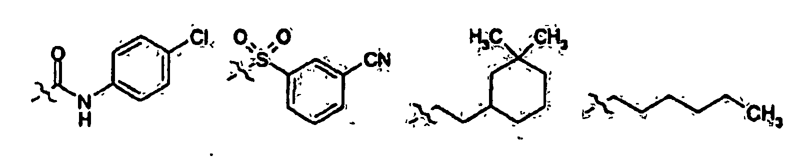
der Verbindung 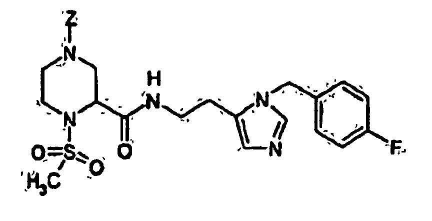 worin Z ausgewahlt ist aus: 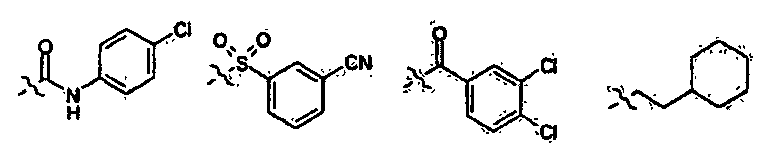 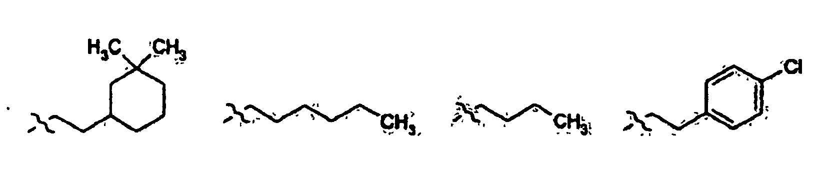 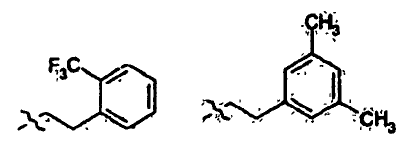 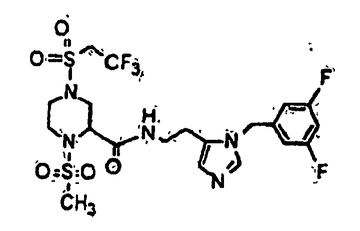 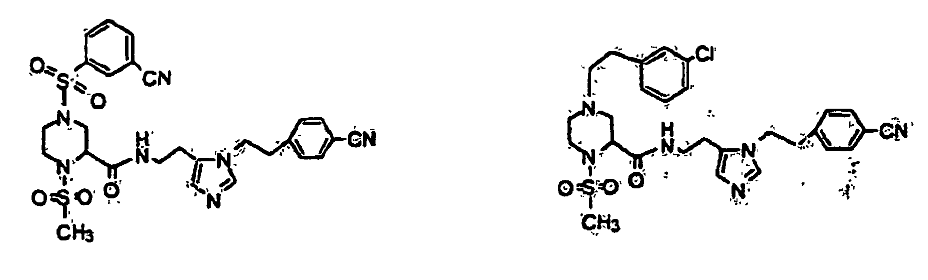
der Verbindung 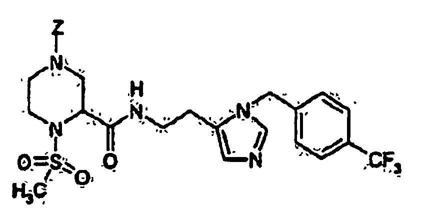 worin Z ausgewahlt ist aus: 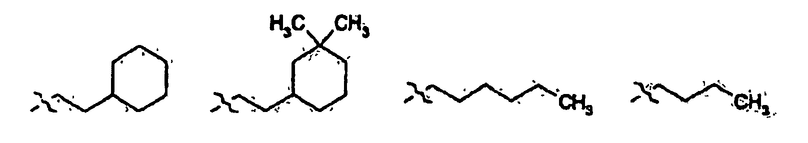 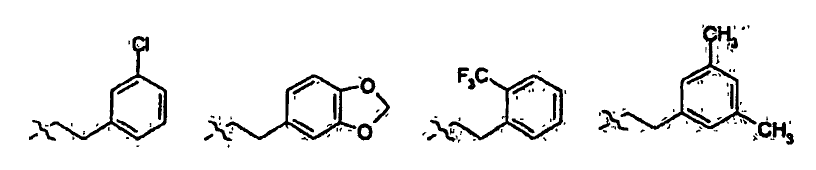
der Verbindung 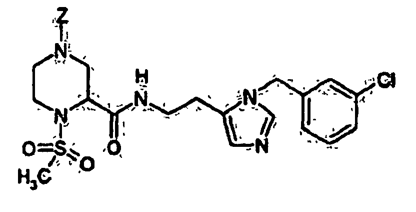 worin Z ausgewählt ist aus: 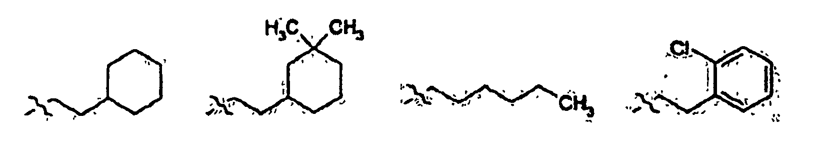 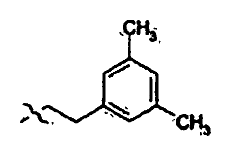
der Verbindung 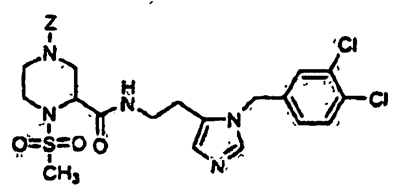 worin Z ausgewählt ist aus: 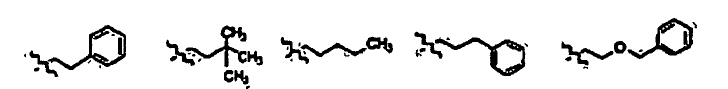 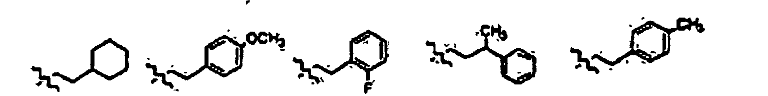 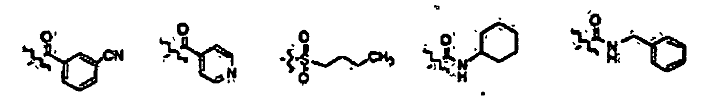 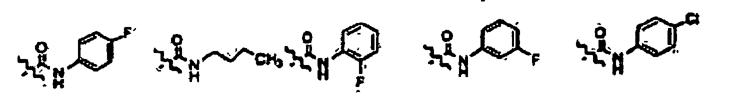 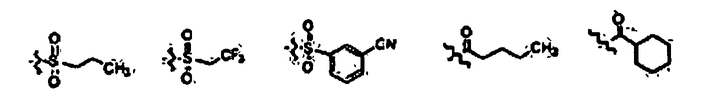 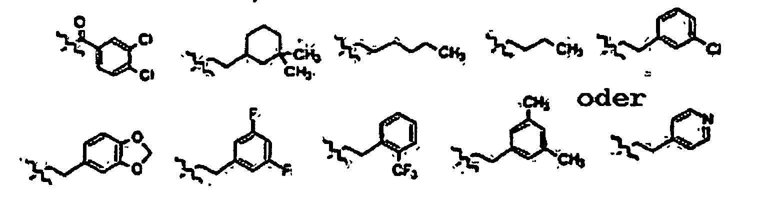
der Verbindung 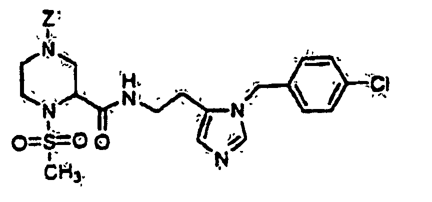 worin Z ausgewählt ist aus: 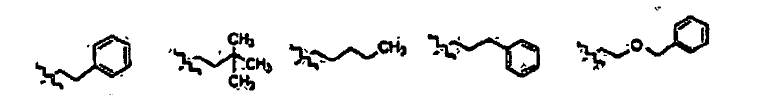 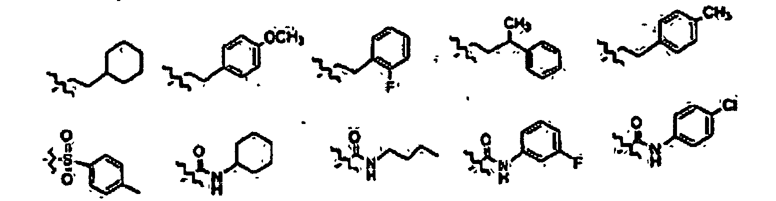 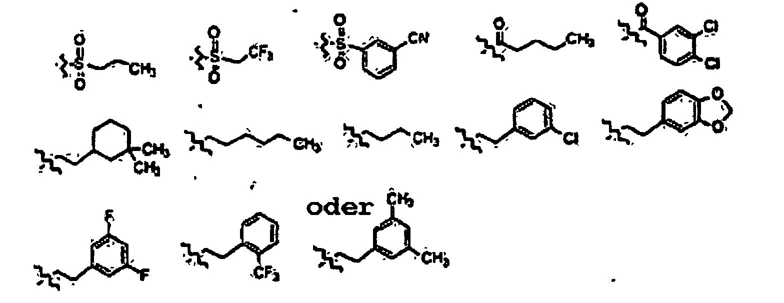
der Verbindung 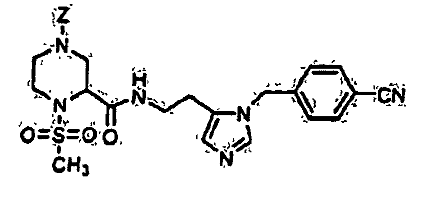 worin Z ausgewählt ist aus: 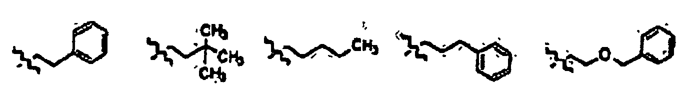 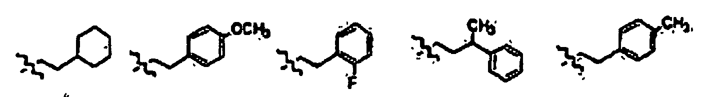 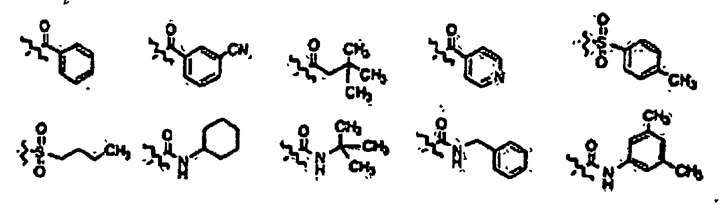 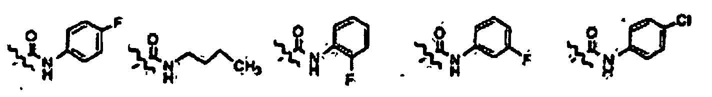 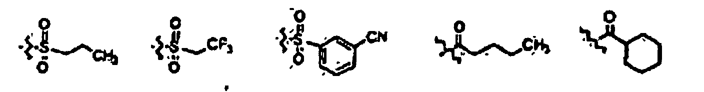 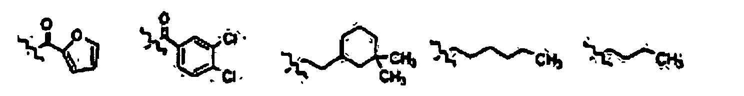 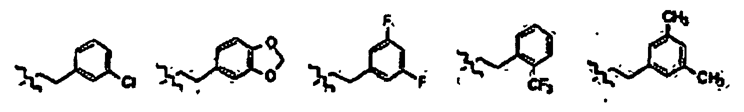 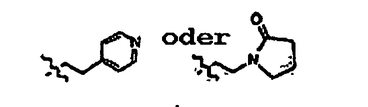 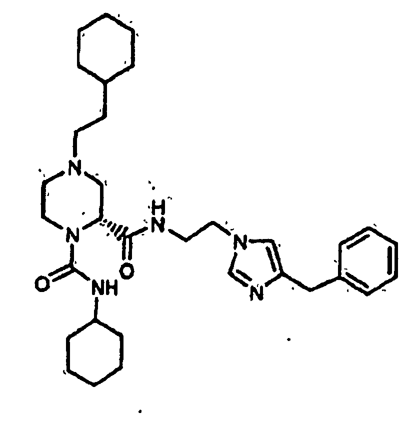 und

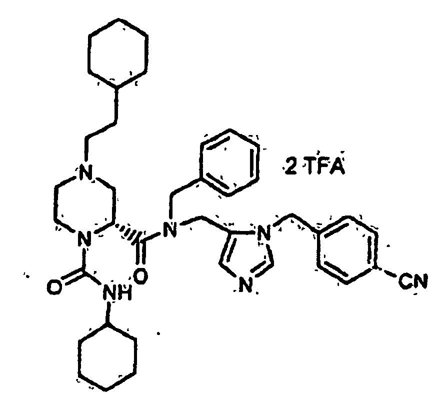

2. Verbindung ausgewahlt aus der Gruppe bestehend aus: 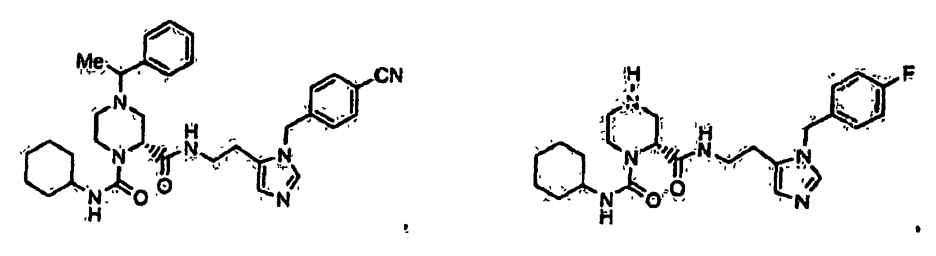 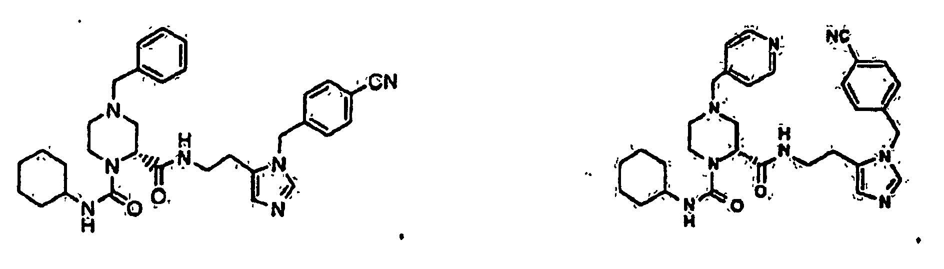 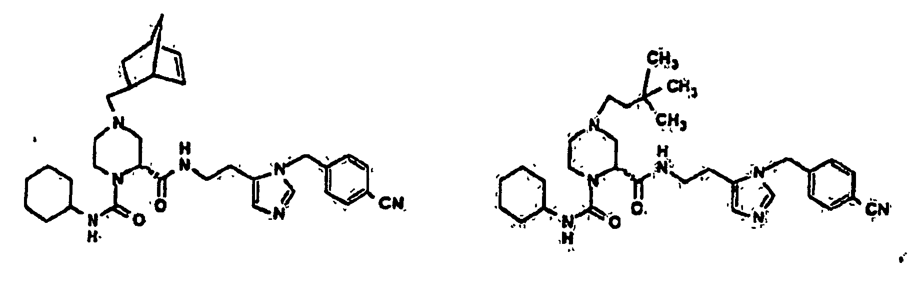 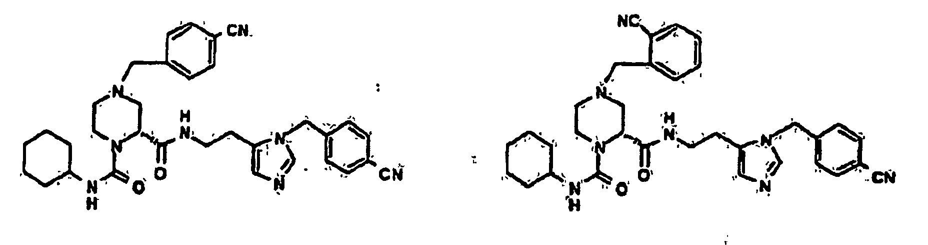 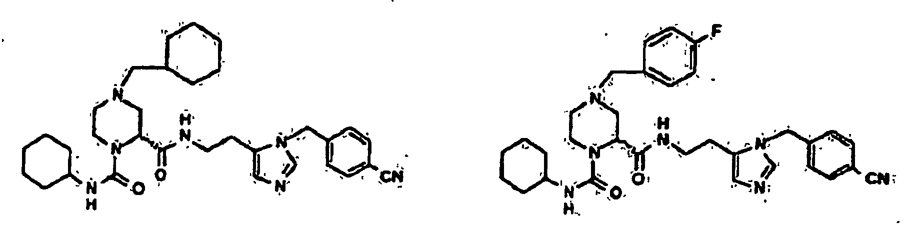 und

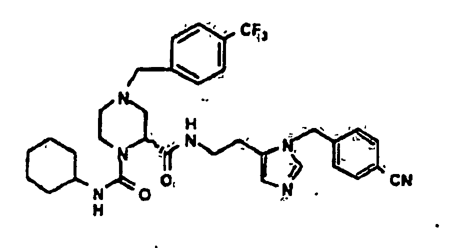

3. Pharmazeutische Zusammensetzung, die eine wirksame Menge einer Verbindung nach einem der Ansprüche 1 bis 2 in Kombination mit einem pharmazeutisch annehmbaren Träger enthält.

4. Verwendung einer Verbindung nach einem der Ansprüche 1 bis 2 zur Herstellung eines Medikaments zur Inhibierung des abnormalen Wachstums von Zellen.

5. Verwendung nach Anspruch 4, bei der die inhibierten Zellen Tumorzellen sind.

6. Verwendung nach Anspruch 4, bei der die inhibierten Zellen Pankreastumorzellen, Lungenkrebszellen, myeloide Leukämietumorzellen, Schilddrusenfollikeltumorzellen, myelodysplastische Tumorzellen, epidermale Carcinomtumorzellen, Blasencarcinomtumorzellen oder Prostatatumorzellen, Melanomtumorzellen, Brusttumorzellen oder Colontumorzellen sind.

7. Verwendung nach Anspruch 4, bei der die Inhibierung des abnormalen Wachstums von Zellen durch Inhibierung von ras-Farnesylproteintransferase erfolgt.

8. Verwendung nach Anspruch 4, wobei die Inhibierung von Tumorzellen erfolgt, bei denen das Ras-Protein infolge von onkogener Mutation in anderen Genen als dem Ras-Gen aktiviert ist.

9. Verwendung einer Verbindung nach einem der Ansprüche 1 bis 2 zur Herstellung eines Medikaments zur Inhibierung von Pankreastumorzellen, Lungenkrebstumorzellen, myeloiden Leukamietumorzellen, Schilddrüsenfollikeltumorzellen, myelodysplastischen Tumorzellen, epidermalen Karzinomtumorzellen, Blasenkarzinomtumorzellen oder Prostatatumorzellen, Melanomtumorzellen, Brusttumorzellen oder Colontumorzellen.

10. Verwendung einer Verbindung nach einem der Ansprüche 1 bis 2 zur Herstellung eines Medikaments zur Inhibierung von ras-Farnesylproteintransferase.

## Revendications

1. Composé choisi dans l'ensemble constitué par les suivants :
- les composés de formule 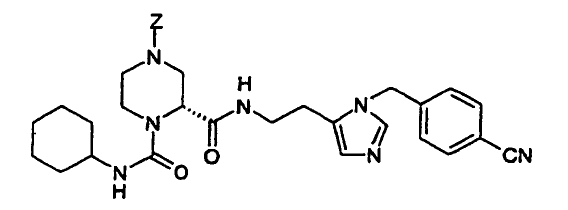 dans laquelle Z est choisi parmi les groupes suivants : 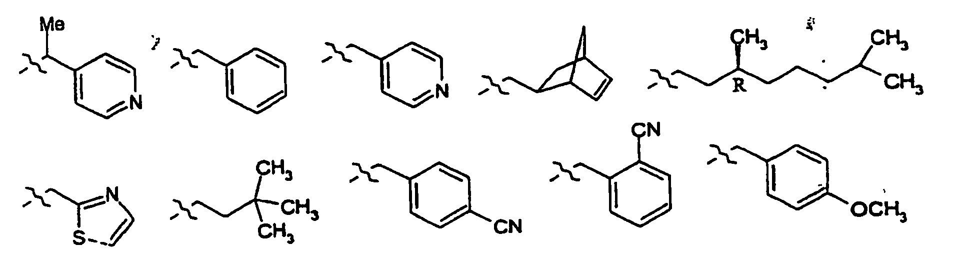 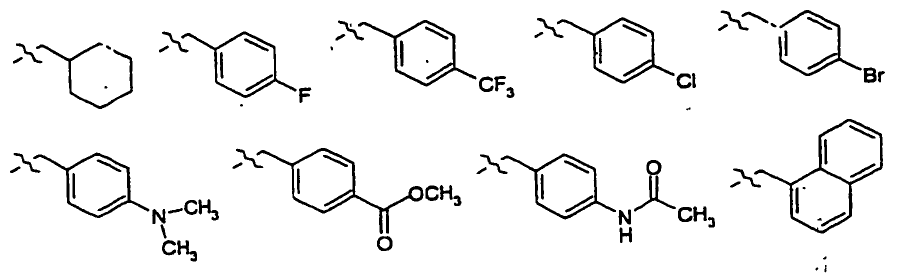 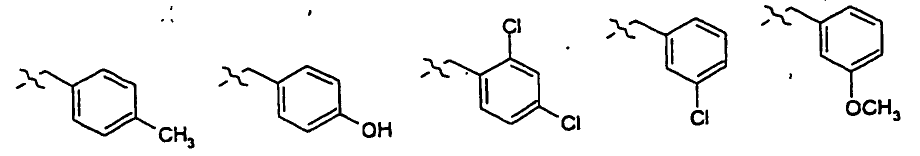 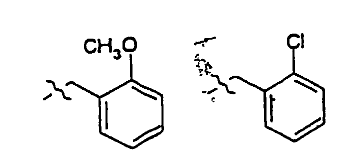 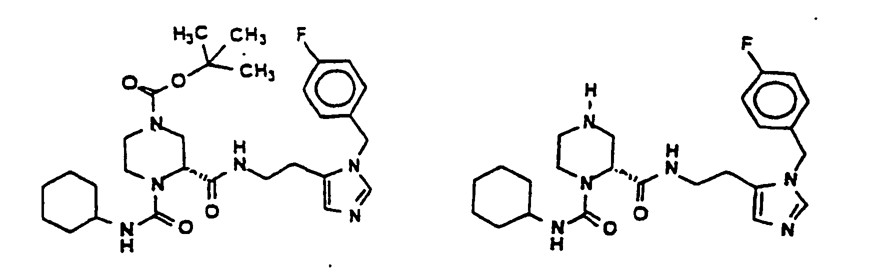 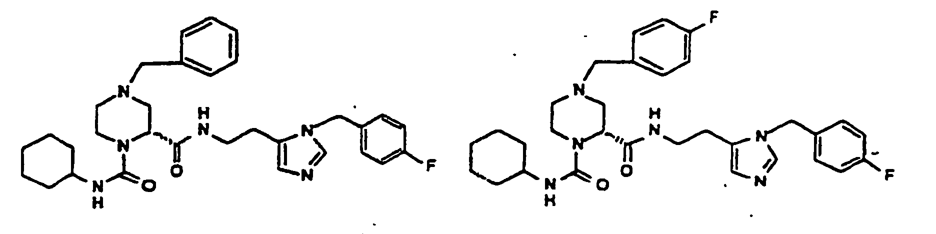 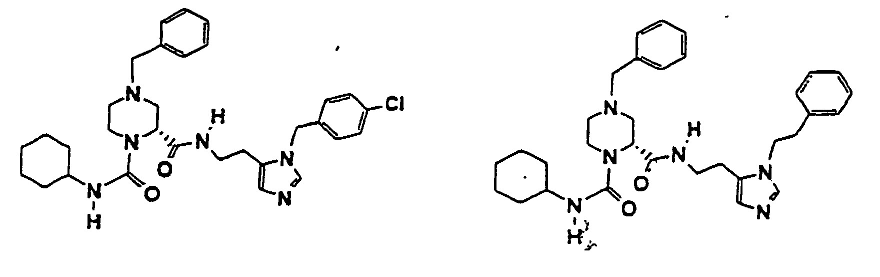
- les composés de formule 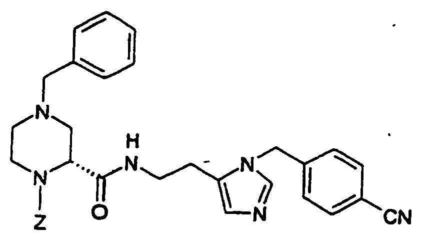 dans laquelle Z est choisi parmi les groupes suivants : 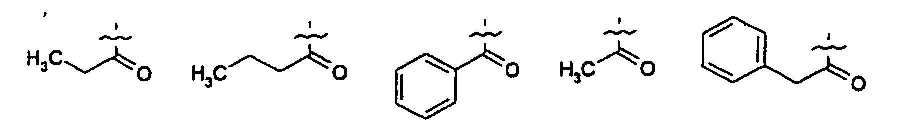 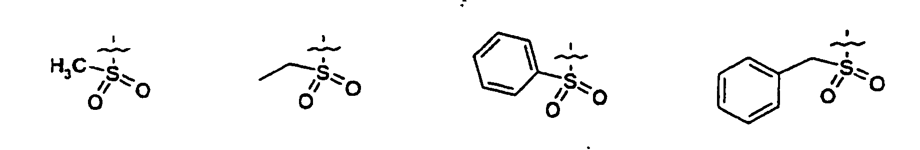 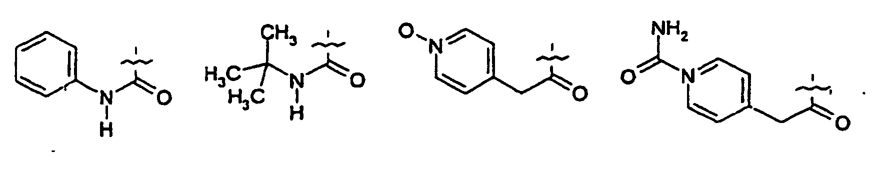 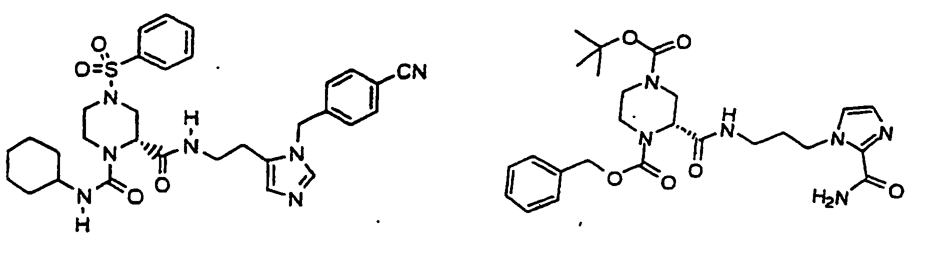 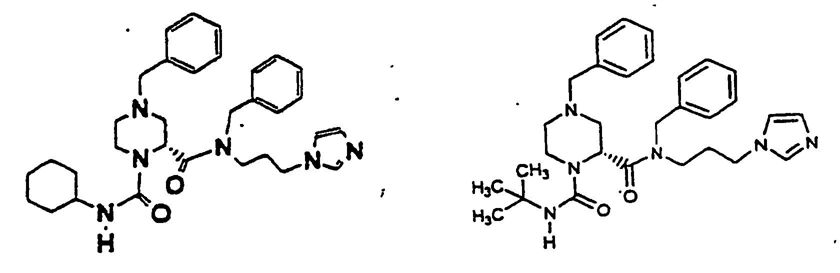 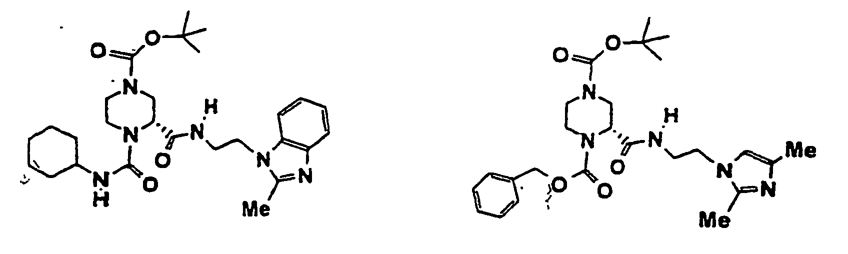 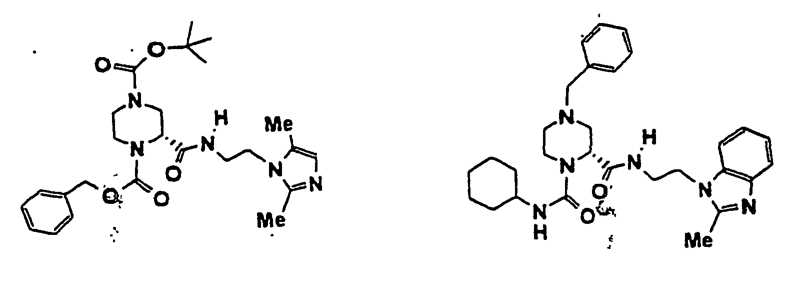 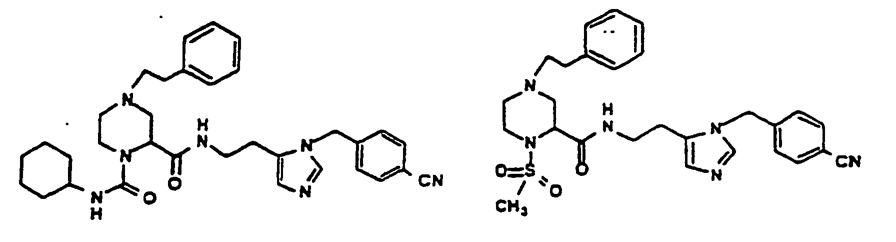 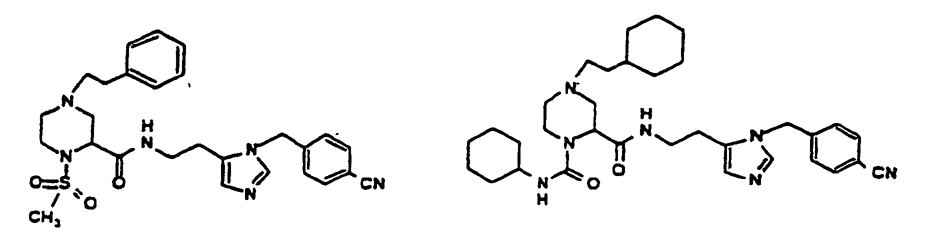 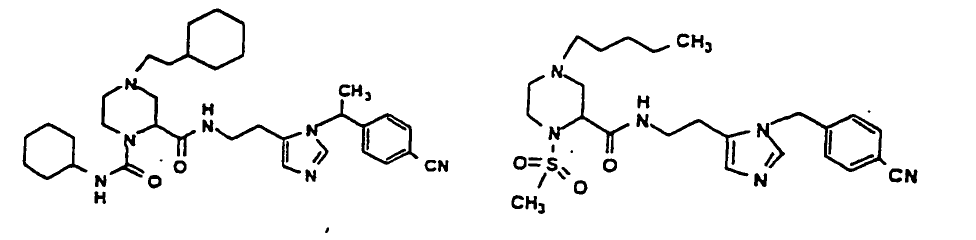 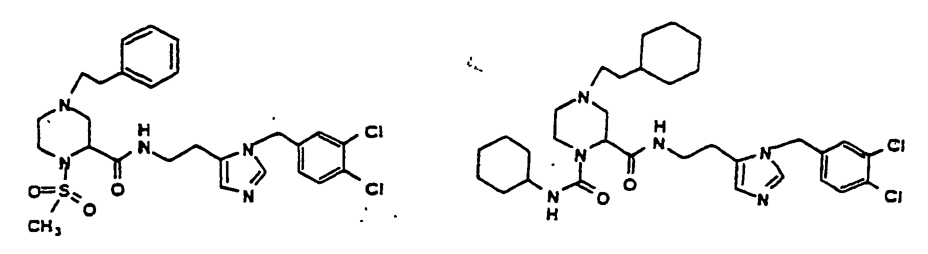 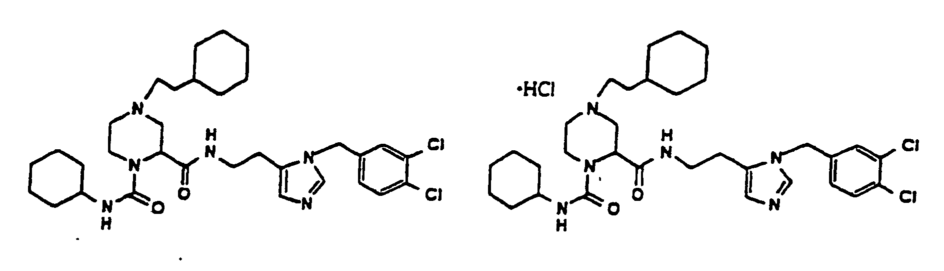 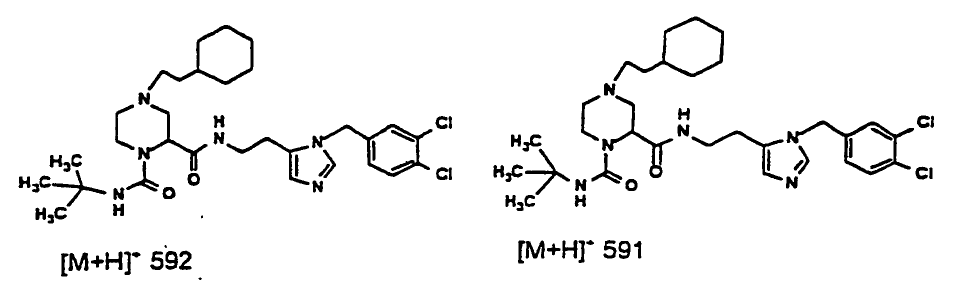 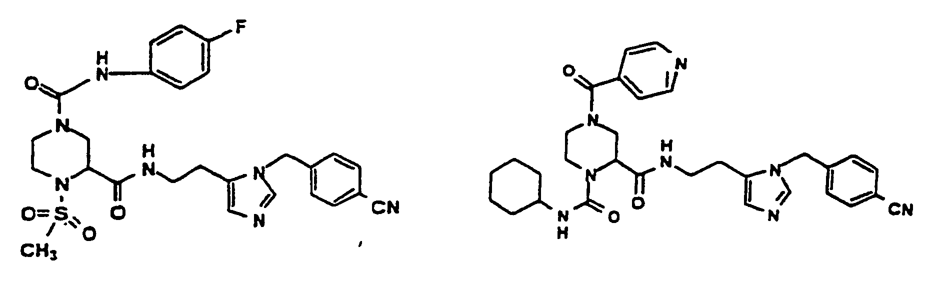 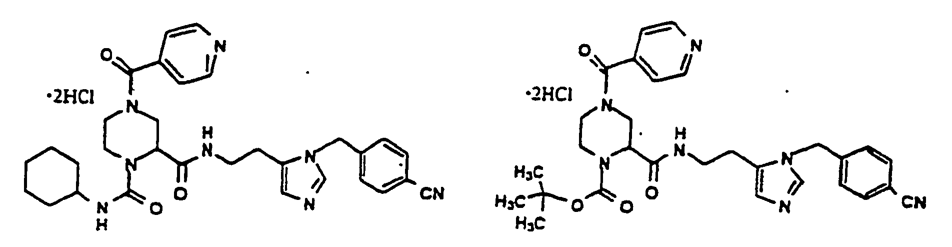 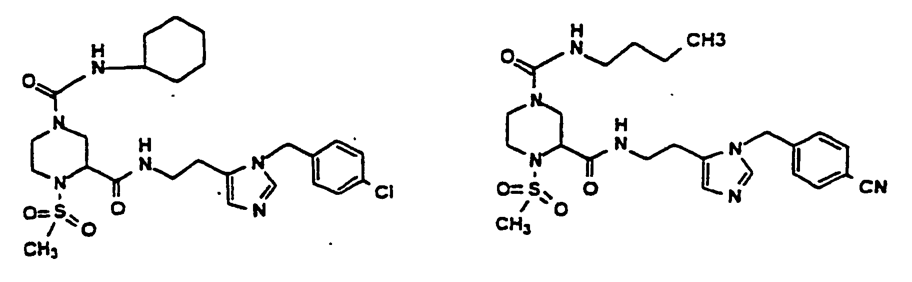 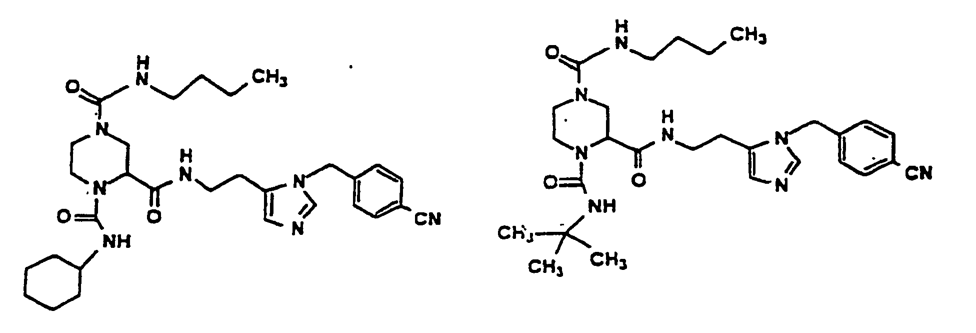 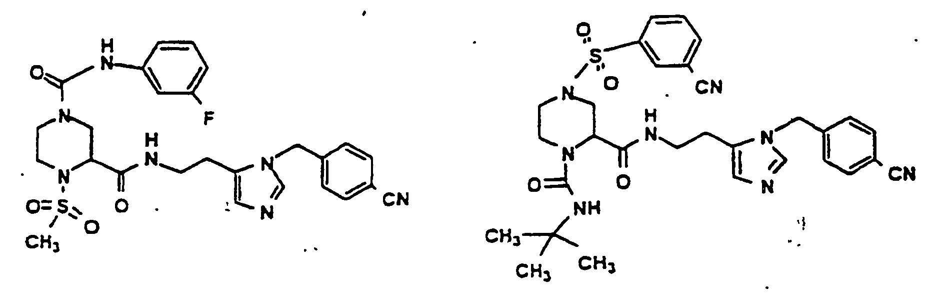 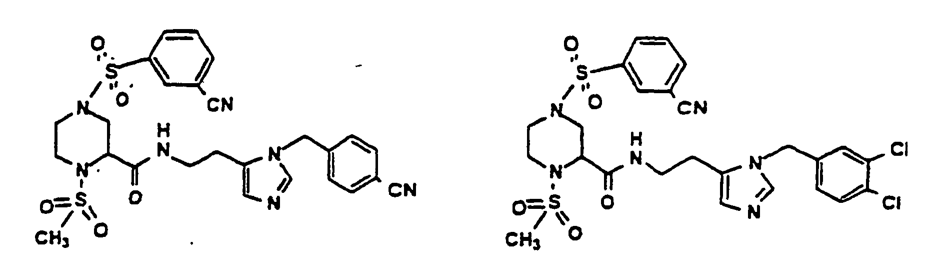 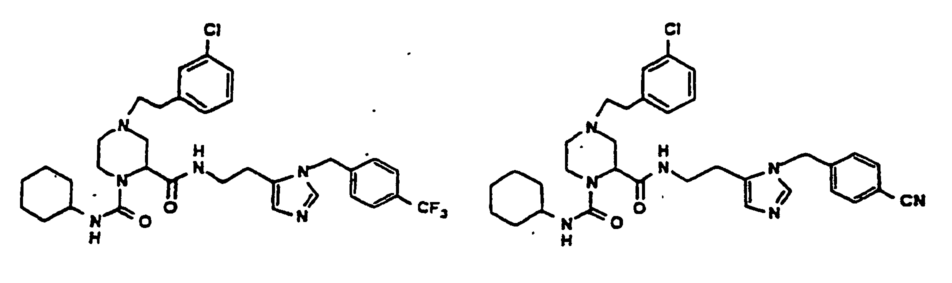 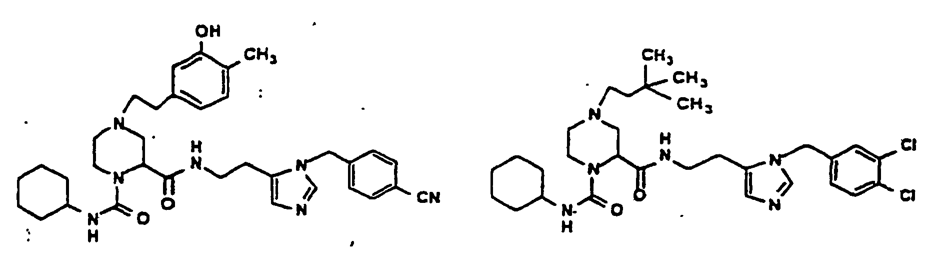 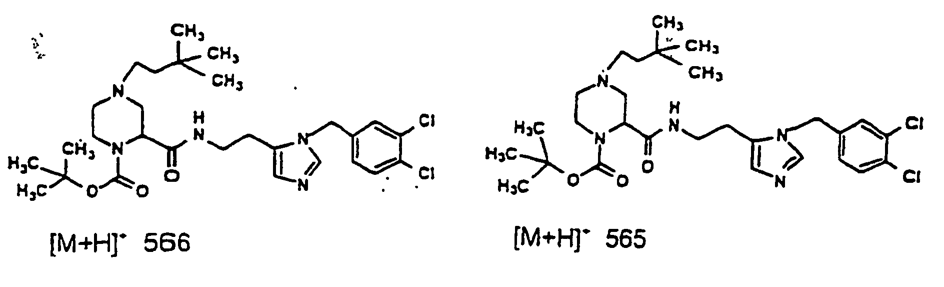 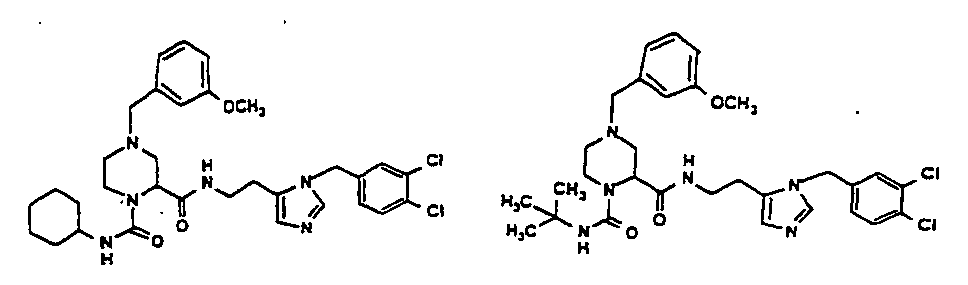 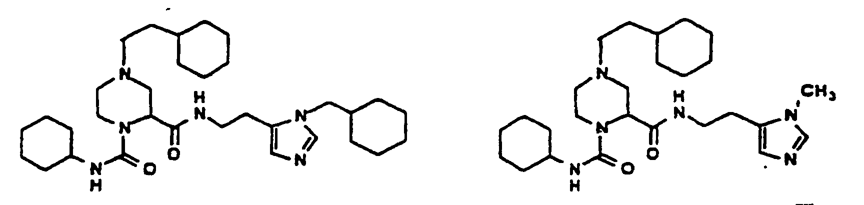 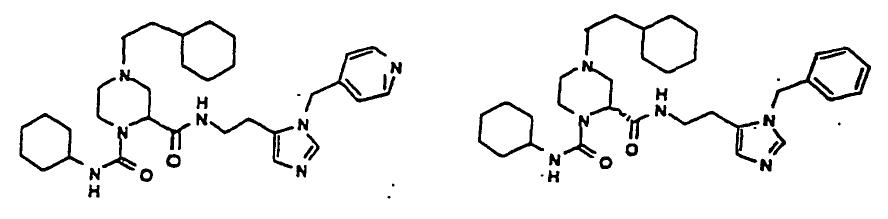 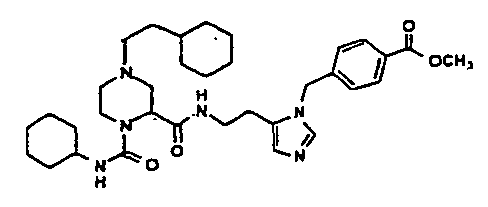
- les composés de formule 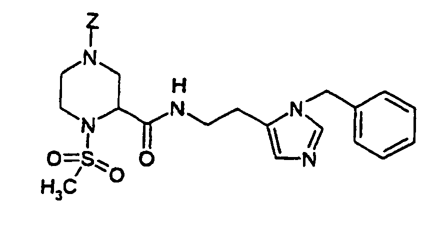 dans laquelle Z est choisi parmi les groupes suivants : 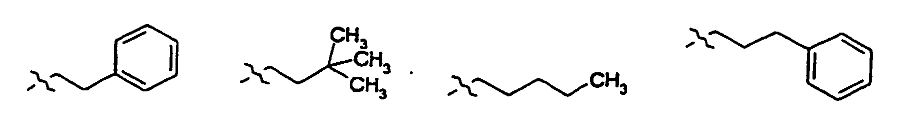 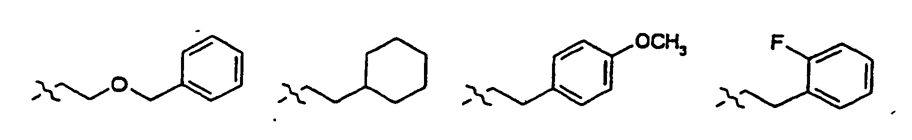 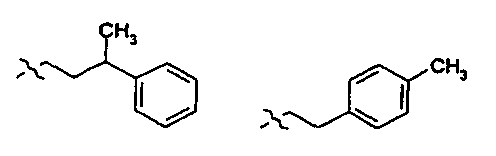
- les composés de formule 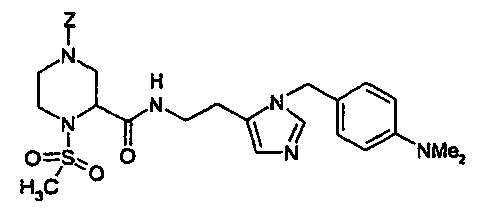 dans laquelle Z est choisi parmi les groupes suivants : 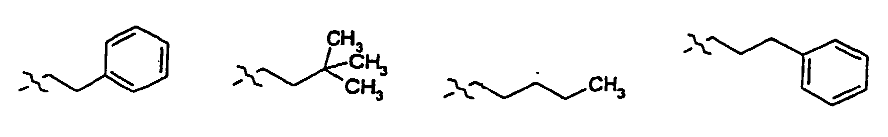 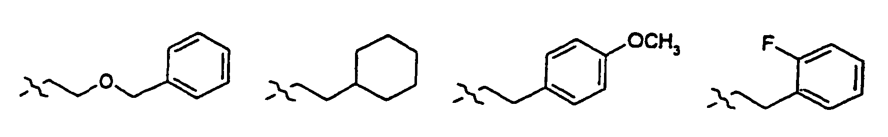 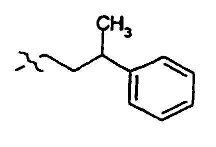
- les composés de formule 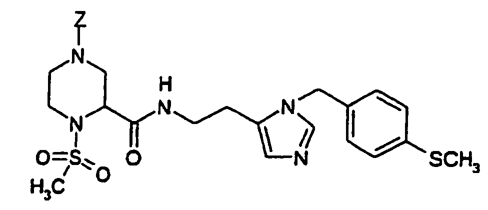 dans laquelle Z est choisi parmi les groupes suivants : 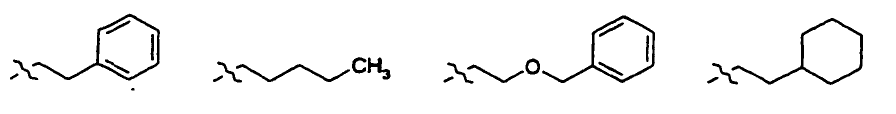 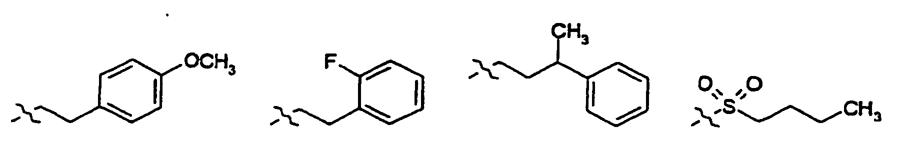 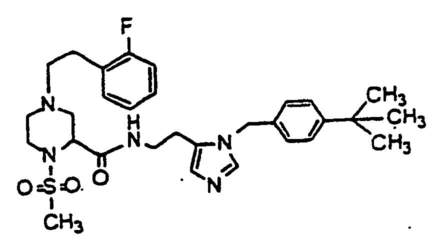
- les composés de formule 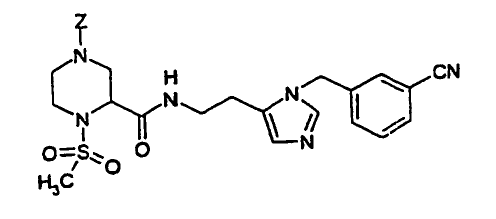 dans laquelle Z est choisi parmi les groupes suivants : 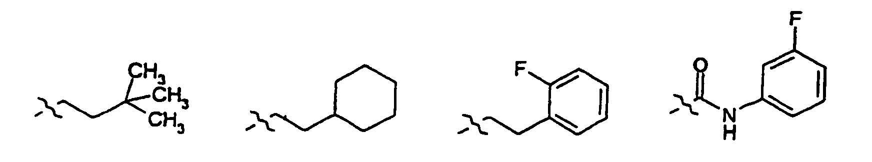 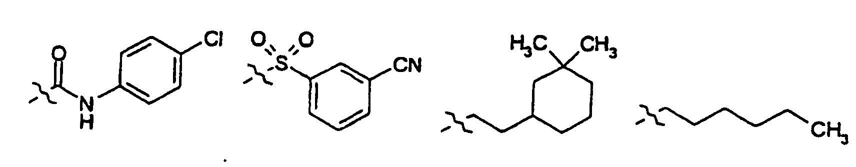
- les composés de formule 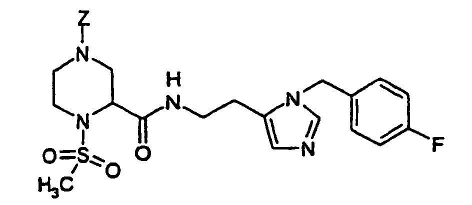 dans laquelle Z est choisi parmi les groupes suivants : 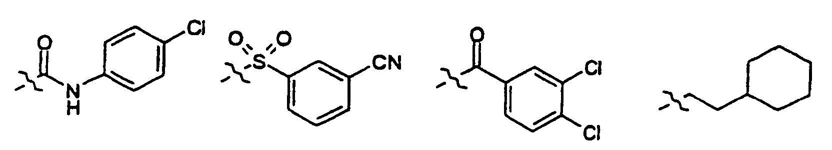 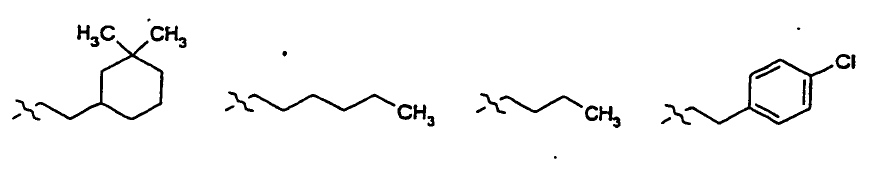 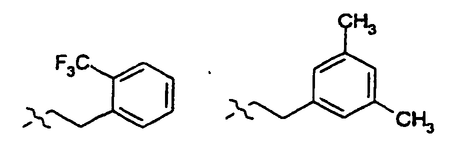 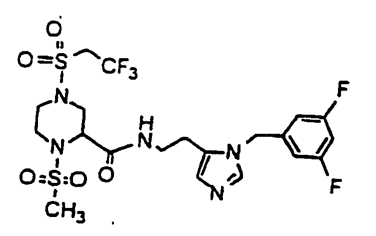 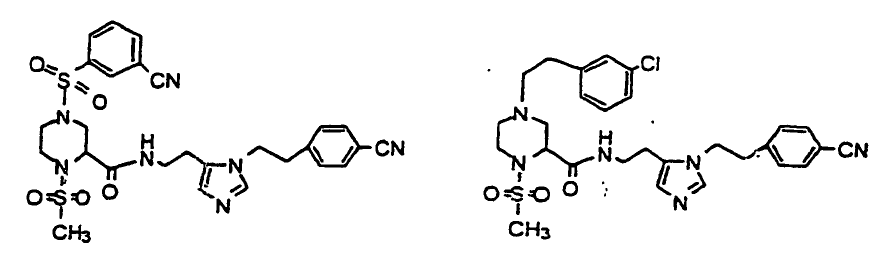
- les composés de formule 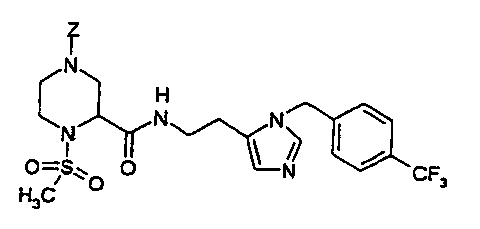 dans laquelle Z est choisi parmi les groupes suivants : 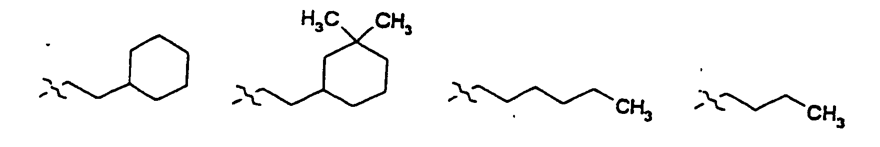 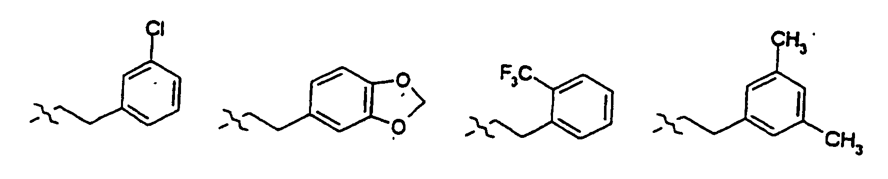
- les composés de formule 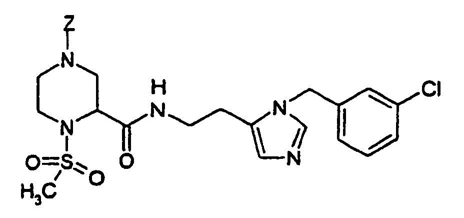 dans laquelle Z est choisi parmi les groupes suivants : 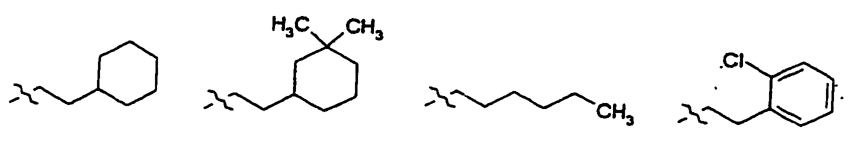 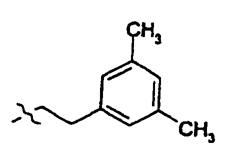
- les composés de formule 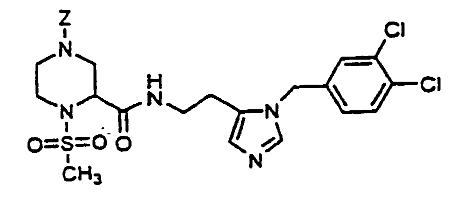 dans laquelle Z est choisi parmi les groupes suivants : 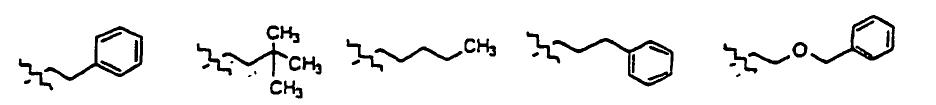 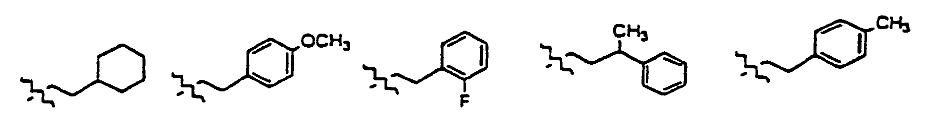 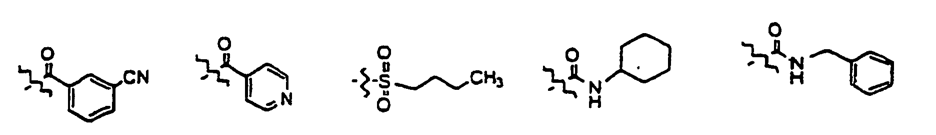 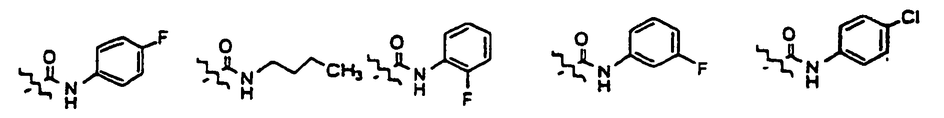 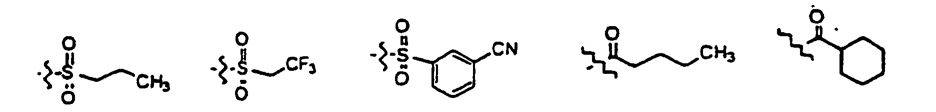 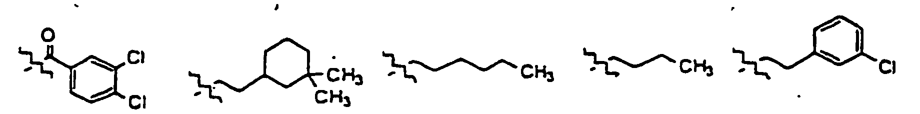 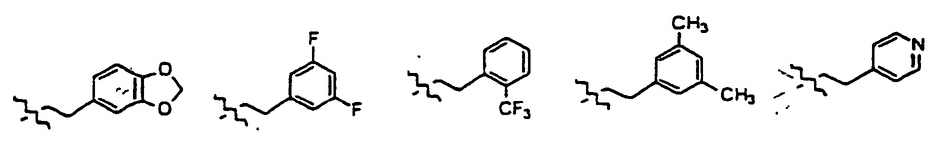
- les composés de formule 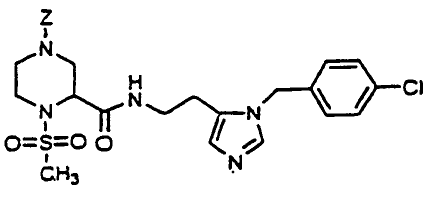 dans laquelle Z est choisi parmi les groupes suivants : 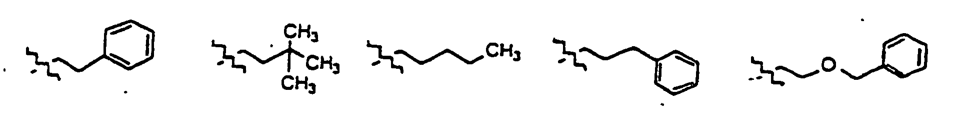 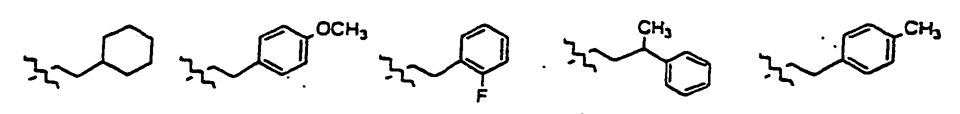 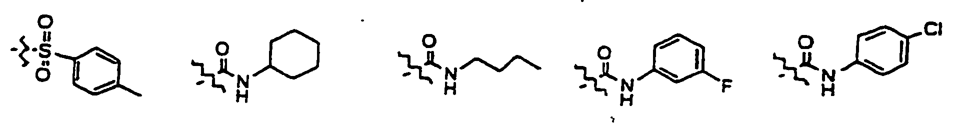 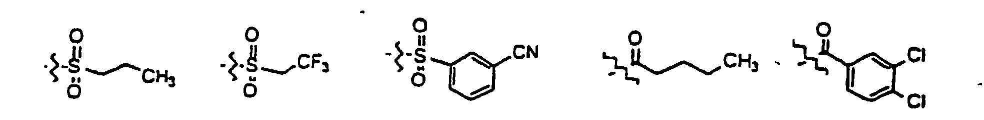 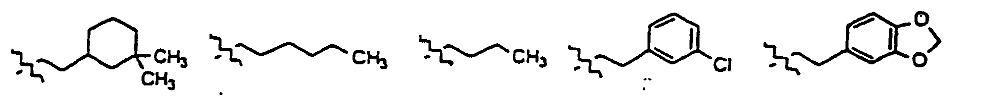 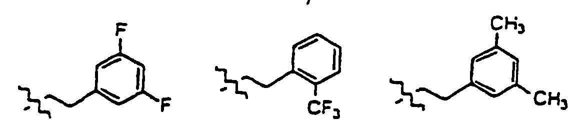
- les composés de formule 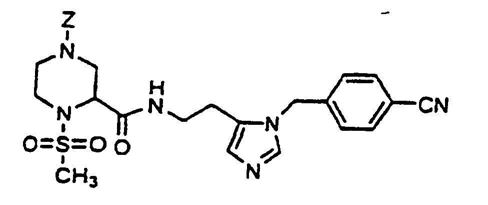 dans laquelle Z est choisi parmi les groupes suivants :

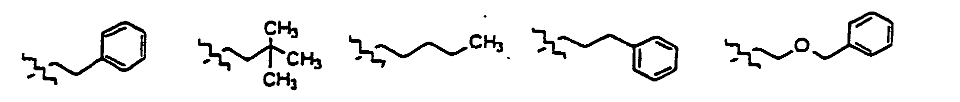

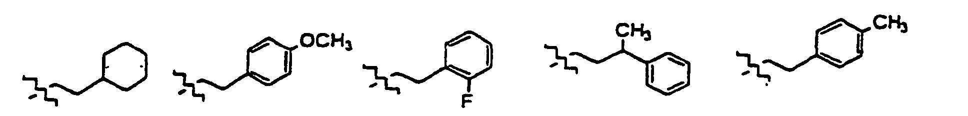

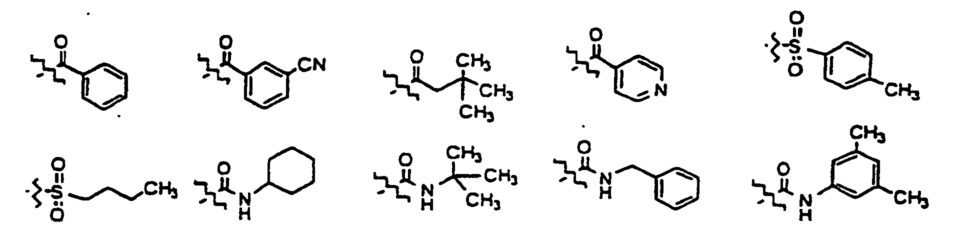

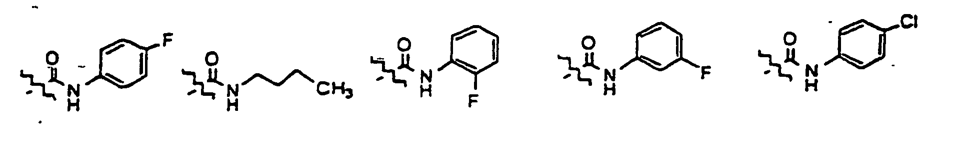

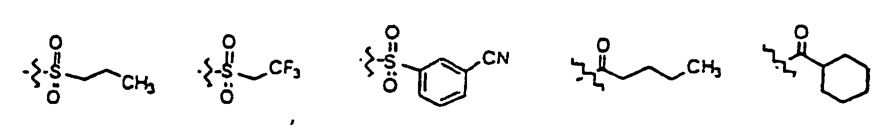

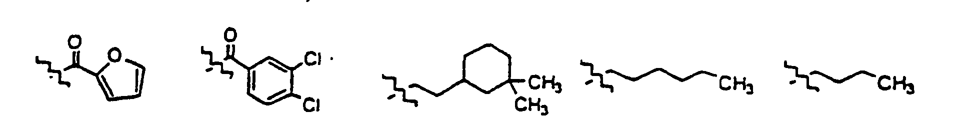

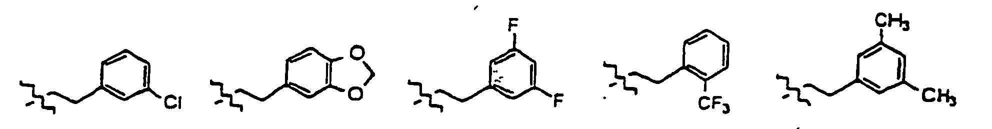

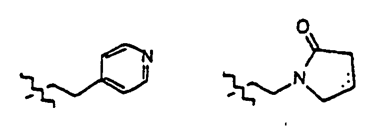

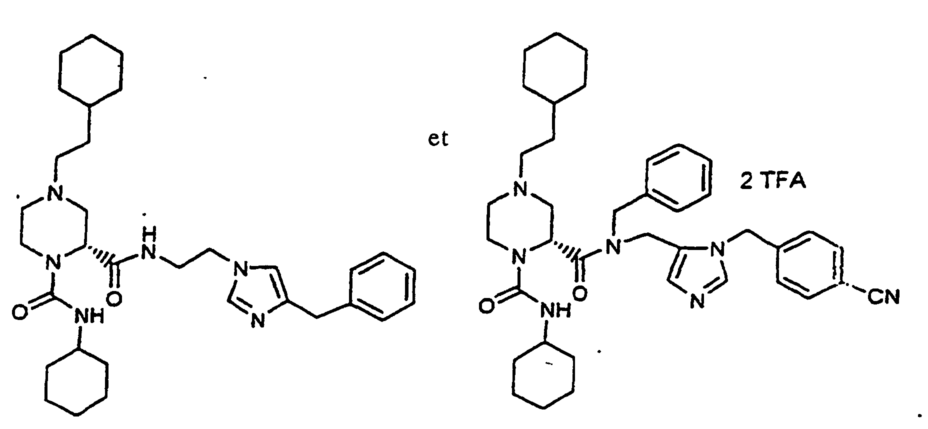

2. Composé choisi dans l'ensemble constitué par les suivants :

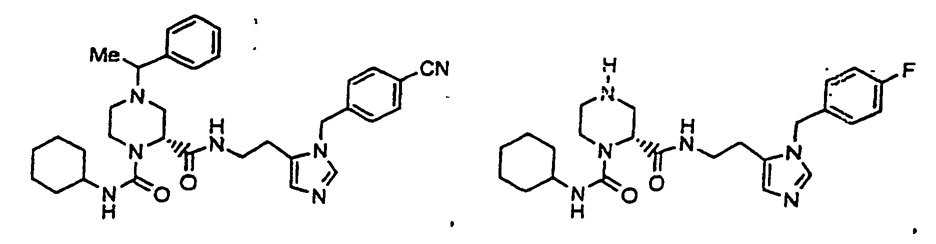

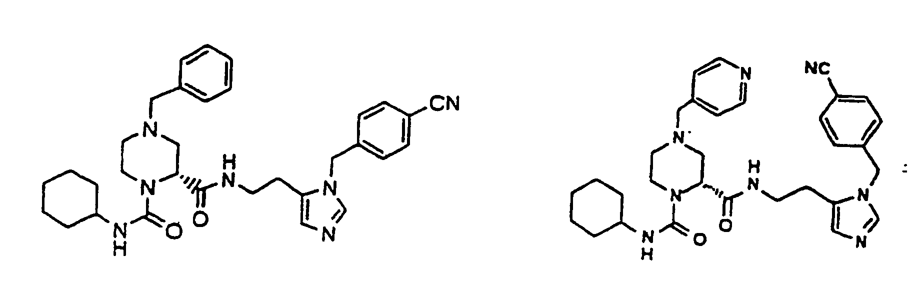

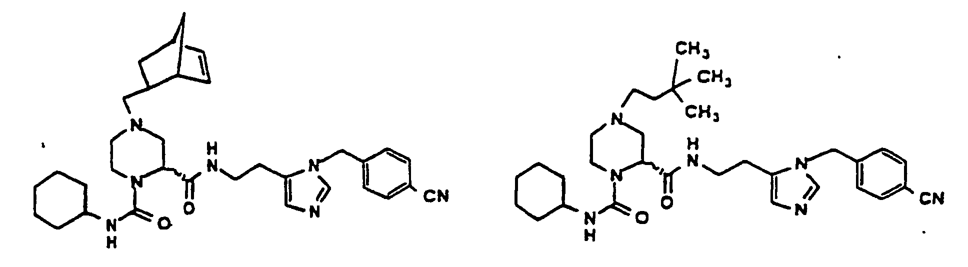

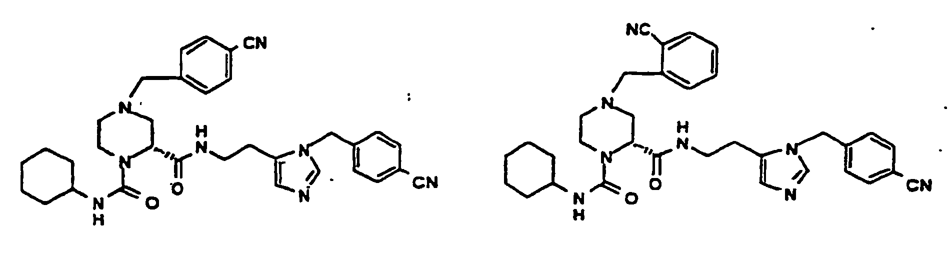

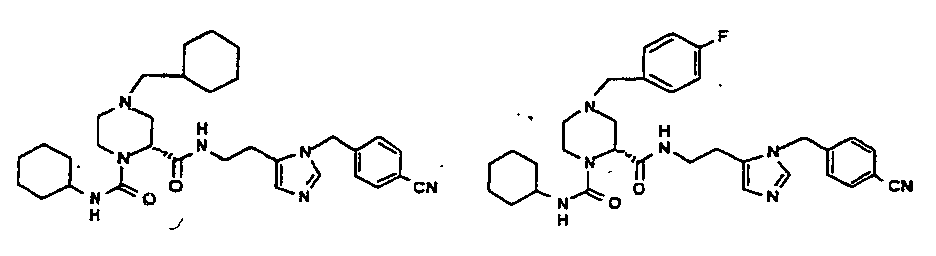

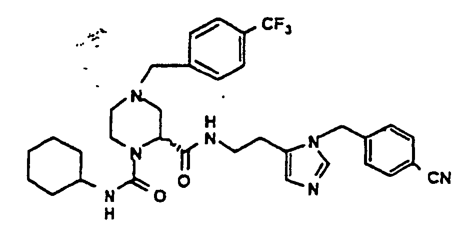

3. Composition pharmaceutique comprenant un composé conforme à l'une des revendications 1 et 2, en une quantité efficace, associé à un véhicule pharmacologiquement admissible.

4. Emploi d'un composé conforme à l'une des revendications 1 et 2, en vue de la fabrication d'un médicament destiné à inhiber une prolifération anormale de cellules.

5. Emploi conforme à la revendication 4, les cellules inhibées étant des cellules tumorales.

6. Emploi conforme à la revendication 4, les cellules inhibées étant des cellules de tumeur du pancréas, des cellules de cancer du poumon, des cellules tumorales de leucémie myéloïde, des cellules de tumeur des vésicules thyroïdiennes, des cellules tumorales de syndrome myélodysplasique, des cellules tumorales de carcinome épidermoide, des cellules tumorales de cancer de la vessie, des cellules de tumeur de la prostate, des cellules tumorales de mélanome, des cellules de tumeur du sein ou des cellules de tumeur du côlon.

7. Emploi conforme à la revendication 4, l'inhibition d'une prolifération anormale de cellules intervenant par inhibition de Ras farnésyl-transférase.

8. Emploi conforme à la revendication 4, les cellules inhibées étant des cellules tumorales dans lesquelles la protéine Ras est activée par suite d'une mutation oncogénique intervenue dans un gène autre que le gène *ras.*

9. Emploi d'un composé conforme à l'une des revendications 1 et 2, en vue de la fabrication d'un médicament destiné à inhiber des cellules de tumeur du pancréas, des cellules de cancer du poumon, des cellules tumorales de leucémie myéloïde, des cellules de tumeur des vésicules thyroïdiennes, des cellules tumorales de syndrome myélodysplasique, des cellules tumorales de carcinome épidermoïde, des cellules tumorales de cancer de la vessie, des cellules de tumeur de la prostate, des cellules tumorales de mélanome, des cellules de tumeur du sein ou des cellules de tumeur du côlon.

10. Emploi d'un composé conforme à l'une des revendications 1 et 2, en vue de la fabrication d'un médicament destiné à inhiber la Ras-farnésyl-transférase.
